(19)

Europäisches Patentamt

European Patent Office

Office européen des brevets

(11) **EP 4 320 276 B1**

(12) **EUROPEAN PATENT SPECIFICATION**

(45) Date of publication and mention of the grant of the patent:
**23.04.2025 Bulletin 2025/17**

(21) Application number: **22728889.1**

(22) Date of filing: **13.05.2022**

(51) International Patent Classification (IPC):
***C12Q 1/6886*** (2018.01)

(52) Cooperative Patent Classification (CPC):
**C12Q 1/6886**; C12Q 2600/154

(86) International application number:
**PCT/EP2022/063005**

(87) International publication number:
**WO 2022/238560 (17.11.2022 Gazette 2022/46)**

(54) **METHODS FOR DISEASE DETECTION**

VERFAHREN ZUR KRANKHEITSERKENNUNG

PROCÉDÉS DE DÉTECTION DE MALADIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.05.2021 US 202163189001 P**
**02.08.2021 EP 21189077**

(43) Date of publication of application:
**14.02.2024 Bulletin 2024/07**

(73) Proprietor: **Universal Diagnostics S.A.**
**41013 Sevilla (ES)**

(72) Inventors:
• **KRISTI, Kruusmaa**
**41013 Sevilla (ES)**
• **NOGUER, Pol Canal**
**41013, Sevilla (ES)**
• **BERTOSSI, Arianna**
**1000 Ljubljana (SI)**
• **CHERSICOLA, Marko**
**1000 Ljubljana (SI)**
• **KNAP, Primoz**
**1000 Ljubljana (SI)**

(74) Representative: **Carlos Hernando, Borja Garrigues IP, S.L.P.**
**Hermosilla, 3**
**28001 Madrid (ES)**

(56) References cited:
WO-A1-2013/097868    WO-A1-2015/153283
WO-A1-2017/043497    WO-A1-2020/069350
WO-A1-2020/239896    WO-A2-2020/239895
US-A1- 2013 012 410    US-A1- 2015 072 866
US-A1- 2019 032 149    US-A1- 2021 139 948

• **ZHANG SHUHUA ET AL: "CRISPR/Cas9-mediated knockout of NSD1 suppresses the hepatocellular carcinoma development via the NSD1/H3/Wnt10b signaling pathway", JOURNAL OF EXPERIMENTAL AND CLINICAL CANCER RESEARCH., vol. 38, no. 1, 14 December 2019 (2019-12-14), IT, pages 467, XP055865347, ISSN: 0392-9078, DOI: 10.1186/s13046-019-1462-y**

Remarks:
The complete document including Reference Table(s) and the Sequence Listing(s) can be downloaded from the EPO website

EP 4 320 276 B1

**Description**

TECHNICAL FIELD

This invention relates generally to methods, and systems for identifying biomarkers for detection of a disease or condition, such as cancer.

BACKGROUND

Disease detection is an important component of prevention of disease progression, diagnosis, and treatment. For example, early detection of colorectal cancer (CRC) has been shown to drastically improve outcomes of those suffering from CRC through early treatment of CRC. However, despite the availability of current tools to screen for and diagnose CRC and other cancers, millions of individuals still die annually from diseases, such as CRC, which are treatable through early intervention and detection. Current tools to screen for and diagnose diseases are insufficient.

DNA methylation is a control mechanism that impacts numerous cellular processes including, for example, cellular differentiation. Dysregulation of methylation, therefore, can lead to disease, including cancer. Accumulated changes in DNA methylation (e.g., hypermethylation or hypomethylation), especially when the changes are located in crucial genes, can result in cancerous cells. These changes in methylation status, if detected, can be used to predict susceptibility of a subject to developing cancer, as well as the development or presence of cancer and, potentially, other diseases.

The most common method for analyzing genome-wide methylation status of a given organism is whole genome bisulfite sequencing (WGBS). In this method, the methylation status of single cytosines of sample DNA is determined by first treating the DNA (e.g., in fragmented form) with sodium bisulfite before sequencing. DNA methylation is present in mammals mostly at CpG dinucleotides - a CpG dinucleotide is a region of DNA where a cytosine nucleotide is followed by a guanine nucleotide in the linear sequence of bases along its 5' → 3' direction. In WGBS, sodium bisulfite is used to convert unmethylated cytosines into uracil, while methylated forms of cytosine (e.g., 5-methylcytosine and 5-hydroxymethylcytosine) remain unchanged. The bisulfite-treated DNA fragments are then sequenced, e.g., via a next generation sequencing technique. Specific algorithms and/or tools such as Bismark are required for mapping the sequence reads and methylation calling in CpG, CHG and CHH context at single base resolution. Thus, the WGBS technique identifies single-cytosine methylation sites genome-wide. However, the method may have low resolution of specific short genomic regions that could be useful as biomarkers of a particular disease or condition on further differentially methylated region analysis context.

Thus, there is a need for improved methods, systems and apparatus for analyzing methylation status of DNA and identifying methylation biomarkers. In particular, there is a need for methods for use in diagnosis and/or classification of colorectal neoplasms.

SUMMARY

The present disclosure provides for, among other things, various systems, methods, and apparatus for identifying biomarkers for detection of a disease or condition. A disease or condition discussed herein can be, e.g., advanced adenoma, colorectal cancer, other cancers, or other diseases or conditions associated with an aberrant methylation status (e.g., neurodegenerative diseases, gastrointestinal disorders, and the like).

In various embodiments, the present disclosure provides methods for detecting colorectal cancer and/or advanced adenoma that include analysis of one or more methylation biomarkers in cfDNA (e.g., ctDNA) of a subject. In various embodiments, the present disclosure provides methods for colorectal cancer and/or advanced adenoma detection that includes determining the methylation status of one or more methylation biomarkers in DNA e.g., cfDNA using a next generation sequencing (NGS) technique (e.g., a targeted NGS technique, a hybrid-capture based NGS technique). Various methods provided herein are useful in colorectal cancer and/or advanced adenoma screening by analysis of an accessible tissue sample of a subject, e.g., a tissue sample that is blood or a blood component (e.g., cfDNA, e.g., ctDNA).

In various embodiments, the methods described herein include screening for mutations of one or more mutation markers in cfDNA e.g., ctDNA. Mutations identified through detection methods described herein may be used to further classify and/or diagnose a disease or condition in combination with the methylation status(es) of the methylation biomarkers. For example, the presence of mutations in mutation markers and methylation status(es) of methylation markers may be acquired (e.g., simultaneously) in the same assay (e.g., a NGS assay) conducted on a single sample. Obtaining information corresponding to methylation and mutation markers in the same assay allows for decreased costs and increased efficiency by not having to conduct separate assays.

Additionally or alternatively, mutation markers may allow for further classification of a disease or condition (e.g.,

cancer). The presence and/or absence of one or more mutations may also allow for identification or recommendation of therapies for treatment of the disease and/or condition.

In various embodiments, the present disclosure relates to methods and/or systems for identifying methylation status of a methylation biomarker in cfDNA of a subject (e.g., a human subject) and/or detecting (e.g., screening for) a disease and/or condition (e.g., cancer) based on the methylation status of one or more known biomarkers.

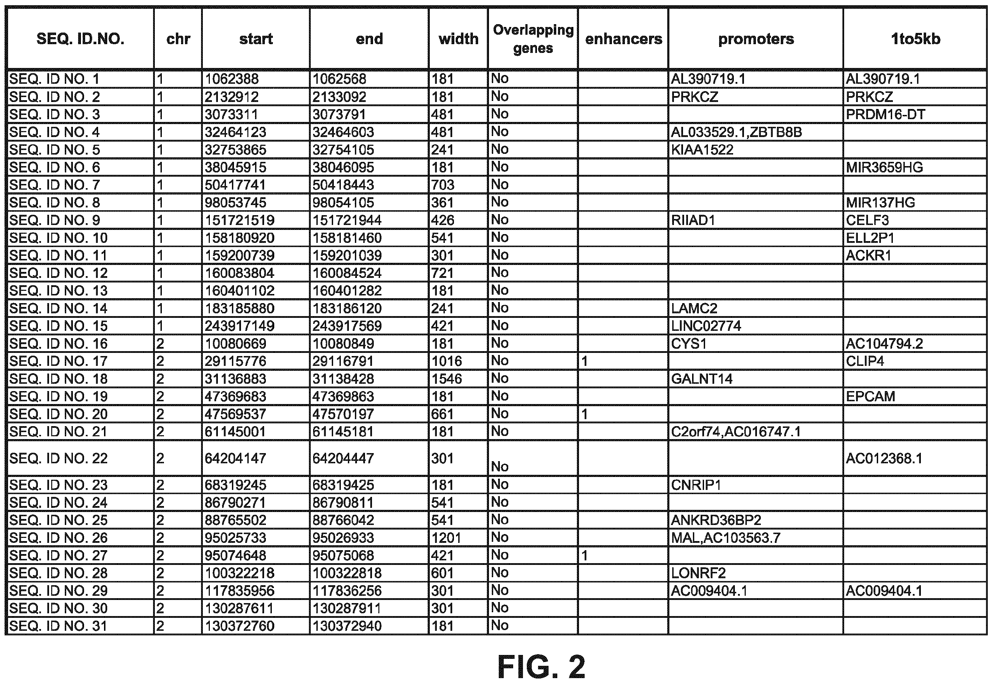

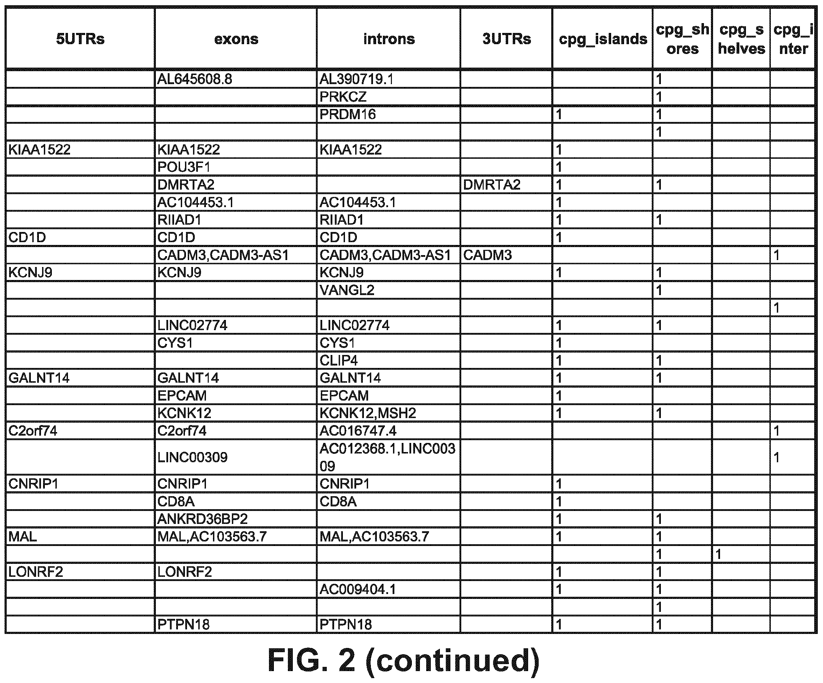

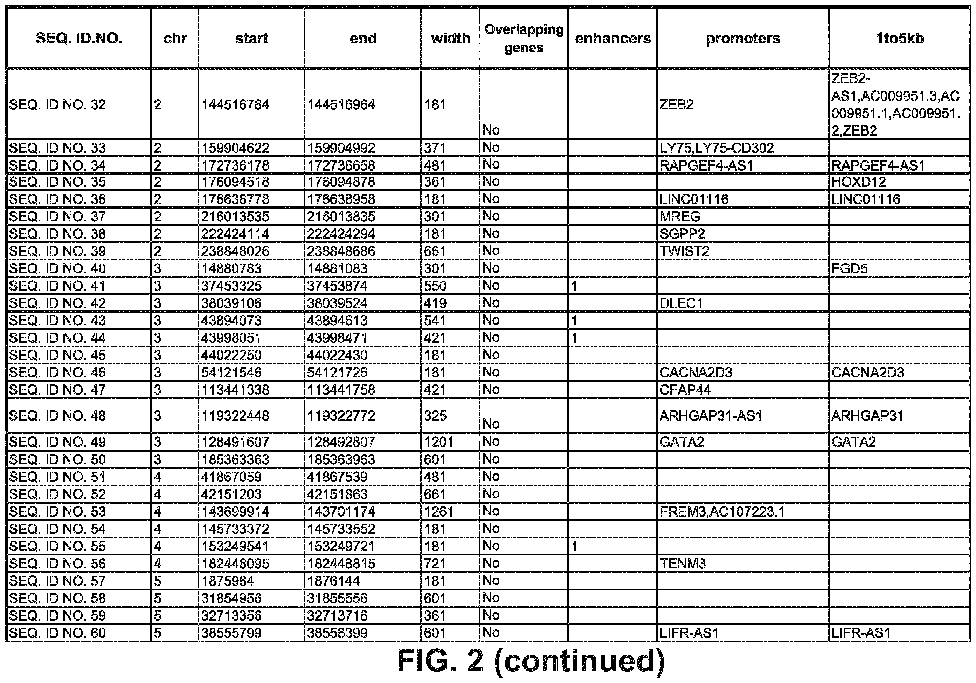

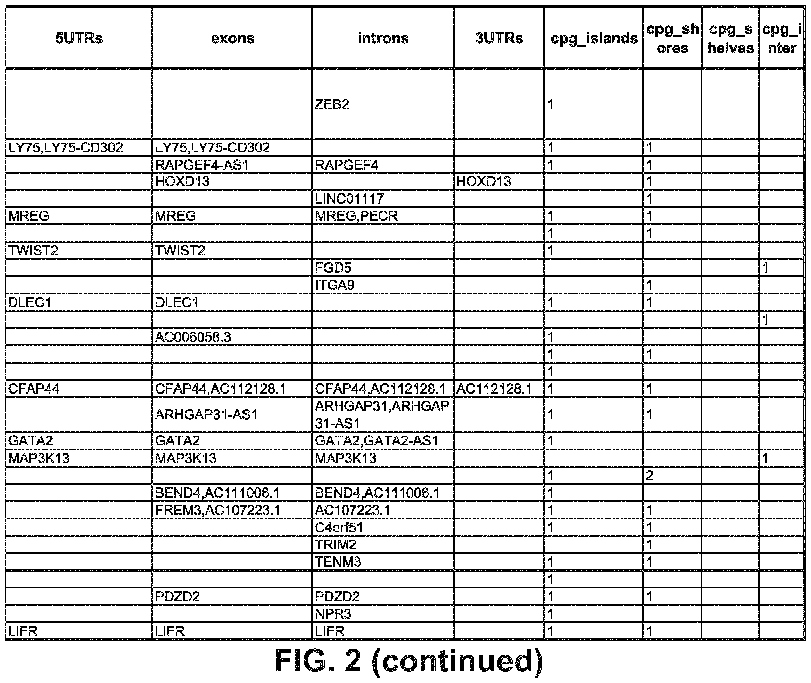

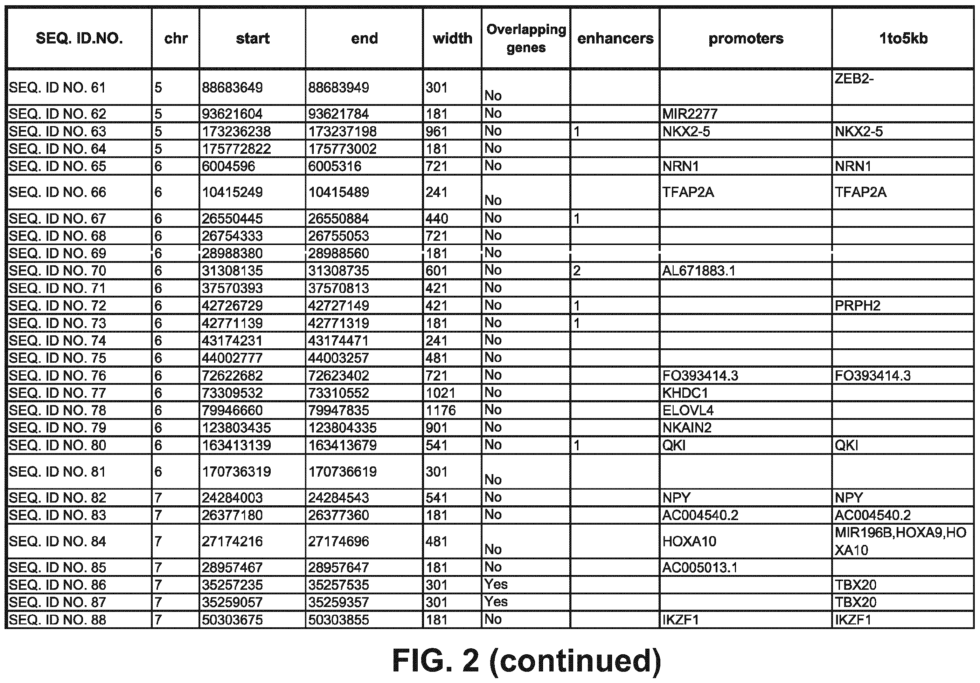

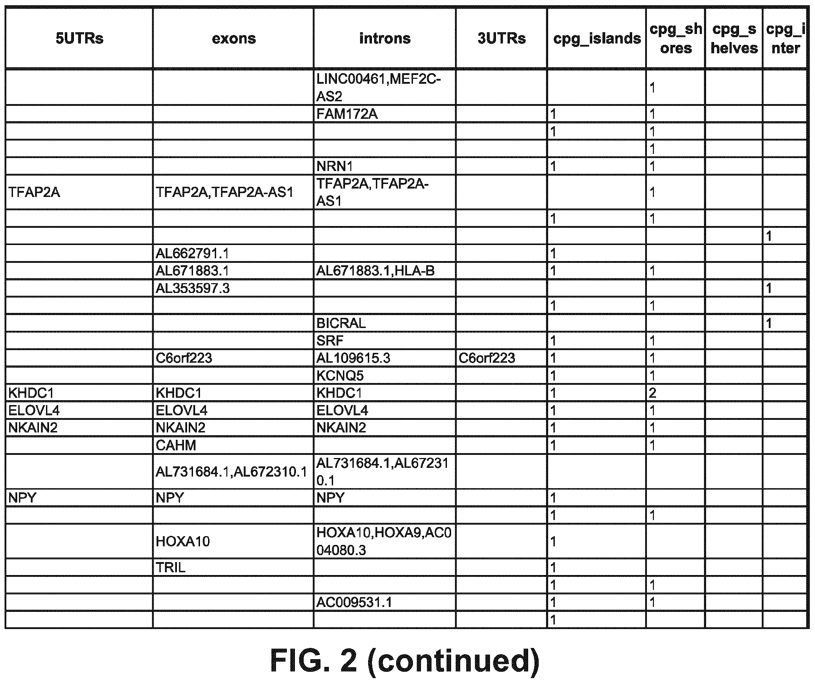

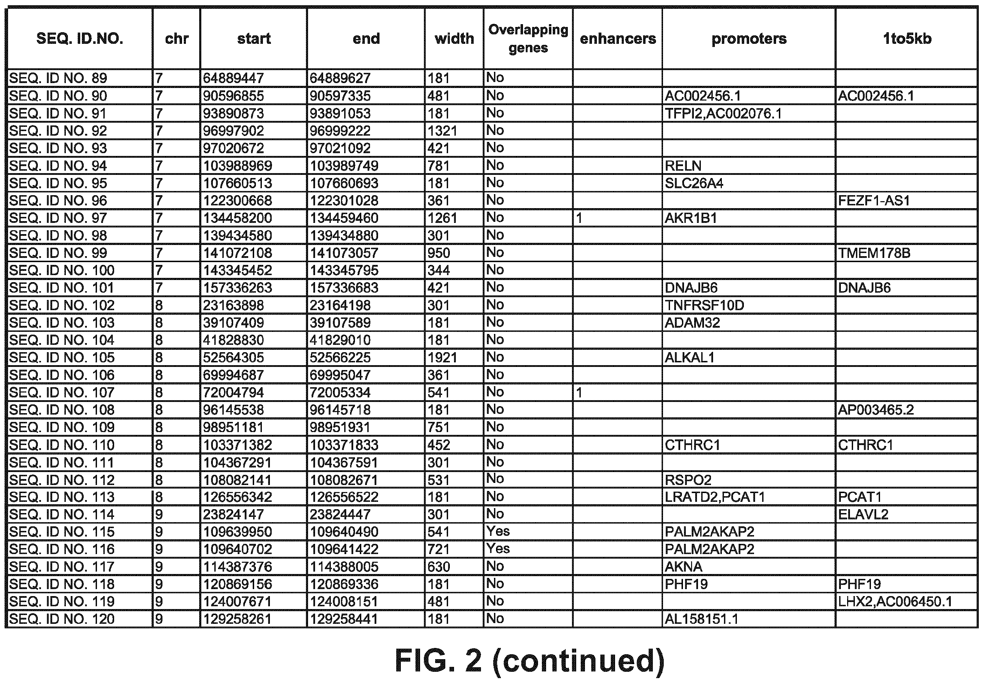

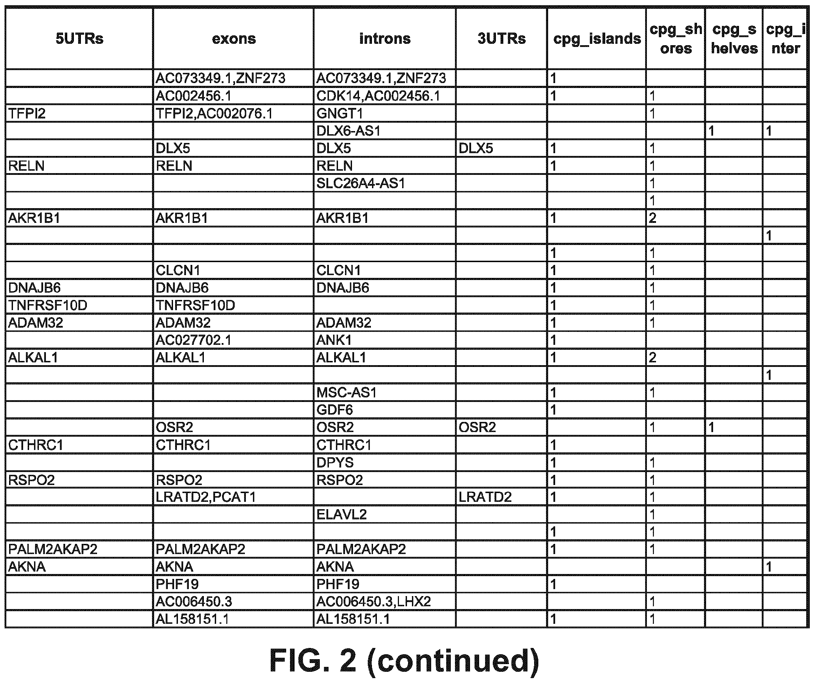

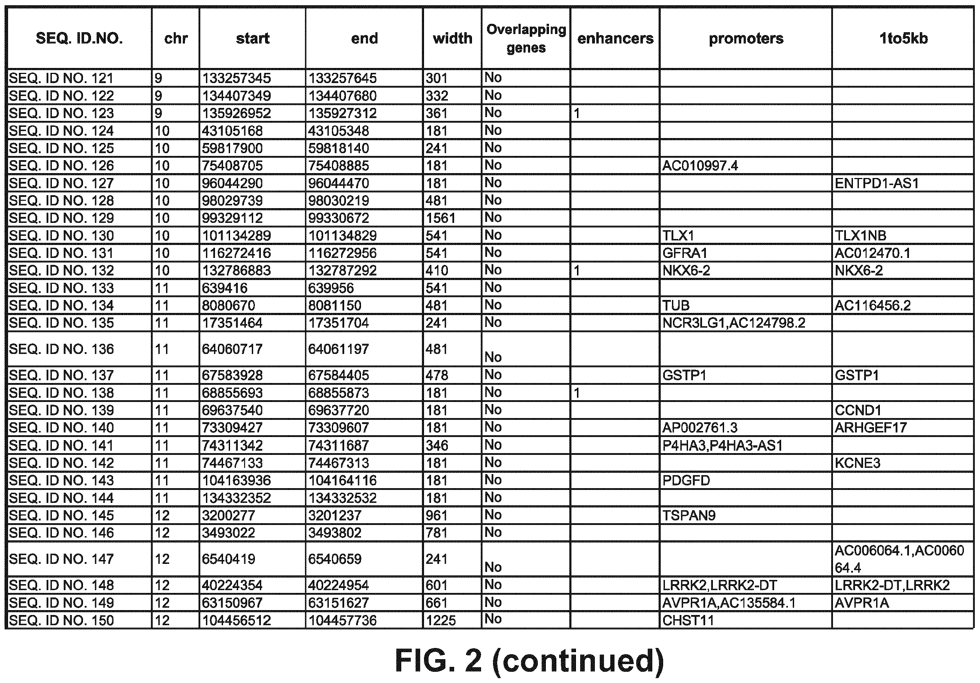

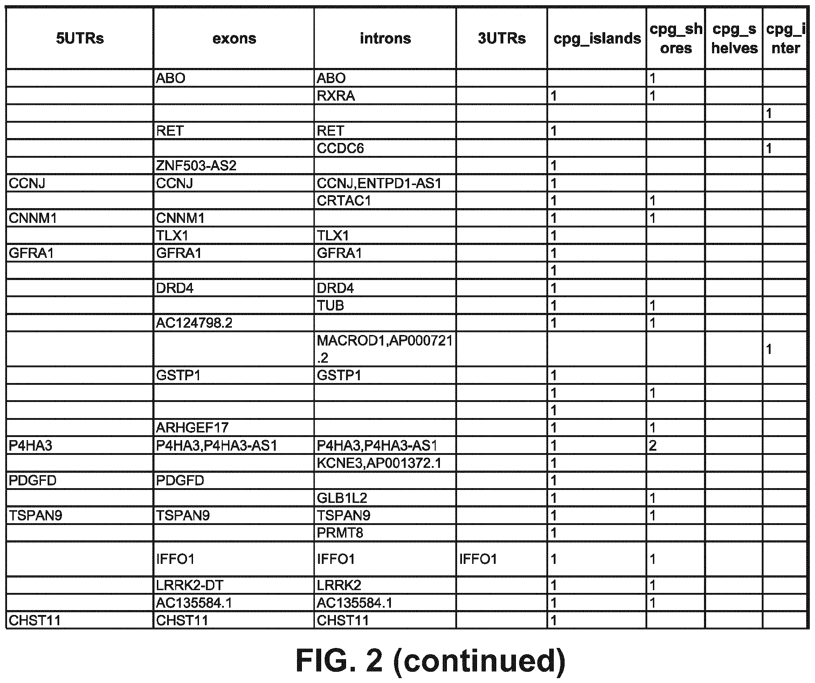

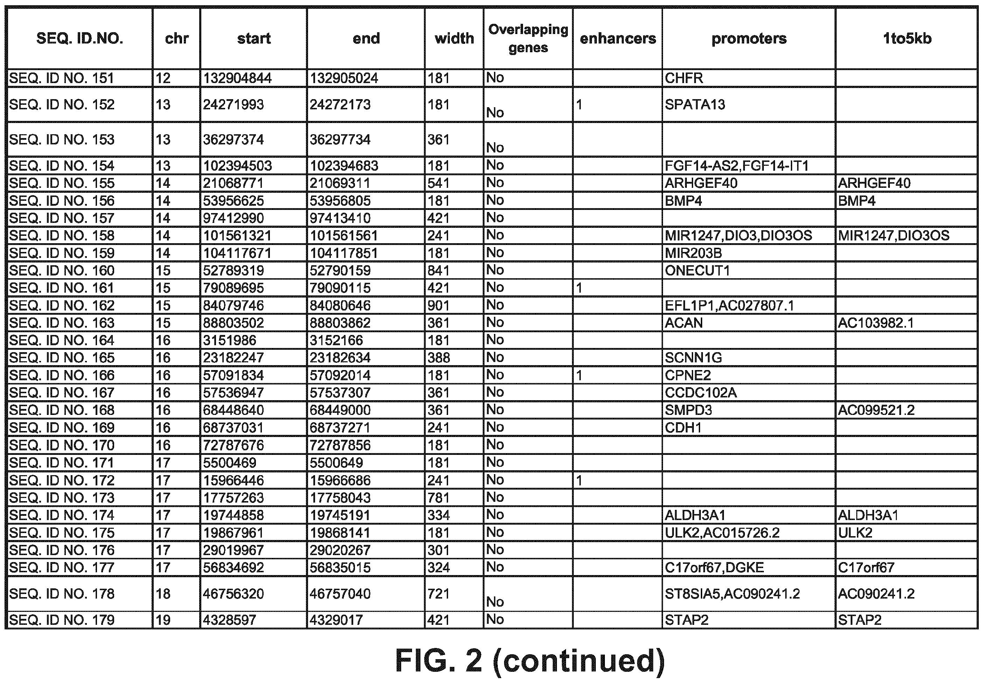

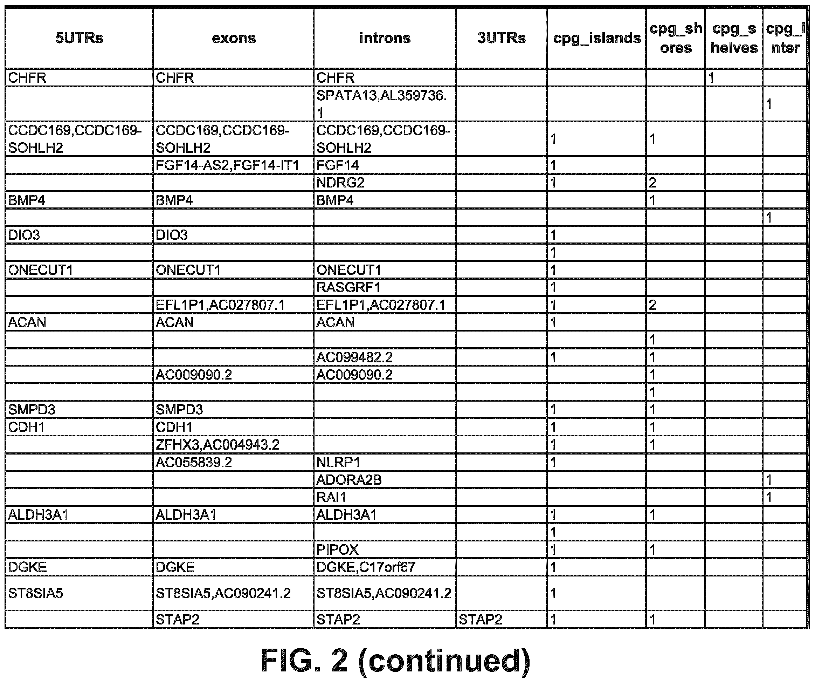

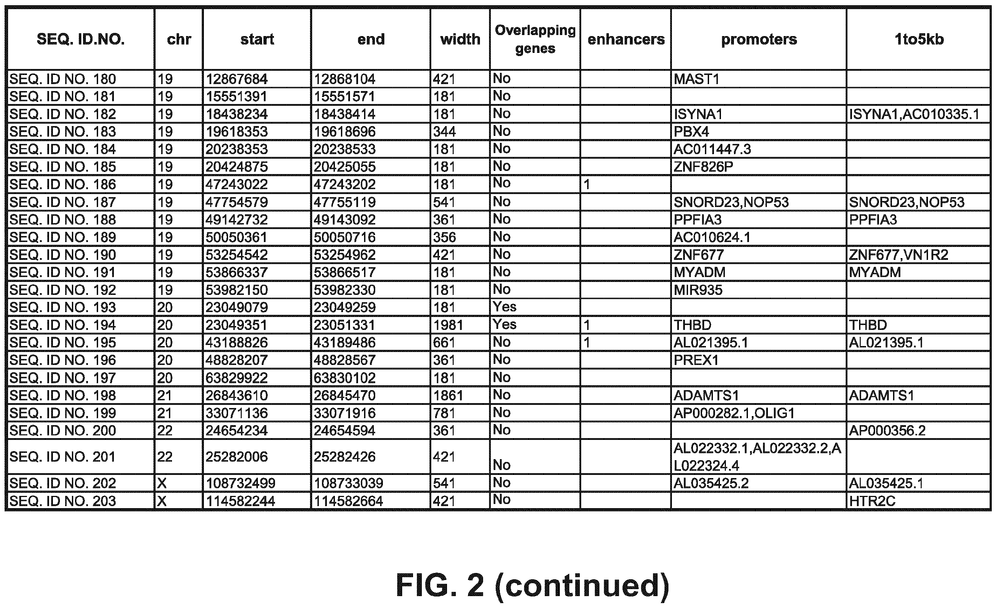

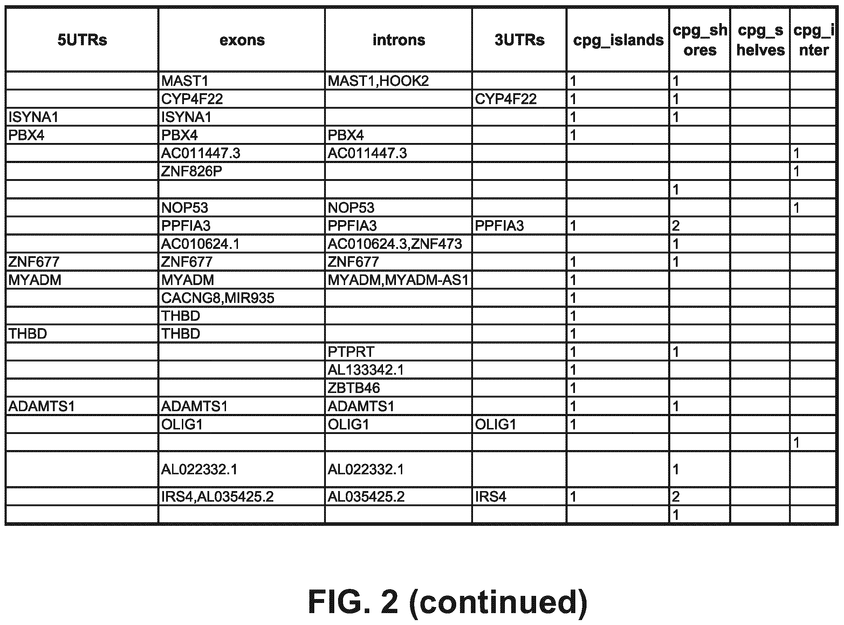

The present disclosure relates to a method of detecting colorectal cancer in a human subject, the method comprising: determining a methylation status of at least a portion of each of the DMRs of Table 9, Table 10, Table 11 and/or Figure 2; identified in DNA from a sample obtained from the subject, and determining whether the subject has colorectal cancer based at least in part on the determined methylation status of at least a portion of each of the DMRs of Table 9, 10, 11 and/or Figure 2 being higher or lower than when compared to a reference sample from a subject not affected by the disease.

In certain embodiments each of the DMRs of Table 9, Table 10, Table 11 and/or Figure 2 is a methylation locus comprising at least a portion comprising at least three (3) CpGs and each said methylation locus having a length equal to or less than 5000 bp.

In certain embodiments, read-wise methylation values obtained from reads of methylation biomarkers are used to identify or diagnose a disease, e.g., using a classification model. In certain embodiments, a read-wise methylation value for a methylation biomarker may be based on a comparison a number of methylated reads of a control DNA sample not affected by the disease and/or condition (e.g., cfDNA from a "healthy" subject, buffy coat DNA, DNA from a "healthy" tissue) as compared to a number of methylated reads of a pathological DNA sample affected by the disease or condition (e.g., cfDNA, e.g., ctDNA).

In various embodiments, the present disclosure relates to methods and/or systems to obtain read-wise methylation values of one or more target biomarkers (e.g., DMRs) using NGS sequencing data. While it is understood that methylation status of individual markers may change in DNA of a subject afflicted by a disease, current bioinformatics-based tools used to identify abnormal methylation are insufficient to accurately detect abnormal methylation patterns. For example, current tools are not sufficiently sensitive to changes in methylation states between control and disease states to detect significant methylation changes in methylation markers. Additionally, such tools suffer from high signal to noise ratio, particularly when using cfDNA as a sample source as, in certain diseases, the amount of cfDNA in a sample may be small in blood or plasma samples. A read-wise assessment of methylation allows for a more appropriate identification and assessment of methylation.

In various embodiments, the present disclosure relates to methods and/or systems for conducting next-generation sequencing (NGS) on samples of DNA, e.g., cfDNA. NGS sequencing on DNA samples is typically conducted using standard sets of manufactured kits and techniques. However, standard NGS techniques may insufficiently cover target regions, particularly as GC content of regions may vary widely from region to region. For example, methylation markers may have high GC content while mutation markers may have low GC content. Under certain NGS sequencing conditions, variations in GC content may lead to over-representation of regions having high GC content and/or underrepresentation of low GC content regions. Steps taken to improve GC coverage of high GC content regions may, in turn, lower coverage of low GC content regions (or vice versa). In addition, current NGS sequencing techniques lack sufficient means for determining data quality of samples.

In certain embodiments, methods and systems disclosed herein may improve NGS sequencing data quality. In certain embodiments, coverage of low GC content regions may be improved by altering probe design and/or experimental parameters related to DNA sample processing. For example, sequencing of a mutation marker with low CG content may be improved by increasing probe tiling density and/or overlapping probes over the low GC content region. In certain embodiments, GC dropout rate is used as a quality control value to assess NGS sequencing coverage. GC dropout rate is indicative of coverage of high GC content target regions (e.g., regions having greater than 50% GC content). In certain embodiments, sequencing data having a low GC dropout rate (e.g., 6% or less) are desirable.

In certain embodiments, conversion rates of DNA is used to quantitatively assess NGS data quality. For example, conversion of a control sequence of DNA (e.g., a spike-in control) may be used to assess conversion (e.g., bisulfite or enzymatic conversion) rates of unmethylated and/or methylated cytosines to uracil. A high conversion efficiency of unmethylated cytosines to uracil is desirable when, for example, bisulfite or enzymatically treating DNA. Unconverted cytosines are typically identified as being methylated when reviewing data of sequenced, converted DNA. In certain embodiments, low conversion rates of methylated cytosines to uracil is desirable. In certain embodiments, parameters related to DNA conversion are changed to enhance or alter conversion rates. For example, increasing a ratio of bisulfite reagent to DNA may be used to alter conversion rates. In certain embodiments, altering a thermocycler step including a number of cycles (e.g., a number of denaturation and conversion steps), time of a step in a cycle, temperature of a cycle, or combination thereof may be adjusted to affect a conversion rate.

In one aspect, the invention is directed to a method comprising: converting unmethylated cytosines of a plurality of DNA fragments in a sample into uracils to generate a plurality of converted DNA fragments, wherein the plurality of DNA fragments were obtained from a biological sample; and sequencing the plurality of converted DNA fragments to generate a plurality of sequence reads, wherein each sequence read corresponds to a converted DNA fragment.

In certain embodiments, converting the unmethylated cytosines of the plurality of DNA fragments comprises subjecting the plurality of DNA fragments to bisulfite treatment. In certain embodiments, the plurality of DNA fragments comprises double-stranded DNA fragments and the bisulfite treatment comprises: (i) denaturing the plurality of DNA fragments in the sample to generate a plurality of single stranded DNA fragments, and (ii) converting unmethylated cytosines of the plurality of single stranded DNA fragments into uracils to generate the plurality of converted DNA fragments. In certain embodiments, the denaturing step of (i) is performed at a temperature of 90-97 °C. In certain embodiments, the denaturing step of (i) is performed for less than 10 minutes, less than 5 minutes, or less than 2 minutes (e.g., for each repetition if step (i) is repeated). In certain embodiments, the converting step of (ii) is performed at a temperature of 55-65 °C. In certain embodiments, the converting step of (ii) is performed for less than 5 hours, less than 4.5 hours, less than 4 hours, less than 3 hours, less than 2 hours, less than 1 hour, less than 30 minutes, or less than 15 minutes, less than 5 min (e.g., for each repetition if step (i) is repeated). In certain embodiments, the denaturing step of (i) is performed at a temperature in a range from 93 °C to 97 °C (e.g., at about 95 °C) for about 1 minute, and the converting step of (ii) is performed at a temperature in a range from 58 °C to 62 °C (e.g., at 60 °C) for about 10 minutes. In certain embodiments, the denaturing step of (i) and the converting step of (ii) are repeated. In certain embodiments, the denaturing step of (i) and the converting step of (ii) are repeated at least five times, at least ten times, at least fifteen times, or at least 20 times.

In certain embodiments, converting the unmethylated cytosines of the plurality of DNA fragments comprises subjecting the plurality of DNA fragments to an enzymatic treatment.

In certain embodiments, converting the unmethylated cytosines of the plurality of DNA fragments comprises denaturing the plurality of DNA fragments (e.g., using formamide, using sodium hydroxide).

In certain embodiments, denaturing the plurality of DNA fragments is performed prior to subjecting the plurality of DNA fragments to the enzymatic treatment.

In certain embodiments, the enzymatic treatment comprises contacting the plurality of DNA fragments with a member of the Apolipoprotein B mRNA Editing Catalytic Polypeptide-like (APOBEC) family (e.g., APOBEC-1, APOBEC-2, APOBEC-3A, APOBEC-3B, APOBEC-3C, APOBEC-3D, APOBEC-3E, APOBEC-3F, APOBEC-3G. APOBEC-3H, APOBEC-4, and/or Activation-induced (cytidine) deaminase (AID)) (e.g., wherein the plurality of DNA fragments are contacted with APOBEC, e.g., an APOBEC reaction buffer).

In certain embodiments, subjecting the plurality of DNA fragments to an enzymatic treatment is performed for less than 4 hours, less than 3 hours, less than 2 hours, less than 1 hour, less than 30 minutes, or less than 15 minutes (e.g., wherein the plurality of DNA fragments are subjected to treatment with APOBEC from about 2 to about 4h, e.g., about 3h).

In certain embodiments, the plurality of DNA fragments comprise a plurality of cell-free DNA (cfDNA) fragments.

In certain embodiments, the plurality of DNA fragments comprise cellular DNA fragments.

In certain embodiments, the plurality of DNA fragments (in total) comprise at least 1 ng, at least 5 ng, at least 10 ng, or at least 20 ng of DNA.

In certain embodiments, the plurality of DNA fragments consist essentially of DNA fragments each of which has a length in a range from 100bp to 600bp (e.g., from about 125bp to about 200bp, or from about 140bp to about 160bp (e.g., for cfDNA))(e.g., about 150bp to about 350bp, or from about 200bp to about 300bp (e.g., for sheared DNA)).

In certain embodiments, the plurality of DNA fragments consist essentially of DNA fragments each of which has a length in a range from 1000bp to 200,000bp [e.g., having an average length of about 10,000bp (e.g., for genomic DNA, e.g., from a sample comprising tissue or buffy coat)].

In certain embodiments, the plurality of sequence reads are at least 50bp, at least 100bp, at least 150bp, at least 200bp, at least 300bp, at least 400bp, at least 500bp or more.

In certain embodiments, the sequencing step comprises pair-end sequencing.

In certain embodiments, the sequencing step comprises single-end sequencing.

In certain embodiments, the biological sample comprises blood, serum, urine, plasma, or stool.

In certain embodiments, the biological sample comprises colorectal cells, polyp cells, glandular cells, or cancer cells.

In certain embodiments, the biological sample is a biological sample from a mammal.

In certain embodiments, the biological sample is a biological sample from a human.

In certain embodiments, the method further comprises fragmenting DNA from the biological sample to generate the plurality of DNA fragments.

In certain embodiments, the method comprises attaching adapters to the plurality of converted DNA fragments (e.g., prior to the sequencing step). In certain embodiments, the method comprises attaching an adapter to both the 5' and 3' ends of the plurality of converted DNA fragments. In certain embodiments, the attaching step is performed such that at least 40%, at least 50%, at least 60%, at least 70% of the converted DNA fragments are attached to adapter. In certain embodiments, the adapters comprise a sample index. In certain embodiments, the adapters comprise a fragment barcode. In certain embodiments, attaching an adapter comprises ligation. In certain embodiments, attaching an adapter comprises PCR. In certain embodiments, the attaching step is performed such that at least 40%, at least 50%, at least 60%, at least 70% of the converted DNA fragments have an adapter attached at both the 5' and 3' ends. In certain embodiments, the adapter at the 5' end, the 3' end, or both of the converted single stranded DNA fragments comprises a sample index. In certain embodiments, the adapter at the 5' end, the 3' end, or both of converted single stranded DNA fragments comprises a fragment barcode. In certain embodiments, the fragment barcode on each of the converted single stranded DNA fragments is different. In certain embodiments, the fragment barcode on at least two of the converted single stranded DNA fragments is the same, wherein the at least two converted single stranded DNA fragments are not in a Watson-Crick pair with each other in the biological sample.

In certain embodiments, the method comprises amplifying the plurality of converted DNA fragments to which adapters have been attached (e.g., wherein the method comprises amplifying a library prepared using converted DNA fragments).

In certain embodiments, the method further comprises enriching the converted DNA fragments. In certain embodiments, enriching comprises selectively enriching. In certain embodiments, selectively enriching comprises amplification. In certain embodiments, selectively enriching comprises hybrid capture. In certain embodiments, hybrid capture comprises capturing a subset of converted DNA fragments with capture probes that target one or more genomic regions (e.g., DMRs) in a genome of interest.

In certain embodiments, the one or more genomic regions comprises regions comprising one or more CpG sites.

In certain embodiments, the one or more genomic regions comprise regions of high GC content (e.g, from about 70% to about 80% GC content) or low GC content (e.g., from about 30% to about 40% GC content).

In certain embodiments, the one or more genomic regions comprise regions of GC content ranging from about 50% to about 60% GC content.

In certain embodiments, the one or more genomic regions comprise one or more genomic mutations. In certain embodiments, the one or more genomic regions comprise both (i) regions comprising one or more CpG sites and (ii) regions known to include one or more genomic mutations.

In certain embodiments, the one or more genomic mutations comprise a single nucleotide variant, an inversion, a deletion, an insertion, a transversion, a translocation, a fusion, a truncation, an amplification, or a combination thereof.

In certain embodiments, the one or more genomic regions comprise at least a portion of a NRAS gene, a PTEN gene, a PIK3CA gene, a STK11 gene, a TP53 gene, a KIT gene, a MET gene, a KRAS gene, a BRAF gene, or a EGFR gene.

In certain embodiments, the capture probes are overlapping capture probes (e.g., wherein the capture probes are tiled). In certain embodiments, the capture probes are overlapping by at least 10bp, at least 20bp, at least 30bp, at least 40bp, at least 50bp, at least 60bp, at least 70bp, at least 80bp, at least 90bp, or at least 100bp.

In certain embodiments, the capture probes are from about 50bp to about 200bp in length (e.g., about 120bp in length).

In certain embodiments, the capture probes comprise at least one capture probe that targets a fully methylated genomic region.

In certain embodiments, the capture probes comprise at least one capture probe that targets a fully unmethylated genomic region.

In certain embodiments, the capture probes comprise at least one capture probe that targets a partially methylated genomic region (e.g., wherein at least 1 CpG site is methylated).

In certain embodiments, capture probes targeted to a genomic region (e.g., a fully methylated region, a partially methylated region, a fully unmethylated region) do not capture a DNA fragment which corresponds exactly to the targeted sequence. A capture probe may bind (e.g., hybridize) to a DNA fragment and form a mismatched bond between some base pairs (e.g., non-Watson-Crick base pairings). For example, in certain embodiments up to 8 mismatches (e.g., non-Watson-Crick base pairings) may be tolerated when a capture probe binds to (e.g., hybridizes to) a DNA fragment. Mismatches allow for capture probes that, for example, target a fully methylated genomic region to hybridize to DNA fragments which may be partially methylated.

In certain embodiments, the capture probes comprise at least one capture probe that targets a coding strand.

In certain embodiments, the capture probes comprise at least one capture probe that targets a template strand (e.g., non-coding strand).

In certain embodiments, the capture probes comprise a group of capture probes that target at least one of the one or more genomic regions, wherein the group of capture probes comprise: (i) at least one capture probe that targets a fully methylated template strand of the genomic region; (ii) at least one capture probe that targets a fully methylated coding strand of the genomic region; (iii) at least one capture probe that targets a fully unmethylated template strand of the genomic region; and (iv) at least one capture probe that targets a fully unmethylated coding strand of the genomic region (e.g., wherein the group of capture probes has at least four capture probes targeting the genomic region - one capture probe targeted to a methylated version of the forward (e.g., coding) strand, one capture probe targeted to an unmethylated version of the forward (e.g., coding) strand, one capture probe targeted to a methylated version of the reverse (e.g., non-coding) strand, and one capture probe targeted to an unmethylated version of the reverse (e.g., non-coding) strand.

In certain embodiments, the capture probes that target one or more genomic regions in the genome of interest target no more than 1000 similar regions, no more than 500 similar regions, no more than 400 similar regions, no more than 300 similar regions, no more than 200 similar regions, no more than 100 similar regions, no more than 25 similar regions, no more than 10 similar regions, no more than 5 similar regions to the one or more genomic regions in the genome of interest (e.g., wherein the capture probe targeting one of the one or more genomic regions hybridizes to no more than 1000 regions similar to the targeted region of interest) (e.g., wherein the similarity of a target genomic region and another similar region is quantified using a 24bp sequence window). In certain embodiments, (i) a first set of capture probes targets one or more genomic regions comprising regions that comprise one or more CpG sites and a second set of capture probes targets one or more genomic regions known to include one or more genomic mutations, and (ii) the first set of capture probes does not include overlapping capture probes and the second set of capture probes does include overlapping capture probes. In certain embodiments, the overlapping probes overlap at least 10%, at least 20%, at least 30%, at least 40%, at least 50%, at least 60% or more.

In certain embodiments, at least two of the capture probes target the same genomic region.

In certain embodiments, at least 2, at least 4, at least 9, at least 10, at least 25, at least 50, at least 75, at least 100, at least 150, at least 200, at least 203, at least 220 genomic regions are targeted by capture probes.

In certain embodiments, the capture probes are RNA probes.

In certain embodiments, the capture probes are DNA probes.

In certain embodiments, each of the one or more genomic regions are equal to or less than 5000bp, 4000 bp, 3000 bp, 2000 bp, 1000bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, or 10 bp in length.

In certain embodiments, at least one of the one or more genomic regions has a length of at least 5000bp, 4000 bp, 3000 bp, 2000 bp, 1000bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, or 10 bp.

In certain embodiments, the method further comprises amplifying the converted DNA fragments. In certain embodiments, the step of amplifying occurs after the step of selectively enriching.

In certain embodiments, the plurality of converted DNA fragments that are sequenced correspond to at least 30%, at least 40% or at least 50% of the converted DNA fragments that were captured by hybrid capture.

In certain embodiments, the method further comprises adding control DNA molecules to the sample, wherein the sequence, number of methylated bases, and number of unmethylated bases of the control DNA molecules had been determined prior to addition of the control DNA to the sample. In certain embodiments, the method further comprises converting unmethylated cytosines of the control DNA molecules in the sample into uracils to generate converted control DNA molecules; and sequencing the converted control DNA molecules to generate a plurality of control sequence reads. In certain embodiments, converting unmethylated cytosines of the control DNA molecules and the DNA fragments occurs simultaneously. In certain embodiments, sequencing the converted control DNA molecules and DNA fragments occurs simultaneously. In certain embodiments, the method further comprises determining the number of unmethylated cytosines of the control DNA molecules that were converted into uracils.

In certain embodiments, at least 90%, at least 95%, at least 96%, at least 97%, at least 98%, at least 99% of unmethylated cytosines of the control DNA molecules are converted into uracils.

In certain embodiments, the method further comprises determining the number of methylated cytosines of the control DNA molecules that were converted into uracils. In certain embodiments, at most 5%, at most 4%, at most 3%, at most 2% or at most 1% of methylated cytosines of the plurality of DNA fragments are converted into uracils.

In another aspect, the invention is directed to a method comprising: mapping a plurality of sequence reads to a reference sequence, wherein the plurality of sequence reads corresponds to a plurality of converted DNA fragments in a sample (e.g., a cfDNA sample from a subject for which it is unknown if the subject is suffering from a disease, disorder or condition associated with aberrant levels of DNA methylation), wherein unmethylated cytosines of a plurality of DNA fragments in the sample had been converted into uracils to generate the plurality of converted DNA fragments, the plurality of sequence reads comprises one or more subsets of sequence reads that (e.g., collectively) map to one or more genomic regions, and each subset of sequence reads comprises sequence reads that map to a unique genomic region (e.g., a DMR); and determining a methylation level for each of the one or more subsets of sequence reads [(e.g., determining a methylation level for each subset of sequence reads) (e.g., determining a methylation level for each genomic region) (e.g., wherein the methylation level for a given subset corresponds to a number of sequence reads in the subset that meet a threshold condition (e.g., normalized by a library size), e.g., wherein the threshold condition comprises a minimum total CpG count and a minimum methylated CpG count (e.g., minimum % CpGs that are methylated)) (e.g., wherein a given read for a DMR does not necessarily cover the entire DMR)].

In certain embodiments, the method further comprises: mapping the plurality of sequence reads to a reference sequence, wherein the plurality of sequence reads corresponds to a plurality of converted DNA fragments in a sample, wherein unmethylated cytosines of a plurality of DNA fragments in a sample had been converted into uracils to generate the plurality of converted DNA fragments, the plurality of sequence reads comprises one or more subsets of sequence reads that (e.g., collectively) map to one or more genomic regions, and each subset of sequence reads comprises sequence reads that map to a unique genomic region (e.g., a DMR); and determining a methylation level for each of the one or more subsets of sequence reads [(e.g., determining a methylation level for each subset of sequence reads) (e.g., a methylation level for each genomic region) (e.g., wherein the methylation level for a given subset corresponds to a number of sequence reads in the subset that meet a threshold condition (e.g., normalized by a library size), e.g., wherein the threshold condition comprises a minimum total CpG count and a minimum methylated CpG count (e.g., minimum % CpGs that are methylated)) (e.g., wherein a given read for a DMR does not necessarily cover the entire DMR)].

In certain embodiments, each of the one or more genomic regions are equal to or less than 5000bp, 4000 bp, 3000 bp, 2000 bp, 1000bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, or 10 bp in length.

In certain embodiments, at least one of the one or more genomic regions has a length of at least 5000bp, 4000 bp, 3000 bp, 2000 bp, 1000bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, 10 bp, or 8 bp.

In certain embodiments, the one or more genomic regions comprise at least 2, at least 4, at least 9, at least 10, at least 25, at least 50, at least 75, at least 100, at least 150, at least 200, at least 203, at least 220 genomic regions.

In certain embodiments, the reference sequence is a bisulfite-converted genome.

In certain embodiments, the reference sequence is a bisulfite-converted human genome. In certain embodiments, determining a methylation level for each of the one or more subsets of sequence reads comprises: determining, for a given sequence read, a total number of CpG sites and a total number of methylated CpG sites. In certain embodiments, determining a methylation level for each of the one or more subsets of sequence reads comprises: assigning a value (e.g., a binary value) to the given sequence read, wherein the value is determined by a comparison of (i) the total number of CpG sites in the read, or the total number of methylated CpG sites in the read, or a number based on the total number of CpG sites and the total number of methylated CpG sites in the read (e.g., a percentage of the total number of CpG sites that are methylated) with (ii) one or more reference values (e.g., a threshold for total number of CpG sites and a threshold for percentage of CpG sites that are methylated).

In certain embodiments, determining the methylation level for a given subset of sequence reads that map to a given genomic region comprises summing the values assigned to sequence reads in the subset, and repeating this determination for each subset of sequence reads (e.g., wherein each subset of sequence reads maps to a unique DMR, and wherein a methylation level is determined for each DMR). In certain embodiments, the method comprises detecting (e.g., using a machine learning algorithm) a disease, disorder or condition associated with aberrant levels of DNA methylation based at least in part on the summed values.

In certain embodiments, the method comprises detecting (e.g., using a machine learning algorithm) a disease, disorder or condition associated with aberrant levels of DNA methylation based at least in part on the methylation levels for each of the one or more subsets of sequence reads.

In certain embodiments, the method further comprises deduplicating sequencing reads. In certain embodiments, deduplicating sequencing reads is performed prior to determining a methylation level for individual sequence reads in the one or more subsets of sequence reads.

In certain embodiments, deduplicating sequence reads includes deduplicating optical duplicate sequence reads. In certain embodiments, optical duplicate sequence reads share a tile (e.g., a spot on a flow cell used for NGS). In certain embodiments, a distance between the optical duplicate sequence reads is less than 2500bp, less than 2000bp, less than 1500bp, less than 1000bp, less than 500bp, less than 100bp.

In certain embodiments, deduplicating sequence reads includes deduplicating PCR duplicate sequencing reads and/or over-sequencing duplicate sequence reads. In certain embodiments, two or more sequence reads are considered PCR duplicate sequencing reads and/or over-sequencing duplicate sequence reads if the two or more sequence reads have (1) a 5' end coordinate, (2) a 3' end coordinate, and (3) a methylation status of each given specific CpG location on the read are the same (e.g., a binary value assigned to each given specific CpG location on the read is the same), wherein the 5' end coordinate and the 3' end coordinate of a sequence read correspond to the position at which the 5'-most nucleotide and the 3'-most nucleotide, respectively, of the sequence read map to the reference sequence.

In certain embodiments, deduplicating sequence reads does not comprise removing duplicate sequence reads that have a different methylation status at a specific CpG location.

In certain embodiments, deduplicating sequence reads includes deduplication of sequence reads corresponding to strands found in a Watson-Crick pair with each other in a biological sample.

In certain embodiments, the method comprises removing one or more nucleic acid bases from one or both ends of each of the plurality of sequence reads prior to mapping (e.g., wherein the one or more nucleic acid bases correspond to an adapter sequence, index, and/or barcode).

In certain embodiments, the method further comprises determining a GC dropout rate for each subset of sequence reads. In certain embodiments, the GC dropout rate is less than 6%.

In certain embodiments, mapping the plurality of sequence reads to the reference sequence further comprises determining an on-target ratio (e.g., percentage), wherein the on-target ratio is a ratio of the number of on-target and/or near-target bases of the plurality of sequence reads to the total number of mapped bases of the plurality of sequence reads. In certain embodiments, the on-target ratio is at least 10%, least 20%, least 30%, least 40%, least 50%, least 60%, least 70%, at least 80%, at least 90%, at least 95%, at least 99%.

In certain embodiments, mapping the plurality of sequence reads to the reference sequence further comprises determining an off-target ratio (e.g., percentage), wherein the off-target ratio is a ratio of the number of off-target bases of the plurality of sequence reads to the total number of mapped bases of the plurality of sequence reads. In certain embodiments, the off-target ratio is less than 95%, less than 90%, less than 85%, less than 80%, less than 70%, less than 60%, less than 50%, less than 40%, less than 30%, less than 20%, less than 10%, less than 5%.

In certain embodiments, mapping the plurality of sequence reads to the reference sequence further comprises determining a mapping quality score for each of the plurality of mapped sequence reads, wherein the mapping quality score is a value corresponding to the probability that a sequence read is misplaced (e.g., wherein the mapping position of the sequence read is incorrect) (e.g., wherein the mapping quality score is a function of the logarithm of the probability that the sequence read is misplaced).

In certain embodiments, the method comprises determining a methylation level for the sequence reads in the one or more subsets if the mapping quality score for the sequence read is at least 10, at least 15, at least 20, at least 25, at least 30.

In certain embodiments, the mapping quality score is a single end mapping quality score. In certain embodiments, the mapping quality score is a paired-end mapping quality score.

In certain embodiments, the method further comprises detecting the presence or absence of one or more mutations based on sequence information from the plurality of sequence reads. In certain embodiments, the one or more genomic mutations comprise a single nucleotide variant, an inversion, a deletion, an insertion, a transversion, a translocation, a fusion, a truncation, an amplification, or a combination thereof. In certain embodiments, the one or more mutations are present in one or more of an NRAS gene, PTEN gene, PIK3CA gene, STK11 gene, TP53 gene, KIT gene, MET gene, KRAS gene, BRAF gene, and EGFR gene.

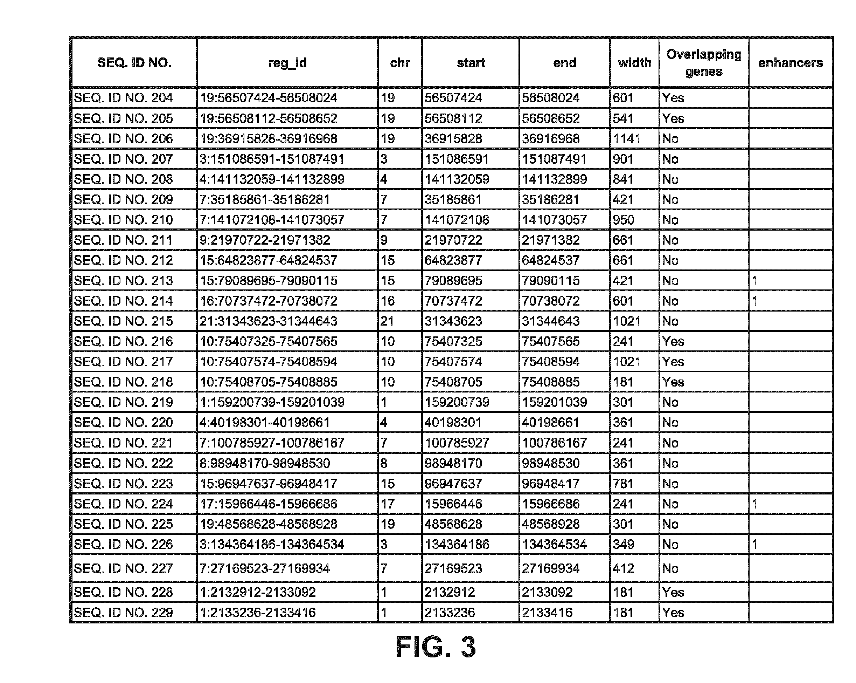

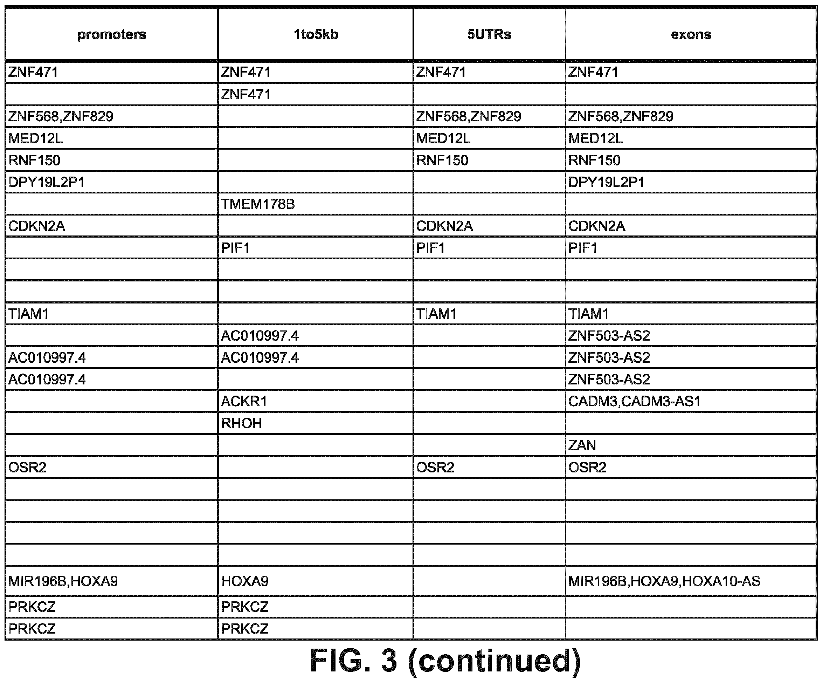

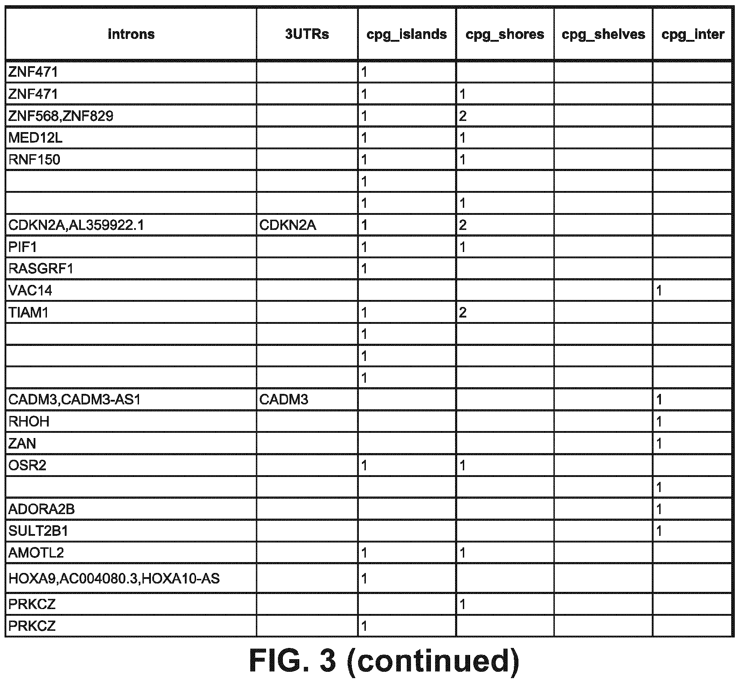

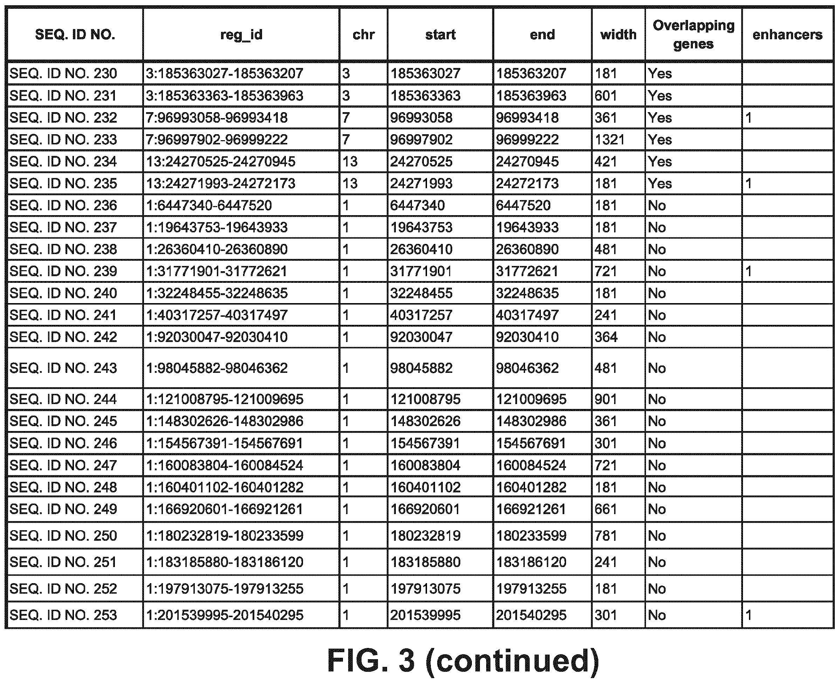

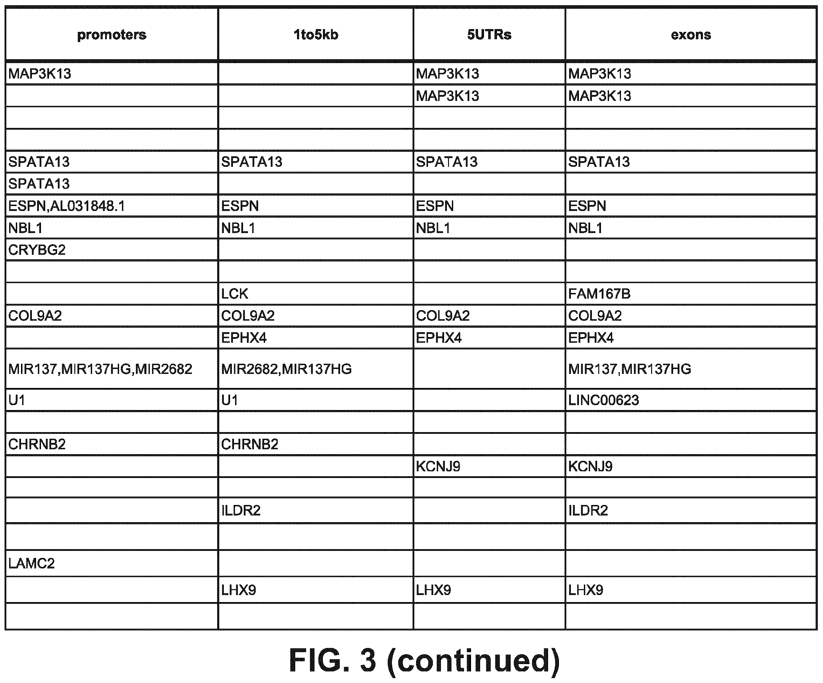

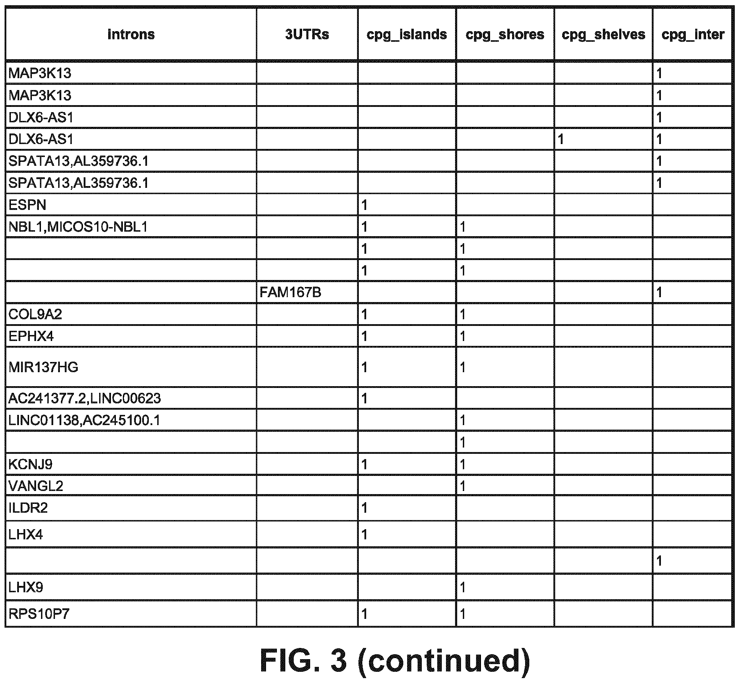

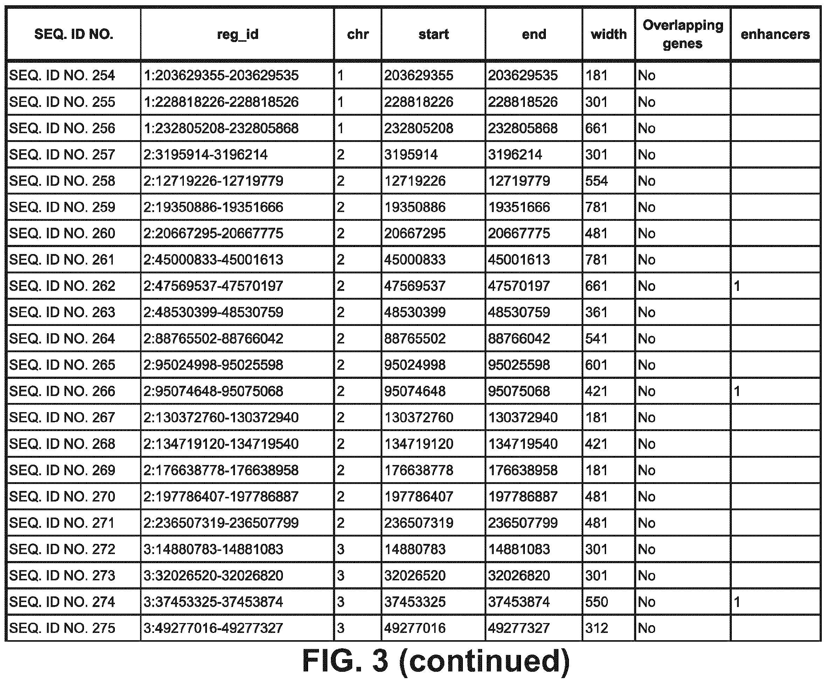

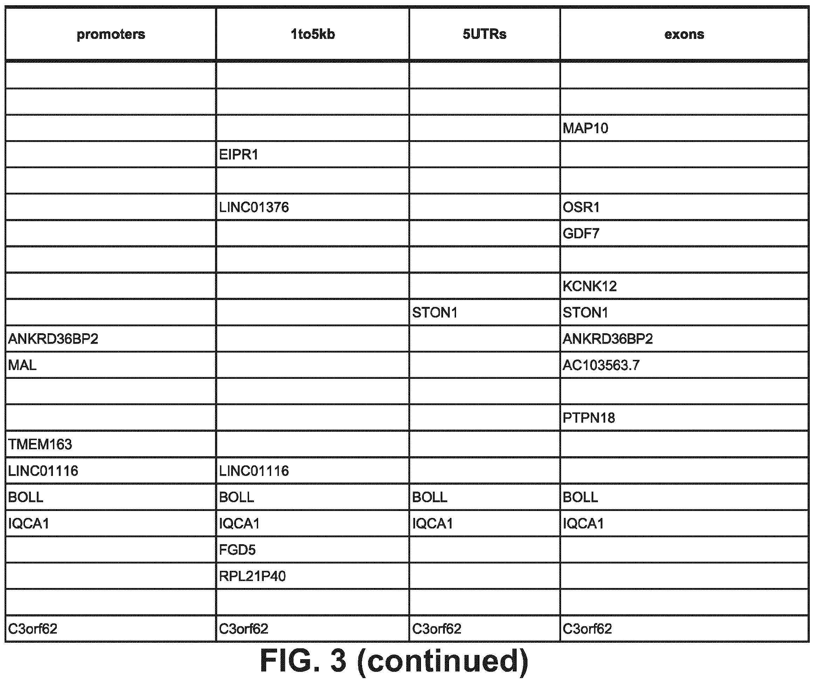

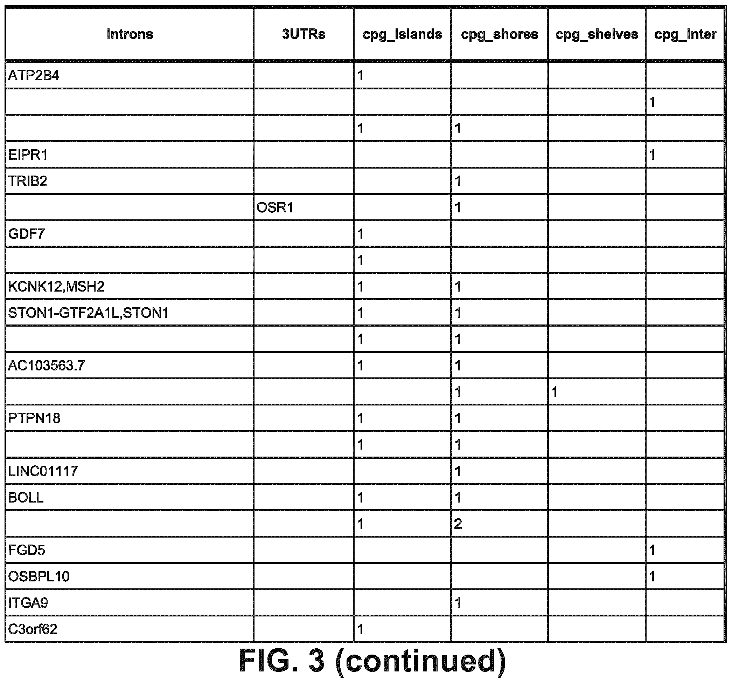

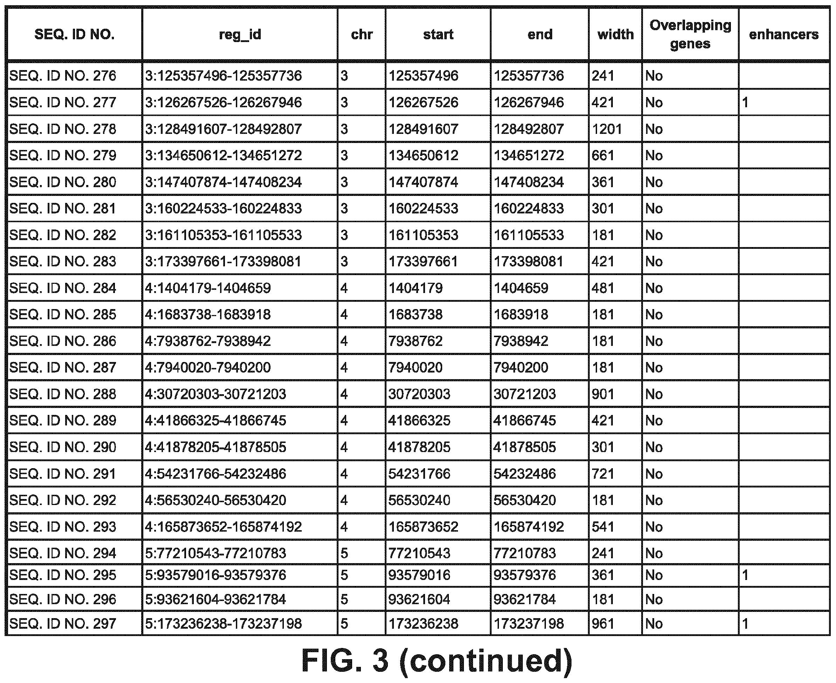

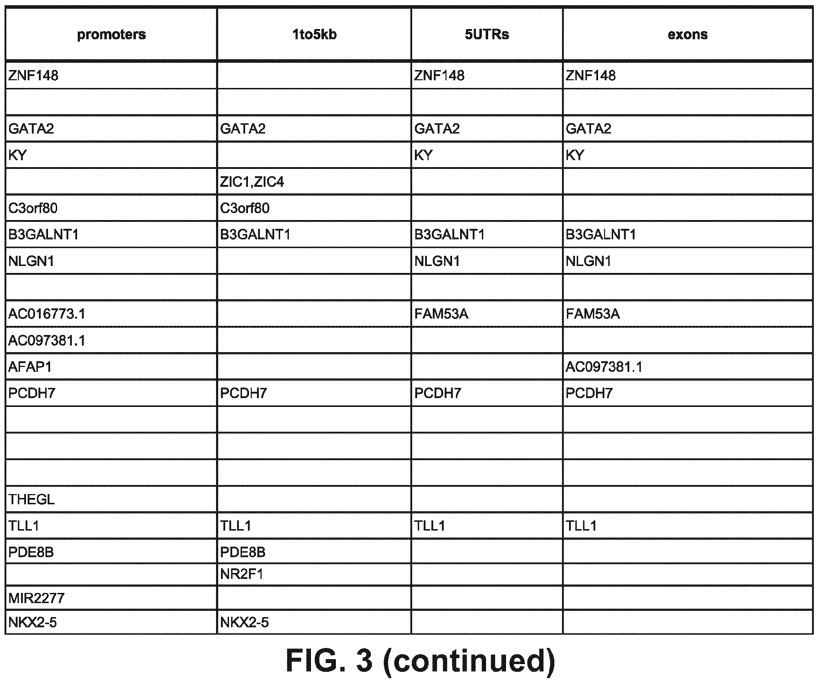

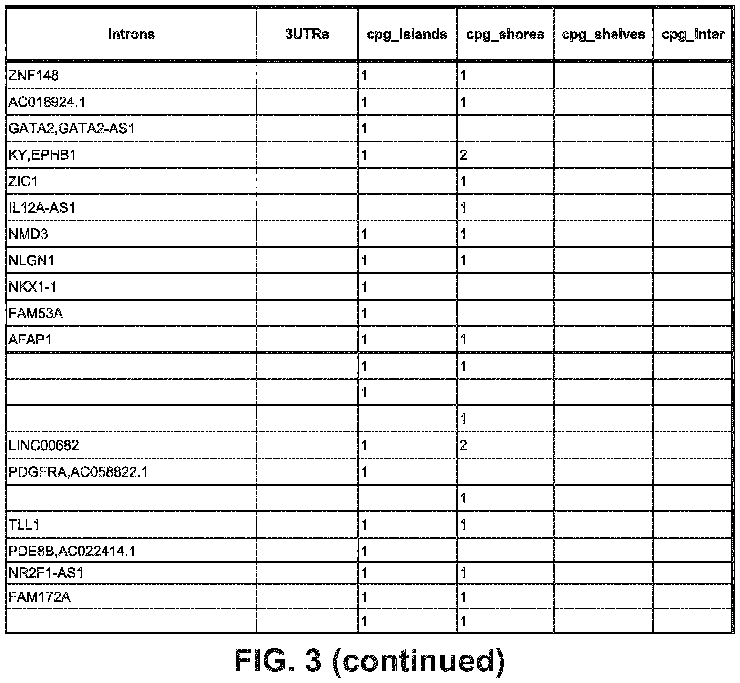

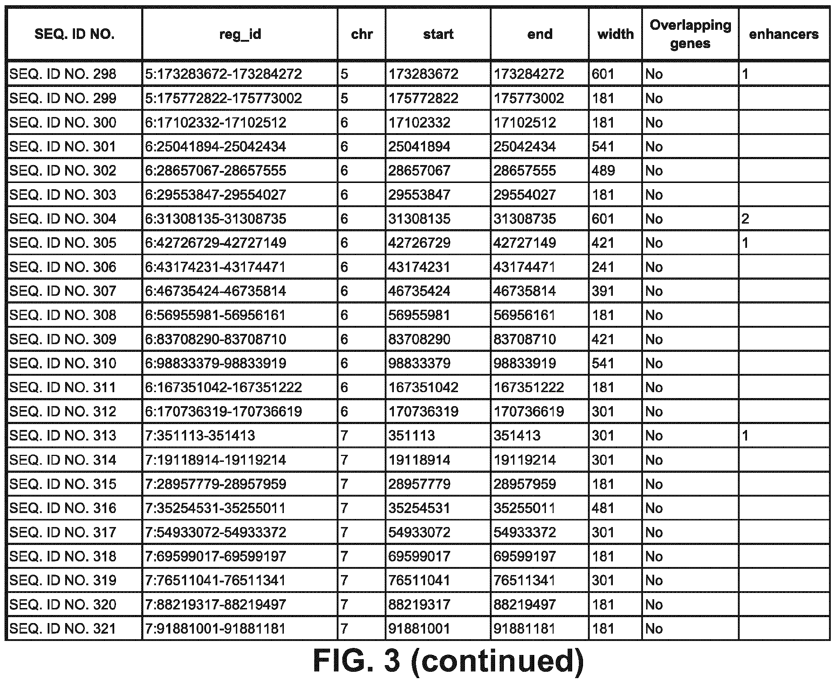

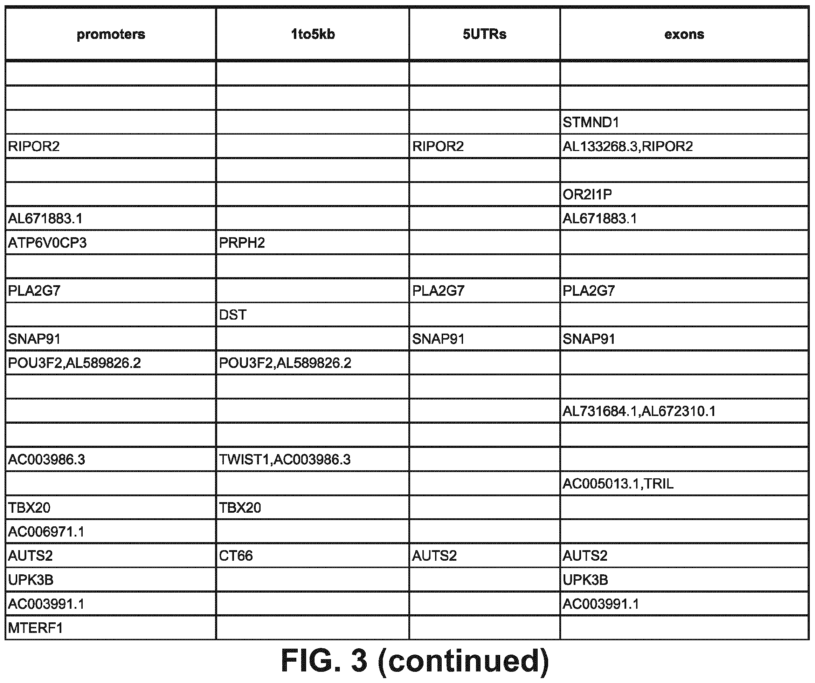

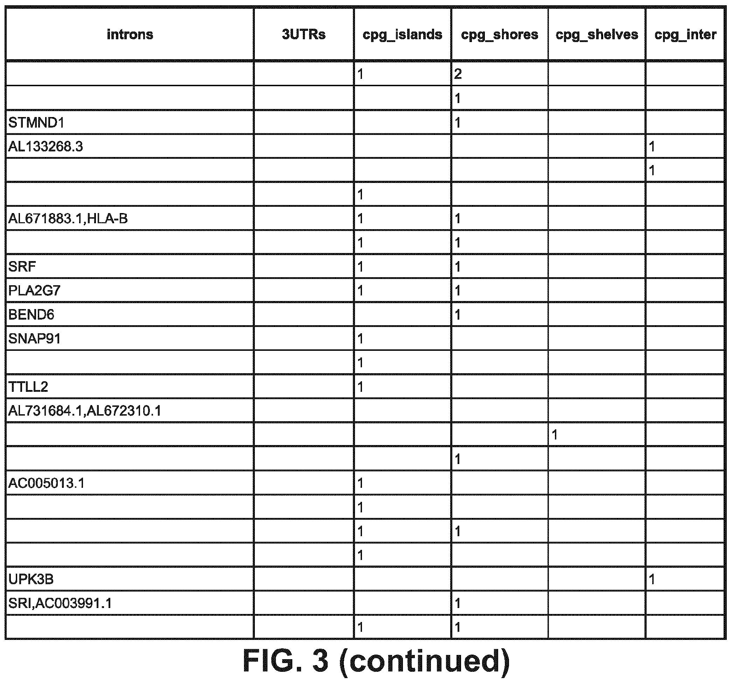

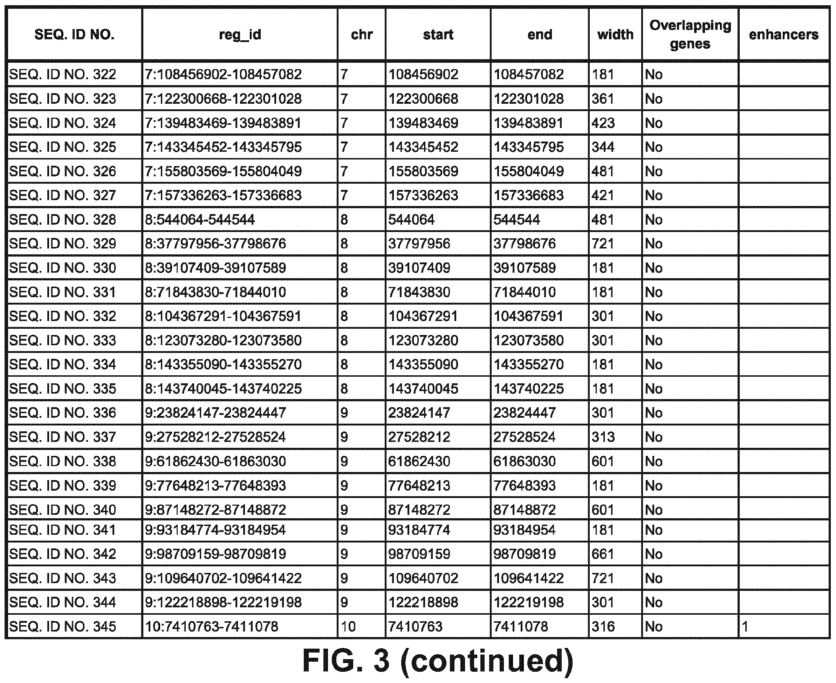

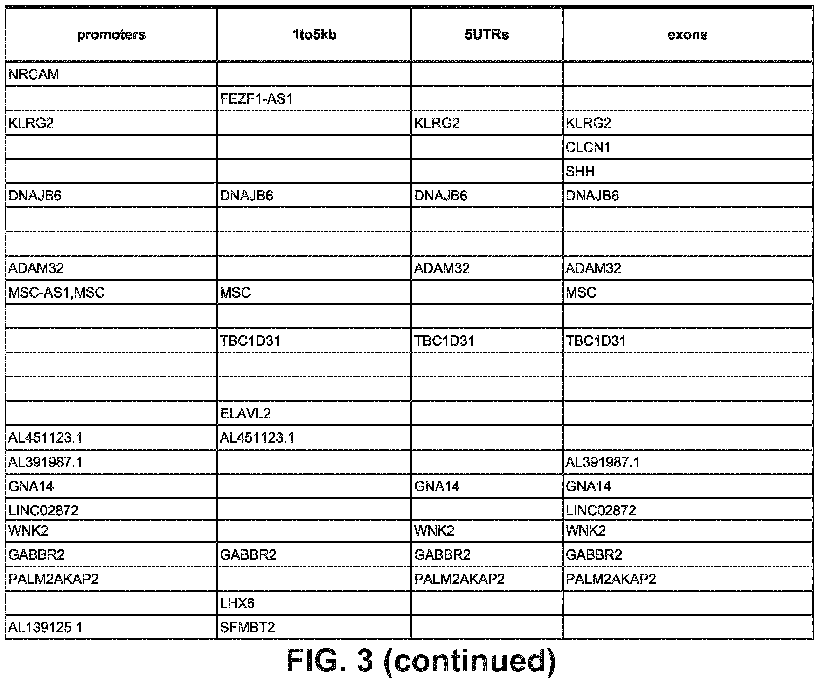

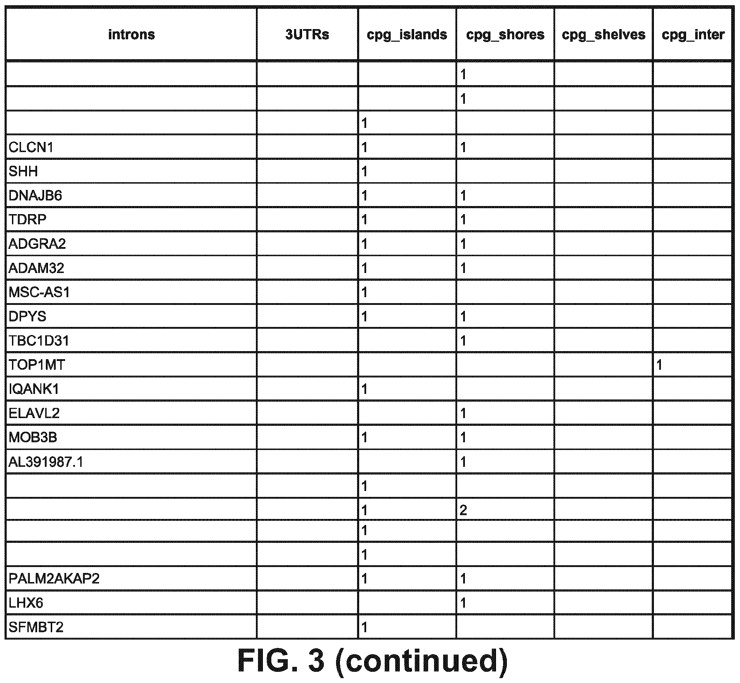

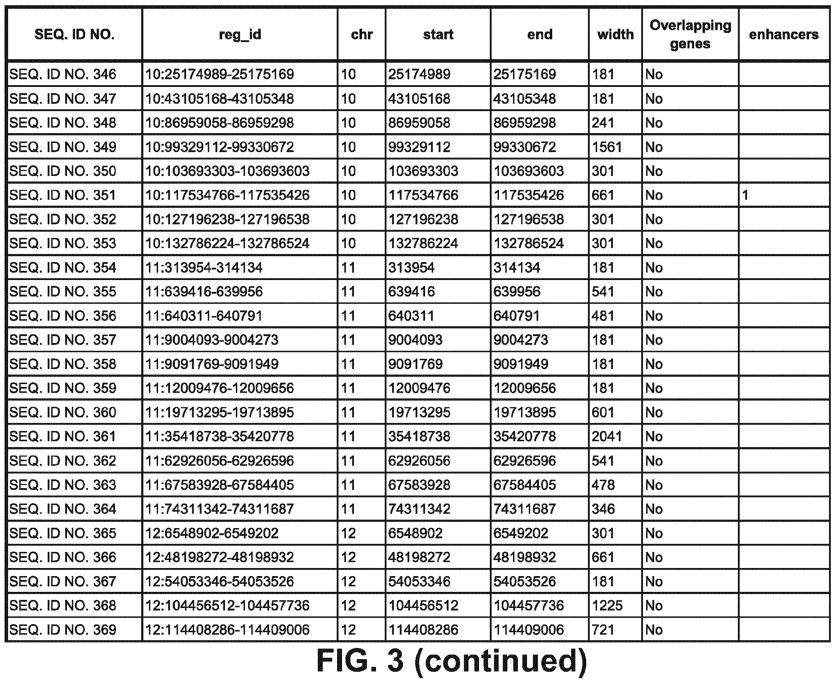

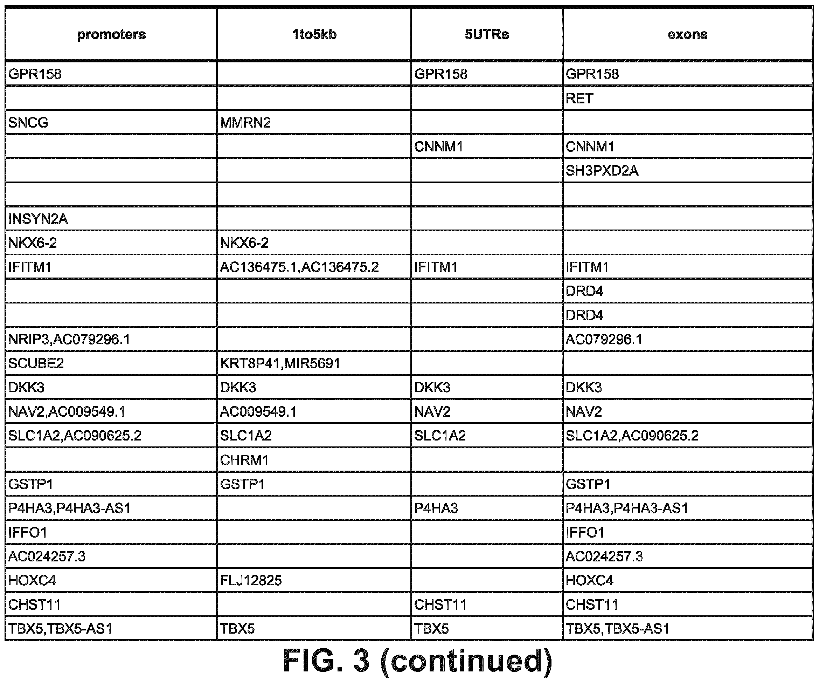

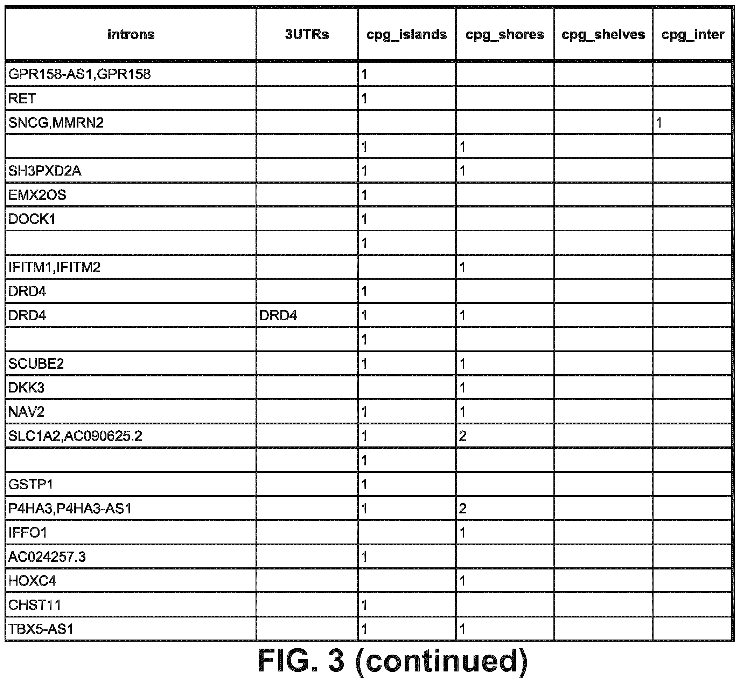

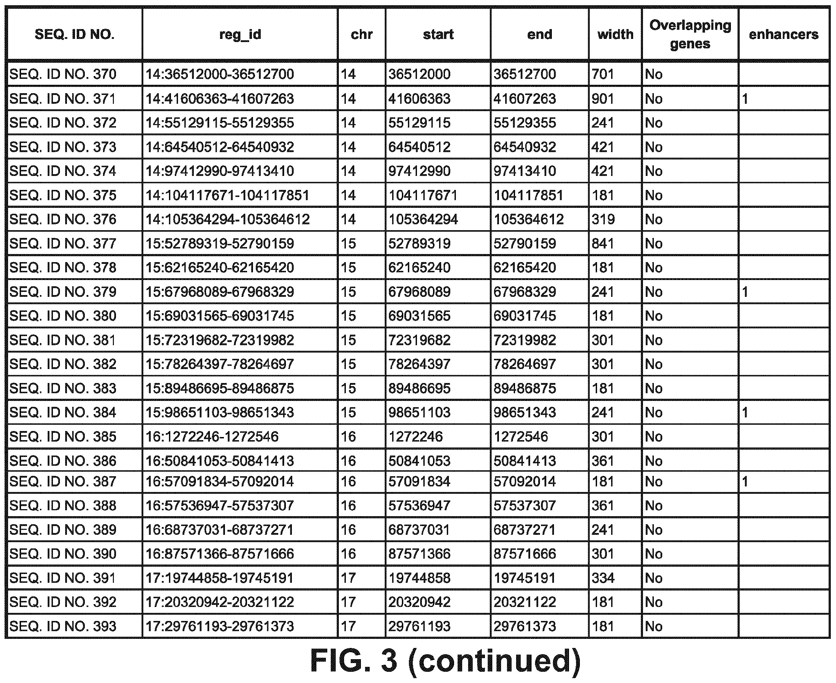

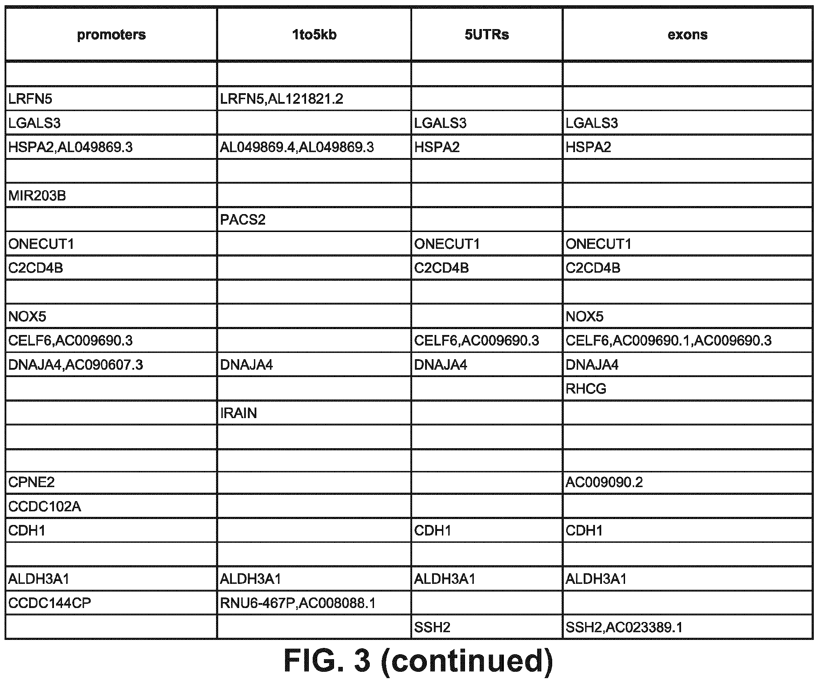

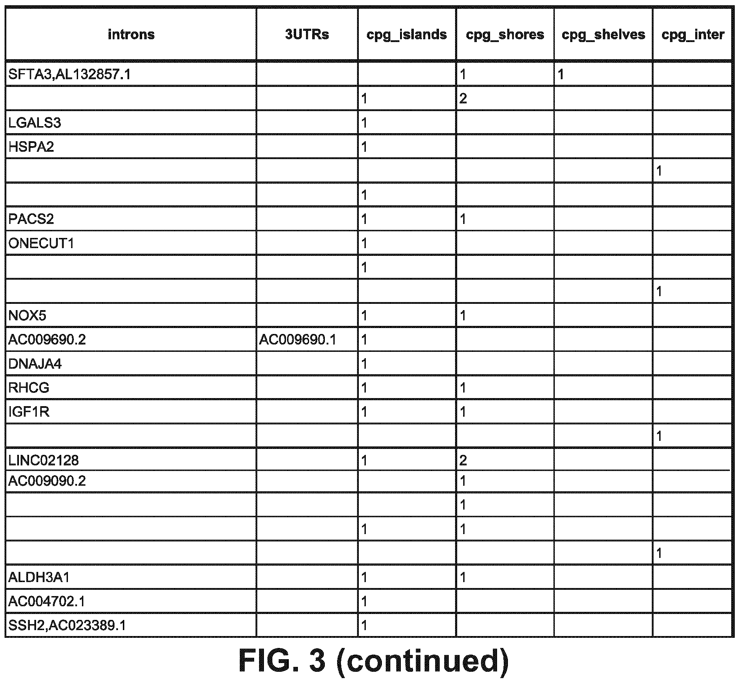

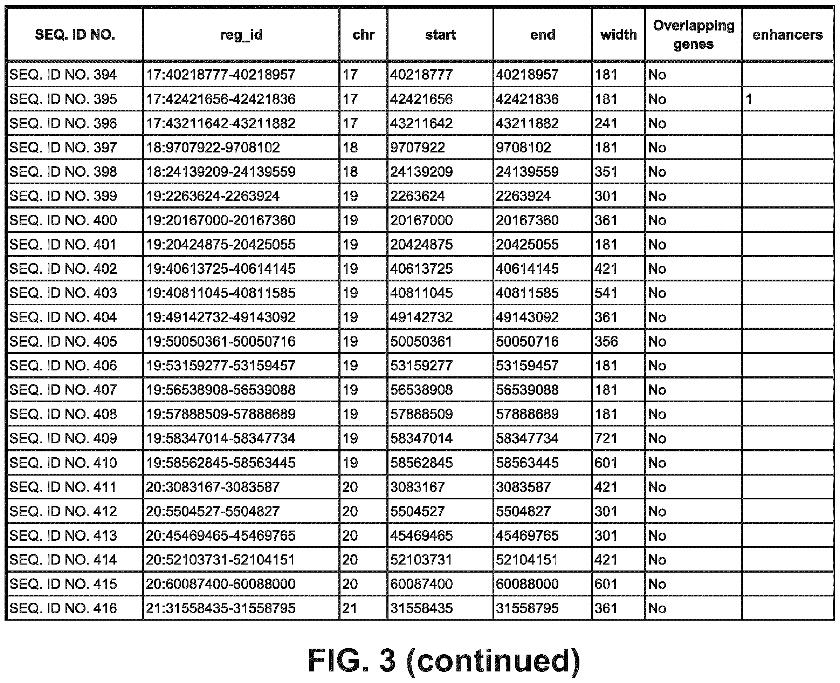

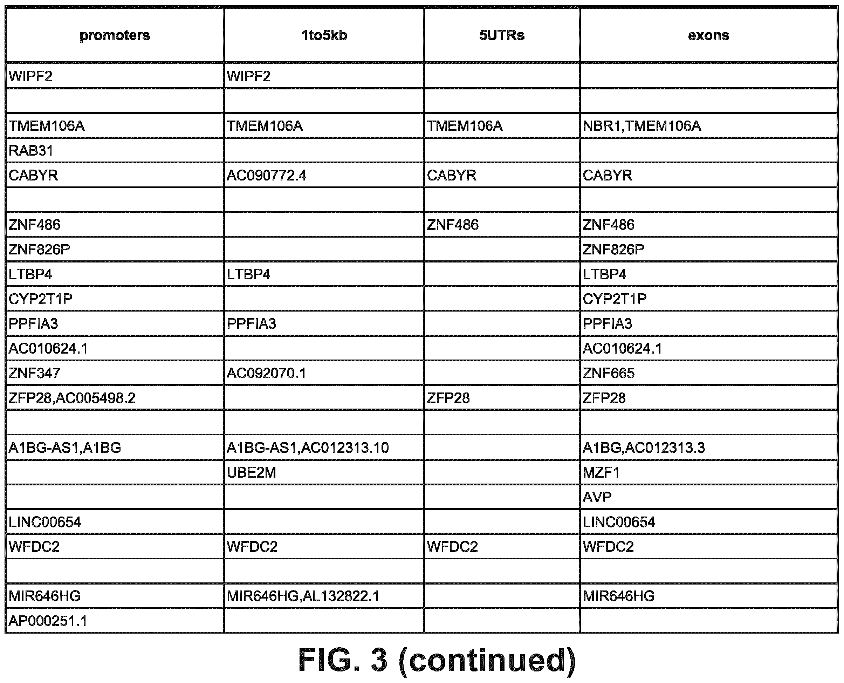

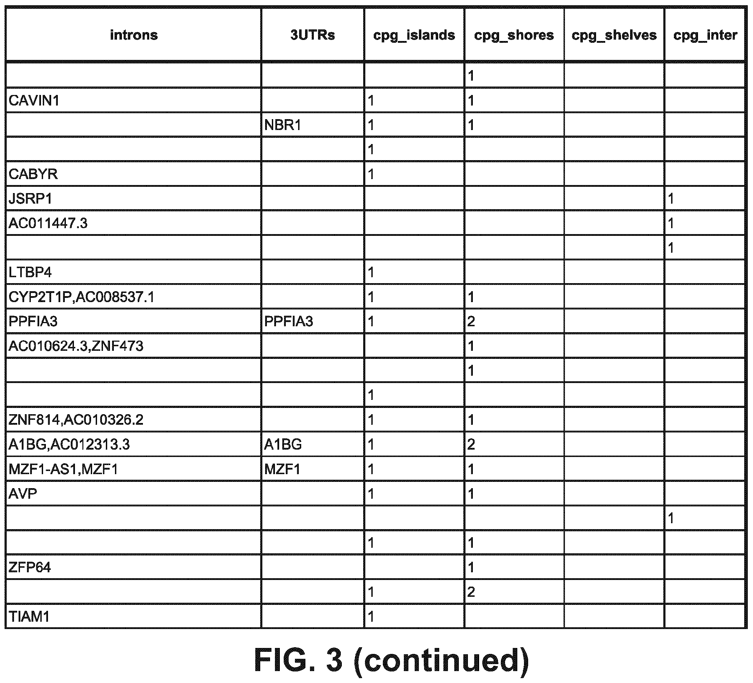

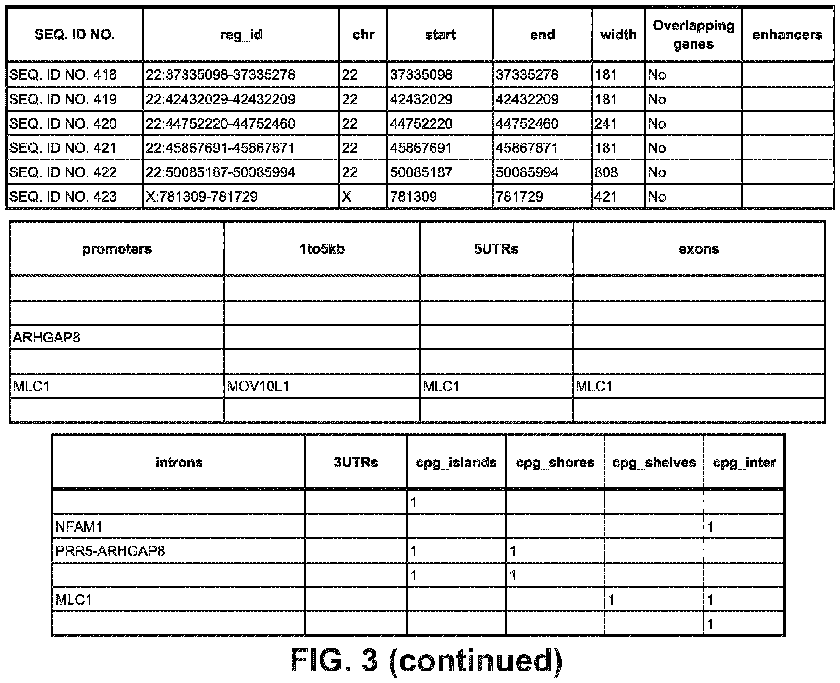

In certain embodiments, one of the one or more genomic regions is a methylation locus comprising at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) a differentially methylated region (DMR) selected from the DMRs of FIG. 2 or FIG. 3.

In certain embodiments, the one or more genomic regions comprise a methylation locus within gene DLX6-AS1; and a methylation locus within gene GDF6.

In certain embodiments, the one or more genomic regions comprise a methylation locus within gene ZAN. In certain embodiments, the one or more genomic regions further comprise a methylation locus within [chr14:97412990-97413410](SEQ ID NO: 374).

In certain embodiments, each of the one or more genomic regions are equal to or less than 5000bp, 4000 bp, 3000 bp, 2000 bp, 1000bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, or 10 bp in length.

In certain embodiments, at least one of the one or more genomic regions has a length of at least 5000bp, 4000 bp, 3000 bp, 2000 bp, 1000bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, or 10 bp.

In certain embodiments, the method is a method of detecting one or more biomarkers associated with cancer.

In certain embodiments, the method is a method of detecting one or more biomarkers associated with advanced adenoma.

In certain embodiments, the method is a method of detecting one or more biomarkers associated with colorectal cancer.

In certain embodiments, the method is a method of detecting one or more biomarkers associated with a disease, disorder or condition associated with aberrant levels of DNA methylation. In certain embodiments, the disease, disorder or condition is or comprises a gastrointestinal disorder or a neurodegenerative disorder.

In another aspect, the invention is directed to a method of detecting (e.g., screening for) colorectal cancer in a human subject, the method comprising: determining a methylation status of each of at least two or more markers identified in DNA from a sample obtained from the subject, and determining whether the subject has colorectal cancer based at least in part on the determined methylation status of each of the two or more markers, wherein each of the two or more markers is a methylation locus comprising at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) a differentially methylated region (DMR) selected from the DMRs of FIG. 2.

In certain embodiments, the method comprises determining a methylation status of at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) each of the following DMRs:

Table 8: 2-DMR Panel for CRC

| chr | start | end | SEQ ID NO. |
|-----|-------|-----|------------|
| 7 | 96997902 | 96999222 | SEQ ID NO.: 92 |
| 8 | 96145538 | 96145718 | SEQ ID NO.: 108 |

In certain embodiments, the method comprises determining a methylation status of at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) each of the following DMRs:

Table 9: 4-DMR Panel for CRC

| chr | start | end | SEQ ID NO. |
|-----|-------|-----|------------|
| 7 | 96997902 | 96999222 | SEQ ID NO.: 92 |
| 8 | 96145538 | 96145718 | SEQ ID NO.: 108 |
| 2 | 100322218 | 100322818 | SEQ ID NO.: 28 |
| 2 | 29115776 | 29116791 | SEQ ID NO.: 17 |

In certain embodiments, the method comprises determining a methylation status of at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) each of the following DMRs:

Table 10: 9-DMR panel for CRC

| chr | start | end | SEQ ID NO. |
|-----|-------|-----|------------|
| 7 | 96997902 | 96999222 | SEQ ID NO.: 92 |
| 8 | 96145538 | 96145718 | SEQ ID NO.: 108 |
| 2 | 100322218 | 100322818 | SEQ ID NO.: 28 |
| 2 | 29115776 | 29116791 | SEQ ID NO.: 17 |
| 2 | 88765502 | 88766042 | SEQ ID NO.: 25 |
| 4 | 153249541 | 153249721 | SEQ ID NO.: 55 |
| 2 | 86790271 | 86790811 | SEQ ID NO.: 24 |
| 2 | 176094518 | 176094878 | SEQ ID NO.: 35 |
| 3 | 37453325 | 37453874 | SEQ ID NO.: 41 |

In certain embodiments, the sample is a tissue sample (e.g., colorectal tissue, e.g., a polyp, an adenoma), a blood sample, a stool sample, or a blood product sample (e.g., a plasma sample)

In certain embodiments, the sample comprises DNA that is isolated from blood or plasma of the2 human subject.

In certain embodiments, the DNA is cell-free DNA (cfDNA) of the human subject.

In certain embodiments, the method comprises determining the methylation status of each of the one or more markers using next generation sequencing (NGS).

In certain embodiments, the method comprises using one or more capture baits that enrich for a target region to capture one or more corresponding methylation locus / loci. In certain embodiments, each methylation locus is equal to or less than 5000bp in length. In certain embodiments, the method further comprises for a subject determined by the method to have colorectal cancer, determining (e.g., simultaneously determining) a presence of a mutation (e.g., a single nucleotide variation) in one or more mutation markers identified in a sample obtained from the subject.

In certain embodiments, the one or more mutation markers comprise at least a portion of one or more of the following genes: NRAS, PTEN, KRAS, PIK3CA, EGFR, BRAF, STK11, TP53, KIT, and MET.

In certain embodiments, the method further comprises classifying the colorectal cancer based on the presence of one or more identified mutations in the mutation markers. In certain embodiments, the classifying comprises identifying that the colorectal cancer is treatable by a particular therapy (e.g., therapeutic agent, drug, etc.) based at least in part on the one or more identified mutations in the mutation markers.

In another aspect, the invention is directed to a method of detecting colorectal cancer in a human subject, the method comprising: determining a methylation status for both of the following in deoxyribonucleic acid (DNA) from a sample of a human subject: (i) a methylation locus within gene DLX6-AS1 ; and (ii) a methylation locus within gene GDF6; and diagnosing colorectal cancer in the human subject based on at least said determined methylation status.

In certain embodiments, the method comprises determining a methylation status for a methylation locus within gene DLX6-AS1, wherein the methylation locus within gene DLX6-AS1 comprises at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) [chr7: 96997902- 96999222] (SEQ ID NO.: 92).

In certain embodiments, the method comprises determining a methylation status for a methylation locus within gene GDF6, wherein the methylation locus within gene GDF6 comprises at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) [chr8: 96145538- 96145718](SEQ ID NO.: 108).

In certain embodiments, the method further comprises identifying the presence of one or more mutations (e.g., a single nucleotide variation) in one or more of the following genes: NRAS, PTEN, KRAS, PIK3CA, EGFR, BRAF, STK11, TP53, KIT, and MET.

In certain embodiments, the method further comprises classifying the colorectal cancer based at least in part on the identified mutation(s). In certain embodiments, the classifying comprises identifying that the colorectal cancer is treatable by a particular therapy (e.g., therapeutic agent, drug, etc.) based at least in part on the identified mutation(s).

In certain embodiments, the DNA is cell-free DNA of the human subject.

In certain embodiments, the method comprises determining the methylation status is determined using next generation sequencing (NGS).

In certain embodiments, the DNA is isolated from blood or plasma of the human subject. In certain embodiments, each methylation locus is equal to or less than 5000bp in length.

In certain embodiments, the methylation status is a read-wise methylation value.

In another aspect, the invention is directed to a method of detecting (e.g., screening for) advanced adenoma in a human subject, the method comprising: determining a methylation status of each of at least two or more markers identified in DNA from a sample obtained from the subject, and determining whether the subject has advanced adenoma based at least in part on the determined methylation status of each of the two or more markers, wherein each of the two or more markers is a methylation locus comprising at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) a differentially methylated region (DMR) selected from the DMRs of FIG. 3 (e.g., the 220 marker table).

In certain embodiments, the method comprises determining a methylation status of at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) each of the following DMRs:

Table 15: 2-DMR Panel for AA

| chr | start | end | SEQ ID NO. |
|---|---|---|---|
| 7 | 100785927 | 100786167 | SEQ ID NO.: 221 |
| 14 | 97412990 | 97413410 | SEQ ID NO.: 374 |

In certain embodiments, the method comprises determining a methylation status of at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) each of the following DMRs:

Table 16:4-DMR panel for AA

| chr | start | end | SEQ ID NO. |
|---|---|---|---|
| 7 | 100785927 | 100786167 | SEQ ID NO.: 221 |
| 14 | 97412990 | 97413410 | SEQ ID NO.: 374 |
| 20 | 3083167 | 3083587 | SEQ ID NO.: 411 |
| 8 | 37797956 | 37798676 | SEQ ID NO.: 329 |

In certain embodiments, the method comprises determining a methylation status of at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) each of the following DMRs:

Table 17: 10-DMR panel for AA

| chr | start | end | SEQ ID NO. |
|---|---|---|---|
| 7 | 100785927 | 100786167 | SEQ ID NO.: 221 |
| 14 | 97412990 | 97413410 | SEQ ID NO.: 374 |
| 20 | 3083167 | 3083587 | SEQ ID NO.: 411 |
| 8 | 37797956 | 37798676 | SEQ ID NO.: 329 |
| 16 | 57091834 | 57092014 | SEQ ID NO.: 387 |
| 4 | 7940020 | 7940200 | SEQ ID NO.: 287 |
| 19 | 40811045 | 40811585 | SEQ ID NO.: 403 |
| 1 | 154567391 | 154567691 | SEQ ID NO.: 246 |
| 14 | 105364294 | 105364612 | SEQ ID NO.: 376 |
| 9 | 61862430 | 61863030 | SEQ ID NO.: 338 |

In certain embodiments, the sample is a tissue sample (e.g., colorectal tissue, e.g., a polyp, an adenoma), a blood sample, a stool sample, or a blood product sample (e.g., a plasma sample).

In certain embodiments, the sample comprises DNA that is isolated from blood or plasma of the human subject.

In certain embodiments, the DNA is cell-free DNA (cfDNA) of the human subject.

In certain embodiments, the method comprises determining the methylation status of each of the one or more markers using next generation sequencing (NGS).

In certain embodiments, the method comprises using one or more capture baits that enrich for a target region to capture one or more corresponding methylation locus / loci. In certain embodiments, each methylation locus is equal to or less than 5000bp in length. In certain embodiments, the method further comprises for a subject determined by the method to have advanced adenoma, determining (e.g., simultaneously determining) a presence of a mutation (e.g., a single nucleotide variation) in one or more mutation markers identified in a sample obtained from the subject.

In certain embodiments, the one or more mutation markers comprise at least a portion of one or more of the following genes: NRAS, PTEN, KRAS, PIK3CA, EGFR, BRAF, STK11, TP53, KIT, and MET.

In certain embodiments, the method further comprises classifying the advanced adenoma based on the presence of one or more identified mutations in the mutation markers. In certain embodiments, the classifying comprises identifying that the advanced adenoma is treatable by a particular therapy (e.g., therapeutic agent, drug, etc.) based at least in part on the one or more identified mutations in the mutation markers.

In another aspect, the invention is directed to a method of detecting advanced adenoma in a human subject, the method comprising: determining a methylation status for both of the following in deoxyribonucleic acid (DNA) from a sample of a human subject: (i) a methylation locus within gene ZAN; and (ii) a methylation locus comprising at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) [chr14:97412990-97413410]( SEQ ID NO.: 374); and diagnosing advanced adenoma in the human subject based on at least said determined methylation status.

In certain embodiments, the method comprises determining a methylation status for a methylation locus within gene ZAN, wherein the methylation locus within gene ZAN comprises at least a portion of (e.g., at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, or at least 95% of) [chr7:100785927-100786167]( SEQ ID NO.: 221).

In certain embodiments, the method further comprises identifying the presence of one or more mutations (e.g., a single nucleotide variation) in one or more of the following genes: NRAS, PTEN, KRAS, PIK3CA, EGFR, BRAF, STK11, TP53, KIT, and MET.

In certain embodiments, the method further comprises classifying the advanced adenoma based at least in part on the identified mutation(s). In certain embodiments, the classifying comprises identifying that the advanced adenoma is treatable by a particular therapy (e.g., therapeutic agent, drug, etc.) based at least in part on the identified mutation(s).

In certain embodiments, the DNA is cell-free DNA of the human subject.

In certain embodiments, the method comprises determining the methylation status is determined using next generation sequencing (NGS).

In certain embodiments, the DNA is isolated from blood or plasma of the human subject. In certain embodiments, each methylation locus is equal to or less than 5000bp in length. In certain embodiments, the methylation status is a read-wise methylation value.

In another aspect, the invention is directed to a system comprising: a processor of a computing device; and a memory having instructions stored thereon, wherein the instructions, when executed by the processor, cause the processor to perform one or more steps of any of the methods described herein.

In various aspects, methods and compositions of the present invention can be used in combination with biomarkers known in the art, e.g., as disclosed in U.S. Patent No. 10,006,925 and U.S. Patent No. 63, 011970.

In other aspects, the invention is directed to a system for performing any of the methods referred to in the preceding paragraphs, the system comprising a processor; and a memory having instructions thereon, the instructions, when executed by the processor, causing the processor to perform one or more (up to all) steps of the method.

DEFINITIONS

*A or An:* The articles "a" and "an" are used herein to refer to one or to more than one (*i.e.,* to at least one) of the grammatical object of the article. By way of example, "an element" refers to one element or more than one element.

*About:* The term "about", when used herein in reference to a value, refers to a value that is similar, in context, to the referenced value. In general, those skilled in the art, familiar with the context, will appreciate the relevant degree of variance encompassed by "about" in that context. For example, in some embodiments, e.g., as set forth herein, the term "about" can encompass a range of values that within 25%, 20%, 19%, 18%, 17%, 16%, 15%, 14%, 13%, 12%, 11%, 10%, 9%, 8%, 7%, 6%, 5%, 4%, 3%, 2%, 1%, or with a fraction of a percent, of the referred value.

*Advanced Adenoma:* As used herein, the term "advanced adenoma" typically refers to refer to cells that exhibit first indications of relatively abnormal, uncontrolled, and/or autonomous growth but are not yet classified as cancerous alterations. In the context of colon tissue, "advanced adenoma" refers to neoplastic growth that shows signs of high grade dysplasia, and/or size that is >=10mm, and/or villious histological type, and/or serrated histological type with any type of dysplasia.

*Administration:* As used herein, the term "administration" typically refers to the administration of a composition to a subject or system, for example to achieve delivery of an agent that is, is included in, or is otherwise delivered by, the composition.

*Agent:* As used herein, the term "agent" refers to an entity (e.g., for example, a small molecule, peptide, polypeptide, nucleic acid, lipid, polysaccharide, complex, combination, mixture, system, or phenomenon such as heat, electric current, electric field, magnetic force, magnetic field, etc.).

*Amelioration:* As used herein, the term "amelioration" refers to the prevention, reduction, palliation, or improvement of a state of a subject. Amelioration includes, but does not require, complete recovery or complete prevention of a disease, disorder or condition. *Amplicon or amplicon molecule:* As used herein, the term "amplicon" or "amplicon molecule" refers to a nucleic acid molecule generated by transcription from a template nucleic acid molecule, or a nucleic acid molecule having a sequence complementary thereto, or a double-stranded nucleic acid including any such nucleic acid molecule. Transcription can be initiated from a primer.

*Amplification:* As used herein, the term "amplification" refers to the use of a template nucleic acid molecule in combination with various reagents to generate further nucleic acid molecules from the template nucleic acid molecule, which further nucleic acid molecules may be identical to or similar to (e.g., at least 70% identical, e.g., at least 75%, 80%, 85%, 90%, 91%, 92%, 93%, 94%, 95%, 96%, 97%, 98%, 99%, or 100% identical to) a segment of the template nucleic acid molecule and/or a sequence complementary thereto.

*Amplification reaction mixture:* As used herein, the terms "amplification reaction mixture" or "amplification reaction" refer to a template nucleic acid molecule together with reagents sufficient for amplification of the template nucleic acid molecule.

*Biological Sample:* As used herein, the term "biological sample" typically refers to a sample obtained or derived from a biological source (e.g., a tissue or organism or cell culture) of interest, as described herein. In some embodiments, e.g., as set forth herein, a biological source is or includes an organism, such as an animal or human. In some embodiments, e.g., as set forth herein, a biological sample is or include biological tissue or fluid. In some embodiments, e.g., as set forth herein, a biological sample can be or include cells, tissue, or bodily fluid. In some embodiments, e.g., as set forth herein, a biological sample can be or include blood, blood cells, cell-free DNA, free floating nucleic acids, ascites, biopsy samples, surgical specimens, cell-containing body fluids, sputum, saliva, feces, urine, cerebrospinal fluid, peritoneal fluid, pleural fluid, lymph, gynecological fluids, secretions, excretions, skin swabs, vaginal swabs, oral swabs, nasal swabs, washings or lavages such as a ductal lavages or broncheoalveolar lavages, aspirates, scrapings, bone marrow. In some embodiments, e.g., as set forth herein, a biological sample is or includes cells obtained from a single subject or from a plurality of subjects. A sample can be a "primary sample" obtained directly from a biological source, or can be a "processed sample." A biological sample can also be referred to as a "sample."

*Biomarker:* As used herein, the term "biomarker," consistent with its use in the art, refers to a to an entity whose presence, level, or form, correlates with a particular biological event or state of interest, so that it is considered to be a "marker" of that event or state. Those of skill in the art will appreciate, for instance, in the context of a DNA biomarker, that a biomarker can be or include a locus (such as one or more methylation loci) and/or the status of a locus (e.g., the status of one or more methylation loci). To give but a few examples of biomarkers, in some embodiments, e.g., as set forth herein, a biomarker can be or include a marker for a particular disease, disorder or condition, or can be a marker for qualitative of quantitative probability that a particular disease, disorder or condition can develop, occur, or reoccur, e.g., in a subject. In some embodiments, e.g., as set forth herein, a biomarker can be or include a marker for a particular therapeutic outcome, or qualitative of quantitative probability thereof. Thus, in various embodiments, e.g., as set forth herein, a biomarker can be predictive, prognostic, and/or diagnostic, of the relevant biological event or state of interest. A biomarker can be an entity of any chemical class. For example, in some embodiments, e.g., as set forth herein, a biomarker can be or include a nucleic acid, a polypeptide, a lipid, a carbohydrate, a small molecule, an inorganic agent (e.g., a metal or ion), or a combination thereof. In some embodiments, e.g., as set forth herein, a biomarker is a cell surface marker. In some embodiments, e.g., as set forth herein, a biomarker is intracellular. In some embodiments, e.g., as set forth herein, a biomarker is found outside of cells (e.g., is secreted or is otherwise generated or present outside of cells, e.g., in a body fluid such as blood, urine, tears, saliva, cerebrospinal fluid, and the like). In some embodiments, e.g., as set forth herein, a biomarker is methylation status of a methylation locus. In some instances, e.g., as set forth herein, a biomarker may be referred to as a "marker." To give but one example of a biomarker, in some embodiments e.g., as set forth herein, the term refers to expression of a product encoded by a gene, expression of which is characteristic of a particular tumor, tumor subclass, stage of tumor, etc. Alternatively or additionally, in some embodiments, e.g., as set forth herein, presence or level of a particular marker can correlate with activity (or activity level) of a particular signaling pathway, for example, of a signaling pathway the activity of which is characteristic of a particular class of tumors.

Those of skill in the art will appreciate that a biomarker may be individually determinative of a particular biological event or state of interest, or may represent or contribute to a determination of the statistical probability of a particular biological event or state of interest. Those of skill in the art will appreciate that markers may differ in their specificity and/or sensitivity as related to a particular biological event or state of interest.

*Blood component:* As used herein, the term "blood component" refers to any component of whole blood, including red blood cells, white blood cells, plasma, platelets, endothelial cells, mesothelial cells, epithelial cells, and cell-free DNA. Blood components also include the components of plasma, including proteins, metabolites, lipids, nucleic acids, and carbohydrates, and any other cells that can be present in blood, e.g., due to pregnancy, organ transplant, infection, injury, or disease.

*Cancer:* As used herein, the terms "cancer," "malignancy," "neoplasm," "tumor," and "carcinoma," are used interchangeably to refer to a disease, disorder, or condition in which cells exhibit or exhibited relatively abnormal, uncontrolled, and/or autonomous growth, so that they display or displayed an abnormally elevated proliferation rate and/or aberrant growth phenotype. In some embodiments, e.g., as set forth herein, a cancer can include one or more tumors. In some embodiments e.g., as set forth herein, a cancer can be or include cells that are precancerous (e.g., benign), malignant, pre-metastatic, metastatic, and/or non-metastatic. In some embodiments e.g., as set forth herein, a cancer can be or include a solid tumor. In some embodiments e.g., as set forth herein, a cancer can be or include a hematologic tumor. In general, examples of different types of cancers known in the art include, for example, colorectal cancer, hematopoietic cancers including leukemias, lymphomas (Hodgkin's and non-Hodgkin's), myelomas and myeloproliferative disorders; sarcomas, melanomas, adenomas, carcinomas of solid tissue, squamous cell carcinomas of the mouth, throat, larynx, and lung, liver cancer, genitourinary cancers such as prostate, cervical, bladder, uterine, and endometrial cancer and renal cell carcinomas, bone cancer, pancreatic cancer, skin cancer, cutaneous or intraocular melanoma, cancer of the endocrine system, cancer of the thyroid gland, cancer of the parathyroid gland, head and neck cancers, breast cancer, gastro-intestinal cancers and nervous system cancers, benign lesions such as papillomas, and the like.

*Chemotherapeutic agent:* As used herein, the term "chemotherapeutic agent," consistent with its use in the art, refers to one or more agents known, or having characteristics known to, treat or contribute to the treatment of cancer. In particular, chemotherapeutic agents include pro-apoptotic, cytostatic, and/or cytotoxic agents. In some embodiments e.g., as set forth herein, a chemotherapeutic agent can be or include alkylating agents, anthracyclines, cytoskeletal disruptors (e.g., microtubule targeting moieties such as taxanes, maytansine, and analogs thereof, of), epothilones, histone deacetylase inhibitors HDACs), topoisomerase inhibitors (e.g., inhibitors of topoisomerase I and/or topoisomerase II), kinase inhibitors, nucleotide analogs or nucleotide precursor analogs, peptide antibiotics, platinum-based agents, retinoids, vinca alkaloids, and/or analogs that share a relevant anti-proliferative activity. In some particular embodiments e.g., as set forth herein, a chemotherapeutic agent can be or include of Actinomycin, All-trans retinoic acid, an Auiristatin, Azacitidine, Azathioprine, Bleomycin, Bortezomib, Carboplatin, Capecitabine, Cisplatin, Chlorambucil, Cyclophosphamide, Curcumin, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Irinotecan, Maytansine and/or analogs thereof (e.g., DM1) Mechlorethamine, Mercaptopurine, Methotrexate, Mitoxantrone, a Maytansinoid, Oxaliplatin, Paclitaxel, Pemetrexed, Teniposide, Tioguanine, Topotecan, Valrubicin, Vinblastine, Vincristine, Vindesine, Vinorelbine, or a combination thereof. In some embodiments e.g., as set forth herein, a chemotherapeutic agent can be utilized in the context of an antibody-drug conjugate. In some embodiments e.g., as set forth herein, a chemotherapeutic agent is one found in an antibody-drug conjugate selected from the group consisting of: hLL1-doxorubicin, hRS7-SN-38, hMN-14-SN-38, hLL2-SN-38, hA20-SN-38, hPAM4-SN-38, hLL1-SN-38, hRS7-Pro-2-P-Dox, hMN-14-Pro-2-P-Dox, hLL2-Pro-2-P-Dox, hA20-Pro-2-P-Dox, hPAM4-Pro-2-P-Dox, hLL1-Pro-2-P-Dox, P4/D10-doxorubicin, gemtuzumab ozogamicin, brentuximab vedotin, trastuzumab emtansine, inotuzumab ozogamicin, glembatumomab vedotin, SAR3419, SAR566658, BIIB015, BT062, SGN-75, SGN-CD19A, AMG-172, AMG-595, BAY-94-9343, ASG-5ME, ASG-22ME, ASG-16M8F, MDX-1203, MLN-0264, anti-PSMA ADC, RG-7450, RG-7458, RG-7593, RG-7596, RG-7598, RG-7599, RG-7600, RG-7636, ABT-414, IMGN-853, IMGN-529, vorsetuzumab mafodotin, and lorvotuzumab mertansine. In some embodiments e.g., as set forth herein, a chemotherapeutic agent can be or comprise of farnesyl-thiosalicylic acid (FTS), 4-(4-Chloro-2-methylphenoxy)-N-hydroxybutanamide (CMH), estradiol (E2), tetramethoxystilbene (TMS), $\delta$-tocatrienol, salinomycin, or curcumin.

*Combination therapy:* As used herein, the term "combination therapy" refers to administration to a subject of to two or more agents or regimens such that the two or more agents or regimens together treat a disease, condition, or disorder of the subject. In some embodiments, e.g., as set forth herein, the two or more therapeutic agents or regimens can be administered simultaneously, sequentially, or in overlapping dosing regimens. Those of skill in the art will appreciate that combination therapy includes but does not require that the two agents or regimens be administered together in a single composition, nor at the same time.

*Comparable:* As used herein, the term "comparable" refers to members within sets of two or more conditions, circumstances, agents, entities, populations, etc., that may not be identical to one another but that are sufficiently similar to permit comparison there between, such that one of skill in the art will appreciate that conclusions can reasonably be drawn based on differences or similarities observed. In some embodiments, e.g., as sort forth herein, comparable sets of conditions, circumstances, agents, entities, populations, etc. are typically characterized by a plurality of substantially identical features and zero, one, or a plurality of differing features. Those of ordinary skill in the art will understand, in context, what degree of identity is required to render members of a set comparable. For example, those of ordinary skill in the art will appreciate that members of sets of conditions, circumstances, agents, entities, populations, etc., are comparable to one another when characterized by a sufficient number and type of substantially identical features to warrant a reasonable conclusion that differences observed can be attributed in whole or part to non-identical features thereof.

*Corresponding to:* As used herein, the term "corresponding to" refers to a relationship between two or more entities. For example, the term "corresponding to" may be used to designate the position/identity of a structural element in a compound or composition relative to another compound or composition (e.g., to an appropriate reference compound or composition). For example, in some embodiments, a monomeric residue in a polymer (e.g., a nucleic acid residue in a polynucleotide) may be identified as "corresponding to" a residue in an appropriate reference polymer. Those of ordinary skill in the art readily appreciate how to identify *"corresponding"* nucleic acids. For example, those skilled in the art will be aware of various sequence alignment strategies, including software programs such as, for example, BLAST, CS-BLAST, CUSASW++, DIAMOND, FASTA, GGSEARCH/GLSEARCH, Genoogle, HMMER, HHpred/HHsearch, IDF, Infernal, KLAST, USEARCH, parasail, PSI-BLAST, PSI-Search, ScalaBLAST, Sequilab, SAM, SSEARCH, SWAPHI, SWAPHI-LS, SWIMM, or SWIPE that can be utilized, for example, to identify "corresponding" residues in nucleic acids in accordance with the present disclosure. Those of skill in the art will also appreciate that, in some instances, the term "corresponding to" may be used to describe an event or entity that shares a relevant similarity with another event or entity (e.g., an appropriate reference event or entity). To give but one example, a fragment of DNA in a sample from a subject may be described as "corresponding to" a gene in order to indicate, in some embodiments, that it shows a particular degree of sequence identity or homology, or shares a particular characteristic sequence element.

*Detectable moiety:* The term "detectable moiety" as used herein refers to any element, molecule, functional group, compound, fragment, or other moiety that is detectable. In some embodiments, e.g., as sort forth herein, a detectable moiety is provided or utilized alone. In some embodiments, e.g., as sort forth herein, a detectable moiety is provided and/or utilized in association with (e.g., joined to) another agent. Examples of detectable moieties include, but are not limited to, various ligands, radionuclides (e.g., $^{3}$H, $^{14}$C, $^{18}$F, $^{19}$F, $^{32}$P, $^{35}$S, $^{135}$I, $^{125}$I, $^{123}$I, $^{64}$Cu, $^{187}$Re, $^{111}$In, $^{90}$Y, $^{99m}$Tc, $^{177}$Lu, $^{89}$Zr etc.), fluorescent dyes, chemiluminescent agents, bioluminescent agents, spectrally resolvable inorganic fluorescent semiconductors nanocrystals (i.e., quantum dots), metal nanoparticles, nanoclusters, paramagnetic metal ions, enzymes, colorimetric labels, biotin, dioxigenin, haptens, and proteins for which antisera or monoclonal antibodies are available.

*Diagnosis:* As used herein, the term "Diagnosis" refers to determining whether, and/or the qualitative of quantitative probability that, a subject has or will develop a disease, disorder, condition, or state. For example, in diagnosis of cancer, diagnosis can include a determination regarding the risk, type, stage, malignancy, or other classification of a cancer. In some instances, e.g., as sort forth herein, a diagnosis can be or include a determination relating to prognosis and/or likely response to one or more general or particular therapeutic agents or regimens.

*Diagnostic information:* As used herein, the term "diagnostic information" refers to information useful in providing a diagnosis. Diagnostic information can include, without limitation, biomarker status information.

*Differentially methylated*: As used herein, the term "differentially methylated" describes a methylation site for which the methylation status differs between a first condition and a second condition. A methylation site that is differentially methylated can be referred to as a differentially methylated site. In some instances, e.g., as sort forth herein, a DMR is defined by the amplicon produced by amplification using oligonucleotide primers, e.g., a pair of oligonucleotide primers selected for amplification of the DMR or for amplification of a DNA region of interest present in the amplicon. In some instances, e.g., as sort forth herein, a DMR is defined as a DNA region amplified by a pair of oligonucleotide primers, including the region having the sequence of, or a sequence complementary to, the oligonucleotide primers. In some instances, e.g., as sort forth herein, a DMR is defined as a DNA region amplified by a pair of oligonucleotide primers, excluding the region having the sequence of, or a sequence complementary to, the oligonucleotide primers. As used herein, a specifically provided DMR can be unambiguously identified by the name of an associated gene followed by three digits of a starting position, such that, for example, a DMR starting at position 100785927 of ZAN can be identified as ZAN '927. As used herein, a specifically provided DMR can be unambiguously identified by the chromosome number followed by the starting and ending positions of a DMR.

*Differentially methylated region:* As used herein, the term "differentially methylated region" (DMR) refers to a DNA region that includes one or more differentially methylated sites. A DMR that includes a greater number or frequency of methylated sites under a selected condition of interest, such as a cancerous state, can be referred to as a hypermethylated DMR. A DMR that includes a smaller number or frequency of methylated sites under a selected condition of interest, such as a cancerous state, can be referred to as a hypomethylated DMR. A DMR that is a methylation biomarker for colorectal cancer can be referred to as a colorectal cancer DMR. A DMR that is a methylation biomarker for advanced adenoma can be referred to as an advanced adenoma DMR. In some instances, e.g., as set forth herein, a DMR can be a single nucleotide, which single nucleotide is a methylation site. In some instances, e.g., as set forth herein, a DMR has a length of at least 10, at least 15, at least 20, at least 30, at least 50, or at least 75 base pairs. In some instances, e.g., as set forth herein, a DMR has a length of equal to or less than 5000 bp, 4,000 bp, 3,000 bp, 2,000 bp, 1,000 bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, or 10 bp (e.g., where methylation status is determined using quantitative polymerase chain reaction (qPCR), e.g., methylation sensitive restriction enzyme quantitative polymerase chain reaction (MSRE-qPCR)) (e.g., where methylation status is determined using a next generation sequencing technique, e.g., targeted next generation sequencing). In some instances, e.g., as set forth herein, a DMR that is a methylation biomarker for advanced adenoma may also be useful in identification of colorectal cancer and vice versa.

*DNA region:* As used herein, "DNA region" refers to any contiguous portion of a larger DNA molecule. Those of skill in the art will be familiar with techniques for determining whether a first DNA region and a second DNA region correspond, based, e.g., on sequence similarity (e.g, sequence identity or homology) of the first and second DNA regions and/or context (e.g., the sequence identity or homology of nucleic acids upstream and/or downstream of the first and second DNA regions).

Except as otherwise specified herein, sequences found in or relating to humans (e.g., that hybridize to human DNA) are found in, based on, and/or derived from the example representative human genome sequence commonly referred to, and known to those of skill in the art, as Homo sapiens (human) genome assembly GRCh38, hg38, and/or Genome Reference Consortium Human Build 38. Those of skill in the art will further appreciate that DNA regions of hg38 can be referred to by a known system including identification of particular nucleotide positions or ranges thereof in accordance with assigned numbering.

*Dosing regimen:* As used herein, the term "dosing regimen" can refer to a set of one or more same or different unit doses administered to a subject, typically including a plurality of unit doses administration of each of which is separated from administration of the others by a period of time. In various embodiments, e.g., as set forth herein, one or more or all unit doses of a dosing regimen may be the same or can vary (e.g., increase over time, decrease over time, or be adjusted in accordance with the subject and/or with a medical practitioner's determination). In various embodiments, e.g., as set forth herein, one or more or all of the periods of time between each dose may be the same or can vary (e.g., increase over time, decrease over time, or be adjusted in accordance with the subject and/or with a medical practitioner's determination). In some embodiments, e.g., as set forth herein, a given therapeutic agent has a recommended dosing regimen, which can involve one or more doses. Typically, at least one recommended dosing regimen of a marketed drug is known to those of skill in the art. In some embodiments, e.g., as set forth herein, a dosing regimen is correlated with a desired or beneficial outcome when administered across a relevant population (i.e., is a therapeutic dosing regimen).

*Downstream:* As used herein, the term" downstream" means that a first DNA region is closer, relative to a second DNA region, to the C-terminus of a nucleic acid that includes the first DNA region and the second DNA region.

*Gene:* As used herein, the term "gene" refers to a single DNA region, e.g., in a chromosome, that includes a coding sequence that encodes a product (e.g., an RNA product and/or a polypeptide product), together with all, some, or none of the DNA sequences that contribute to regulation of the expression of coding sequence. In some embodiments, e.g., as set forth herein, a gene includes one or more non-coding sequences. In some particular embodiments, e.g., as set forth herein, a gene includes exonic and intronic sequences. In some embodiments, e.g., as set forth herein, a gene includes one or more regulatory elements that, for example, can control or impact one or more aspects of gene expression (e.g., cell-type-specific expression, inducible expression, etc.). In some embodiments, e.g., as set forth herein, a gene includes a promoter. In some embodiments, e.g., as set forth herein, a gene includes one or both of a (i) DNA nucleotides extending a predetermined number of nucleotides upstream of the coding sequence and (ii) DNA nucleotides extending a predetermined number of nucleotides downstream of the coding sequence. In various embodiments, e.g., as set forth herein, the predetermined number of nucleotides can be 500 bp, 1 kb, 2 kb, 3 kb, 4 kb, 5 kb, 10 kb, 20 kb, 30 kb, 40 kb, 50 kb, 75 kb, or 100 kb.

*Homology:* As used herein, the term "homology" refers to the overall relatedness between polymeric molecules, *e.g.,* between nucleic acid molecules *(e.g.,* DNA molecules and/or RNA molecules) and/or between polypeptide molecules. Those of skill in the art will appreciate that homology can be defined, e.g., by a percent identity or by a percent homology (sequence similarity). In some embodiments, e.g., as set forth herein, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% identical. In some embodiments, e.g., as set forth herein, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% similar.

*Hybridize:* As used herein, "hybridize" refers to the association of a first nucleic acid with a second nucleic acid to form a double-stranded structure, which association occurs through complementary pairing of nucleotides. Those of skill in the art will recognize that complementary sequences, among others, can hybridize. In various embodiments, e.g., as set forth herein, hybridization can occur, for example, between nucleotide sequences having at least 70% complementarity, e.g., at least 75%, 80%, 85%, 90%, 91%, 92%, 93%, 94%, 95%, 96%, 97%, 98%, 99%, or 100% complementarity. Those of skill in the art will further appreciate that whether hybridization of a first nucleic acid and a second nucleic acid does or does not occur can dependence upon various reaction conditions. Conditions under which hybridization can occur are known in the art.

*Hypomethylation:* As used herein, the term "hypomethylation" refers to the state of a methylation locus having at least one fewer methylated nucleotides in a state of interest as compared to a reference state (e.g., at least one fewer methylated nucleotides in colorectal cancer than in a healthy control).

*Hypermethylation:* As used herein, the term "hypermethylation" refers to the state of a methylation locus having at least one more methylated nucleotide in a state of interest as compared to a reference state (e.g., at least one more methylated nucleotide in colorectal cancer than in a healthy control).

*Identity, identical:* As used herein, the terms "identity" and "identical" refers to the overall relatedness between polymeric molecules, *e.g.,* between nucleic acid molecules *(e.g.,* DNA molecules and/or RNA molecules) and/or between polypeptide molecules.

Methods for the calculation of a percent identity as between two provided sequences are known in the art. Calculation of the percent identity of two nucleic acid or polypeptide sequences, for example, can be performed by aligning the two sequences (or the complement of one or both sequences) for optimal comparison purposes (e.g., gaps can be introduced in one or both of a first and a second sequences for optimal alignment and non-identical sequences can be disregarded for comparison purposes). The nucleotides or amino acids at corresponding positions are then compared. When a position in the first sequence is occupied by the same residue (e.g., nucleotide or amino acid) as the corresponding position in the second sequence, then the molecules are identical at that position. The percent identity between the two sequences is a function of the number of identical positions shared by the sequences and, optionally, taking into account the number of gaps and the length of each gap, which may need to be introduced for optimal alignment of the two sequences. The comparison of sequences and determination of percent identity between two sequences can be accomplished using a computational algorithm, such as BLAST (basic local alignment search tool).

*"Improved," "increased,"* or *"reduced":* As used herein, these terms, or grammatically comparable comparative terms, indicate values that are relative to a comparable reference measurement. For example, in some embodiments, e.g., as set forth herein, an assessed value achieved with an agent of interest may be "improved" relative to that obtained with a comparable reference agent or with no agent. Alternatively or additionally, in some embodiments, e.g., as set forth herein, an assessed value in a subject or system of interest may be "improved" relative to that obtained in the same subject or system under different conditions or at a different point in time (e.g., prior to or after an event such as administration of an agent of interest), or in a different, comparable subject (e.g., in a comparable subject or system that differs from the subject or system of interest in presence of one or more indicators of a particular disease, disorder or condition of interest, or in prior exposure to a condition or agent, etc.). In some embodiments, e.g., as set forth herein, comparative terms refer to statistically relevant differences (e.g., differences of a prevalence and/or magnitude sufficient to achieve statistical relevance). Those of skill in the art will be aware, or will readily be able to determine, in a given context, a degree and/or prevalence of difference that is required or sufficient to achieve such statistical significance.

*Methylation:* As used herein, the term "methylation" includes methylation at any of (i) C5 position of cytosine; (ii) N4 position of cytosine; and (iii) the N6 position of adenine. Methylation also includes (iv) other types of nucleotide methylation. A nucleotide that is methylated can be referred to as a "methylated nucleotide" or "methylated nucleotide base." In certain embodiments, e.g., as set forth herein, methylation specifically refers to methylation of cytosine residues. In some instances, methylation specifically refers to methylation of cytosine residues present in CpG sites.

*Methylation assay:* As used herein, the term "methylation assay" refers to any technique that can be used to determine the methylation status of a methylation locus.

*Methylation biomarker:* As used herein, the term "methylation biomarker" refers to a biomarker that is or includes at least one methylation locus and/or the methylation status of at least one methylation locus, e.g., a hypermethylated locus. In particular, a methylation biomarker is a biomarker characterized by a change between a first state and a second state (e.g., between a cancerous state and a non-cancerous state) in methylation status of one or more nucleic acid loci.

*Methylation locus:* As used herein, the term "methylation locus" refers to a DNA region that includes at least one differentially methylated region. A methylation locus that includes a greater number or frequency of methylated sites under a selected condition of interest, such as a cancerous state, can be referred to as a hypermethylated locus. A methylation locus that includes a smaller number or frequency of methylated sites under a selected condition of interest, such as a cancerous state, can be referred to as a hypomethylated locus. In some instances, e.g., as set forth herein, a methylation locus has a length of at least 10, at least 15, at least 20, at least 30, at least 50, or at least 75 base pairs. In some instances, e.g., as set forth herein, a methylation locus has a length of less than 5000 bp, 4,000 bp, 3,000 bp, 2,000 bp, 1,000 bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, or 10 bp (e.g., where methylation status is determined using quantitative polymerase chain reaction (qPCR), e.g., methylation sensitive restriction enzyme quantitative polymerase chain reaction (MSRE-qPCR)).

*Methylation site:* As used herein, a methylation site refers to a nucleotide or nucleotide position that is methylated in at least one condition. In its methylated state, a methylation site can be referred to as a methylated site.

*Methylation status:* As used herein, "methylation status," "methylation state," or "methylation profile" refer to the number, frequency, or pattern of methylation at methylation sites within a methylation locus. Accordingly, a change in methylation status between a first state and a second state can be or include an increase in the number, frequency, or pattern of methylated sites, or can be or include a decrease in the number, frequency, or pattern of methylated sites. In various instances, a change in methylation status in a change in methylation value.

*Methylation value:* As used herein, the term "methylation value" refers to a numerical representation of a methylation status, e.g., in the form of number that represents the frequency or ratio of methylation of a methylation locus. In some instances, e.g., as set forth herein, a methylation value can be generated by a method that includes quantifying the amount of intact nucleic acid present in a sample following restriction digestion of the sample with a methylation dependent restriction enzyme. In some instances, e.g., as set forth herein, a methylation value can be generated by a method that includes comparing amplification profiles after bisulfite reaction of a sample. In some instances, e.g., as set forth herein, a methylation value can be generated by comparing sequences of bisulfite-treated and untreated nucleic acids. In some instances, e.g., as set forth herein, a methylation value is, includes, or is based on a quantitative PCR result. In some instances, e.g., as set forth herein, a methylation value

*Mutation:* As used herein, the term "mutation" refers to a genetic variation in a biomolecule (e.g., a nucleic acid or a protein) as compared to a reference biomolecule. For example, a mutation in a nucleic acid may, in some embodiments, comprise a nucleobase substitution, a deletion of one or more nucleobases, an insertion of one or more nucleobases, an inversion of two or more nucleobases, or a truncation, as compared to a reference nucleic acid molecule. Similarly, a mutation in a protein may comprise an amino acid substitution, insertion, inversion, or truncation, as compared to a reference polypeptide. Additional mutations, e.g., fusions and indels, are known to those of skill in the art. In some embodiments, a mutation comprises a genetic variant that is associated with a loss of function of a gene product. A loss of function may be a complete abolishment of function, e.g., an abolishment of the enzymatic activity of an enzyme, or a partial loss of function, e.g., a diminished enzymatic activity of an enzyme. In some embodiments, a mutant comprises a genetic variant that is associated with a gain of function, e.g., with a negative or undesirable alteration in a characteristic or activity in a gene product. In some embodiments, a mutant is characterized by a reduction or loss in a desirable level or activity as compared to a reference; in some embodiments, a mutant is characterized by an increase or gain of an undesirable level or activity as compared to a reference. In some embodiments, the reference biomolecule is a wild-type biomolecule.

*Nucleic acid:* As used herein, in its broadest sense, the term "nucleic acid" refers to any compound and/or substance that is or can be incorporated into an oligonucleotide chain. In some embodiments e.g., as set forth herein, a nucleic acid is a compound and/or substance that is or can be incorporated into an oligonucleotide chain via a phosphodiester linkage. As will be clear from context, in some embodiments e.g., as set forth herein, the term nucleic acid refers to an individual nucleic acid residue (e.g., a nucleotide and/or nucleoside), and in some embodiments e.g., as set forth herein refers to an polynucleotide chain comprising a plurality of individual nucleic acid residues. A nucleic acid can be or include DNA, RNA, or a combinations thereof. A nucleic acid can include natural nucleic acid residues, nucleic acid analogs, and/or synthetic residues. In some embodiments e.g., as set forth herein, a nucleic acid includes natural nucleotides (e.g., adenosine, thymidine, guanosine, cytidine, uridine, deoxyadenosine, deoxythymidine, deoxy guanosine, and deoxycytidine). In some embodiments e.g., as set forth herein, a nucleic acid is or includes of one or more nucleotide analogs (e.g., 2-aminoadenosine, 2-thiothymidine, inosine, pyrrolo-pyrimidine, 3 -methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 0(6)-methylguanine, 2-thiocytidine, methylated bases, intercalated bases, and combinations thereof).

In some embodiments e.g., as set forth herein, a nucleic acid has a nucleotide sequence that encodes a functional gene product such as an RNA or protein. In some embodiments e.g., as set forth herein, a nucleic acid includes one or more introns. In some embodiments e.g., as set forth herein, a nucleic acid includes one or more genes. In some embodiments e.g., as set forth herein, nucleic acids are prepared by one or more of isolation from a natural source, enzymatic synthesis by polymerization based on a complementary template *(in vivo* or *in vitro),* reproduction in a recombinant cell or system, and chemical synthesis.

In some embodiments e.g., as set forth herein, a nucleic acid analog differs from a nucleic acid in that it does not utilize a phosphodiester backbone. For example, in some embodiments e.g., as set forth herein, a nucleic acid can include one or more peptide nucleic acids, which are known in the art and have peptide bonds instead of phosphodiester bonds in the backbone. Alternatively or additionally, in some embodiments e.g., as set forth herein, a nucleic acid has one or more phosphorothioate and/or 5'-N-phosphoramidite linkages rather than phosphodiester bonds. In some embodiments e.g., as set forth herein, a nucleic acid comprises one or more modified sugars (e.g., 2'-fluororibose, ribose, 2'-deoxyribose, arabinose, and hexose) as compared with those in natural nucleic acids.

In some embodiments, e.g., as set forth herein, a nucleic acid is or includes at least 3, 4, 5, 6, 7, 8, 9, 10, 15, 20, 25, 30, 35, 40, 45, 50, 55, 60, 65, 70, 75, 80, 85, 90, 95, 100, 1 10, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000 or more residues. In some embodiments, e.g., as set forth herein, a nucleic acid is partly or wholly single stranded, or partly or wholly double stranded.

*Nucleic acid detection assay:* As used herein, the term "nucleic acid detection assay" refers to any method of determining the nucleotide composition of a nucleic acid of interest. Nucleic acid detection assays include but are not limited to, DNA sequencing methods (e.g., next generation sequencing methods), polymerase chain reaction-based methods, probe hybridization methods, ligase chain reaction, etc.

*Nucleotide:* As used herein, the term "nucleotide" refers to a structural component, or building block, of polynucleotides, e.g., of DNA and/or RNA polymers. A nucleotide includes of a base (e.g., adenine, thymine, uracil, guanine, or cytosine) and a molecule of sugar and at least one phosphate group. As used herein, a nucleotide can be a methylated nucleotide or an un-methylated nucleotide. Those of skill in the art will appreciate that nucleic acid terminology, such as, as examples, "locus" or "nucleotide" can refer to both a locus or nucleotide of a single nucleic acid molecule and/or to the cumulative population of loci or nucleotides within a plurality of nucleic acids (e.g., a plurality of nucleic acids in a sample and/or representative of a subject) that are representative of the locus or nucleotide (e.g., having the same identical nucleic acid sequence and/or nucleic acid sequence context, or having a substantially identical nucleic acid sequence and/or nucleic acid context).

*Oligonucleotide primer:* As used herein, the term oligonucleotide primer, or primer, refers to a nucleic acid molecule used, capable of being used, or for use in, generating amplicons from a template nucleic acid molecule. Under transcription-permissive conditions (e.g., in the presence of nucleotides and a DNA polymerase, and at a suitable temperature and pH), an oligonucleotide primer can provide a point of initiation of transcription from a template to which the oligonucleotide primer hybridizes. Typically, an oligonucleotide primer is a single-stranded nucleic acid between 5 and 200 nucleotides in length. Those of skill in the art will appreciate that optimal primer length for generating amplicons from a template nucleic acid molecule can vary with conditions including temperature parameters, primer composition, and transcription or amplification method. A pair of oligonucleotide primers, as used herein, refers to a set of two oligonucleotide primers that are respectively complementary to a first strand and a second strand of a template double-stranded nucleic acid molecule. First and second members of a pair of oligonucleotide primers may be referred to as a "forward" oligonucleotide primer and a "reverse" oligonucleotide primer, respectively, with respect to a template nucleic acid strand, in that the forward oligonucleotide primer is capable of hybridizing with a nucleic acid strand complementary to the template nucleic acid strand, the reverse oligonucleotide primer is capable of hybridizing with the template nucleic acid strand, and the position of the forward oligonucleotide primer with respect to the template nucleic acid strand is 5' of the position of the reverse oligonucleotide primer sequence with respect to the template nucleic acid strand. It will be understood by those of skill in the art that the identification of a first and second oligonucleotide primer as forward and reverse oligonucleotide primers, respectively, is arbitrary inasmuch as these identifiers depend upon whether a given nucleic acid strand or its complement is utilized as a template nucleic acid molecule.

*Overlapping:* The term "overlapping" is used herein in reference to two regions of DNA, each of which contains a sub-sequence that is substantially identical to a sub-sequence of the same length in the other region (e.g., the two regions of DNA have a common sub-sequence). "Substantially identical" means that the two identically-long sub-sequences differ by fewer than a given number of base pairs. In certain instances, e.g., as set forth herein, each sub-sequence has a length of at least 20 base pairs that differ by fewer than 4, 3, 2, or 1 base pairs from each other (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, each sub-sequence has a length of at least 24 base pairs that differ by fewer than 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). **In** certain instances, e.g., as set forth herein, each sub-sequence has a length of at least 50 base pairs that differ by fewer than 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). **In** certain instances, e.g., as set forth herein, each sub-sequence has a length of at least 100 base pairs that differ by fewer than 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). **In** certain instances, e.g., as set forth herein, each sub-sequence has a length of at least 200 base pairs that differ by fewer than 40, 30, 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). **In** certain instances, e.g., as set forth herein, each sub-sequence has a length of at least 250 base pairs that differ by fewer than 50, 40, 30, 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). **In** certain instances, e.g., as set forth herein, each sub-sequence has a length of at least 300 base pairs that differ by fewer than 60, 50, 40, 30, 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). **In** certain instances, e.g., as set forth herein, each sub-sequence has a length of at least 500 base pairs that differ by fewer than 100, 60, 50, 40, 30, 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). **In** certain instances, e.g., as set forth herein, each sub-sequence has a length of at least 1000 base pairs that differ by fewer than 200, 100, 60, 50, 40, 30, 20, 15, 10, 9, 8, 7, 6, 5, 4, 3, 2, or 1 base pairs (e.g., the two sub-sequences having at least 80%, at least 85%, at least 90%, at least 95% similarity, at least 97% similarity, at least 98% similarity, at least 99% similarity, or at least 99.5% similarity). In certain instances, e.g., as set forth herein, the subsequence of a first region of the two regions of DNA may comprise the entirety of the second region of the two regions of DNA (or vice versa) (e.g., the common sub-sequence may contain the whole of either or both regions). In certain embodiments, where a methylation locus has a sequence that comprises at "least a portion of" a DMR sequence listed herein (e.g., at least 20%, at least 30%, at least 40%, at least 50%, at least 60%, at least 70%, at least 80%, or at least 90% of the DMR sequence), the overlapping portion of the methylation locus has at least 95% similarity, at least 98% similarity, or at least 99% similarity with the overlapping portion of the DMR sequence (e.g., if the overlapping portion is 100bp, the portion of the methylation locus that overlaps with the portion of the DMR differs by no more than 1 bp, no more than 2 bp, or no more than 5 bp). In certain embodiments, where a methylation locus has a sequence that comprises "at least a portion of" a DMR sequence listed herein, this means the methylation locus has a subsequence in common with the DMR sequence that has a consecutive series of bases that covers at least 20%, at least 30%, at least 40%, at least 50%, at least 60%, at least 70%, at least 80%, or at least 90% of the DMR sequence, e.g., wherein the subsequence in common differs by no more than 1 bp, no more than 2 bp, or no more than 5 bp). In certain embodiments, where a methylation locus has a sequence that comprises "at least a portion of" a DMR sequence listed herein, this means the methylation locus contains at least a portion of (e.g., at least 20%, at least 30%, at least 40%, at least 50%, at least 60%, at least 70%, at least 80%, or at least 90% of) the CpG dinucleotides corresponding to the CpG dinucleotides within the DMR sequence.

*Pharmaceutical composition:* As used herein, the term "pharmaceutical composition" refers to a composition in which an active agent is formulated together with one or more pharmaceutically acceptable carriers. In some embodiments, e.g., as set forth herein, the active agent is present in a unit dose amount appropriate for administration to a subject, e.g., in a therapeutic regimen that shows a statistically significant probability of achieving a predetermined therapeutic effect when administered to a relevant population. In some embodiments, e.g., as set forth herein, a pharmaceutical composition can be formulated for administration in a particular form (e.g., in a solid form or a liquid form), and/or can be specifically adapted for, for example: oral administration (for example, as a drenche (aqueous or non-aqueous solutions or suspensions), tablet, capsule, bolus, powder, granule, paste, etc., which can be formulated specifically for example for buccal, sublingual, or systemic absorption); parenteral administration (for example, by subcutaneous, intramuscular, intravenous or epidural injection as, for example, a sterile solution or suspension, or sustained-release formulation, etc.); topical application (for example, as a cream, ointment, patch or spray applied for example to skin, lungs, or oral cavity); intravaginal or intrarectal administration (for example, as a pessary, suppository, cream, or foam); ocular administration; nasal or pulmonary administration, etc.

*Pharmaceutically acceptable:* As used herein, the term "pharmaceutically acceptable," as applied to one or more, or all, component(s) for formulation of a composition as disclosed herein, means that each component must be compatible with the other ingredients of the composition and not deleterious to the recipient thereof.

*Pharmaceutically acceptable carrier:* As used herein, the term "pharmaceutically acceptable carrier" refers to a pharmaceutically-acceptable material, composition, or vehicle, such as a liquid or solid filler, diluent, excipient, or solvent encapsulating material, that facilitates formulation and/or modifies bioavailability of an agent, e.g., a pharmaceutical agent. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

*Polyposis syndromes:* The terms "polyposis" and "polyposis syndrome", as used herein, refer to hereditary conditions that include, but are not limited to, familial adenomatous polyposis (FAP), hereditary nonpolyposis colorectal cancer (HNPCC)/Lynch syndrome, Gardner syndrome, Turcot syndrome, MUTYH polyposis, Peutz-Jeghers syndrome, Cowden disease, familial juvenile polyposis, and hyperplastic polyposis. In certain embodiments, polyposis includes serrated polyposis syndrome. Serrated polyposis is classified by a subject having 5 or more serrated polyps proximal to the sigmoid colon with two or more at least 10 mm in size, having a serrated polyp proximal to the sigmoid colon in the context of a family history of serrated polyposis, and/or having 20 or more serrated polyps throughout the colon.

*Prevent* or *prevention:* The terms "prevent" and "prevention," as used herein in connection with the occurrence of a disease, disorder, or condition, refers to reducing the risk of developing the disease, disorder, or condition; delaying onset of the disease, disorder, or condition; delaying onset of one or more characteristics or symptoms of the disease, disorder, or condition; and/or to reducing the frequency and/or severity of one or more characteristics or symptoms of the disease, disorder, or condition. Prevention can refer to prevention in a particular subject or to a statistical impact on a population of subjects. Prevention can be considered complete when onset of a disease, disorder, or condition has been delayed for a predefined period of time.

*Probe:* As used herein, the terms "probe", "capture probe", or "bait" refer to a single- or double-stranded nucleic acid molecule that is capable of hybridizing with a complementary target and includes a detectable moiety. In certain embodiments, e.g., as set forth herein, a probe is a restriction digest product or is a synthetically produced nucleic acid, e.g., a nucleic acid produced by recombination or amplification. In some instances, e.g., as set forth herein, a probe is a capture probe useful in detection, identification, and/or isolation of a target sequence, such as a gene sequence. In various instances, e.g., as set forth herein, a detectable moiety of probe can be, e.g., an enzyme (e.g., ELISA, as well as enzyme-based histochemical assays), fluorescent moiety, radioactive moiety, or moiety associated with a luminescence signal.

*Prognosis:* As used herein, the term "prognosis" refers to determining the qualitative of quantitative probability of at least one possible future outcome or event. As used herein, a prognosis can be a determination of the likely course of a disease, disorder, or condition such as cancer in a subject, a determination regarding the life expectancy of a subject, or a determination regarding response to therapy, e.g., to a particular therapy. *Prognostic information:* As used herein, the term "prognostic information" refers to information useful in providing a prognosis. Prognostic information can include, without limitation, biomarker status information.

*Promoter:* As used herein, a "promoter" can refer to a DNA regulatory region that directly or indirectly (e.g., through promoter-bound proteins or substances) associates with an RNA polymerase and participates in initiation of transcription of a coding sequence.

*Reference:* As used herein describes a standard or control relative to which a comparison is performed. For example, in some embodiments, e.g., as set forth herein, an agent, subject, animal, individual, population, sample, sequence, or value of interest is compared with a reference or control agent, subject, animal, individual, population, sample, sequence, or value. In some embodiments, e.g., as set forth herein, a reference or characteristic thereof is tested and/or determined substantially simultaneously with the testing or determination of the characteristic in a sample of interest. In some embodiments, e.g., as set forth herein, a reference is a historical reference, optionally embodied in a tangible medium. Typically, as would be understood by those of skill in the art, a reference is determined or characterized under comparable conditions or circumstances to those under assessment, e.g., with regard to a sample. Those skilled in the art will appreciate when sufficient similarities are present to justify reliance on and/or comparison to a particular possible reference or control.

*Risk:* As used herein with respect to a disease, disorder, or condition, the term "risk" refers to the qualitative of quantitative probability (whether expressed as a percentage or otherwise) that a particular individual will develop the disease, disorder, or condition. In some embodiments, e.g., as set forth herein, risk is expressed as a percentage. In some embodiments, e.g., as set forth herein, a risk is a qualitative of quantitative probability that is equal to or greater than 0, 1, 2, 3, 4, 5, 6, 7, 8, 9, 10, 20, 30, 40, 50, 60, 70, 80, 90, or 100%. In some embodiments, e.g., as set forth herein, risk is expressed as a qualitative or quantitative level of risk relative to a reference risk or level or the risk of the same outcome attributed to a reference. In some embodiments, e.g., as set forth herein, relative risk is increased or decreased in comparison to the reference sample by a factor of 1.1, 1.2, 1.3, 1.4, 1.5, 1.6, 1.7,. 1.8, 1.9, 2, 3, 4, 5, 6, 7, 8, 9, 10, or more.

*Sample:* As used herein, the term "sample" typically refers to an aliquot of material obtained or derived from a source of interest. In some embodiments, e.g., as set forth herein, a source of interest is a biological or environmental source. In some embodiments, e.g., as set forth herein, a sample is a "primary sample" obtained directly from a source of interest. In some embodiments, e.g., as set forth herein, as will be clear from context, the term "sample" refers to a preparation that is obtained by processing of a primary sample (e.g., by removing one or more components of and/or by adding one or more agents to a primary sample). Such a "processed sample" can include, for example cells, nucleic acids, or proteins extracted from a sample or obtained by subjecting a primary sample to techniques such as amplification or reverse transcription of nucleic acids, isolation and/or purification of certain components, *etc.*

In certain instances, e.g., as set forth herein, a processed sample can be a DNA sample that has been amplified (e.g., pre-amplified). Thus, in various instances, e.g., as set forth herein, an identified sample can refer to a primary form of the sample or to a processed form of the sample. In some instances, e.g., as set forth herein, a sample that is enzyme-digested DNA can refer to primary enzyme-digested DNA (the immediate product of enzyme digestion) or a further processed sample such as enzyme-digested DNA that has been subject to an amplification step (e.g., an intermediate amplification step, e.g., pre-amplification) and/or to a filtering step, purification step, or step that modifies the sample to facilitate a further step, e.g., in a process of determining methylation status (e.g., methylation status of a primary sample of DNA and/or of DNA as it existed in its original source context).

*Screening:* As used herein, the term "screening" refers to any method, technique, process, or undertaking intended to generate diagnostic information and/or prognostic information. Accordingly, those of skill in the art will appreciate that the term screening encompasses method, technique, process, or undertaking that determines whether an individual has, is likely to have or develop, or is at risk of having or developing a disease, disorder, or condition, e.g., colorectal cancer, advanced adenoma.

*Specificity:* As used herein, the "specificity" of a biomarker refers to the percentage of samples that are characterized by absence of the event or state of interest for which measurement of the biomarker accurately indicates absence of the event or state of interest (true negative rate). In various embodiments, e.g., as set forth herein, characterization of the negative samples is independent of the biomarker, and can be achieved by any relevant measure, e.g., any relevant measure known to those of skill in the art. Thus, specificity reflects the probability that the biomarker would detect the absence of the event or state of interest when measured in a sample not characterized that event or state of interest. In particular embodiments in which the event or state of interest is colorectal cancer, e.g., as set forth herein, specificity refers to the probability that a biomarker would detect the absence of colorectal cancer in a subject lacking colorectal cancer. Lack of colorectal cancer can be determined, e.g., by histology.

*Sensitivity:* As used herein, the "sensitivity" of a biomarker refers to the percentage of samples that are characterized by the presence of the event or state of interest for which measurement of the biomarker accurately indicates presence of the event or state of interest (true positive rate). In various embodiments, e.g., as set forth herein, characterization of the positive samples is independent of the biomarker, and can be achieved by any relevant measure, e.g., any relevant measure known to those of skill in the art. Thus, sensitivity reflects the probability that a biomarker would detect the presence of the event or state of interest when measured in a sample characterized by presence of that event or state of interest. In particular embodiments in which the event or state of interest is colorectal cancer, e.g., as set forth herein, sensitivity refers to the probability that a biomarker would detect the presence of colorectal cancer in a subject that has colorectal cancer. Presence of colorectal cancer can be determined, e.g., by histology.

*Single Nucleotide Polymorphism (SNP):* As used herein, the term "single nucleotide polymorphism" or "SNP" refers to a particular base position in the genome where alternative bases are known to distinguish one allele from another. In some embodiments, one or a few SNPs and/or CNPs is/are sufficient to distinguish complex genetic variants from one another so that, for analytical purposes, one or a set of SNPs and/or CNPs may be considered to be characteristic of a particular variant, trait, cell type, individual, species, etc, or set thereof. In some embodiments, one or a set of SNPs and/or CNPs may be considered to define a particular variant, trait, cell type, individual, species, etc, or set thereof.

*Solid Tumor:* As used herein, the term "solid tumor" refers to an abnormal mass of tissue including cancer cells. In various embodiments, e.g., as set forth herein, a solid tumor is or includes an abnormal mass of tissue that does not contain cysts or liquid areas. In some embodiments, e.g., as set forth herein, a solid tumor can be benign; in some embodiments, a solid tumor can be malignant. Examples of solid tumors include carcinomas, lymphomas, and sarcomas. In some embodiments, e.g., as set forth herein, solid tumors can be or include adrenal, bile duct, bladder, bone, brain, breast, cervix, colon, endometrium, esophagum, eye, gall bladder, gastrointestinal tract, kidney, larynx, liver, lung, nasal cavity, nasopharynx, oral cavity, ovary, penis, pituitary, prostate, retina, salivary gland, skin, small intestine, stomach, testis, thymus, thyroid, uterine, vaginal, and/or vulval tumors.

*Stage of cancer:* As used herein, the term "stage of cancer" refers to a qualitative or quantitative assessment of the level of advancement of a cancer. In some embodiments, e.g., as set forth herein, criteria used to determine the stage of a cancer can include, but are not limited to, one or more of where the cancer is located in a body, tumor size, whether the cancer has spread to lymph nodes, whether the cancer has spread to one or more different parts of the body, etc. In some embodiments, e.g., as set forth herein, cancer can be staged using the so-called TNM System, according to which T refers to the size and extent of the main tumor, usually called the primary tumor; N refers to the number of nearby lymph nodes that have cancer; and M refers to whether the cancer has metastasized. In some embodiments, e.g., as set forth herein, a cancer can be referred to as Stage 0 (abnormal cells are present but have not spread to nearby tissue, also called carcinoma *in situ,* or CIS; CIS is not cancer, but it can become cancer), Stage I-III (cancer is present; the higher the number, the larger the tumor and the more it has spread into nearby tissues), or Stage IV (the cancer has spread to distant parts of the body). In some embodiments, e.g., as set forth herein, a cancer can be assigned to a stage selected from the group consisting of: *in situ* (abnormal cells are present but have not spread to nearby tissue); localized (cancer is limited to the place where it started, with no sign that it has spread); regional (cancer has spread to nearby lymph nodes, tissues, or organs): distant (cancer has spread to distant parts of the body); and unknown (there is not enough information to identify cancer stage).

*Susceptible to:* An individual who is "susceptible to" a disease, disorder, or condition is at risk for developing the disease, disorder, or condition. In some embodiments, e.g., as set forth herein, an individual who is susceptible to a disease, disorder, or condition does not display any symptoms of the disease, disorder, or condition. In some embodiments, e.g., as set forth herein, an individual who is susceptible to a disease, disorder, or condition has not been diagnosed with the disease, disorder, and/or condition. In some embodiments, e.g., as set forth herein, an individual who is susceptible to a disease, disorder, or condition is an individual who has been exposed to conditions associated with, or presents a biomarker status (e.g., a methylation status) associated with, development of the disease, disorder, or condition. In some embodiments, e.g., as set forth herein, a risk of developing a disease, disorder, and/or condition is a population-based risk (e.g., family members of individuals suffering from the disease, disorder, or condition).

*Subject:* As used herein, the term "subject" refers to an organism, typically a mammal (e.g., a human). **In** some embodiments, e.g., as set forth herein, a subject is suffering from a disease, disorder or condition. **In** some embodiments, e.g., as set forth herein, a subject is susceptible to a disease, disorder, or condition. **In** some embodiments, e.g., as set forth herein, a subject displays one or more symptoms or characteristics of a disease, disorder or condition. **In** some embodiments, e.g., as set forth herein, a subject is not suffering from a disease, disorder or condition. **In** some embodiments, e.g., as set forth herein, a subject does not display any symptom or characteristic of a disease, disorder, or condition. **In** some embodiments, e.g., as set forth herein, a subject is someone with one or more features characteristic of susceptibility to or risk of a disease, disorder, or condition. **In** some embodiments, e.g., as set forth herein, a subject is a patient. **In** some embodiments, e.g., as set forth herein, a subject is an individual to whom diagnosis has been performed and/or to whom therapy has been administered. **In** some instances, e.g., as set forth herein, a human subject can be interchangeably referred to as an "individual."

*Therapeutic agent:* As used herein, the term "therapeutic agent" refers to any agent that elicits a desired pharmacological effect when administered to a subject. **In** some embodiments, e.g., as set forth herein, an agent is considered to be a therapeutic agent if it demonstrates a statistically significant effect across an appropriate population. **In** some embodiments, e.g., as set forth herein, the appropriate population can be a population of model organisms or a human population. **In** some embodiments, e.g., as set forth herein, an appropriate population can be defined by various criteria, such as a certain age group, gender, genetic background, preexisting clinical conditions, etc. **In** some embodiments, e.g., as set forth herein, a therapeutic agent is a substance that can be used for treatment of a disease, disorder, or condition. **In** some embodiments, e.g., as set forth herein, a therapeutic agent is an agent that has been or is required to be approved by a government agency before it can be marketed for administration to humans. **In** some embodiments, e.g., as set forth herein, a therapeutic agent is an agent for which a medical prescription is required for administration to humans.

*Therapeutically effective amount:* As used herein, the term "therapeutically effective amount" refers to an amount that produces a desired effect for which it is administered.

In some embodiments, e.g., as set forth herein, the term refers to an amount that is sufficient, when administered to a population suffering from or susceptible to a disease, disorder, or condition, in accordance with a therapeutic dosing regimen, to treat the disease, disorder, or condition. Those of ordinary skill in the art will appreciate that the term therapeutically effective amount does not in fact require successful treatment be achieved in a particular individual. Rather, a therapeutically effective amount can be an amount that provides a particular desired pharmacological response in a significant number of subjects when administered to individuals in need of such treatment. In some embodiments, e.g., as set forth herein, reference to a therapeutically effective amount can be a reference to an amount as measured in one or more specific tissues (e.g., a tissue affected by the disease, disorder or condition) or fluids (e.g., blood, saliva, serum, sweat, tears, urine, etc.). Those of ordinary skill in the art will appreciate that, in some embodiments, a therapeutically effective amount of a particular agent can be formulated and/or administered in a single dose. In some embodiments, e.g., as set forth herein, a therapeutically effective agent can be formulated and/or administered in a plurality of doses, for example, as part of a multi-dose dosing regimen.

*Treatment:* As used herein, the term "treatment" (also "treat" or "treating") refers to administration of a therapy that partially or completely alleviates, ameliorates, relieves, inhibits, delays onset of, reduces severity of, and/or reduces incidence of one or more symptoms, features, and/or causes of a particular disease, disorder, or condition, or is administered for the purpose of achieving any such result. In some embodiments, e.g., as set forth herein, such treatment can be of a subject who does not exhibit signs of the relevant disease, disorder, or condition and/or of a subject who exhibits only early signs of the disease, disorder, or condition. Alternatively or additionally, such treatment can be of a subject who exhibits one or more established signs of the relevant disease, disorder and/or condition. In some embodiments, e.g., as set forth herein, treatment can be of a subject who has been diagnosed as suffering from the relevant disease, disorder, and/or condition. In some embodiments, e.g., as set forth herein, treatment can be of a subject known to have one or more susceptibility factors that are statistically correlated with increased risk of development of the relevant disease, disorder, or condition. In various examples, treatment is of a cancer.

*Upstream:* As used herein, the term "upstream" means a first DNA region is closer, relative to a second DNA region, to the N-terminus of a nucleic acid that includes the first DNA region and the second DNA region.

*Unit dose:* As used herein, the term "unit dose" refers to an amount administered as a single dose and/or in a physically discrete unit of a pharmaceutical composition. In many embodiments, e.g., as set forth herein, a unit dose contains a predetermined quantity of an active agent. In some embodiments, e.g., as set forth herein, a unit dose contains an entire single dose of the agent. In some embodiments, e.g., as set forth herein, more than one unit dose is administered to achieve a total single dose. In some embodiments, e.g., as set forth herein, administration of multiple unit doses is required, or expected to be required, in order to achieve an intended effect. A unit dose can be, for example, a volume of liquid (e.g., an acceptable carrier) containing a predetermined quantity of one or more therapeutic moieties, a pre-determined amount of one or more therapeutic moieties in solid form, a sustained release formulation or drug delivery device containing a predetermined amount of one or more therapeutic moieties, etc. It will be appreciated that a unit dose can be present in a formulation that includes any of a variety of components in addition to the therapeutic agent(s). For example, acceptable carriers (e.g., pharmaceutically acceptable carriers), diluents, stabilizers, buffers, preservatives, etc., can be included. It will be appreciated by those skilled in the art, in many embodiments, e.g., as set forth herein, a total appropriate daily dosage of a particular therapeutic agent can comprise a portion, or a plurality, of unit doses, and can be decided, for example, by a medical practitioner within the scope of sound medical judgment. In some embodiments, e.g., as set forth herein, the specific effective dose level for any particular subject or organism can depend upon a variety of factors including the disorder being treated and the severity of the disorder; activity of specific active compound employed; specific composition employed; age, body weight, general health, sex and diet of the subject; time of administration, and rate of excretion of the specific active compound employed; duration of the treatment; drugs and/or additional therapies used in combination or coincidental with specific compound(s) employed, and like factors well known in the medical arts.

*Unmethylated:* As used herein, the terms "unmethylated" and "non-methylated" are used interchangeably and mean that an identified DNA region includes no methylated nucleotides.

*Variant:* As used herein, the term "variant" refers to an entity that shows significant structural identity with a reference entity but differs structurally from the reference entity in the presence, absence, or level of one or more chemical moieties as compared with the reference entity. In some embodiments, e.g., as set forth herein, a variant also differs functionally from its reference entity. In general, whether a particular entity is properly considered to be a "variant" of a reference entity is based on its degree of structural identity with the reference entity. A variant can be a molecule comparable, but not identical to, a reference. For example, a variant nucleic acid can differ from a reference nucleic acid at one or more differences in nucleotide sequence. In some embodiments, e.g., as set forth herein, a variant nucleic acid shows an overall sequence identity with a reference nucleic acid that is at least 85%, 86%, 87%, 88%, 89%, 90%, 91%, 92%, 93%, 94%, 95%, 96%, 97%, or 99%. In many embodiments, e.g., as set forth herein, a nucleic acid of interest is considered to be a "variant" of a reference nucleic acid if the nucleic acid of interest has a sequence that is identical to that of the reference but for a small number of sequence alterations at particular positions. In some embodiments, e.g., as set forth herein, a variant has 10, 9, 8, 7, 6, 5, 4, 3, 2, or 1 substituted residues as compared with a reference. In some embodiments, e.g., as set forth herein, a variant has not more than 5, 4, 3, 2, or 1 residue additions, substitutions, or deletions as compared with the reference. In various embodiments, e.g., as set forth herein, the number of additions, substitutions, or deletions is fewer than about 25, about 20, about 19, about 18, about 17, about 16, about 15, about 14, about 13, about 10, about 9, about 8, about 7, about 6, and commonly are fewer than about 5, about 4, about 3, or about 2 residues.

BRIEF DESCRIPTION OF THE DRAWINGS

The foregoing and other objects, aspects, features, and advantages of the present disclosure will become more apparent and better understood by referring to the following description taken in conjunction with the accompanying drawings, in which:

FIG. 1 is a flow diagram of an exemplary hybrid capture method, according to an illustrative embodiment.
FIG. 2 is a list of 203 DMRs (differentially methylated regions) identified for use in colorectal cancer detection, according to an illustrative embodiment.
FIG. 3 is a list of 220 DMRs (differentially methylated regions) identified for use in advanced adenoma detection, according to an illustrative embodiment.
FIG. 4 is a list of mutation biomarker regions, according to an illustrative embodiment.
FIG. 5 is a flow diagram of an exemplary hybrid capture method, according to an illustrative embodiment.
FIG. 6 is a flow diagram of an exemplary library preparation method, according to an illustrative embodiment.
FIG. 7 is an exemplary bioinformatics workflow, according to an illustrative embodiment.
FIG. 8 is a graph showing a receiver operating characteristic (ROC) curve of a 203 marker panel on a validation set for colorectal cancer, according to an illustrative embodiment.
FIG. 9 is a bar graph showing sensitivity values and overall specificity of an exemplary classification model for colorectal cancer.
FIG. 10A and 10B are box plots showing exemplary values for determining methylation sample thresholds, for each sample in a validation set of subjects for two individual methylation marker regions, according to an illustrative embodiment.
FIG. 11 is a bar graph showing sensitivity values and overall specificity according to advanced adenoma type for an exemplary classification model for advanced adenoma.
FIG. 12 is a bar graph showing sensitivity values and overall specificity of an exemplary classification model for advanced adenoma.
FIG. 13 is a flow diagram for bioinformatics processing steps, according to an illustrative embodiment.
FIG. 14 is a series of bar graphs comparing sample quality for bisulfite (BS) converted samples and enzymatic (EM) converted samples, according to an illustrative embodiment.
FIG. 15 is a PCA plot comparing sample groups prepared using bisulfite (BS) conversion or enzymatic (EM) conversion, according to an illustrative embodiment.
FIG. 16 is a block diagram of an exemplary cloud computing environment used in certain embodiments.
FIG. 17 is a block diagram of an example computing device and an example mobile computing device used in certain embodiments.

DETAILED DESCRIPTION

It is contemplated that systems, architectures, devices, methods, and processes of the claimed invention encompass variations and adaptations developed using information from the embodiments described herein. Adaptation and/or modification of the systems, architectures, devices, methods, and processes described herein may be performed, as contemplated by this description.

Throughout the description, where articles, devices, systems, and architectures are described as having, including, or comprising specific components, or where processes and methods are described as having, including, or comprising specific steps, it is contemplated that, additionally, there are articles, devices, systems, and architectures of the present invention that consist essentially of, or consist of, the recited components, and that there are processes and methods according to the present invention that consist essentially of, or consist of, the recited processing steps.

It should be understood that the order of steps or order for performing certain action is immaterial so long as the invention remains operable. Moreover, two or more steps or actions may be conducted simultaneously.

The mention herein of any publication, for example, in the Background section, is not an admission that the publication serves as prior art with respect to any of the claims presented herein. The Background section is presented for purposes of clarity and is not meant as a description of prior art with respect to any claim. Where there is any discrepancy in the meaning of a particular term, the meaning provided in the Definition section above is controlling.

Detection of Methylation in Colorectal Neoplasias using a UDX Colon Test

In certain embodiments, methods, systems, and techniques described herein are used to conduct a UDX Colon test. The UDX Colon test is qualitative next generation sequencing (NGS)-based in vitro diagnostic that uses high throughput, targeted hybridization-based capture with bioinformatics processes to detect methylation in more than 300 colorectal neoplasia (e.g., colorectal cancer, advanced adenoma) associated methylation loci in DNA, e.g., cfDNA of a human subject. In certain embodiments, a UDX Colon test utilizes cell-free DNA (cfDNA) from plasma of whole blood in order to identify methylated loci.

A positive result may indicate a presence of colorectal cancer (CRC) or advanced adenoma (AA). A positive result may be followed by diagnostic colonoscopy or other diagnosis-confirmatory assay. A UDX Colon test may be used to detect (e.g., screen) for a colorectal neoplasia in adults 45 years or older, who are at an average risk for CRC. In certain embodiments, a UDX Colon Test includes reagents, software, procedures, or a combination thereof, for testing cfDNA from whole blood samples.

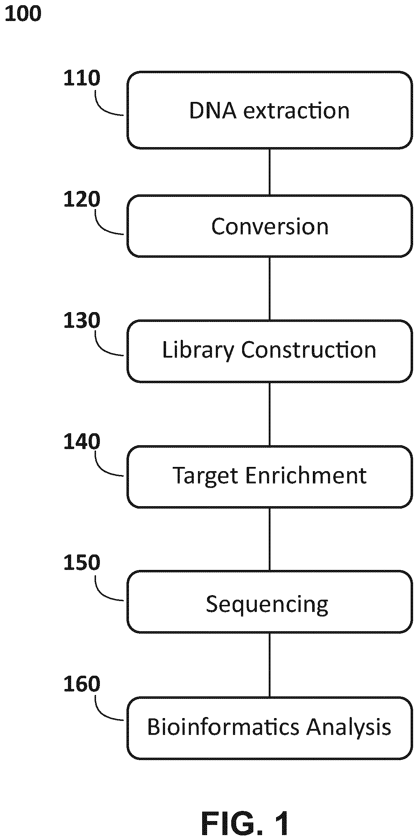

FIG. 1 shows a flow diagram of an exemplary method (100) for processing a DNA samples using methods and techniques disclosed herein. In certain embodiments, a test uses about 10 to about 20 ng of cfDNA extracted from about 4 ml of plasma (110). Extracted cfDNA may be subjected to conversion (e.g., bisulfite or enzymatic conversion) (120). Then a sequencing library is constructed (130) using converted DNA. Regions of interest (e.g., methylation markers and mutation markers) may be enriched using a target enrichment strategy (e.g., hybridized-capture) (140). The captured targets are then sequenced using a next generation sequencing (150) technique. Sequencing data is processed using a customized bioinformatics analysis pipeline (160) designed to detect (e.g., screen for) genomic alterations including methylation and mutations (e.g., nucleotide substitutions).

Detection of Colorectal Cancer and Advanced Adenoma

In various embodiments, a methylation biomarker of the present disclosure used for detection of colorectal cancer is selected from a methylation locus that is or includes at least a portion of a DMR listed in FIG. 2. FIG. 2 lists the region of DNA on which the DMR is found, which includes the chromosome number (chr), the start ("start") and end ("end") positions of the DMR on the chromosome, and the size (e.g., "width") of the DMR region ("size of the region"). Additional features of the DMR also listed, including the presence of any enhancers (1 for "present", blank for "not present"), any names of genes known to have a promoter in the region ("promoters"), any 1-5kb regions upstream of the transcription start site (TSS) ("1to5kb") of genes, 5' untranslated regions ("5'UTR"), names of genes having exons in the region ("exons"), names of genes having introns in the region, 3' untranslated regions ("3'UTRs"), and notations regarding the presence of CpG islands, CpG shores, CpG shelves, and CpG open seas (CpG_inter). If a gene of a DMR is mentioned as being associated with another, different DMR, the column "overlapping genes" will say "yes".

In various embodiments, a methylation biomarker of the present disclosure used for detection of advanced adenoma is selected from a methylation locus that is or includes at least a portion of a DMR listed in FIG. 3. FIG. 3 lists the region of DNA on which the DMR is found, which includes the chromosome number (chr), the start ("start") and end ("end") positions of the DMR on the chromosome, and the size (e.g., "width") of the DMR region ("size of the region") is also listed. Additional features of the DMRs are also listed, which are identified above.

For the avoidance of any doubt, any methylation biomarker provided herein in either FIG. 2 or FIG. 3 can be, or be included in, among other things, a colorectal cancer marker and/or an advanced adenoma marker. Additionally, any methylation biomarker herein can be, or be included in, an advanced adenoma methylation biomarker.

In some embodiments, said methylation biomarker can be or include a single methylation locus. In some embodiments, a methylation biomarker can be or include two or more methylation loci. In some embodiments, a methylation biomarker can be or include a single differentially methylated region (DMR) (e.g., (i) a DMR selected from those listed in FIG. 2 or FIG. 3, (ii) a DMR that encompasses a DMR selected from those listed in FIG. 2 or FIG. 3, (iii) a DMR that overlaps with one or more DMRs selected from those listed in FIG. 2 or FIG. 3, or (iv) a DMR that is a portion of a DMR selected from those listed in FIG. 2 or FIG. 3). In some embodiments, a methylation locus can be or include two or more DMRs (e.g., two, three, four, or more DMRs selected from those listed in FIG. 2 or FIG. 3, or two, three, four, five, six, seven, eight, nine, ten, eleven, twelve, thirteen, fourteen, fifteen, sixteen, seventeen, eighteen, nineteen, twenty, twenty-one, twenty two, twenty three, twenty four, or more DMRs, each of which overlap with and/or encompass a DMR selected from those listed in FIG. 2 or FIG. 3). In some embodiments, a methylation biomarker can be or include a single methylation site (e.g., a single CpG site, a methylated cytosine residue). In other embodiments, a methylation biomarker can be or include two or more methylation sites. In some embodiments, a methylation locus can include two or more DMRs and further include DNA regions adjacent to one or more of the included DMRs.

In some instances, a methylation locus is or includes a gene, such as a gene provided in FIG. 2 or FIG. 3. In some instances a methylation locus is or includes a portion of a gene, e.g., a portion of a gene provided in FIG. 2 or FIG. 3. In some instances, a methylation locus includes but is not limited to identified nucleic acid boundaries of a gene. For example, a methylation locus may include a region 1 to 5kb upstream of the transcription start site (TSS) of a gene. A methylation locus may not be currently associated with any known gene.

In some instances, a methylation locus is or includes a coding region of a gene, such as a coding region of a gene provided in FIG. 2 or FIG. 3. In some instances a methylation locus is or includes a portion of the coding region of gene, e.g., a portion of the coding region a gene provided in FIG. 2 or FIG. 3. In some instances, a methylation locus includes but is not limited to identified nucleic acid boundaries of a coding region of gene. In some instances, a methylation locus is or includes a promoter, enhancer, and/or other regulatory region of a gene, such as a gene provided in FIG. 2 or FIG. 3. In some instances a methylation locus is or includes a portion of the promoter, enhancer, and/or regulatory region of a gene, e.g., a portion of promoter and/or regulatory region a gene provided in FIG. 2 or FIG. 3. In some instances, a methylation locus includes but is not limited to identified nucleic acid boundaries of a promoter and/or other regulatory region of gene. In some embodiments a methylation locus is or includes a high CpG density promoter, or a portion thereof.

In some embodiments, a methylation locus is or includes a non-coding sequence. In some embodiments, a methylation locus is or includes one or more exons, and/or one or more introns.

In some embodiments, a methylation locus includes a DNA region extending a predetermined number of nucleotides upstream of a coding sequence, and/or a DNA region extending a predetermined number of nucleotides downstream of a coding sequence. In various instances, a predetermined number of nucleotides upstream and/or downstream and be or include, e.g., 500 bp, 1 kb, 2 kb, 3 kb, 4 kb, 5 kb, 10 kb, 20 kb, 30 kb, 40 kb, 50 kb, 75 kb, or 100 kb. Those of skill in the art will appreciate that methylation biomarkers capable of impacting expression of a coding sequence may typically be within any of these distances of the coding sequence, upstream and/or downstream.

Those of skill in the art will appreciate that a methylation locus identified as a methylation biomarker need not necessarily be assayed in a single experiment, reaction, or amplicon. A single methylation locus identified as a colorectal cancer methylation biomarker can be assayed, e.g., in a method including separate amplification (or providing oligonucleotide primers and conditions sufficient for amplification of) of one or more distinct or overlapping DNA regions within a methylation locus, e.g., one or more distinct or overlapping DMRs. Those of skill in the art will further appreciate that a methylation locus identified as a methylation biomarker need not be analyzed for methylation status of each nucleotide, nor each CpG, present within the methylation locus. Rather, a methylation locus that is a methylation biomarker may be analyzed, e.g., by analysis of a single DNA region within the methylation locus, e.g., by analysis of a single DMR within the methylation locus.

DMRs of the present disclosure can be a methylation locus or include a portion of a methylation locus. In some instances, a DMR is a DNA region with a methylation locus that is, e.g., 1 to 5,000 bp in length. In various embodiments, a DMR is a DNA region with a methylation locus that is equal to or less than 5000 bp, 4,000 bp, 3,000 bp, 2,000 bp, 1,000 bp, 950 bp, 900 bp, 850 bp, 800 bp, 750 bp, 700 bp, 650 bp, 600 bp, 550 bp, 500 bp, 450 bp, 400 bp, 350 bp, 300 bp, 250 bp, 200 bp, 150 bp, 100 bp, 50 bp, 40 bp, 30 bp, 20 bp, or 10 bp in length. In some embodiments, a DMR is 1, 2, 3, 4, 5, 6, 7, 8 or 9 bp in length.

Methylation biomarkers, including without limitation methylation loci and DMRs provided herein, can include at least one methylation site that is a colorectal cancer biomarker.

For clarity, those of skill in the art will appreciate that term methylation biomarker is used broadly, such that a methylation locus can be a methylation biomarker that includes one or more DMRs, each of which DMRs is also itself a methylation biomarker, and each of which DMRs can include one or more methylation sites, each of which methylation sites is also itself a methylation biomarker. Moreover, a methylation biomarker can include two or more methylation loci. Accordingly, status as a methylation biomarker does not turn on the contiguousness of nucleic acids included in a biomarker, but rather on the existence of a change in methylation status for included DNA region(s) between a first state and a second state, such as between colorectal cancer and controls, advanced adenoma and controls, or both colorectal cancer and advanced adenoma and controls. As provided herein, a methylation locus can be any of one or more methylation loci each of which methylation loci is, includes, or is a portion of a gene (or specific DMR) identified in FIG. 2 or FIG. 3. In some embodiments, a colorectal cancer and/or advanced adenoma methylation biomarker includes a single methylation locus that is, includes, or is a portion of a gene identified in FIG. 2 or FIG. 3.

In some embodiments, a methylation biomarker includes two or more methylation loci, each of which is, includes, or is a portion of a gene identified in FIG. 2 or FIG. 3. In some embodiments, a colorectal cancer and/or advanced adenoma methylation biomarker includes a plurality of methylation loci, each of which is, includes, or is a portion of a gene identified in FIG. 2 or FIG. 3.

In various embodiments, a methylation biomarker can be or include one or more individual nucleotides (e.g., a single individual cytosine residue in the context of a CpG) or a plurality of individual cytosine residues (e.g., of a plurality of CpGs) present within one or more methylation loci (e.g, one or more DMRs) provided herein. Thus, in certain embodiments a methylation biomarker is or includes methylation status of a plurality of individual methylation sites.

In various embodiments, a methylation biomarker is, includes, or is characterized by change in methylation status that is a change in the methylation of one or more methylation sites within one or more methylation loci (e.g., one or more DMRs). In various embodiments, a methylation biomarker is or includes a change in methylation status that is a change in the number of methylated sites within one or more methylation loci (e.g., one or more DMRs)(e.g., one or more CpG sites). In various embodiments, a methylation biomarker is or includes a change in methylation status that is a change in the frequency of methylation sites within one or more methylation loci (e.g., one or more DMRs). In various embodiments, a methylation biomarker is or includes a change in methylation status that is a change in the pattern of methylation sites within one or more methylation loci (e.g., one or more DMRs).

In various embodiments, methylation status of one or more methylation loci (e.g., one or more DMRs) is expressed as a fraction or percentage of the one or more methylation loci (e.g., the one or more DMRs) present in a sample that are methylated, e.g., as a fraction of the number of individual DNA strands of DNA in a sample that are methylated at one or more particular methylation loci (e.g., one or more particular DMRs). Those of skill in the art will appreciate that, in some instances, the fraction or percentage of methylation can be calculated from the ratio of methylated DMRs to unmethylated DMRs for one or more analyzed DMRs, e.g., within a sample.

In various embodiments, methylation status of one or more methylation loci (e.g., one or more DMRs) is compared to a reference methylation status value and/or to methylation status of the one or more methylation loci (e.g., one or more DMRs) in a reference sample or a group of reference samples. For example, in certain embodiments, the group of reference samples is a plurality of samples obtained from individuals where said samples are known to represent a particular state (e.g., a "normal" non-cancer state, or a cancer state). In certain instances, a reference is a non-contemporaneous sample from the same source, e.g., a prior sample from the same source, e.g., from the same subject. In certain instances, a reference for the methylation status of one or more methylation loci (e.g., one or more DMRs) is the methylation status of the one or more methylation loci (e.g., one or more DMRs) in a sample (e.g., a sample from a subject), or a plurality of samples, known to represent a particular state (e.g., a cancer state or a non-cancer state). Thus, a reference can be or include one or more predetermined thresholds, which thresholds can be quantitative (e.g., a methylation value) or qualitative. Those of skill in the art will appreciate that a reference measurement is typically produced by measurement using a methodology identical to, similar to, or comparable to that by which the non-reference measurement was taken.

In various embodiments, methylation status of one or more methylation loci (e.g., one or more DMRs) is compared to a reference methylation status value and/or to methylation status of the one or more methylation loci (e.g., one or more DMRs) in a reference sample. In certain instances, a reference is a non-contemporaneous sample from the same source, e.g., a prior sample from the same source, e.g., from the same subject. In certain instances, a reference for the methylation status of one or more methylation loci (e.g., one or more DMRs) is the methylation status of the one or more methylation loci (e.g., one or more DMRs) in a sample (e.g., a sample from a subject), or a plurality of samples, known to represent a particular state (e.g., a cancer state or a non-cancer state). Thus, a reference can be or include one or more predetermined thresholds, which thresholds can be quantitative (e.g., a methylation value) or qualitative. Those of skill in the art will appreciate that a reference measurement is typically produced by measurement using a methodology identical to, similar to, or comparable to that by which the non-reference measurement was taken.

In various embodiments, a methylation status of a methylation loci may be based on methylation of one or more reads (e.g., obtained using a NGS technique) mapped to the methylation loci. For example, when analyzing sequencing data obtained from a sequencing technique, e.g., a NGS sequencing technique, e.g., a targeted NGS sequencing technique, sequencing data may include an inferred or probabilistic sequence of base pairs of a DNA fragment. The inferred or probabilistic sequence of base pairs of the DNA fragment is known as a read. The read may be mapped to a methylation loci (e.g., a DMR, a mutation marker) reference sequence, for example, in a genome (e.g., a reference genome, e.g., a reference bisulfite converted genome). Based on a comparison of the read sequence to a reference sequence, individual CpGs or cytosine residues may be identified as being hypermethylated or hypomethylated as compared to a reference state. In certain embodiments, a read-wise methylation value (e.g., a read-wise methylation score) is determined for a read, based pre-determined minimal thresholds that takes into account a number of methylation sites (e.g., CpGs) and a percentage of methylation In certain embodiments, a read-wise methylation value is a binary value.

Advanced Adenomas

In certain embodiments, methods and compositions presented herein are useful for screening for advanced adenomas. Advanced adenomas include, without limitation: neoplastic adenomatous growth in colon and/or in rectum, adenomas located in the proximal part of the colon, adenomas located in the distal part of the colon and/or rectum, adenomas of low grade dysplasia, adenomas of high grade dysplasia, neoplastic growth(s) of colorectum tissue that shows signs of high grade dysplasia of any size, neoplastic growth(s) of colorectum tissue having a size greater than or equal to 10mm of any histology and/or dysplasia grade, neoplastic growth(s) of colorectum tissue with villious histological type of any type of dysplasia and any size, and colorectum tissue having a serrated histological type with any dysplasia grade and/or size.

Colorectal Cancers

In certain embodiments, methods and compositions of the present disclosure are useful for screening for colorectal cancer. Colorectal cancers include, without limitation, colon cancer, rectal cancer, and combinations thereof. Colorectal cancers include metastatic colorectal cancers and non-metastatic colorectal cancers. Colorectal cancers include cancer located in the proximal part of the colon cancer and cancer located in the distal part of the colon.

Colorectal cancers include colorectal cancers at any of the various possible stages known in the art, including, e.g., Stage I, Stage II, Stage III, and Stage IV colorectal cancers (e.g., stages 0, I, IIA, IIB, IIC, IIIA, IIIB, IIIC, IVA, IVB, and IVC). Colorectal cancers include all stages of the Tumor/Node/Metastasis (TNM) staging system. With respect to colorectal cancer, T can refer to whether the tumor grown into the wall of the colon or rectum, and if so by how many layers; N can refer to whether the tumor has spread to lymph nodes, and if so how many lymph nodes and where they are located; and M can refer to whether the cancer has spread to other parts of the body, and if so which parts and to what extent. Particular stages of T, N, and M are known in the art. T stages can include TX, T0, Tis, T1, T2, T3, T4a, and T4b; N stages can include NX, N0, N1a, N1b, N1c, N2a, and N2b; M stages can include M0, M1a, and M1b. Moreover, grades of colorectal cancer can include GX, G1, G2, G3, and G4. Various means of staging cancer, and colorectal cancer in particular, are well known in the art summarized, e.g., on the world wide web at cancer.net/cancer-types/colorectal-cancer/stages.

In certain instances, the present disclosure includes screening of early stage colorectal cancer. Early stage colorectal cancers can include, e.g., colorectal cancers localized within a subject, e.g., in that they have not yet spread to lymph nodes of the subject, e.g., lymph nodes near to the cancer (stage N0), and have not spread to distant sites (stage M0). Early stage cancers include colorectal cancers corresponding to, e.g., Stages 0 to IIC.

Thus, colorectal cancers of the present disclosure include, among other things, pre-malignant colorectal cancer and malignant colorectal cancer. Methods and compositions of the present disclosure are useful for screening of colorectal cancer in all of its forms and stages, including without limitation those named herein or otherwise known in the art, as well as all subsets thereof. Accordingly, the person of skill in art will appreciate that all references to colorectal cancer provided here include, without limitation, colorectal cancer in all of its forms and stages, including without limitation those named herein or otherwise known in the art, as well as all subsets thereof.

Subjects and Samples

A sample analyzed using methods and compositions provided herein can be any biological sample and/or any sample including nucleic acids. In various particular embodiments, a sample analyzed using methods and compositions provided herein can be a sample from a mammal. In various particular embodiments, a sample analyzed using methods and compositions provided herein can be a sample from a human subject. In various particular embodiments, a sample analyzed using methods and compositions provided herein can be a sample form a mouse, rat, pig, horse, chicken, or cow.

In various instances, a human subject is a subject diagnosed or seeking diagnosis as having, diagnosed as or seeking diagnosis as at risk of having, and/or diagnosed as or seeking diagnosis as at immediate risk of having, a colorectal neoplasm (e.g., colorectal cancer, advanced adenoma). In various instances, a human subject is a subjected identified as a subject in need of screening for a colorectal neoplasm (e.g., colorectal cancer, advanced adenoma). In certain instances, a human subject is a subject identified as in need of colorectal cancer screening by a medical practitioner. In various instances, a human subject is identified as in need of colorectal cancer screening due to age, e.g., due to an age equal to or greater than 40 years, e.g., an age equal to or greater than 49, 45, 50, 55, 60, 65, 70, 75, 80, 85, or 90 years, though in some instances a subject 18 years old or older may be identified as at risk and/or in need of screening for a colorectal neoplasm (e.g., colorectal cancer, advanced adenoma). In various instances, a human subject is identified as being high risk and/or in need of screening for a colorectal neoplasm (e.g., colorectal cancer, advanced adenoma) based on, without limitation, familial history, prior diagnoses, and/or an evaluation by a medical practitioner. In various instances, a human subject is a subject not diagnosed as having, not at risk of having, not at immediate risk of having, not diagnosed as having, and/or not seeking diagnosis for a cancer such as a colorectal cancer, or any combination thereof.

A sample from a subject, e.g., a human or other mammalian subject, can be a sample of, e.g., blood, blood component (e.g., plasma, buffy coat), cfDNA (cell free DNA), ctDNA (circulating tumor DNA), stool, or tissue (e.g., advanced adenoma and/or colorectal tissue). In some particular embodiments, a sample is an excretion or bodily fluid of a subject (e.g., stool, blood, plasma, lymph, or urine of a subject) or a tissue sample of a colorectal neoplasm, such as a colonic polyp, an advanced adenoma, and/or colorectal cancer. A sample from a subject can be a cell or tissue sample, e.g., a cell or tissue sample that is of a cancer or includes cancer cells, e.g., of a tumor or of a metastatic tissue. For example, the sample may include colorectal cells, polyp cells, or glandular cells. In various embodiments, a sample from a subject, e.g., a human or other mammalian subject, can be obtained by biopsy (e.g., colonoscopy resection, fine needle aspiration or tissue biopsy) or surgery.

In various particular embodiments, a sample is a sample of cell-free DNA (cfDNA). cfDNA is typically found in biological fluids (e.g., plasma, serum, or urine) in short, double-stranded fragments. The concentration of cfDNA is typically low, but can significantly increase under particular conditions, including without limitation pregnancy, autoimmune disorder, myocardial infraction, and cancer. Circulating tumor DNA (ctDNA) is the component of circulating DNA specifically derived from cancer cells. ctDNA can be present in human fluids. For example in some instances, ctDNA can be found bound to and/or associated with leukocytes and erythrocytes. In some instances, ctDNA can be found not bound to and/or associated with leukocytes and erythrocytes. Various tests for detection of tumor-derived cfDNA are based on detection of genetic or epigenetic modifications that are characteristic of cancer (e.g., of a relevant cancer). Genetic or epigenetic modifications characteristic of cancer can include, without limitation, oncogenic or cancer-associated mutations in tumor-suppressor genes, activated oncogenes, hypermethylation, and/or chromosomal disorders. Detection of genetic or epigenetic modifications characteristic of cancer or pre-cancer can confirm that detected cfDNA is ctDNA.

cfDNA and ctDNA provide a real-time or nearly real-time metric of the methylation status of a source tissue. cfDNA and ctDNA have a half-life in blood of about 2 hours, such that a sample taken at a given time provides a relatively timely reflection of the status of a source tissue.

Various methods of isolating nucleic acids from a sample (e.g., of isolating cfDNA from blood or plasma) are known in the art. Nucleic acids can be isolated, e.g., without limitation, standard DNA purification techniques, by direct gene capture (e.g., by clarification of a sample to remove assay-inhibiting agents and capturing a target nucleic acid, if present, from the clarified sample with a capture agent to produce a capture complex, and isolating the capture complex to recover the target nucleic acid).

In certain embodiments, a sample may have a required minimum amount of DNA (e.g., cfDNA, gDNA) (e.g.,DNA fragments) for later determining a methylation status. For example, in certain embodiments, a sample may be required to have at least 5 ng, at least 10 ng, at least 20 ng (or more) DNA.

Methods of measuring methylation status

Methylation status can be measured by a variety of methods known in the art and/or by methods provided in this specification. Those of skill in the art will appreciate that a method for measuring methylation status can generally be applied to samples from any source and of any kind, and will further be aware of processing steps available to modify a sample into a form suitable for measurement by a given methodology.

In certain embodiments, the processing steps involve fragmenting or shearing DNA of the sample. For example, genomic DNA (e.g., gDNA) obtained from a cell, tissue, or other source may require fragmentation prior to sequencing. In certain embodiments, DNA may be fragmented prior to measurement of methylation status using a physical method (e.g., using an ultra-sonicator, a nebulizer technique, hydrodynamic shearing, etc.). In certain embodiments, DNA may be fragmented using an enzymatic method (e.g., using an endonuclease or a transposase). Certain samples, e.g., cfDNA samples, may not require fragmentation. cfDNA fragments are about 200bp in length and may be appropriate for certain methods provided herein. DNA fragments of about 100-1000 bp in length are suitable for analysis in certain NGS techniques described herein including, for example, Illumina® based techniques. Certain technologies may require DNA fragments of about 100-1000bp range. In contrast, DNA fragments of about 10kb or longer are suitable for long read sequencing technologies.

Methods of measuring methylation status include, without limitation, methods including whole genome bisulfite sequencing, targeted bisulfite sequencing, targeted enzymatic methylation sequencing, methylation-status-specific polymerase chain reaction (PCR), methods including mass spectrometry, methylation arrays, methods including methylation-specific nucleases, methods including mass-based separation, methods including target-specific capture (e.g., hybrid capture), and methods including methylation-specific oligonucleotide primers. Certain particular assays for methylation utilize a bisulfite reagent (e.g., hydrogen sulfite ions) or enzymatic conversion reagents (e.g., Tet methylcytosine dioxygenase 2).

Bisulfite reagents can include, among other things, bisulfite, disulfite, hydrogen sulfite, sodium metabisulphite, or combinations thereof, which reagents can be useful in distinguishing methylated and unmethylated nucleic acids. Bisulfite interacts differently with cytosine and 5-methylcytosine. In typical bisulfite-based methods, contacting of DNA (e.g., single stranded DNA, double stranded DNA) with bisulfite deaminates (e.g., converts) unmethylated cytosine to uracil, while methylated cytosine remains unaffected. Methylated cytosines, but not unmethylated cytosines, are selectively retained. Thus, in a bisulfite processed sample, uracil residues stand in place of, and thus provide an identifying signal for, unmethylated cytosine residues, while remaining (methylated) cytosine residues thus provide an identifying signal for methylated cytosine residues. Bisulfite processed samples can be analyzed, e.g., by next generation sequencing (NGS) or other methods disclosed herein.

In some embodiments, bisulfite processed samples may be treated using a bisulfite ratio of bisulfite to DNA that is at least. In certain embodiments, the bisulfite processed sample comprises single stranded DNA fragments or double stranded DNA fragments.

In some embodiments, bisulfite treatment includes subjecting DNA fragments (e.g., double stranded DNA) to one or more denaturation-conversion cycles in order to convert unmethylation cytosines to uracils in the DNA fragments. Denaturation converts double stranded DNA fragments in the sample to single stranded DNA fragments. Conversion changes the unmethylated cytosines of the single stranded DNA into uracils. In some embodiments, only one denaturation-conversion cycle are performed. In some embodiments, two, three, four, five, six, seven, eight, nine, ten, fifteen, twenty, or more denaturation-conversion cycles are performed. In some embodiments, the temperature of the denaturation step is performed at a temperature of about 80-100°C (e.g., about 90-97°C, e.g., about 96°C). In some embodiments, the denaturation step is performed for less than 10 minutes (e.g., less than 5 minutes, less than 5 minutes, less than 2 minutes, or less). In certain embodiments, the conversion step is performed for less than 2.5 hr (e.g., less than 2 hr, less than 1 hr, less than 30 minutes, less than 15 minutes, or less). In certain embodiments, the conversion step is performed at a temperature of 55 to 65°C. In certain embodiments, the converted DNA fragments may be stored at a temperature of about 4°C after performing the denaturation-conversion cycle(s). In some embodiments, bisulfite treatment may be applied prior to library preparation. In some embodiments, bisfulfite treatment may be applied after library preparation.

Enzymatic conversion reagents can include Tet methylcytosine dioxygenase 2 (TET2). TET2 oxidizes 5-methylcytosine and thus protects it from the consecutive deamination by APOBEC. APOBEC deaminates unmethylated cytosine to uracil, while oxidized 5-methylcytosine remains unaffected. Thus, in a TET2 processed sample, uracil residues stand in place of, and thus provide an identifying signal for, unmethylated cytosine residues, while remaining (methylated) cytosine residues thus provide an identifying signal for methylated cytosine residues. TET2 processed samples can be analyzed, e.g., by next generation sequencing (NGS). In certain embodiments, APOBEC refers to a member (or plurality of members) of the Apolipoprotein B mRNA Editing Catalytic Polypeptide-like (APOBEC) family. In certain embodiments, APOBEC may refer to APOBEC-1, APOBEC-2, APOBEC-3A, APOBEC-3B, APOBEC-3C, APOBEC-3D, APOBEC-3E, APOBEC-3F, APOBEC-3G. APOBEC-3H, APOBEC-4, and/or Activation-induced (cytidine) deaminase (AID).

Methods of measuring methylation status can include, without limitation, massively parallel sequencing (e.g., next-generation sequencing) to determine methylation state, e.g., sequencing by- synthesis, real-time (e.g., single-molecule) sequencing, bead emulsion sequencing, nanopore sequencing, or other sequencing techniques known in the art. In some embodiments, a method of measuring methylation status can include whole-genome sequencing, e.g., measuring whole genome methylation status from bisulfite or enzymatically treated material with base-pair resolution.

In some embodiments, a method of measuring methylation status includes reduced representation bisulfite sequencing e.g., utilizing use of restriction enzymes to measure methylation status of high CpG content regions from bisulfite or enzymatically treated material with base-pair resolution.

In some embodiments, a method of measuring methylation status can include targeted sequencing e.g., measuring methylation status of pre-selected genomic location from bisulfite or enzymatically treated material with base-pair resolution.

In some embodiments, the pre-selection (capture) (e.g., enrichment) of regions of interest (e.g., DMRs) can be done by complementary *in vitro* synthesized oligonucleotide sequences (e.g., capture baits/probes). Capture probes (e.g., oligonucleotide capture probes, oligonucleotide capture baits) are useful in targeted sequencing (e.g., NGS) techniques to enrich for particular regions of interest in an oligonucleotide (e.g., DNA) sequence. For example, enrichment of target regions is useful when sequences of particular pre-determined regions of DNA are sequenced. In certain embodiments, capture probes are about 10 to 1000bp long (e.g., about 10 to about 200bp long) (e.g., about 120bp long). In certain embodiments, one or more capture probes are targeted to capture a region of interest (e.g., a genomic marker) corresponding to one or more methylation loci (e.g., methylation loci comprising at least a portion of one or more DMRs, e.g., as found in FIG. 2 and/or FIG. 3). In certain embodiments, capture probes are targeted to methylation loci that are hypomethylated or hypermethylated. For example, a capture probe may be targeted to a particular methylation loci. However, if fragments of DNA corresponding to a methylation loci are converted (e.g., bisulfite or enzymatic converted) prior to enrichment using a capture probe, the sequence of the converted DNA fragments will change as described herein due to particular cytosine residues being unmethylated. Therefore, targeting an unconverted DNA region may result in some mismatches if cytosines are hypomethylated. Though capture probe-target sequence hybridization may tolerate some mismatches, a second probe may be required to enrich for DNA regions which are hypomethylated.

In certain embodiments, capture probes are evaluated (e.g., prior to sequencing) for their ability to target multiple regions of the genome of interest. For example, when designing a capture probe to target a particular region of interest (e.g., a DMR), the ability for a capture probe to target multiple regions of the genome may be considered. As discussed herein, mismatches in pairing (e.g., non-Watson-Crick pairing) allow for capture probes to hybridize to other, unintended regions of a genome. In addition, a particular target sequence may be repeated elsewhere in a genome. Repeat sequences are common for sequences that are highly repetitive. In certain embodiments, capture probes are designed such that they only target a few similar regions of the genome. In certain embodiments, capture probes may hybridize to 500 or fewer, 100 or fewer, 50 or fewer, 10 or fewer, 5 or fewer similar regions in a genome. In certain embodiments, a similar region to the target of region of interest is calculated using a 24bp window moving around a genome and matching the region of the window to a reference sequence according to sequence order similarity. Other size windows and/or techniques may be used.

For example, hybrid-capture of one or more DNA fragments (e.g., ctDNA, fragmented gDNA) may be performed using capture probes targeted to predetermined regions of interested of a genome. In certain embodiments, capture probes target at least 2 (e.g, at least 3, 4, 5, 6, 7, 8, 9, 10, 11, 12, 13, 14, 15, 16, 17, 18, 19, 20, 21, 22, 23, 24, 25, 50, 75, 100, 150, or more) predetermined regions of interest (e.g., genomic markers, e.g., DMRs). In certain embodiments, the capture probes overlap. In certain embodiments, the overlapping probes overlap at least 10%, at least 20%, at least 30%, at least 40%, at least 50%, at least 60% or more.

In certain embodiments, the capture probes are nucleic acid probes (e.g., DNA probes, RNA probes). In some embodiments, a method may also include identifying mutated regions (e.g., individual nucleotide bases) using targeted sequencing e.g., determining the presence of a mutation in one or more pre-selected genomic locations (e.g., a genomic marker, e.g., a mutation marker). In certain embodiments, mutations may also be identified from bisulfite or enzymatically treated DNA with base-pair resolution.

In some embodiments, a method for measuring methylation status can include Illumina Methylation Assays e.g., measuring over 850,000 methylation sites quantitatively across a genome at single-nucleotide resolution.

Various methylation assay procedures can be used in conjunction with bisulfite treatment to determine methylation status of a target sequence such as a DMR. Such assays can include, among others, Methylation-Specific Restriction Enzyme qPCR, sequencing of bisulfite-treated nucleic acid, PCR (e.g., with sequence-specific amplification), Methylation Specific Nuclease-assisted Minor-allele Enrichment PCR, and Methylation-Sensitive High Resolution Melting. In some embodiments, DMRs are amplified from converted (e.g., bisulfite or enzyme converted) DNA fragments for library preparation.

In some embodiments, a sequencing library may be prepared using converted (e.g., bisulfite or enzyme converted) oligonucleotide fragments (e.g., cfDNA, gDNA fragments, synthetic nucleotide sequences, etc.) according to, e.g., an Illumina protocol, an Accel-NGS® Methyl-Seq DNA Library Kit (Swift Bioscience) protocol, a transpose-based Nextera XT protocol, or the like. In some embodiments, the oligonucleotide fragments are DNA fragments which have been converted (e.g., bisulfite or enzyme converted). In certain embodiments, DNA fragments used in preparation of a sequencing library may be single stranded DNA fragments or double stranded DNA fragments. In certain embodiments, a library may be prepared by attaching adapters to DNA fragments. Adapters contain short (e.g., about 100 to about 1000bp) sequences (e.g., oligonucleotide sequences) that allow oligonucleotide fragments of a library (e.g., a DNA library) to bind to and generate clusters on a flow cell used in, for example, next generation sequencing (NGS). Adapters may be ligated to library fragments prior to NGS. In certain embodiments, a ligase enzyme covalently links the adapter and library fragments. In certain embodiments, adapters are attached to either one or both of the 5' and 3' ends of converted DNA fragments. In certain embodiments, the attaching step is performed such that at least 40%, at least 50%, at least 60%, at least 70% of the converted DNA fragments are attached to adapter. In certain embodiments, the attaching step is performed such that at least 40%, at least 50%, at least 60%, at least 70% of the converted DNA fragments have an adapter attached at both the 5' and 3' ends

In certain embodiments, adapters used herein contain a sequence of oligonucleotides that aid in sample identification. For example, in certain embodiments, adapters include a sample index. A sample index is a short sequence (e.g., about 8 to about 10 bases) of nucleic acids (e.g., DNA, RNA) that serve as sample identifiers and allow for, among other things, multiplexing and/or pooling of multiple samples in a single sequencing run and/or on a flow cell (e.g., used in a NGS technique). In certain embodiments, an adapter at a 5' end, a 3' end, or both of a converted single stranded DNA fragment includes a sample index. In certain embodiments, an adapter sequence may include a molecular barcode. A molecular barcode may serve as a unique molecular identifier to identify a target molecule during, for example, DNA sequencing. In certain embodiments, DNA barcodes may be randomly generated. In certain embodiments, DNA barcodes may be predetermined or predesigned. In certain embodiments, the DNA barcodes are different on each DNA fragment. In certain embodiments, the DNA barcodes may be the same for two single stranded DNA fragments that are not complementary to one another (e.g., in a Watson-Crick pair with each other) in the biological sample. In certain embodiments, DNA fragments may be amplified (e.g., using PCR) after ligation of adapters to DNA fragments. In certain embodiments, at least 40% (e.g., at least at least 50%, at least 60%, at least 70%) of the converted DNA fragments have an adapter attached at both the 5' and 3' ends.

In certain embodiments, high-throughput and/or next-generation sequencing (NGS) techniques are used to achieve base-pair level resolution of an oligonucleotide (e.g., a DNA) sequence, permitting analysis of methylation status and/or identification of mutations. For example, in certain embodiments, NGS may include single-end or paired-end sequencing. In single-end sequencing, a technique reads a sequenced fragment in one direction - from one end of a fragment to the opposite end of the fragment. In certain embodiments, this produces a single DNA sequence that then may be aligned to a reference sequence. In paired-end sequencing, a sequenced fragment is read in a first direction from one end of the fragment to the opposite end of the fragment. The sequenced fragment may be read until a specified read length is reached. Then, the sequenced fragment is read in a second direction, which is opposite to the first direction. In certain embodiments, having multiple read pairs may help to improve read alignment and/or identify mutations (e.g., insertions, deletions, inversion, etc.) that may not be detected by single-end reading.

Another method, that can be used for methylation detection includes PCR amplification with methylation-specific oligonucleotide primers (MSP methods), e.g., as applied to bisulfite-treated sample (see, e.g., Herman 1992 Proc. Natl. Acad. Sci. USA 93: 9821-9826,). Use of methylation-status-specific oligonucleotide primers for amplification of bisulfite-treated DNA allows differentiation between methylated and unmethylated nucleic acids. Oligonucleotide primer pairs for use in MSP methods include at least one oligonucleotide primer capable of hybridizing with sequence that includes a methylation site, e.g., a CpG site. An oligonucleotide primer that includes a T residue at a position complementary to a cytosine residue will selectively hybridize to templates in which the cytosine was unmethylated prior to bisulfite treatment, while an oligonucleotide primer that includes a G residue at a position complementary to a cytosine residue will selectively hybridize to templates in which the cytosine was methylated cytosine prior to bisulfite treatment. MSP results can be obtained with or without sequencing amplicons, e.g., using gel electrophoresis. MSP (methylation-specific PCR) allows for highly sensitive detection (detection level of 0.1% of the alleles, with full specificity) of locus-specific DNA methylation, using PCR amplification of bisulfite-converted DNA.

Another method that can be used to determine methylation status after bisulfite treatment of a sample is Methylation-Sensitive High Resolution Melting (MS-HRM) PCR (see, e.g., Hussmann 2018 Methods Mol Biol. 1708:551-571). MS-HRM is an intube, PCR-based method to detect methylation levels at specific loci of interest based on hybridization melting. Bisulfite treatment of the DNA prior to performing MS-HRM ensures a different base composition between methylated and unmethylated DNA, which is used to separate the resulting amplicons by high resolution melting. A unique primer design facilitates a high sensitivity of the assays enabling detection of down to 0.1-1% methylated alleles in an unmethylated background. Oligonucleotide primers for MS-HRM assays are designed to be complementary to the methylated allele, and a specific annealing temperature enables these primers to anneal both to the methylated and the unmethylated alleles thereby increasing the sensitivity of the assays.

Another method that can be used to determine methylation status after bisulfite treatment of a sample is Quantitative Multiplex Methylation-Specific PCR (QM-MSP). QM-MSP uses methylation specific primers for sensitive quantification of DNA methylation (see, e.g., Fackler 2018 Methods Mol Biol. 1708:473-496). QM-MSP is a two-step PCR approach, where in the first step, one pair of gene-specific primers (forward and reverse) amplifies the methylated and unmethylated copies of the same gene simultaneously and in multiplex, in one PCR reaction. This methylation-independent amplification step produces amplicons of up to $10^9$ copies per µL after 36 cycles of PCR. In the second step, the amplicons of the first reaction are quantified with a standard curve using real-time PCR and two independent fluorophores to detect methylated/unmethylated DNA of each gene in the same well (e.g., 6FAM and VIC). One methylated copy is detectable in 100,000 reference gene copies.

Another method that can be used to determine methylation status after bisulfite treatment of a sample is Methylation Specific Nuclease-assisted Minor-allele Enrichment (MS-NaME) (see, e.g., Liu 2017 Nucleic Acids Res. 45(6):e39). Ms-NaME is based on selective hybridization of probes to target sequences in the presence of DNA nuclease specific to double-stranded (ds) DNA (DSN), such that hybridization results in regions of double-stranded DNA that are subsequently digested by the DSN. Thus, oligonucleotide probes targeting unmethylated sequences generate local double stranded regions resulting to digestion of unmethylated targets; oligonucleotide probes capable of hybridizing to methylated sequences generate local double-stranded regions that result in digestion of methylated targets, leaving methylated targets intact. Moreover, oligonucleotide probes can direct DSN activity to multiple targets in bisulfite-treated DNA, simultaneously. Subsequent amplification can enrich non-digested sequences. Ms-NaME can be used, either independently or in combination with other techniques provided herein.

Another method that can be used to determine methylation status after bisulfite treatment of a sample is Methylation-sensitive Single Nucleotide Primer Extension (Ms-SNuPE™) (see, e.g., Gonzalgo 2007 Nat Protoc. 2(8):1931-6). In Ms-SNuPE, strand-specific PCR is performed to generate a DNA template for quantitative methylation analysis using Ms-SNuPE. SNuPE is then performed with oligonucleotide(s) designed to hybridize immediately upstream of the CpG site(s) being interrogated. Reaction products can be electrophoresed on polyacrylamide gels for visualization and quantitation by phosphor-image analysis. Amplicons can also carry a directly or indirectly detectable labels such as a fluorescent label, radionuclide, or a detachable molecule fragment or other entity having a mass that can be distinguished by mass spectrometry. Detection may be carried out and/or visualized by means of, e.g., matrix assisted laser desorption/ionization mass spectrometry (MALDI) or using electron spray mass spectrometry (ESI).

Certain methods that can be used to determine methylation status after bisulfite treatment of a sample utilize a first oligonucleotide primer, a second oligonucleotide primer, and an oligonucleotide probe in an amplification-based method. For instance, the oligonucleotide primers and probe can be used in a method of real-time polymerase chain reaction (PCR) or droplet digital PCR (ddPCR). In various instances, the first oligonucleotide primer, the second oligonucleotide primer, and/or the oligonucleotide probe selectively hybridize methylated DNA and/or unmethylated DNA, such that amplification or probe signal indicate methylation status of a sample.

Other bisulfite-based methods for detecting methylation status (e.g., the presence of level of 5-methylcytosine) are disclosed, e.g., in Frommer (1992 Proc Natl Acad Sci U S A. 1;89(5):1827-31).

In certain MSRE-qPCR embodiments, the amount of total DNA is measured in an aliquot of sample in native (e.g., undigested) form using, e.g., real-time PCR or digital PCR.

Various amplification technologies can be used alone or in conjunction with other techniques described herein for detection of methylation status. Those of skill in the art, having reviewed the present specification, will understand how to combine various amplification technologies known in the art and/or described herein together with various other technologies for methylation status determination known in the art and/or provided herein. Amplification technologies include, without limitation, PCR, e.g., quantitative PCR (qPCR), real-time PCR, and/or digital PCR. Those of skill in the art will appreciate that polymerase amplification can multiplex amplification of multiple targets in a single reaction. PCR amplicons are typically 100 to 2000 base pairs in length. In various instances, an amplification technology is sufficient to determine methylations status.

Digital PCR (dPCR) based methods involve dividing and distributing a sample across wells of a plate with 96-, 384-, or more wells, or in individual emulsion droplets (ddPCR) e.g., using a microfluidic device, such that some wells include one or more copies of template and others include no copies of template. Thus, the average number of template molecules per well is less than one prior to amplification. The number of wells in which amplification of template occurs provides a measure of template concentration.

If the sample has been contacted with MSRE, the number of wells in which amplification of template occurs provides a measure of the concentration of methylated template.

In various embodiments a fluorescence-based real-time PCR assay, such as MethyLight™, can be used to measure methylation status (see, e.g., Campan 2018 Methods Mol Biol. 1708:497-513). MethyLight is a quantitative, fluorescence-based, real-time PCR method to sensitively detect and quantify DNA methylation of candidate regions of the genome. MethyLight is uniquely suited for detecting low-frequency methylated DNA regions against a high background of unmethylated DNA, as it combines methylation-specific priming with methylation-specific fluorescent probing. Additionally, MethyLight can be combined with Digital PCR, for the highly sensitive detection of individual methylated molecules, with use in disease detection and screening.

Real-time PCR-based methods for use in determining methylation status typically include a step of generating a standard curve for unmethylated DNA based on analysis of external standards. A standard curve can be constructed from at least two points and can permit comparison of a real-time Ct value for digested DNA and/or a real-time Ct value for undigested DNA to known quantitative standards. In particular instances, sample Ct values can be determined for MSRE-digested and/or undigested samples or sample aliquots, and the genomic equivalents of DNA can be calculated from the standard curve. Ct values of MSRE-digested and undigested DNA can be evaluated to identify amplicons digested (e.g., efficiently digested; e.g., yielding a Ct value of 45). Amplicons not amplified under either digested or undigested conditions can also be identified. Corrected Ct values for amplicons of interest can then be directly compared across conditions to establish relative differences in methylation status between conditions. Alternatively or additionally, delta-difference between the Ct values of digested and undigested DNA can be used to establish relative differences in methylation status between conditions.

In certain particular embodiments, targeted bisulfite sequencing (e.g., using hybrid capture) among other techniques, can be used to determine the methylation status of a methylation biomarker for a disease and/or condition. For example, a colorectal neoplasm (e.g., advanced adenoma and/or colorectal cancer) methylation biomarker that is or includes a single methylation locus. In certain particular embodiments, targeted bisulfite sequencing, among other techniques, can be used to determine the methylation status of a methylation biomarker that is or includes two or more methylation loci.

Those of skill in the art will appreciate that in embodiments in which a plurality of methylation loci (e.g., a plurality of DMRs) are analyzed for methylation status in a method of screening for colorectal cancer provided herein, methylation status of each methylation locus can be measured or represented in any of a variety of forms, and the methylation statuses of a plurality of methylation loci (preferably each measured and/or represented in a same, similar, or comparable manner) be together or cumulatively analyzed or represented in any of a variety of forms. In various embodiments, methylation status of each methylation locus can be measured as methylation portion. In various embodiments, methylation status of each methylation locus can be represented as the percentage value of methylated reads from total sequencing reads compared against reference sample. In various embodiments, methylation status of each methylation locus can be represented as a qualitative comparison to a reference, e.g., by identification of each methylation locus as hypermethylated or hypomethylated. In some embodiments in which a single methylation locus is analyzed, hypermethylation of the single methylation locus constitutes a diagnosis that a subject is suffering from or possibly suffering from a condition (e.g., cancer) (e.g., advanced adenoma, colorectal cancer), while absence of hypermethylation of the single methylation locus constitutes a diagnosis that the subject is likely not suffering from a condition. In some embodiments, hypermethylation of a single methylation locus (e.g., a single DMR) of a plurality of analyzed methylation loci constitutes a diagnosis that a subject is suffering from or possibly suffering from the condition, while the absence of hypermethylation at any methylation locus of a plurality of analyzed methylation loci constitutes a diagnosis that a subject is likely not suffering from the condition. In some embodiments, hypermethylation of a determined percentage (e.g., a predetermined percentage) of methylation loci (e.g., at least 10% (e.g., at least 10%, at least 20%, at least 30%, at least 40%, at least 50%, at least 60%, at least 70%, at least 80%, at least 90%, or 100%)) of a plurality of analyzed methylation loci constitutes a diagnosis that a subject is suffering from or possibly suffering from the condition, while the absence of hypermethylation of a determined percentage (e.g., a predetermined percentage) of methylation loci (e.g., at least 10% (e.g., at least 10%, at least 20%, at least 30%, at least 40%, at least 50%, at least 60%, at least 70%, at least 80%, at least 90%, or 100%)) of a plurality of analyzed methylation loci constitutes a diagnosis that a subject is not likely suffering from the condition. In some embodiments, hypermethylation of a determined number (e.g., a predetermined number) of methylation loci (e.g., at least 1, 2, 3, 4, 5, 6, 7, 8, 9, 10, 11, 12, 13, 14, 15, 16, 17, 18, 19, 20, 21, 22, 23, 24, 25, 26, 50, 100, 150, or more DMRs) of a plurality of analyzed methylation loci (e.g 2, 3, 4, 5, 6, 7, 8, 9, 10, 11, 12, 13, 14, 15, 16, 17, 18, 19, 20, 21, 22, 23, 24, 25, 26, 50, 100, 150, or more DMRs) constitutes a diagnosis that a subject is suffering from or possibly suffering from the condition, while the absence of hypermethylation of a determined number (e.g., a predetermined number) of methylation loci (e.g., at least 1, 2, 3, 4, 5, 6, 7, 8, 9, 10, 11, 12, 13, 14, 15, 16, 17, 18, 19, 20, 21, 22, 23, 24, 25, 26, 50, 100, 150, or more DMRs) of a plurality of analyzed methylation loci (e.g., 2, 3, 4, 5, 6, 7, 8, 9, 10, 11, 12, 13, 14, 15, 16, 17, 18, 19, 20, 21, 22, 23, 24, 25, 26, 50, 100, 150, or more DMRs) constitutes a diagnosis that a subject is not likely suffering from the condition.

In some embodiments, methylation status of a plurality of methylation loci (e.g., a plurality of DMRs) is measured qualitatively or quantitatively and the measurement for each of the plurality of methylation loci are combined to provide a diagnosis. In some embodiments, the quantitatively measured methylation status of each of a plurality of methylation loci is individually weighted, and weighted values are combined to provide a single value that can be comparative to a reference in order to provide a diagnosis.

In some embodiments, methylation status may include determination of methylated and/or unmethylated reads mapped to a genomic region (e.g., a DMR). For example, when using particular sequencing technologies as disclosed herein (e.g., NGS, whole genome bisulfite sequencing, etc.), sequence reads are produced. A sequence read is an inferred sequence of base pairs (e.g., a probabilistic sequence) corresponding to all or part of a sequenced oligonucleotide (e.g., DNA) fragment (e.g., cfDNA fragments, gDNA fragments). In certain embodiments, sequence reads may be mapped (e.g., aligned) to a particular region of interest using a reference sequence (e.g., a bisulfite converted reference sequence) in order to determine if there are any alterations or variations in a read. Alterations may include methylation and/or mutations. A region of interest may include one or more genomic markers including a methylation marker (e.g., a DMR), a mutation marker, or other marker as disclosed herein.

For example, in the case of bisulfite or enzymatically treated DNA fragments, treatment converts unmethylated cytosines to uracils, while methylated cytosines are not converted to uracils. Accordingly, a sequence read produced for a DNA fragment that has methylated cytosines will be different from a sequence read produced for the same DNA fragment that does not have methylated cytosine. Methylation at sites where a cytosine nucleotide is followed by a guanine nucleotide (e.g., CpG sites) may be of particular interest.

Quality Control Protocol

In certain embodiments, quality control steps may be implemented. Quality control steps are used to determine whether or not particular steps or processes were conducted within particular parameters. In certain embodiments, quality control steps may be used to determine the validity of results of a given analysis. In addition or alternatively, quality control steps may be used to determine sequenced data quality. For example, quality control steps may be used to determine read coverage of one or more regions of DNA. Quantitative metrics for quality control include, but are not limited to AT dropout rate, GC dropout rate, bisulfite conversion rate (e.g., bisulfite conversion efficiency), and the like. Failure to meet a threshold quality control condition (e.g., a minimum conversion rate, a maximum CG dropout rate, etc.) may indicate, for example, that one or more of the conversion steps were not performed within appropriate parameters.

For example, in the methods described herein, various steps of a conversion protocol may be optimized to decrease AT and/or GC dropout rate. As is understood by those of skill in the art, AT and GC dropout metrics indicate the degree of inadequate coverage of a particular target region based on its AT or GC content. In certain embodiments, samples having a low GC dropout rate is useful in identifying which samples were processed appropriately. For example, a GC dropout rate found to be less than 10%, less than 9%, less than 8%, less than 7%, less than 6%, less than 5%, less than 4%, or less may be useful in identifying appropriately processed samples.

In certain embodiments, a quality control step may involve determining an on and/or off-target ratio. Sequence reads that align to a region of interest (e.g., a DMR) are considered to be on-target, while sequence reads that do not align to the region of interest (e.g., a DMR) are considered to be off-target. In certain embodiments, the on-target ratio is represented as a percentage of on-target bases to the total number of aligned bases. In certain embodiments, the on-target ratio is represented as a percentage of on-target and near-target bases to the total number of aligned bases. Near-target bases may be a base within a certain number of bases (e.g., within 500bp, within 200bp, within 100bp) of the target region. In certain embodiments, the on-target ratio is at least 10%, least 20%, least 30%, least 40%, least 50%, least 60%, least 70%, at least 80%, at least 90%, at least 95%, at least 99% or more for a sequencing experiment to pass quality control. In certain embodiments, the off-target ratio is represented as a percentage of off-target bases to the total number of aligned bases. In certain embodiments, an off-target ratio is less than 95%, less than 90%, less than 85%, less than 80%, less than 70%, less than 60%, less than 50%, less than 40%, less than 30%, less than 20%, less than 10%, less than 1% for a sequencing experiment to pass quality control.

In certain embodiments, a quality control step may include determining quality scores for mapped sequence reads. A quality score is a value which quantifies a probability that a sequence read is incorrectly mapped. For example, when mapping short or repetitive sequences, it is possible that a sequence will be mapped to multiple places in a reference genome. The quality score considers the best alignment of the sequence read to the reference genome as compared to other possible alignments of the sequence read to the reference genome. In certain embodiments, the quality score is a Mapping Quality (MAPQ) score. The MAPQ is the negative, log-scaled probability that a read is misaligned. A high score indicates a high confidence that a read is aligned correctly, while a low score indicates a low confidence that a read is aligned correctly. In certain embodiments, the MAPQ score may be calculated using the following equation:

$$\text{MAPQ score} = -10 \log_{10} \Pr\{ \text{ mapping position is wrong } \}.$$

In certain embodiments, the MAPQ score is rounded to the nearest integer. In certain embodiments Pr is a probability that the sequence read is incorrectly mapped as obtained from an alignment (e.g., mapping) tool. In certain embodiments, the scaling factor is 1 (instead of 10), or another number.

Artificial Spike-in Control

Control nucleic acid (e.g., DNA) molecules (e.g., "spike-in controls") may be used to evaluate or estimate conversion efficiency of unmethylated and methylated cytosines to uracils. Control nucleic acid molecules may be used in sequencing methods involving conversion (e.g., bisulfite or enzymatic conversion) of DNA samples.

When DNA is subjected to conversion (e.g., bisulfite or enzymatic conversion) as described herein, conversion may be incomplete. That is, some number of unmethylated cytosines may not be converted to uracils. If the conversion is not complete such that unmethylated cytosines are not mostly converted, the unconverted unmethylated cytosines may be identified as methylated when the DNA sequenced. Accordingly, in order to determine whether or not bisulfite conversion is complete, a control DNA molecule may be subjected to conversion along with DNA fragments from a sample. In certain embodiments, sequencing the converted control DNA molecules (e.g., using an NGS technique as described herein) generates a plurality of control sequence reads. Control sequence reads may be used to determine conversion rates of unmethylated and/or methylated cytosines to uracils.

Prior techniques did not recognize that controls (e.g., a control DNA molecule) were useful to include in each sample. Rather, they presumed that conversion efficiencies remained relatively consistent between samples for a given run. However, the inventors have identified that conversion rate of unmethylated cytosines to uracils in DNA fragments may vary significantly from on sample to another. For example, conversion efficiency may range from 10% to 110% within a single batch of processed samples. Note, there can be overconversion such that conversion efficiency can be greater than 100%, e.g., the conversion efficiency is 110% when 10% of the methylated cytosine gets converted. In certain embodiments, the conversion efficiency ranges from 30% to 110%. In other embodiments, the conversion efficiency ranges from 50% to 100%.

In certain embodiments, a control DNA molecule may be added to a sample after fragmentation and before conversion using e.g., bisulfite or enzymatic reagents. In certain embodiments, a plurality (e.g., two, three, four or more) control DNA sequences may be added to DNA fragments of a sample. A control DNA molecule may be a known sequence. For example, the sequence, number of methylated bases, and number of unmethylated bases of the control sequence had been determined prior to addition of the control DNA molecule to the sample. In certain embodiments, a control sequence may be a DNA sequence which is produced *in vitro* to contain artificially methylated or unmethylated nucleotides (e.g., methylated cytosines). In certain embodiments, a control sequence may be a DNA sequence which is produced to contain completely unmethylated DNA nucleotides.

A high conversion efficiency of the spike-in control sequence may be used to infer the conversion efficiency of a DNA fragments undergoing the same conversion process as a spike-in control. For example, deamination of at least 98% of unmethylated cytosines in the unmethylated spike-in control DNA sequence indicates that conversion efficiency is high and that a sample may pass a quality control assessment. In certain embodiments, at least 90%, at least 95%, at least 96%, at least 97%, at least 98%, at least 99% of unmethylated cytosines of a plurality of DNA fragments of a control DNA sequence are converted into uracils. A high conversion efficiency is important as it is ideal for all (or nearly all) of the unmethylated cytosines to be converted to uracils when subjecting DNA to bisulfite or enzymatic treatments. As described above, unconverted, unmethylated cytosines may serve as a source of noise in the data.

In addition, conversion of methylated cytosines to uracils is undesirable when DNA is treated using a conversion process. Conversion of methylated cytosines of a spike-in control is indicative that methylated cytosines have been converted to uracils in a DNA sample subjected to the same treatment as the methylated spike-in control. Methylated cytosines in a methylated spike-in control should not convert to uracils. For the same reasons as described above, methylated cytosines being converted to uracils may result in misidentification of purportedly unmethylated cytosines during methylation analysis. In certain embodiments, at most 5%, at most 4%, at most 3%, at most 2% or at most 1% of methylated cytosines of a plurality of DNA fragments of a control DNA sequence are converted into uracils. For example, deamination of at most 2% of methylated cytosines in a methylated spike-in control DNA sequence indicates that conversion efficiency is high and that a sample may pass a quality control assessment.

Identifying Mutations

In certain embodiments as disclosed herein, genomic mutations may be identified in one or more predetermined mutation biomarkers. In various embodiments, a mutation biomarker of the present disclosure is used for further detection (e.g., screening) and/or classification of a condition in addition to methylation biomarkers. In certain embodiments, information regarding a methylation status of one or more colorectal cancer biomarkers may be combined with a mutation biomarker in order to further classify the identified colorectal cancer. In addition or alternatively, mutation biomarkers may be used to determine or recommend (e.g., either for or against) a particular course of treatment for the identified disease and/or condition.

In certain embodiments, identifying genomic mutations may be performed using a sequencing technique as discussed herein (e.g., a NGS sequencing technique). In certain embodiments, oligonucleotides (e.g., cfDNA fragments, gDNA fragments) are sequenced to a read depth sufficient to detect a genomic mutation (e.g., in a mutation biomarker, in a tumor markers) at a frequency in a sample as low as 1.0%, 0.75%, 0.5%, 0.25%, 0.1%, 0.075%, 0.05%, 0.025%, 0.01%, or 0.005%.

Genomic mutations generally include any variation in nucleotide base pair sequences of DNA as is understood in the art. A mutation in a nucleic acid may, in some embodiments, include a single nucleotide variant, an inversion, a deletion, an insertion, a transversion, a translocation, a fusion, a truncation, an amplification, or a combination thereof, as compared to a reference DNA sequence.

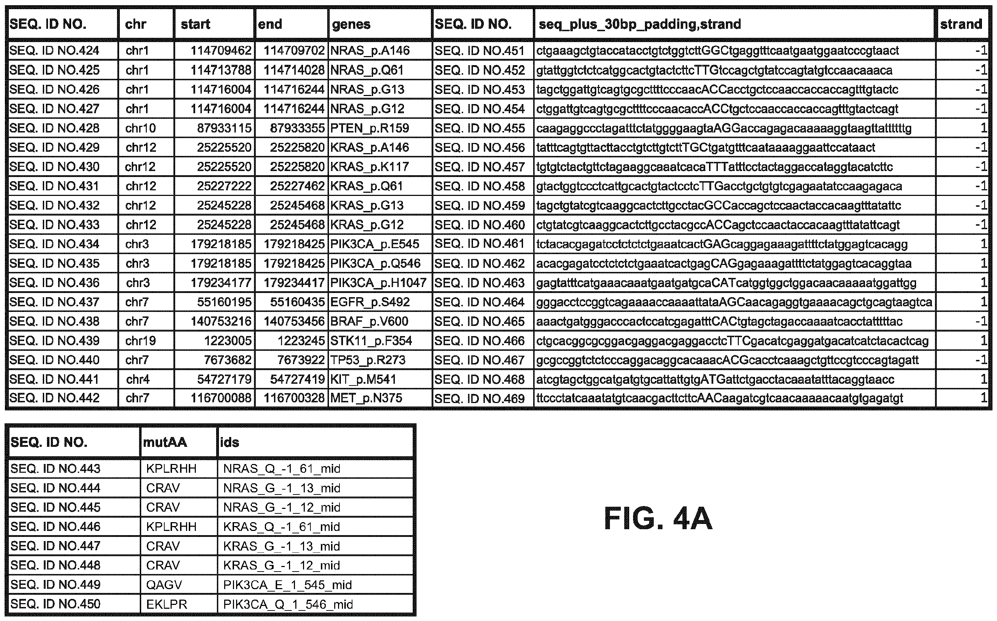

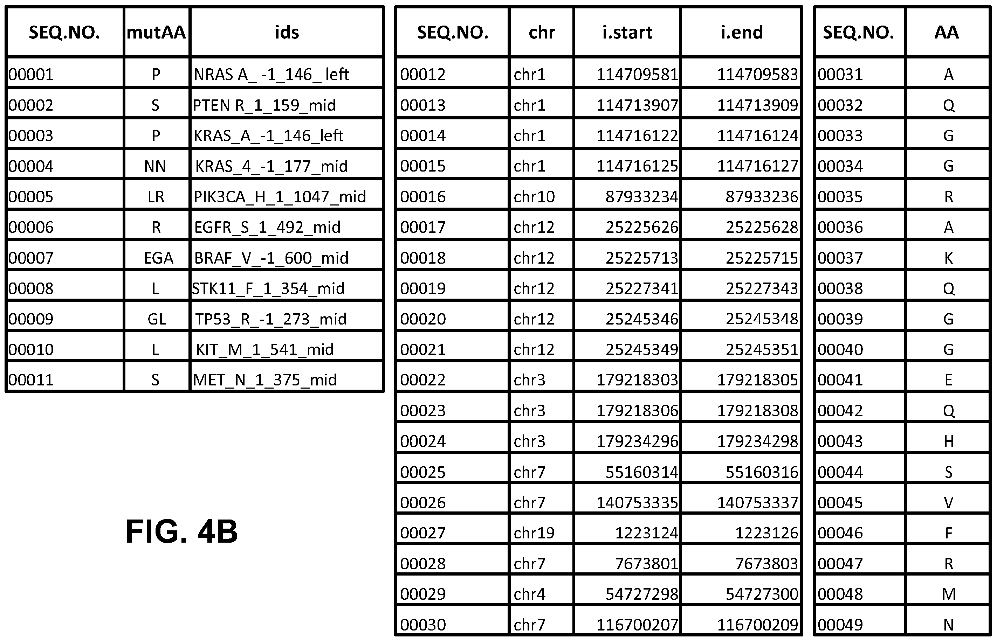

FIG. 4 lists regions of DNA in which useful genomic mutations are found. The DNA regions include the chromosome number (chr), start ("start") and end ("end") positions of a target region containing a mutation marker on a chromosome, and the gene names (e.g., NRAS, PTEN, KRAS, PIK3CA, EGFR, BRAF, STK22, TP53, KIT, and MET) in which the mutation markers are found along with an identifier for the codon at which the mutation has been identified. For the avoidance of doubt, each of the listed genes in FIG. 4 may also include other mutations not listed in FIG. 4.

By way of example, NRAS_p.A146, listed in the first row of FIG. 4, identifies that the gene NRAS has a mutation at codon 146. A region of DNA which is targeted by one or more probes is identified by the chromosome number 1 and the start and end positions 114709462 and 114709702, respectively. On the same chromosome, the start and end positions 114709581 ("i.start") and 114709583 ("i.end") correspond to the DNA sequence which encodes for the mutated codon. In the identified mutation, the amino acid alanine ("A") is altered to proline ("P") as is shown in FIG. 4. In the particular assay, the mutation is identified in the non-coding ("-1") strand of the DNA fragment. The provided sequence corresponds to the base pairs of the mutated codon, which are identified using capital letters. A buffer of 30 bp on either side of the codon is provided for further identification of the region.

Mutations may be identified using NGS sequencing techniques (e.g., targeted NGS sequencing techniques, hybridization NGS sequencing techniques, or the like) or other sequencing techniques disclosed herein. In certain embodiments as disclosed herein, mutations may be identified in converted (e.g., bisulfite or enzymatic converted) DNA fragments. In certain embodiments, mutations and methylated loci may be identified in parallel (e.g., simultaneously) using a single sequencing assay (e.g., an NGS assay). In certain embodiments, one or more capture probes are targeted to capture and/or enrich for a region of interest of an oligonucleotide (e.g., DNA) sequence corresponding to one or more mutations markers (e.g., mutation regions and sites as found in FIG. 4).

In certain embodiments, mutation markers contain low GC content regions. Due to the low GC content, sufficient coverage of a region may not be obtained when sequencing a low GC content region using protocols adapted for high GC content regions. For example, targeted NGS sequencing (e.g., targeted bisulfite sequencing) of a low GC content region using only 1x tiling density of a target region may not provide sufficient coverage of a mutation region. Tiling (e.g., tiling density, tiling frequency) refers to a number of probes targeted to a region. Increased probe tiling density (e.g., through increasing the number of probes targeting a region) may be used in order to provide additional coverage for a region. For example, coverage of a low GC content region may be improved through increased tiling. Accordingly, increasing tiling density of a region to at least 2x tiling (e.g., 3x, 4x or more) may be beneficial in enhancing enrichment of a targeted region.

For example, with 2x tiling, a region covered by a probe may be covered with at two probes which overlap with one another. In addition or alternatively, probes may be overlapped to permit enhanced coverage of a region. For example, probes may be overlapped by at least 10%, 20%, 30%, 40%, 50% or more. The amount which two probes overlap with one another may depend on desired tiling density, sequence of a targeted region, or other factors. For the avoidance of doubt, tiling and/or overlap of probes may also be changed over high GC content regions (e.g., methylation loci) as well.

Exemplary Deduplication Steps

In certain embodiments as discussed herein, duplicate sequences are found in sequencing data. Duplicate sequences arise from a number of potential sources as discussed herein, and accordingly may need to be removed from sequencing data. Duplicates are particularly important to remove in an analysis as signals from cancer are low. Cancer signals would get lost in noise if duplicates are not removed.

For example, in certain embodiments sequencing data may include a large number of reads obtained from sequencing oligonucleotide fragments (e.g., DNA fragments, e.g., cfDNA, gDNA fragments) of a sample. Multiple reads corresponding to a particular DNA fragment may result in false variant calls (e.g., identification of multiple variants of the same DNA fragment), which would interfere with the identification of a methylated CpG site and/or a mutation. In certain embodiments, duplicate sequences are removed prior to determining read-wise methylation values. In certain embodiments, a bioinformatics package (e.g., Picard, SAMTools) may be used to mark and remove duplicates from sequencing data.

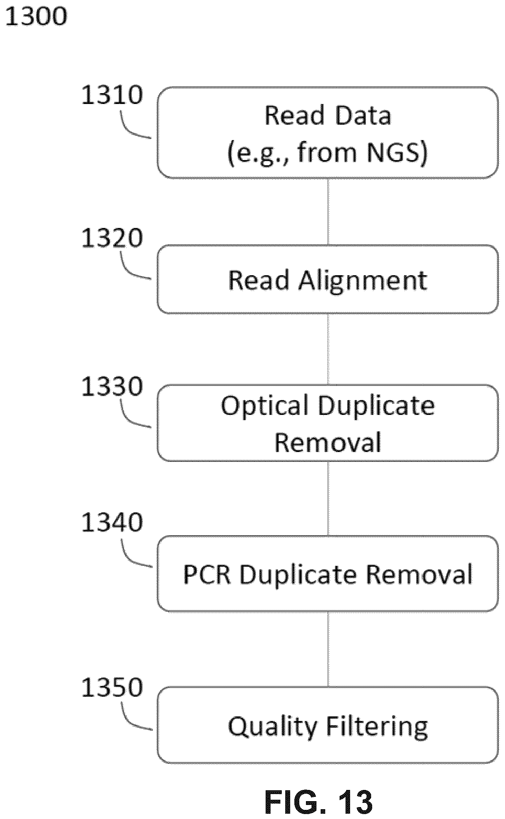

FIG. 13 shows a series of bioinformatics steps (1300) conducted on sequencing data to remove duplicate sequences, according to an illustrative embodiment. Read data acquired from, for example, a NGS sequencing technique is provided and/or acquired (e.g., as described herein) (1310). In certain embodiments, sequencing data is obtained from bisulfite or enzymatically converted DNA fragments as described herein. Reads obtained from the sequencing data may then be aligned to a reference sequence (1320). In certain embodiments, the reference sequence is a bisulfite converted genome (e.g., a bisulfite converted human genome). Reads that correspond to optical duplicates may then be removed (1330). Optical duplicates may arise during preparation of a sample for sequencing. Optical duplicates may be connected to flow cell type. For example, NGS sequencers with patterned flow cells have a problem of template hopping and thus duplication of clusters. Template hopping occurs when a sequence "hops" from one spot on a flow cell to another spot on the flow cell. Duplicate clusters may result in over-representation of particular reads in a data set. Duplicate clusters may also arise from a sensor of the NGS sequencer incorrectly identifying a single amplification cluster on a substrate (e.g., a flow cell) as multiple clusters. In certain embodiments, optical duplicates are identified when two reads share an identical sequence of bases. In certain embodiments, a read is called an optical duplicate if a pair of reads are both on the same tile (e.g., a spot on a flow cell used for NGS), and the distance between reads is less than 100bp (e.g., when using NExtSeq equipment) and 2500bp (e.g., when using NovaSeq equipment).

In certain embodiments, PCR duplicates (also known as library duplicates) and/or over-sequencing duplicates may also be removed (1340). PCR duplicates and over-sequencing duplicates are sequence reads that result from sequencing two or more copies of the exact same DNA fragment. PCR duplicates and over-sequencing duplicates may arise during library preparation. In certain embodiments, sequence reads are considered PCR duplicates or over-sequencing duplicates if the sequence reads have (1) a 5' end coordinate, (2) a 3' end coordinate, and (3) a methylation level that are the same, wherein the 5' end coordinate and the 3' end coordinate of a sequence read correspond to the position at which the 5'-most nucleotide and the 3'-most nucleotide, respectively, of the sequence read map to a reference sequence. Finally, the deduplicated reads are quality filtered (1350), which results in the removal of additional reads.

In certain embodiments, deduplicating sequence reads does not comprise removing duplicate sequence reads that have a different methylation level. For example, a sample may have two sequence reads that are identical. However, one sequence read may have a CpG site that is methylated, while the same CpG site in the other strand is not methylated. In certain embodiments, both strands may be kept for further bioinformatics analysis. Without wishing to be bound to any particular theory, a presence of different methylation levels within duplicate fragments may be due to sequencing errors or a different source of one fragment.

Applications

Methods and compositions of the present disclosure can be used in any of a variety of applications. For example, methods and compositions of the present disclosure can be used to screen, or aid in screening for a condition (e.g., cancer). In particular, the methods and compositions can be used to screen, or aid in screening for a colorectal neoplasm, e.g., advanced adenoma and/or colorectal cancer. In various instances, screening using methods and compositions of the present disclosure can detect any stage of colorectal cancer, including without limitation early-stage colorectal cancer. In some embodiments, screening using methods and compositions of the present disclosure is applied to individuals 40 years of age or older, e.g., 40, 45, 50, 55, 60, 65, 70, 75, 80, 85, or 90 years or older. In particular, individuals 40 years of age or older are of interest for colorectal cancer and/or advanced adenoma screening. In some embodiments, screening using methods and compositions of the present disclosure is applied to individuals 18 years of age or older, e.g., 18, 20, 25, 30, 35, 40, 45, 50, 55, 60, 65, 70, 75, 80, 85, or 90 years or older. In some embodiments, screening using methods and compositions of the present disclosure is applied to individuals 18 to 40 years of age. In various embodiments, screening using methods and compositions of the present disclosure is applied to individuals experiencing abdominal pain or discomfort, e.g., experiencing undiagnosed or incompletely diagnosed abdominal pain or discomfort. In various embodiments, screening using methods and compositions of the present disclosure is applied to individuals experiencing no symptoms likely to be associated with a cancer or a colorectal neoplasm such as advanced adenoma, polyposis, and/or colorectal cancer. Thus, in certain embodiments, screening using methods and compositions of the present disclosure is fully or partially preventative or prophylactic, at least with respect to later or non-early stages of cancer.

In various embodiments, cancer screening using methods and compositions of the present disclosure can be applied to an asymptomatic human subject. In particular, a subject can be referred to as "asymptomatic" if the subject does not report, and/or demonstrate by non-invasively observable indicia (e.g., without one, several, or all of device-based probing, tissue sample analysis, bodily fluid analysis, surgery, or cancer screening), sufficient characteristics of the condition to support a medically reasonable suspicion that the subject is likely suffering from the condition. Detection of a colorectal neoplasm such as advanced adenoma and/or early stage colorectal cancer is particularly likely in asymptomatic individuals screened in accordance with methods and compositions of the present disclosure.

Those of skill in the art will appreciate that regular, preventative, and/or prophylactic screening for a colorectal neoplasm such as advanced adenoma and/or colorectal cancer improves diagnosis. As noted above, early stage cancers include, according to at least one system of cancer staging, Stages 0 to II C of colorectal cancer. Thus, the present disclosure provides, among other things, methods and compositions particularly useful for the diagnosis and treatment of colorectal neoplasms including advanced adenoma, polyposis and/or early stage colorectal cancer. Generally, and particularly in embodiments in which screening in accordance with the present disclosure is carried out annually, and/or in which a subject is asymptomatic at time of screening, methods and compositions of the present invention are especially likely to detect early stage colorectal cancer.

In various embodiments colorectal cancer screening in accordance with the present disclosure is performed once for a given subject or multiple times for a given subject. In various embodiments, colorectal cancer screening in accordance with the present disclosure is performed on a regular basis, e.g., every six months, annually, every two years, every three years, every four years, every five years, or every ten years.

In various embodiments, screening using methods and compositions disclosed herein will provide a diagnosis of a condition (e.g., a type or class of a colorectal neoplasm). In other instances, screening for colorectal neoplasms using methods and compositions disclosed herein will be indicative of having one or more conditions, but not definitive for diagnosis of a particular condition. For example, screening may be used to classify a subject as having one or more conditions or combination of conditions including, but not limited to, advanced adenoma and/or colorectal cancer. In various instances, screening using methods and compositions of the present disclosure can be followed by a further diagnosis-confirmatory assay, which further assay can confirm, support, undermine, or reject a diagnosis resulting from prior screening, e.g., screening in accordance with the present disclosure.

In various embodiments, screening in accordance with methods and compositions of the present disclosure reduces colorectal cancer mortality, e.g., by early colorectal cancer diagnosis. Data supports that colorectal cancer screening reduces colorectal cancer mortality, which effect persisted for over 30 years (see, e.g., Shaukat 2013 N Engl J Med. 369(12):1106-14). Moreover, colorectal cancer is particularly difficult to treat at least in part because colorectal cancer, absent timely screening, may not be detected until cancer is past early stages. For at least this reason, treatment of colorectal cancer is often unsuccessful. To maximize population-wide improvement of colorectal cancer outcomes, utilization of screening in accordance with the present disclosure can be paired with, e.g., recruitment of eligible subjects to ensure widespread screening.

In various embodiments, screening of colorectal neoplasms including one or more methods and/or compositions disclosed herein is followed by treatment of colorectal cancer, e.g., treatment of early stage colorectal cancer. In various embodiments, treatment of colorectal cancer, e.g., early stage colorectal cancer, includes administration of a therapeutic regimen including one or more of surgery, radiation therapy, and chemotherapy. In various embodiments, treatment of colorectal cancer, e.g., early stage colorectal cancer, includes administration of a therapeutic regimen including one or more of treatments provided herein for treatment of stage 0 colorectal cancer, stage I colorectal cancer, and/or stage II colorectal cancer.

In various embodiments, treatment of colorectal cancer includes treatment of early stage colorectal cancer, e.g., stage 0 colorectal cancer or stage I colorectal cancer, by one or more of surgical removal of cancerous tissue e.g., by local excision (e.g., by colonoscope), partial colectomy, or complete colectomy.

In various embodiments, treatment of colorectal cancer includes treatment of early stage colorectal cancer, e.g., stage II colorectal cancer, by one or more of surgical removal of cancerous tissue (e.g., by local excision (e.g., by colonoscope), partial colectomy, or complete colectomy), surgery to remove lymph nodes near to identified colorectal cancer tissue, and chemotherapy (e.g., administration of one or more of 5-FU and leucovorin, oxaliplatin, or capecitabine).

In various embodiments, treatment of colorectal cancer includes treatment of stage III colorectal cancer, by one or more of surgical removal of cancerous tissue (e.g., by local excision (e.g., by colonoscopy-based excision), partial colectomy, or complete colectomy), surgical removal of lymph nodes near to identified colorectal cancer tissue, chemotherapy(e.g., administration of one or more of 5-FU, leucovorin, oxaliplatin, capecitabine, e.g., in a combination of (i) 5-FU and leucovorin, (ii) 5-FU, leucovorin, and oxaliplatin (e.g., FOLFOX), or (iii) capecitabine and oxaliplatin (e.g., CAPEOX)), and radiation therapy.

In various embodiments, treatment of colorectal cancer includes treatment of stage IV colorectal cancer, by one or more of surgical removal of cancerous tissue (e.g., by local excision (e.g., by colonoscope), partial colectomy, or complete colectomy), surgical removal of lymph nodes near to identified colorectal cancer tissue, surgical removal of metastases, chemotherapy (e.g., administration of one or more of 5-FU, leucovorin, oxaliplatin, capecitabine, irinotecan, VEGF- targeted therapeutic agent (e.g., bevacizumab, ziv-aflibercept, or ramucirumab), EGFR-targeted therapeutic agent (e.g., cetuximab or panitumumab), Regorafenib, trifluridine, and tipiracil, e.g., in a combination of or including (i) 5-FU and leucovorin, (ii) 5-FU, leucovorin, and oxaliplatin (e.g., FOLFOX), (iii) capecitabine and oxaliplatin (e.g., CAPEOX), (iv) leucovorin, 5-FU, oxaliplatin, and irinotecan (FOLFOXIRI), and (v) trifluridine and tipiracil (Lonsurf)), radiation therapy, hepatic artery infusion (e.g., if cancer has metastasized to liver), ablation of tumors, embolization of tumors, colon stent, colorectomy, colostomy (e.g., diverting colostomy), and immunotherapy (e.g., pembrolizumab).

Those of skill in the art that treatments of colorectal cancer provided herein can be utilized, e.g., as determined by a medical practitioner, alone or in any combination, in any order, regimen, and/or therapeutic program. Those of skill in the art will further appreciate that advanced treatment options may be appropriate for earlier stage cancers in subjects previously having suffered a cancer or colorectal cancer, e.g., subjects diagnosed as having a recurrent colorectal cancer.

In some embodiments, methods and compositions for colorectal neoplasm screening provided herein can inform treatment and/or payment (e.g., reimbursement for or reduction of cost of medical care, such as screening or treatment) decisions and/or actions, e.g., by individuals, healthcare facilities, healthcare practitioners, health insurance providers, governmental bodies, or other parties interested in healthcare cost. In some embodiments, methods and compositions for colorectal neoplasm screening provided herein can inform decision making relating to whether health insurance providers reimburse a healthcare cost payer or recipient (or not), e.g., for (1) screening itself (e.g., reimbursement for screening otherwise unavailable, available only for periodic/regular screening, or available only for temporally- and/or incidentally-motivated screening); and/or for (2) treatment, including initiating, maintaining, and/or altering therapy, e.g., based on screening results. For example, in some embodiments, methods and compositions for colorectal neoplasm screening provided herein are used as the basis for, to contribute to, or support a determination as to whether a reimbursement or cost reduction will be provided to a healthcare cost payer or recipient. In some instances, a party seeking reimbursement or cost reduction can provide results of a screen conducted in accordance with the present specification together with a request for such reimbursement or cost reduction of a healthcare cost. In some instances, a party making a determination as to whether or not to provide a reimbursement or cost reduction of a healthcare cost will reach a determination based in whole or in part upon receipt and/or review of results of a screen conducted in accordance with the present specification. For the avoidance of any doubt, those of skill in the art will appreciate from the present disclosure that methods and compositions for colorectal cancer diagnosis of the present specification are at least for in vitro use. Accordingly, all aspects and embodiments of the present disclosure can be performed and/or used at least in vitro.

Kits

The present disclosure includes, among other things, kits including one or more compositions for use in screening as provided herein, optionally in combination with instructions for use thereof in screening (e.g., screening for advanced adenoma, colorectal cancer, other cancers, or other diseases or conditions associated with an aberrant methylation status, e.g., neurodegenerative diseases, gastrointestinal disorders, and the like). In various embodiments, a kit for screening a diseases or conditions associated with an aberrant methylation status can include one or more oligonucleotide probes (e.g., one or more biotinylated oligonucleotide probes). In certain embodiments, the kit for screening optionally includes one or more bisulfite conversion reagents as disclosed herein. In certain embodiments, the kit for screening optionally includes one or more enzymatic conversion reagents as disclosed herein. In certain embodiments, the kit for screening may include one or more adapters as described herein. In certain embodiments, the kit may include one or more reagents used in library preparation. In certain embodiments, the kit may include software (e.g., for analyzing methylation status of DMRs).

EXAMPLES

Example 1: Identification of Markers associated with Colorectal Cancer and Advanced Adenoma

The purpose of this Example is to identify differentially methylated regions (DMRs) in DNA of colorectal cancer and colonic adenoma samples (e.g., samples from subjects having advanced adenoma). Identification of DMRs was performed by comparing DNA of subjects having colorectal cancer and/or colonic adenomas with matching control samples. This comparison allowed for development of methods that would elucidate colorectal cancer and advanced adenoma related methylation patterns from cell-free (cfDNA).

Whole genome bisulfite sequencing (WGBS) was used to identify differences in methylation status in samples of genomic DNA (gDNA) and cfDNA obtained from a variety of sources. gDNA was obtained from tissue samples with different histological backgrounds (e.g., colorectal cancer, colonic adenoma, lung cancer, breast cancer, pancreatic cancer, gastric cancer, and matching controls) and buffy coat samples. Genomic DNA (gDNA) from tissue and buffy coat samples was extracted using a DNeasy Blood & Tissue kit (Qiagen) according to a manufacturer's protocol. Extracted gDNA was then further processed in order to fragment it. For example, gDNA was fragmented into segments having lengths of about 400 bp with a Covaris S220 ultra-sonicator. cfDNA from plasma samples was extracted using QIAamp Circulating Nucleic Acid kit (Qiagen) according to the manufacturer's protocol.

The extracted and fragmented gDNA (genomic DNA) and cfDNA was bisulfite-converted with EZ DNA Methylation-Lightning kit (ZymoResearch). Sequencing libraries were prepared from the bisulfite converted DNA fragments by using Accel-NGS Methyl-seq DNA library kit (Swift Biosciences). The converted DNA fragments were sequenced with average depth of 37.5x with NovaSeq6000 (Illumina) equipment, using paired-end sequencing. For this experiment, paired-end sequencing was conducted such that 150 bp of each end of a converted DNA fragment was covered (e.g., 2x150). The sequenced reads were aligned to a bisulfite-converted human genome (Ensembl 91 assembly) using Bisulfite Read Mapper with Bowtie 2. The following steps were used to align sequenced reads to a bisulfite-converted human genome:

1. Evaluation of the sequencing quality
2. Alignment to a reference genome (hG38)
3. Deduplication and cleaning from adapter dimers
4. Methylation calling (e.g., identification of methylated nucleic acids)

Differentially methylated region analysis was done by comparing beta ($\beta$) values of individual CpGs of the colon cancer and/or colonic adenoma tissue samples to a matching control tissue. The $\beta$-value reflects methylation level of CpG reads in a sample. A $\beta$ value of 0 indicates no methylated reads were found at a specific CpG location, while a $\beta$ value of 1 indicates that all reads were fully methylated. Individual CpG methylation value scores were combined into regions of having a minimum of 3 CpGs within 50 bp distance of one another. The q-value of the region, which is the p-value corrected with a between-group label permutation test, was evaluated in order to select for regions of DNA from subjects with colorectal cancer and/or colonic adenoma which were significantly differently methylated from the same region in DNA obtained from a control subject. A q-value < 0.05 was considered to show high statistical significance of a differentially methylated region (DMR). Significant regions were further evaluated to determine if there was a significant methylation signal compared to tissue samples with non-colorectal cancer origin, control tissue samples of non-colorectal origin, buffy coat samples, and cfDNA from healthy individuals.

In total, 6061 DMRs were initially identified as being significant for colorectal cancer and/or advanced adenoma. These DMRs include regions that are more indicative of colorectal cancer, DMRs that are more indicative of different histological subtypes of colonic adenomas, and regions that are indicative of both colorectal cancer and advanced adenoma.

Further cancer signal analysis was done using on the selected target regions from whole genome sequencing data using a read-wise signal scoring method. Thresholds were calculated in tissue-control paired samples to allow maximum separation between cancer and control reads. The calculated scores were applied to each read obtained from plasma cfDNA of subjects.

Example 2: Feature Evaluation and Algorithm Development

The purpose of this Example was to identify which differentially methylated regions (DMRs) from Example 1 were more indicative of colorectal cancer and/or advanced adenoma and could be used for subsequent panel development. The work performed in this Example evaluated about 2000 DMRs that were found to be more indicative of colorectal cancer. The initial target regions and a prediction model were employed on the sample set of about 2000 DMRs in a train-verification setting. Results obtained from the larger sample set served as basis for further QC pipeline definition and optimization of cancer signal detection methods described herein.

Methods

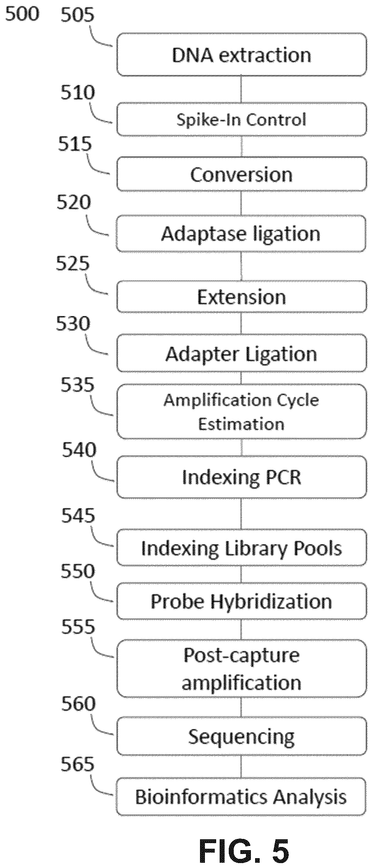

FIG. 5 is a flow diagram of a hybrid capture method (500) as performed herein. Steps of the hybrid capture method (500) are further described herein. In step (505), cfDNA was extracted from plasma. About 10- about 20ng of DNA was required for processing. An artificial spike-in control (510) was then added to the cfDNA sample. The artificial spike-in control was used to monitor conversion rates of methylated cytosines and/or unmethylated cytosines to uracils in later quality control steps. The DNA sample was then subjected to conversion (e.g., bisulfite or enzymatic conversion) (515). The adaptase ligation step (520) was a reaction that simultaneously performed end repair tailing and ligation of a first adapter to the 3' end of DNA fragments (e.g., cfDNA fragments). An extension step (525) generated a complementary, uracil-free library molecule. An adapter ligation step (530) added a second adapter to the newly generated library molecule. An optimal number of cycles for library amplification was then assessed using qPCR (535). Indexing PCR (540) was then performed to increase yield and incorporate full length adapters for either single or dual indexing (545) of the library fragments. Methylation and/or mutation capture probes were then hybridized with indexed library pools (550). Target DNA fragments were then were enriched for by binding the biotinylated, hybridized capture probes hybridized to streptavidin coated beads. After capture, the captured molecules were then amplified using PCR (555). This post-capture amplification step was accompanied by purification and quality control steps. Finally, captured and amplified molecules were then sequenced using a NGS technique to obtain reads corresponding to DNA fragments (560). Subsequent bioinformatics analyses (565) were conducted to identify sequenced targets that were methylated and/or mutated.

Sample set

The study was conducted under the approval of The Research Ethics Committee of the Virgen del Rocio Hospital of Sevilla, Spain (Ethical Committee Approval ref: 2014PI/155). All patients provided written informed consent prior to sample collection.

Patient cohorts

Blood samples were collected in endoscopy units and clinics from average risk patients prior to colonoscopy for screening or due to fecal occult blood (pre-colonoscopy samples). CRC (colorectal cancer) numbers were enriched through prospective enrollment of CRC patients from oncology units before CRC treatment, and samples obtained from biobanks (post-colonoscopy samples). The stage of the colorectal cancer patients was defined as per the AJCC Cancer Staging Manual 8th Edition.

Pre-colonoscopy blood samples were collected a maximum of 60 days prior to colonoscopy through the day of colonoscopy, but prior to administration of any sedatives for the procedure. In order to include a patient, the colonoscopy had to reach the cecum and visibility had to be "Good" or "Excellent" for each segment of the colorectum. Patients were assigned to a condition group based on colonoscopy and pathology findings. Colonoscopy and pathology findings included findings of colorectal cancer (CRC), advanced adenoma (AA), non-advanced adenoma (NAA), hyperplastic polyps, or healthy (e.g., "normal") colorectum. Subjects having AA were defined as subjects having an adenoma equal to or greater than 1 cm, tubulovillous histology, high-grade dysplasia, and/or serrated adenoma with dysplasia, carcinoma in situ.

Blood samples from post-colonoscopy patients awaiting CRC treatment were collected at least 3 days after colonoscopy. The average time for collection was 15 days post-colonoscopy. The time for collection ranged from 3 to 75 days.

Study Inclusion Criteria

Participants could be female or male and were required to be at least 45 years of age.

Study Exclusion Criteria

The exclusion criteria were: (1) prior diagnosis of cancer, except for patients with newly diagnosed colorectal cancer, (2) family history of CRC, (3) personal or family history of genetic cancer predisposition, (4) prior diagnosis of benign gastrointestinal disease, (5) pregnancy, and (6) for participants with colorectal cancer, current or prior therapy for the current cancer. Current or prior therapy included: surgical management beyond that required to establish diagnosis, chemotherapy, immunotherapy, hormone therapy, and/or radiation therapy.

Plasma sample collection protocol

Plasma samples were collected using one of two methods. In the first method, plasma was collected using K2 EDTA Tubes. Plasma collected with these types of tubes was extracted with double-spin centrifugation within 2 hours of collection. In the second method, plasma was collected with Cell-Free DNA BCT® Streck tubes. Plasma was then extracted with double-spin centrifugation within 2 days of collection. In both methods, plasma was stored at -80°C until it was analyzed.

cfDNA extraction from plasma and quality control samples

cfDNA was extracted from 4mL of human plasma using a QIAamp MinElute ccfDNA Midi Kit (Qiagen) according to the manufacturer's specifications.

cfDNA concentration was measured using the Qubit® dsDNA HS Assay Kit (Thermo Fisher Scientific).

cfDNA quality was assessed with DNF-474 NGS fragment kit on a Fragment Analyzer (Agilent).

A minimum 10 ng of extracted cfDNA is required to pass into next step.

Bisulfite and Enzymatic Conversion of DNA

Between 10 ng and 20 ng of cfDNA from each of patient was bisulfite treated using an optimized EZ DNA Methylation-Direct Kit (Zymo). NEBNext Enzymatic Methyl-seq Conversion Module (NEB) kit was used together with formamide denaturation on 16 replicate samples. Results from the replicate samples prepared with an enzymatic method are compared with bisulfite converted samples in Example 3. Deamination of the cfDNA helps in identification of methylated and unmethylated cytosine residues, particularly at CpG sites.

The NEB kit is an enzyme-based alternative method to bisulfite conversion for deamination of the cfDNA. In the enzymatic method, TET2 oxidizes methylated cytosines (both 5-methylcytosine (5-mC) and 5-hydroxymethylcytosine (5-hmC)). Following oxidation, APOBEC (apolipoprotein B mRNA editing enzyme, catalytic polypeptide-like) was used to treat the DNA. Unmodified cytosines (e.g., unmethylated cytosines unaffected by TET2 oxidation), are deaminated to uracils.

In the enzymatic method, 10 μl of TET2 reaction buffer, 1 μl DTT (Dithiothreitol), 1 μl oxidation supplement and 1 μl of TET2 were added to 28 μl of DNA. 5 μl of diluted Fe(II) solution was added and incubated for 1h at 37°C. DNA oxidation was then stopped. Oxidized DNA was then cleaned. 4 μl of formamide was added to 16 μl of oxidized DNA for denaturation. The following was then added to 20 μl of denatured DNA: 68 μl of water, 10 μl APOBEC Reaction Buffer, 1 μl BSA, and 1 μl APOBEC. The solution was then incubated at 37°C for 3 hours.

An optimized version of the EZ DNA Methylation-Direct Kit (Zymo) protocol was used as is presented below. The optimized version of the protocol contains various quality controls and experimental parameters which are important to maintaining the integrity of the sequenced cfDNA and/or identifying if any errors are present in the processes.

Artificially methylated and unmethylated spike-in (Premium RRBS kit [Diagenode]) control sequences were added to all cfDNA samples prior to conversion of the cfDNA. The spike-in control sequences were added in using a 1:10000 ratio (by volume) of spike-in control to cfDNA.

In the optimized version of the protocol presented below, the ratio of the CT Conversion Reagent to sample is lower than a standard ratio. For example, in a standard protocol, 20 μl of sample and 130 μl of CT Conversion Reagent may be used. In the protocol presented below, 117 μl CT conversion reagent was used for 33 μl of sample. The increased amount of sample accounted for low amounts of starting material. In addition, increasing the amount of sample and/or decreasing the amount of bisulfite reagent may be done to compensate for the aggressive nature of the bisulfite reagent, which may further fragment the DNA.

Additionally, the temperatures of the denaturation and conversion cycles and number of denaturation-conversion cycles were changed. In a standard protocol, a denaturation-conversion cycle is run a single time. In addition under standard protocols, denaturation is conducted at 98°C for 8 mins, while conversion is conducted at 64°C for 3.5 hours.

CT Conversion Reagent was a reagent which contained sodium metabisulphite. The reagent was used in bisulfite conversion of cfDNA. As provided by the manufacturer, each tube of CT Conversion Reagent was designed for 10 separate DNA treatments.

CT Conversion Reagent was prepared as follows:

1. 790 μl of M-Solubilization Buffer and 300 μl of M-Dilution Buffer was added to a tube of CT Conversion Reagent.
2. The solution was mixed at room temperature with frequent vortexing or shaking for 10 minutes (Keep in the dark).
3. 160 μl of M-Reaction Buffer was added to the solution and mixed for an additional 1 minute.

Note: It was normal to see trace amounts of undissolved reagent in the CT Conversion Reagent. The CT Conversion Reagent was light sensitive, its exposure to light was minimized.
- 117μl CT conversion reagent was added to 33μl of sample. The solution was pipetted up and down about 5-times.
- The sample was mixed by inversion and then centrifuged briefly to ensure no droplets were in the cap or sides of the tube.
- The samples were put in the thermocycler and run using the following protocol:

| Cycle Step | Temperature | Time | Cycles |
|---|---|---|---|
| Denaturation | 95°C | 1 min | 20 |
| Conversion | 60°C | 10 min | |
| Hold | 4°C | O/N | / |

- If the samples were not ready, the M-Wash buffer was prepared by adding 24 ml of 100% ethanol to the 6 ml M-Wash Buffer concentrate (D5020) or 96 ml of 100% ethanol to the 24 ml M-Wash Buffer concentrate (D5021).
- 600 μl of M-Binding Buffer was added into a Zymo-Spin™ IC Column and the column was placed into a provided Collection Tube.
- The sample was loaded into the Zymo-Spin™ IC Column containing the M-Binding Buffer. The cap was closed and the sample was mixed by inverting the column several times.
- The column was centrifuged at full speed (13000g) for 1 min. The flow-through was discarded.
- 100 μl of M-Wash Buffer was added to the column. The column was centrifuged at full speed (13000g) for 1 min.
- 200 μl of M-Desulphonation Buffer was added to the column and incubated at room temperature (20-30°C) for exactly 30 minutes. After this incubation period, the column was centrifuged at full speed (13000g) for 1 min.
- 200 μl of M-Wash Buffer was added to the column. The column was centrifuged at full speed (13000g) for 1 min. The flow-through was discarded.
- Another 200 μl of M-Wash Buffer was added to the column and the column was centrifuged at a higher speed (15000g) for 1.5min.
- The column was placed into a 1.5 ml microcentrifuge tube. 17 μl of M-Elution Buffer was added directly to the column matrix and incubated for 2 min. The column was centrifuged for 1.5min at full speed (20000g) to elute the DNA.
- A second elution was performed by transferring the eluate to the column membrane and waiting for 2 min. The column was centrifuged for 1.5min at full speed (20000g) to elute the DNA.
- 15μl of each bisulfited cfDNA sample was transferred to 8-strip tubes with RNAse/DNAse-free cap and frozen at -20°C for subsequent library preparation. Converted cfDNA quality was assessed using RNA 6000 Pico Kit (Agilent) on a Fragment Analyzer™ (Agilent).

Library Preparation

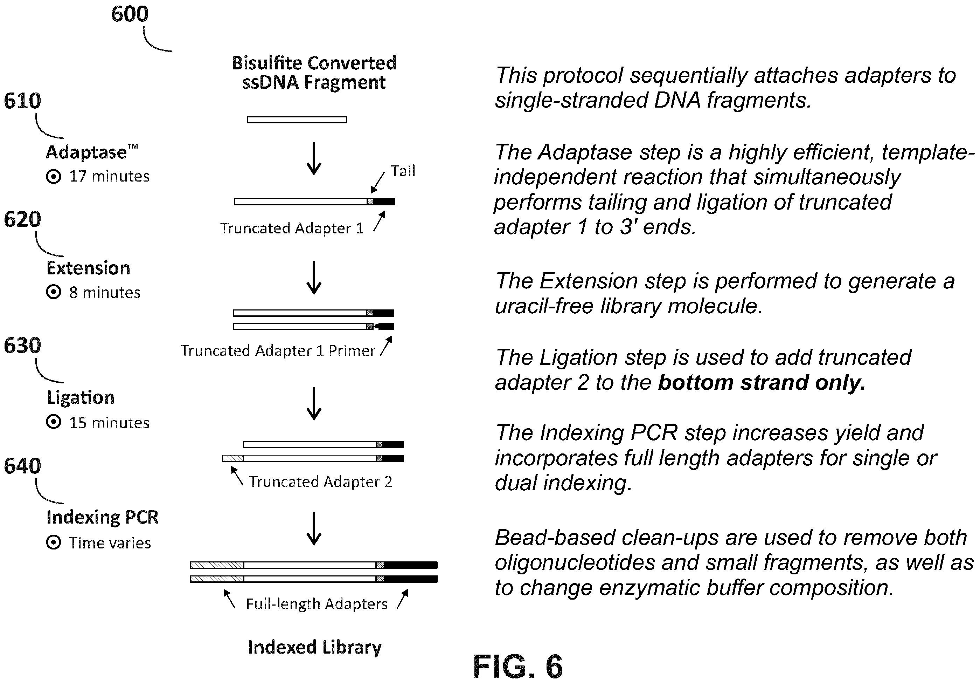

Converted cfDNA was used as input for NGS (next generation sequencing) library preparation. The Accel-NGS® Methyl-Seq DNA Library Kit (Swift Bioscience) was used to prepare a library using the converted cfDNA. FIG. 6 shows a series of steps involved in addition of adaptor sequences to DNA fragments (600),. Bisulfite converted single stranded DNA fragments were first subjected to an adaptase ligation step (610). The adaptase ligation step is a highly efficient, template-independent reaction that simultaneously performs end repair tailing and ligation of a first adapter (truncated adapter 1) to the 3' end of the DNA fragment. Next, an extension step (620) was performed to generate a complementary, uracil-free library molecule. Following extension, ligation (630) added a second adapter (truncated adapter 2) to the newly generated library molecule. Indexing PCR (640) was then performed to increase yield and incorporate full length adapters for either single or dual indexing of the library fragments. Bead-based clean-up steps were used to remove both oligonucleotides and small fragments.

Optimal library amplification was assessed by qPCR using KAPA SYBR® FAST (Sigma-Aldrich) on LightCycler® 96 System (Roche). qPCR was used to measure a total concentration of a prepared library, as described herein. The minimum amount of library material required was about 200ng. qPCR determines the optimal number of PCR cycles that may need to be performed in order to obtain the minimum amount of library material. The generated DNA libraries were first purified on IP-Star® Compact Automated System (Diagenode Cat# B03000002) using Agencourt® AMPure® XP (Beckman Coulter), then quantified using Qubit™ dsDNA HS Assay Kit (Thermo Fisher Scientific) and finally their size assessed with High Sensitivity NGS Fragment Analysis Kit (DNF-474) on a Fragment Analyzer™ (Agilent).

Hybrid Capture

120-bp biotinylated DNA probes were designed for targeted (e.g., selective) enrichment of bisulfite or enzymatically-converted DNA. 120-bp probes were designed to target either hypermethylated fragments (100% methylated CpGs) or hypomethylated fragments (100% unmethylated CpGs) of pre-determined regions (e.g., methylation markers, mutation markers). Probes were designed such that there were at least one probe per strand and one probe per methylation status. That is, there were two probes targeting the coding strand and two probes targeting the non-coding strand. One of the two probes in each pair targeted a methlyated fragment, while the other probe targeted an unmethylated fragment. For capturing partially methylated regions, a mismatch count of 8 is used for the probe and target region (e.g., up to about 8 bases may be mismatched (e.g., form non-Watson-Crick base pairings) between a DNA fragment belonging to a target region and a capture probe).

In this experiment, probes for methylation targets were designed using 1x tiling density. Probes for mutation targets were designed using 3x tiling density with each base in the target being covered by at least 3 different probes (e.g., where there is substantial overlap between the probes). The mutation probes were designed using 3x tiling to assure higher capture efficiency for low CG content regions (e.g., regions having from about 30% to about 40% GC content) that otherwise would be under-represented. Tiling density refers to the coverage of the target region by probes. For example, probes designed having 1x tiling density would cover each base of the target region at least once. Probes with 3x tiling density would cover the target region at least 3x.

A custom algorithm aligned candidate probes to the genome and scored the number potential of on- and off-target mapping events. Probes with >250 genomic regions globally mapping to off-target regions, were omitted from the final panel design. Biotinylated probes were synthesized and combined into a final targeted methylation panel. Off-target mapping was done also after the initial assay design and testing. Targets were omitted if they caused more than 1% off-target capture in an actual panel. Purified libraries (about 187.5 ng each) were pooled together in 8-plex, dried using concentrator plus (Eppendorf) and captured using a Fast Hybridization Target Enrichment Protocol by Twist® and a customized panel probes as described herein, which was designed to capture methylation and mutation targets of interest. Fragments bound to biotinylated probes were captured using streptavidin coated beads.

The captured fragments from the purified libraries were reamplified using PCR amplification for 11 cycles following manufacturer's protocol. The amplified libraries were then purified with Twist beads.

An exemplary method describing the Twist Fast Hybridization Target Enrichment protocol is presented below. Of note, maintaining a temperature of 70°C the Fast Wash Buffer 1 during steps (e.g., steps 23-28) was important for GC rich regions. Differences in temperatures at these steps lead to higher than expected GC dropout rates (e.g., a GC dropout rate greater than 6%). For example, when the Fast Wash Buffer 1 was used at 65°C, GC dropout rates were about 30%, which was surprisingly high. Accordingly, minimizing pipetting time and maintaining the temperature of the Fast Wash Buffer 1 and sample-Fast Wash Buffer 1 mixtures were important.


Twist Fast Hybridization Target Enrichment Protocol


Before Beginning

All required reagents were thawed on ice, then pulse-vortex for 2 seconds to mix and pulse-spin.

In preparation for hybridization of capture probes with library pools, library pools were also thawed on ice: From the Twist Fast Hybridization Reagents:

Fast Hybridization Mix
Hybridization Enhancer

1. The concentration of each amplified, indexed library was used to calculate the volume (in µl) of each library needed for hybridization. The calculated volumes from each amplified, indexed library were transfered to a hybridization reaction tube (e.g., a 0.2-ml thin-walled PCR strip-tube, a 96-well plate) for each hybridization reaction to be performed.


PREPARATION OF THE PRE-HYBRIDIZATION SOLUTION

2. The following volumes of reagents were added to each amplified indexed library to create a pre-hybridization solution as shown in Table 1 below. The solution was mixed by flicking the tubes.


Table 1. Volumes of Reagents for Pre-Hybridization Solution

| Reagent | | Volume |
|---|---|---|
| Twist Probe Panel | | 4 µL |
| Optional: | Secondary Panel (if a secondary panel is not used, do not add water as the entire solution will be dried) | 4 µL |

(continued)

| Reagent | Volume |
|---|---|
| Universal Blocker | 8 µL |
| Blocker Solution | 5 µL |

3. Tubes were pulse-spun and it was ensured that there were a minimal number of bubbles present in the solution.

4. The pre-hybridization solution (including library, probes, blockers) were dried in the tubes used for the hybridization reaction using a SpeedVac system (or a similar evaporator device) using low or no-heat.

IMPORTANT: Step 12 to 18 (below) were performed concurrently to the pre-hybridization solution dry down and for hybridizations under 30 minutes.

The aliquoted libraries and hybridization reaction solution from Step 1 were used, as well as the thawed Fast Hybridization Mix and Hybridization Enhancer.

IMPORTANT: Before proceeding with this step, the compatibility of the thermal cycler and PCR tubes or plates were tested by incubating them at 95°C for up to 5 minutes to ensure they did not crack under heat and pressure. The tightness of the thermal cycler lid was adjusted and/or a spacer specific to the thermal cycler model was used.

Reagents Required

- Dried hybridization reaction (from Step 4)
- Reagents thawed:
- Fast Hybridization Mix
- Hybridization Enhancer

Before Beginning

A 96-well thermocycler was programmed with the following conditions in Table 2 and the heated lid was set to 85°C:

Table 2. Thermocycler Program Steps.

| Step | Temperature | Time |
|---|---|---|
| 1 | 95°C | HOLD |
| 2 | 95°C | 5 min |
| 3 | 60°C | 15 min to 4 hours |

RESUSPENDING THE PRE-HYBRIDIZATION SOLUTION

5. The Fast Hybridization Mix was heated to 65°C for 10 minutes, or until all precipitate was dissolved. The mix was vortexed and used immediately. The Fast Hybridization Mix was not allowed to cool to room temperature.

6. The dried pre-hybridization solution from Step 4 was resuspended in 20 µl Fast Hybridization Mix.

Some notes to consider when following this process are presented as follows. If this resuspended solution required transfer into a secondary vessel for hybridization, the resuspended solution was mixed by flicking and an additional 5 minutes was added to incubation for resuspension. Fast Hybridization Mix is viscous. The mix was pipetted slowly to ensure accuracy. Small white particles present in the capture probes did not affect the final capture product.

7. Tubes were pulse-spun and it was ensured that there were no bubbles present.

8. 30 µl Hybridization Enhancer was added to the top of the pre-hybridization solution.

9. Tubes were pulse-spun to ensure all solution was at the bottom of the tubes.

NOTE: Hybridization Enhancer settled on top of the hybridization reaction after the pulse-spin. This did not affect the final capture product.

Tubes were transfered to the preheated thermocycler. The program was then moved to Steps 2 and 3 of the thermocycler program.

IMPORTANT: Tubes were sealed tightly to prevent evaporation over the incubation time period.

BINDING HYBRIDIZED TARGETS TO STREPTAVIDIN BEADS

Reagents Required

- Hybridization reactions prepared as above.
- From the Twist Fast Wash Buffers:

    Fast Binding Buffer
    Fast Wash Buffer 1
    Wash Buffer 2

- From Twist Binding and Purification Beads:

    Streptavidin Binding Beads

Before Beginning

The following reagents were inspected for precipitate. If a precipitate was observed, the reagent was heated at 48°C until the precipitate was dissolved:

    Fast Binding Buffer
    Fast Wash Buffer 1
    Wash Buffer 2

For each hybridization reaction:

    Preheat 450 μl Fast Wash Buffer 1 to 70°C
    Preheat 700 μl Wash Buffer 2 to 48°C

The Streptavidin Binding Beads were equilibrated to room temperature for at least 30 minutes.
It was important to maintain the temperature of the Fast Wash Buffer 1 at 70°C. Accordingly, pipetting time was minimized. Additionally, the Fast Wash Buffer 1 stayed on a heating block for the whole time of pipetting.
In preparation for the steps of Post-Capture PCR Amplification, Purification, and Performing QC:

    DNA Purification Beads (from the Twist Binding and Purification Beads) were equilibrated to room temperature for at least 30 minutes
    KAPA HiFi HotStart ReadyMix was thawed on ice
    Amplification Primers (from the Twist Fast Hybridization and Wash Kit) were thawed on ice
    Once these reagents were thawed, the reagents were pulse-vortex for 2 seconds to mix.

PREPARATION OF THE BEADS

12. The pre-equilibrated Streptavidin Binding Beads were vortexed until mixed.
13. 100 μl Streptavidin Binding Beads were added to a 1.5-ml microcentrifuge tube. One tube was prepared for each hybridization reaction.
14. 200 μl Fast Binding Buffer was added to each of the tubes and mixed by pipetting.
15. The tubes were placed on a magnetic stand for 1 minute, then removed. The clear supernatant was discarded. The bead pellet was not disturbed. The tube was removed from the magnetic stand.
16. The wash steps (Steps 14 and 15) were repeated two more times for a total of three washes.
17. After removing the clear supernatant from the third wash, a final 200 μl Fast Binding Buffer was added. The beads were resuspended by vortexing until homogenized.
18. After the hybridization was complete, the thermal cycler lid was opened and the volume each hybridization reaction was quickly transfered (including Hybridization Enhancer) into a corresponding tube of washed Streptavidin Binding Beads from Step 18. The solution was mixed by pipetting and flicking.
NOTE: Rapid transfer directly from the thermal cycler at 60°C was a critical step for minimizing off-target binding. The tubes of hybridization reaction were not removed from the thermal cycler or otherwise allowed to cool to less than 60°C before transferring the solution to the washed Streptavidin Binding Beads.

BIND THE TARGETS

19. The tubes of the hybridization reaction were mixed with the Streptavidin Binding Beads for 30 minutes at room temperature on a shaker, rocker, or rotator at a speed sufficient to keep the solution mixed.

NOTE: The solutions were not vortexed. Aggressive mixing was not required.

20. The tubes containing the hybridization reaction with Streptavidin Binding Beads were removed from the mixer and pulse-spun to ensure all solution was at the bottom of the tubes.

21. The tubes were placed on a magnetic stand for 1 minute.

22. The clear supernatant including the Hybridization Enhancer was removed and discarded. The bead pellet was not disturbed.

NOTE: A trace amount of Hybridization Enhancer was visible after supernatant removal and throughout each wash step in certain samples. It did not affect the final capture product.

23. The tubes were removed from the magnetic stand and 200 µl preheated Fast Wash Buffer 1 was added. The solution was mixed by pipetting.

24. The tubes were incubated for 5 minutes at 70°C.

25. The tubes were placed on a magnetic stand for 1 minute.

26. The clear supernatant was removed and discarded. The bead pellet was not disturbed.

27. The tubes were removed from the magnetic stand and an additional 200 µl **of** preheated Fast Wash Buffer 1 was added. The solutions were mixed by pipetting.

28. The tubes were incubated for 5 minutes at 70°C.

29. The entire volume from Step 28 (~200 µl) was transferred into a new 1.5-ml microcentrifuge tube, with one per hybridization reaction. The tubes were placed on a magnetic stand for 1 minute.

NOTE: A tube transfer was required at this step as it reduced background due to non-targeted library that may stick to the surface of the tube.

30. The clear supernatant was removed and discarded. The bead pellet was not disturbed.

31. The tubes were removed from the magnetic stand and 200 µl of 48°C Wash Buffer 2 was added to each. The solution was mix by pipetting, and then pulse-spun to ensure all solution was at the bottom of the tubes.

32. The tubes were incubated for 5 minutes at 48°C.

33. The tubes were placed on a magnetic stand for 1 minute.

34. The clear supernatant was removed and discarded. The bead pellet was not disturbed.

35. The wash (Steps 31-34) was performed two more times, for a total of three washes.

36. After the final wash, a 10 µl pipette was used to remove all traces of supernatant. The next step was immediately followed. The beads were not allowed to dry.

37. The tubes were removed from the magnetic stand and 45 µl water was added. The solution was mixed by pipetting until homogenized. The solution, hereafter referred to as the Streptavidin Binding Bead slurry, was incubated on ice.

POST-CAPTURE PCR AMPLIFICATION, PURIFICATION, AND PERFORMING QC

Reagents Required

Streptavidin Binding Bead slurry (from Step 38)

Ethanol

Molecular biology grade water

Reagents thawed and equilibrated:

DNA Purification Beads
KAPA HiFi HotStart ReadyMix (or equivalent)
Amplification Primers
Agilent Bioanalyzer High Sensitivity DNA Kit (or equivalent)
Thermo Fisher Scientific Qubit dsDNA High Sensitivity Quantitation Assay.
Before Beginning
500 µl 80% ethanol was prepared for each Streptavidin Binding Bead slurry to be processed.

PREPARING THE BEADS, THERMOCYCLER, AND PCR MIX

38. A thermocycler was programmed with the following conditions as presented in Table 3 below. The heated lid was set to 105°C. As stated above, PCR amplification was performed for 11 cycles according to the below program. However, for certain samples more or fewer cycles were run based on the results of the results of a qPCR assessment performed as discussed above.

Table 3. Thermocycler conditions for PCR.

| Step | | Temperature | Time | Number of Cycles |
|---|---|---|---|---|
| 1. | Initialization | 98°C | 45 sec | 1 |
| 2. | Denaturation | 98°C | 15 sec | 11 |
| | Annealing | 60°C | 30 sec | |
| | Extension | 72°C | 30 sec | |
| 3. | Final Extension | 72°C | 1 minute | 1 |
| 4. | Final Hold | 4°C | HOLD | - |
| NOTE: The number of amplification cycles varied depending on hybridization reaction size. | | | | |

39. If the Streptavidin Binding Bead slurry settled, it was mixed by pipetting.

40. 22.5 μl of the Streptavidin Binding Bead slurry was transferred to a 0.2-ml thin-walled PCR strip-tube(s).

41. The solution was kept on ice until ready to use in the next step.

NOTE: The remaining 22.5 μl water/Streptavidin Binding Bead slurry was stored at - 20°C for future use.

A PCR mixture was prepared by adding the following reagents to the tubes containing the Streptavidin Binding Bead slurry. The solution was mixed by pipetting.

PCR AMPLIFICATION

42. The tubes were pulse-spun and transferred to the thermocycler. The cycling program was then started.

43. When the thermal cycler program was completed, the tubes were removed from the block and followed by purification steps.

44. DNA Purification Beads were vortexed to mix.

45. 90 μl (1.8X) homogenized DNA Purification Beads was added to each of the tubes from Step 44. The solution was mixed well by vortexing.

NOTE: It was not necessary to recover supernatant or remove Streptavidin Binding Beads from the amplified PCR product.

46. The solution was incubated for 5 minutes at room temperature.

47. The tubes were placed on a magnetic plate for 1 minute.

48. Without removing the tubes from the magnetic plate, the clear supernatant was removed and discarded.

49. The DNA Purification Bead pellet was washed with 200 μl freshly prepared 80% ethanol for 1 minute, then removed. The ethanol was discarded. This wash was repeated once, for a total of two washes, while the tube was kept on the magnetic plate.

50. Using a 10 μl pipet, all residual ethanol was removed. The bead pellet was not disturbed.

51. The bead pellet was air-dried on a magnetic plate for 5-10 minutes or until the bead pellet was dry. Care was taken to not overdry the bead pellet.

52. The tubes from the magnetic plate were removed and 32 μl water was added. The solution was mixed by pipetting until homogenized and incubated at room temperature for 2 minutes.

53. The tubes were placed on a magnetic plate and let stand for 3 minutes or until the beads fully pelleted.

54. 30 μl of the clear supernatant containing the enriched library was transferred to a clean thin-walled PCR 0.2-ml strip-tube, while making sure not to disturb the bead pellet.

55. Each enriched library was validated and quantified using an Agilent Bioanalyzer High Sensitivity DNA Kit and a Thermo Fisher Scientific Qubit dsDNA High Sensitivity Quantitation Assay.

NOTE: When using the Agilent Bioanalyzer High Sensitivity DNA Kit, 0.5 μl of the final sample was loaded.

56. Average fragment length was about 375-425 bp using a range setting of 150-1,000 bp. Final concentration for samples was greater than or equal to 15 ng/μl, but this depended on the hybridization reaction size, hybridization time, and number of PCR cycles used.

The captured library pools were then pooled together to be sequenced on Illumina NovaSeq SP PE150 (1 lane each 96 samples).

Bioinformatics workflow

Sequencing data analysis

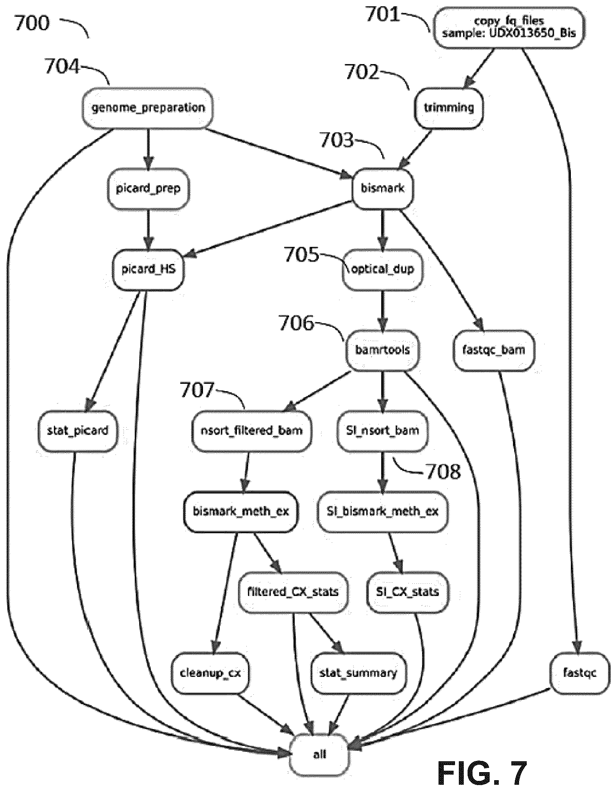

The bioinformatics workflow included genome preparation according to pre-determined regions of interest. The alignment was conducted for pre-determined regions of interest and sequences not within these regions are discarded. A bioinformatics workflow (700) used herein is shown in FIG. 7. Raw FASTQ files (701) were trimmed (702) to remove adaptor ends and combined using an alignment tool (e.g., BISMARK) (703) with the prepared genome (704). FASTQ files are text files that contain sequence data from clusters that pass filter on a flow cell. Optical duplicates (705) were then marked and removed. For pair-end runs, forward and reverse reads were generated for each strand and combined in silico to produce a sequence read that corresponded to a single strand of a double stranded fragment (706). Aligned files were sorted (707, 708), marked for duplicates, and put into either target of interest .bam files (707) or spike-in (SI) control .bam files (708)..bam files were further deduplicated and quality filtered and run through Picard metrics and QC summary workflow.

The final outputs of the bioinformatics workflow were:

.bam files that include trimmed, aligned, deduplicated and quality filtered reads for the targets (e.g., methylation and/or mutation markers) of interest;
.bam files that include trimmed, aligned, deduplicated and quality filtered reads for the spike-in control sequences. Spike-in control sequences were used for conversion quality control (e.g., determination of a conversion rate, e.g., conversion efficiency); and
.xsl files with summaries of statistics per sample and per region analyzed (used for sample and region quality control).

Cancer signal deduction and prediction algorithm

.bam files of the samples were further used for assigning read-wise methylation values. Pre-defined thresholds based on reads having a minimum pre-determined CpG number and a minimum methylation percentage were applied to each sequencing read in a target region of interest. Each read in each target region (e.g., a DMR) received a score of 1 or 0 depending on whether the read passed a threshold or not. The scores were then summed to find a total number of reads of a DMR that passed the threshold condition. Read-wise methylation values were further normalized using an effective library size of an individual sample. The resulting values were log2 transformed and used as inputs in prediction algorithm building, training, and validation.

Colorectal Cancer Model

A training set of colorectal cancer samples and colonoscopy negative control samples was used for initial feature filtering using a 50-fold Monte Carlo cross-validation. In each iteration, 50% of the samples were used as training samples and 50% of the samples were used as validation samples. Sequential Backward Selection (SBS) was used for dimensionality reduction to avoid overfitting by reducing the complexity of the further prediction model building with random forest. Sequential backwards selection learns which features (e.g., DMRs) are most informative at each step, and then chooses the next feature depending on the already selected features. SBS is a sequential process where features from the full feature subset were removed until the new feature subspace contained a set of features upon which a model did not improve. A set of 203 marker regions (listed in FIG. 2) was selected using SBS. A random forest (RF) machine learning algorithm was built on a training set and applied to an independent, validation set which was not used in the feature filtering or prediction algorithm training phase. Individual predictions from the model on the validation set were then compared against true condition of the patient. Sensitivity and specificity values were then calculated.

Advanced Adenoma Model

Preliminary analysis of markers for advanced adenoma detection potential was developed similarly to the colorectal cancer model. Using a pre-selected region list and AMBER score thresholds defined on colorectal cancer tissue samples, results obtained from advanced adenoma and control samples were evaluated separately in cross-validation setting. 50-fold Monte Carlo cross-validation was used in each iteration as above. In each iteration, 50% of the samples were used as training and 50% of samples were used for testing and validation. Sequential Backward Selection (SBS) was used for dimensionality reduction to avoid overfitting by reducing the complexity for prediction model building. Using SBS, a set of 220 marker regions (listed in FIG. 3) were selected. Prediction models were built using random forest (RF), PLS-DA and support vector machine (SVM) models. Individual sample results were presented as consensus predictions for all folds a particular sample was tested in.

Results and Quality Filtering

Based on evaluation of mapping quality, duplication level, conversion, and coverage, 37 samples were deemed invalid for additional analysis and were excluded. This left 70 colorectal cancer samples, 81 advanced adenoma samples, 37 non-advanced adenoma samples, 14 gastrointestinal disease samples, and 142 colonoscopy negative samples for further analysis.

Colorectal Cancer Model

Read-wise methylation values were used to train a machine learning model on 68 ctDNA samples as presented in Table 4 below. Samples analyzed were from 18 early stage (I-II) and 16 late-stage (III-IV) CRC patients and 34 age, BMI, gender and country of origin matched neoplasia-free controls. The median age of subjects was 63 [50-74], the mean BMI was 27 [19.5-37], 50% of subjects were female, 50% of CRCs were distal cancers. Subjects were from either Ukraine or Spain.

Table 4.  Training Subject Demographics.

| Characteristics | Controls (n=34) | CRC (n=34) |
|---|---|---|
| Age (years, mean (IQR)) | 63 (50-74) | 63 (50-74) |
| Gender (n (%)) | | |
| Female | 17 (50%) | 17 (50%) |
| Male | 17 (50%) | 17 (50%) |
| Body mass index (kg/m2, mean (IQR)) | 27 (19.5-32) | 27 (20-37) |
| Stage | | |
| Stage I | | 10 |
| Stage II | | 8 |
| Stage III | | 11 |
| Stage IV | | 5 |
| Cancer location | | |
| Proximal colon | | 17 |
| Distal colon | | 17 |

This model was then applied to an independent, validation set of subjects as presented in Table 5 below. The subjects were from Spain, Ukraine, and Germany. Subjects included 36 stage I-IV cancer patients (median age 61.5 [55-82], BMI 28 [16-39], female 47%, 42% of the tumors were distal) and 159 age and sex matched controls. 87 of the control subjects had a negative colonoscopy finding (cNEG), 19 had hyperplastic polyps (HP), 37 had small non-advanced adenomas (NAA), and 16 were diagnosed with other benign gastrointestinal diseases (GID).

Table 5.  Validation Subject Demographics.

| Characteristics | Controls (n=87) | HP (n=19) | NAA (n=37) | GID (n=16) | CRC (n=36) |
|---|---|---|---|---|---|
| Age (years, mean (IQR)) | 63 (46-79) | 62 (50-71) | 61 (45-78) | 58 (50-78) | 62 (55-82) |
| **Gender (n (%))** | | | | | |
| Female | 48 (55%) | 6 (32%) | 20 (54%) | 9 (56%) | 17 (47%) |
| Male | 39 (45%) | 13 (68%) | 17 (46%) | 7 (44%) | 19 (53%) |
| Body mass index (kg/m2, mean (IQR)) | 27 (20-41.5) | 28 (22-41) | 29 (20-43) | 27 (20-39) | 29 (16-39) |
| **Stage** | | | | | |
| Stage I | | | | | 6 |
| Stage II | | | | | 13 |
| Stage III | | | | | 12 |
| Unknown | | | | | 3 |
| **Cancer location** | | | | | |
| Proximal colon | | | | | 21 |
| Distal colon | | | | | 15 |

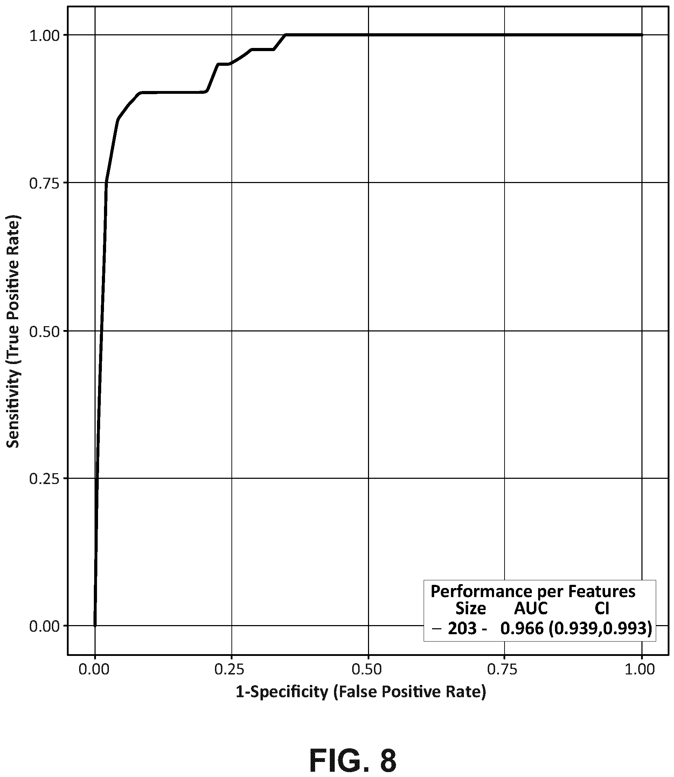

Using a 203 marker panel as presented in FIG. 2, the model correctly classified 92% (33/36) of CRC patients in the validation subject group. FIG. 8 is a ROC curve showing the performance of the 203-marker CRC panel on the validation set.

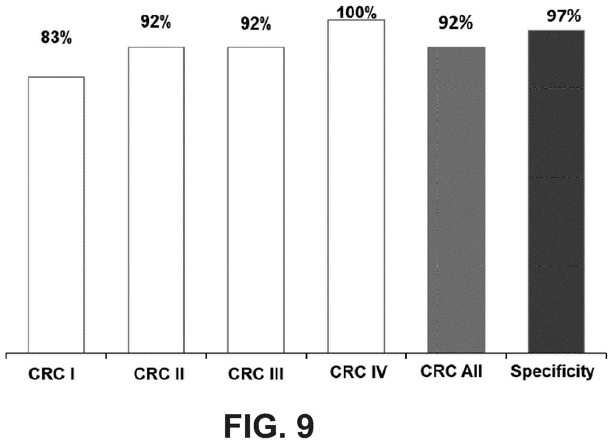

FIG. 9 shows CRC sensitivity values per CRC stage, overall specificities, sensitivity in the validation set. Sensitivity per CRC stage ranged from 83% (5/6) for stage I CRC, 92%(11/12) for stage II CRC, 92% (12/13) for stage III CRC to 100% (5/5) for stage IV CRC. The specificity of the model was 97% (154/159), with 100% (37/37) NAA, 94% (15/16) GID, 95%(18/19) HP and 97% cNEG patients correctly identified as not having CRC. Lesion location, gender, BMI, age and country of origin were not significantly correlated to prediction outcome.

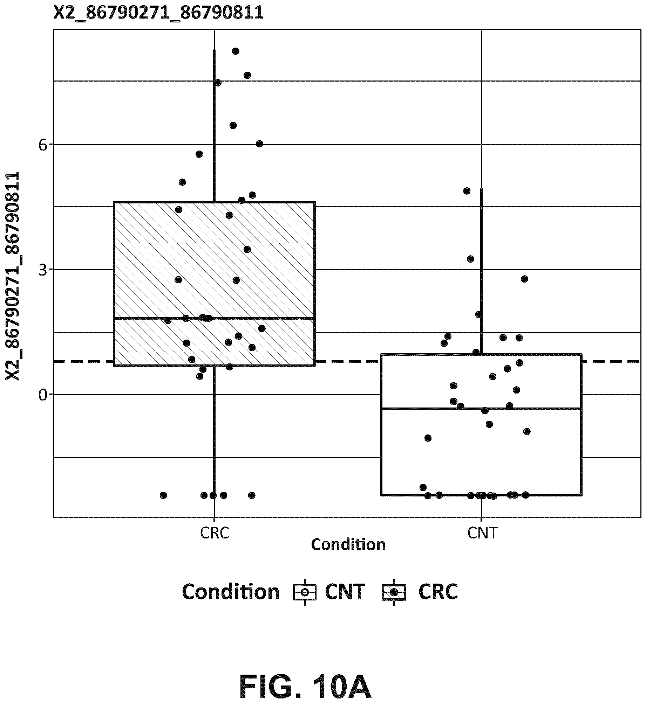

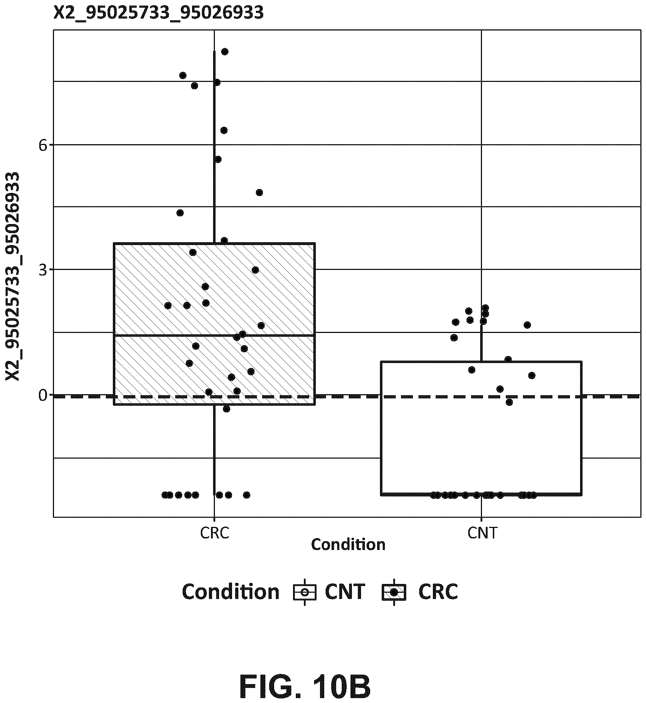

FIGs. 10A and 10B are box-plots of AMBER score values in each sample in the Validation Set for two individual DMRs. As can be seen form the box-plots, the two individual DMRs have a strong ability to distinguish CRC from other conditions. Control samples (CNT) includes all samples from subjects that were not determined to have CRC (e.g., cNEG, HP, NAA, GID).

Further analysis was conducted on the 203 DMRs (FIG. 2) using KEGG pathway analysis. KEGG pathway analysis identifies key pathways involved in cellular metabolic processes. The results of the analysis are presented below in Table 6. The main pathways identified as contributing to the DMR panel were identified to be linked to cancer as well as signaling pathway regulating cell pluripotency, which is commonly affected in cancer.

Table 6. KEGG Pathway Analysis Results.

| DAVID KEGG pathways |
|---|
| |
| **Term** |
| **hsa05216: Thyroid cancer** |
| **hsa04550: Signaling pathways regulating pluripotency** |
| **hsa05200: Pathways in cancer** |
| **hsa04015: Rap1 signaling pathway** |

Subset analysis of 203 DMRs showed that subsets of DMRs performed surprisingly well in distinguishing between CRC and control samples in the validation set. It was found that combinations of 2, 4, 9, and 24 DMRs performed well in identifying CRC subjects from control subjects. For example, a panel of only two DMRs showed an AUC of 78%. Though accuracy improves with increased numbers of DMRs, these smaller DMR panels may also be useful in identifying subjects suffering from CRC. Results of these subsets of DMR combinations are presented below in Table 7.

Table 7. CRC DMR panels statistics.

|  | 2 | 2 | 9 | 24 | 203 |
|---|---|---|---|---|---|
| AUC | 0.78 | 0.83 | 0.87 | 0.92 | 0.97 |
| Sensitivity | 0.54 | 0.80 | 0.83 | 0.90 | 0.92 |
| Specificity | 0.89 | 0.72 | 0.64 | 0.72 | 0.97 |
| Accuracy | 0.82 | 0.74 | 0.68 | 0.76 | 0.93 |
| Kappa | 0.43 | 0.39 | 0.32 | 0.45 | 0.78 |

Tables 8, 9, 10, and 11 (presented below) correspond to the 2, 4, 9, and 24 DMR combinations, respectively, indicated in Table 7. DMRs listed in Tables 8, 9, 10, and 11 are also found in the 203 DMR panel shown in FIG. 2. Though the best performing DMR panel was the 203 DMR panel (FIG. 2), smaller panels proved also to be surprisingly useful. For example, the two marker panel has a surprisingly high AUC, accuracy, and kappa values as compared to the 4, 9, and 24 DMR panels.

Table 8. 2-DMR Panel for CRC.

| chr | start | end | SEQ ID NO. |
|---|---|---|---|
| 7 | 96997902 | 96999222 | SEQ ID NO.: 92 |
| 8 | 96145538 | 96145718 | SEQ ID NO.: 108 |

Table 9. 4-DMR Panel for CRC.

| chr | start | end | SEQ ID NO. |
|---|---|---|---|
| 7 | 96997902 | 96999222 | SEQ ID NO.: 92 |
| 8 | 96145538 | 96145718 | SEQ ID NO.: 108 |
| 2 | 100322218 | 100322818 | SEQ ID NO.: 28 |
| 2 | 29115776 | 29116791 | SEQ ID NO.: 17 |

Table 10. 9-DMR Panel for CRC.

| chr | start | end | SEQ ID NO. |
|---|---|---|---|
| 7 | 96997902 | 96999222 | SEQ ID NO.: 92 |
| 8 | 96145538 | 96145718 | SEQ ID NO.: 108 |
| 2 | 100322218 | 100322818 | SEQ ID NO.: 28 |
| 2 | 29115776 | 29116791 | SEQ ID NO.: 17 |
| 2 | 88765502 | 88766042 | SEQ ID NO.: 25 |
| 4 | 153249541 | 153249721 | SEQ ID NO.: 55 |
| 2 | 86790271 | 86790811 | SEQ ID NO.: 24 |
| 2 | 176094518 | 176094878 | SEQ ID NO.: 35 |
| 3 | 37453325 | 37453874 | SEQ ID NO.: 41 |

Table 11. 24 DMR Panel for CRC.

| chr | start | end | SEQ ID NO. |
|---|---|---|---|
| 7 | 96997902 | 96999222 | SEQ ID NO.: 92 |
| 8 | 96145538 | 96145718 | SEQ ID NO.: 108 |
| 2 | 100322218 | 100322818 | SEQ ID NO.: 28 |

(continued)

| chr | start | end | SEQ ID NO. |
|---|---|---|---|
| 2 | 29115776 | 29116791 | SEQ ID NO.: 17 |
| 2 | 88765502 | 88766042 | SEQ ID NO.: 25 |
| 4 | 153249541 | 153249721 | SEQ ID NO.: 55 |
| 2 | 86790271 | 86790811 | SEQ ID NO.: 24 |
| 2 | 176094518 | 176094878 | SEQ ID NO.: 35 |
| 3 | 37453325 | 37453874 | SEQ ID NO.: 41 |
| 10 | 43105168 | 43105348 | SEQ ID NO.: 124 |
| 7 | 90596855 | 90597335 | SEQ ID NO.: 90 |
| 6 | 28988380 | 28988560 | SEQ ID NO.: 69 |
| 19 | 53254542 | 53254962 | SEQ ID NO.: 190 |
| 7 | 141072108 | 141073057 | SEQ ID NO.: 99 |
| 14 | 104117671 | 104117851 | SEQ ID NO.: 159 |
| 6 | 163413139 | 163413679 | SEQ ID NO.: 80 |
| 5 | 38555799 | 38556399 | SEQ ID NO.: 60 |
| 2 | 95025733 | 95026933 | SEQ ID NO.: 26 |
| 7 | 35257235 | 35257535 | SEQ ID NO.: 86 |
| 1 | 2132912 | 2133092 | SEQ ID NO.: 2 |
| 3 | 128491607 | 128492807 | SEQ ID NO.: 49 |
| 20 | 23049079 | 23049259 | SEQ ID NO.: 193 |
| 1 | 243917149 | 243917569 | SEQ ID NO.: 15 |
| 1 | 38045915 | 38046095 | SEQ ID NO.: 6 |

In some embodiments, the present disclosure includes combinations of DMRs in which each of the DMRs is, includes all of, includes a portion of, or is present in a gene identified as being associated with a DMR of Table 8 (e.g., as shown in FIG. 2). In some embodiments, the present disclosure includes combinations of DMRs in which each of the DMRs is, includes all of, includes a portion of, or is present in a gene identified as being associated with a DMR of Table 9 (e.g., as shown in FIG. 2). In some embodiments, the present disclosure includes combinations of DMRs in which each of the DMRs is, includes all of, includes a portion of, or is present in a gene identified as being associated with a DMR of Table 10 (e.g., as shown in FIG. 2). In some embodiments, the present disclosure includes combinations of DMRs in which each of the DMRs is, includes all of, includes a portion of, or is present in a gene identified as being associated with a DMR of Table 11 (e.g., as shown in FIG. 2).

Advanced Adenoma Model

Calculated read-wise methylation values corresponding to individual DMRs were used to build and cross-validate a machine learning model on 217 ctDNA samples from 81 patients with advanced adenomas and 136 age, BMI, gender and country of origin matched neoplasia-free controls. Statistics regarding the advanced adenomas subjects used in validation and training of the model are presented as follows: median age 63 [46-79], mean BMI 28 [19-48], female 51%. Subjects were from Spanish, Ukrainian, and German populations. The distribution of patients with different forms of advanced adenomas can be seen as listed below in Table 12.

Table 12.  Validation and Training Subject Demographics.

| | | | Controls | Advanced adenoma |
|---|---|---|---|---|
| | Sum | | 136 | 81 |
| Characteristics | Advanced adenoma histology | | | |
| | Tubular | | | 30 |
| | Tubulovillous | | | 29 |
| | Serrated | | | 15 |
| | Carcinoma in situ | | | 7 |
| | Dysplasia grade | | | |
| | Tis | | | 7 |
| | High grade | | | 25 |
| | >= 1cm | | | 41 |
| | <1 cm (serrated with dysplasia or tubulovillous low grade) | | | 8 |
| | Age (years, mean (IQR)) | | 63 (46-78) | 63 (50-79) |
| | Gender (n (% )) | | | |
| | Female | | 69 (51%) | 42 (52%) |
| | Male | | 67 (49%) | 39 (48%) |
| | Body mass index (kg/m2, mean (IQR)) | | 28 (19.5-42) | 28 (19-48) |

The SBS feature filtering method was evaluated in combination with RF, PLS-DA and SVM classification models to identify DMRs of interest. A SVM classification model based SBS selected features (DMRs) showed the best performance in a cross-validation setting.

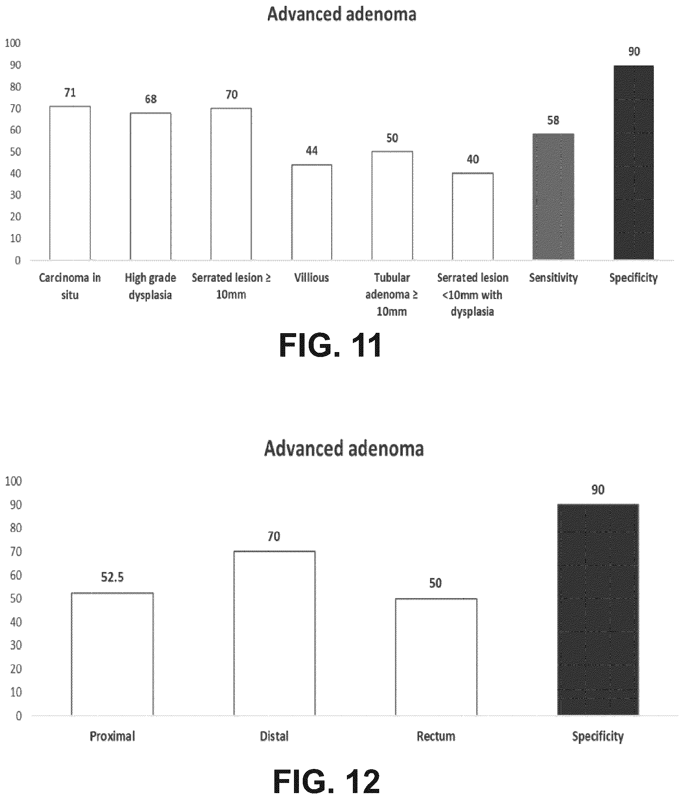

Statistical results regarding the sensitivity of the model to subjects diagnosed with various classifications and types of advanced adenomas are presented in FIGs. 11 and 12. The advanced adenoma model correctly classified 58% (47/81) of AA patients and had an 90% specificity (123/136) overall. Statistics regarding percentage of correctly identified sub-classes of AA using the model are provided as follows: 40% (2/5) for those diagnosed as having advanced adenoma with a serrated lesion <10 mm with dysplasia, 44% (8/18) for adenomas with villious component, 50% (8/16) for low grade >=1cm tubular adenoma, 68% (17/25) for patients with high grade dysplasia, 70% (7/10) for serrated lesion >=1cm, and 71% (5/7) for carcinoma in situ. Additionally, statistics are provided in FIG. 12 regarding the model's sensitivity for AA found in various locations in the colon and rectum. For example, the sensitivity of the model was 52.5% for proximal AAs (AAs proximal to the splenic flexure and located in the colon), 70% for distal AAs (AAs distal to the splenic flexure and located in the colon), and 50% for AAs located in the rectum.

All 220 contributing regions were also further analyzed using a KEGG pathway analysis. The results of the KEGG pathway analysis are presented below in Table 13. The main contributing pathways were linked to cancer as well as signaling pathways, affected in cancer development.

Table 13. KEGG Pathway Analysis Results.

| DAVID KEGG pathways |
|---|
| |
| Term |
| hsa05200:Pathways in cancer |
| hsa04510:Focal adhesion |
| hsa04810:Regulation of actin cytoskeleton |
| hsa04015:Rap1 signaling pathway |
| hsa05214:Glioma |
| hsa04010:MAPK signaling pathway |
| hsa05218: Melanoma |
| hsa04350:TGF-beta signaling pathway |

Subset analysis of 220 DMRs (as shown in FIG. 3) showed that subsets of DMRs performed surprisingly well in distinguishing between AA and control samples in the validation set. It was found that combinations of 2, 4, 9, and 24 DMRs performed well in identifying AA subjects from control subjects. For example, a panel of only two DMRs showed an AUC of 82%. Though accuracy improves with increased numbers of DMRs, these smaller DMR panels may also be useful in identifying subjects suffering from AA. Results of these subsets of DMR combinations are presented below in Table 14.

Table 14. Advanced Adenoma (AA) DMR panels statistics.

|  | 2 | 4 | 10 | 220 |
|---|---|---|---|---|
| AUC | 0.51 | 0.52 | 0.54 | 0.82 |
| Sensitivity | 0.21 | 0.26 | 0.31 | 0.54 |
| Specificity | 0.82 | 0.77 | 0.74 | 0.90 |
| Accuracy | 0.59 | 0.57 | 0.58 | 0.70 |
| Kappa | 0.03 | 0.03 | 0.06 | 0.27 |

Tables 15, 16, and 17 (presented below) correspond to the 2, 4, and 10 DMR combinations, respectively, indicated in Table 14. DMRs listed in Tables 15, 16, and 17 are also found in the 220 DMR panel shown in FIG. 3. Though the best performing DMR panel was the 220 DMR panel, smaller panels proved also to be surprisingly useful. For example, the 2-DMR panel had a surprisingly high specificity as compared to the 4 and 10 DMR panels. Moreover, the AUC value of the 2-marker panel demonstrated that the DMRs of the 2-DMR panel contributed significantly to the AUC of the 220-DMR panel.

Table 15. 2-DMR Panel for AA.

| chr | start | end | SEQ ID NO. |
|---|---|---|---|
| 7 | 100785927 | 100786167 | SEQ ID NO.: 221 |
| 14 | 97412990 | 97413410 | SEQ ID NO.: 374 |

Table 16. 4-DMR Panel for AA.

| chr | start | end | SEQ ID NO. |
|---|---|---|---|
| 7 | 100785927 | 100786167 | SEQ ID NO.: 221 |
| 14 | 97412990 | 97413410 | SEQ ID NO.: 374 |
| 20 | 3083167 | 3083587 | SEQ ID NO.: 411 |
| 8 | 37797956 | 37798676 | SEQ ID NO.: 329 |

Table 17. 10-DMR Panel for AA.

| chr | start | end | SEQ ID NO. |
|---|---|---|---|
| 7 | 100785927 | 100786167 | SEQ ID NO.: 221 |
| 14 | 97412990 | 97413410 | SEQ ID NO.: 374 |
| 20 | 3083167 | 3083587 | SEQ ID NO.: 411 |
| 8 | 37797956 | 37798676 | SEQ ID NO.: 329 |
| 16 | 57091834 | 57092014 | SEQ ID NO.: 387 |
| 4 | 7940020 | 7940200 | SEQ ID NO.: 287 |
| 19 | 40811045 | 40811585 | SEQ ID NO.: 403 |
| 1 | 154567391 | 154567691 | SEQ ID NO.: 246 |
| 14 | 105364294 | 105364612 | SEQ ID NO.: 376 |
| 9 | 61862430 | 61863030 | SEQ ID NO.: 338 |

In some embodiments, the present disclosure includes combinations of DMRs in which each of the DMRs is, includes all of, includes a portion of, or is present in a gene identified as being associated with a DMR of Table 15 (e.g., as shown in FIG. 2). In some embodiments, the present disclosure includes combinations of DMRs in which each of the DMRs is, includes all of, includes a portion of, or is present in a gene identified as being associated with a DMR of Table 16 (e.g., as shown in FIG. 2). In some embodiments, the present disclosure includes combinations of DMRs in which each of the DMRs is, includes all of, includes a portion of, or is present in a gene identified as being associated with a DMR of Table 17 (e.g., as shown in FIG. 2).

Example 3: Bisulfite conversion vs enzymatic conversion

The purpose of this Example is to demonstrate the usefulness of both bisulfite and enzymatic conversion in identifying methylation sites using NGS assays. In particular, both methods are shown herein to be useful in determining the presence of colorectal cancer. In addition, this Example shows the effect of different processing steps, such as removal of duplicates (e.g., optical duplicates, PCR duplicates), on the amount of data obtained from NGS assays. Deduplication is shown to remove a significant number of reads from the bioinformatics analysis pipeline.

FIG. 13 shows a series of bioinformatics steps (1300) conducted on sequencing data to remove duplicate sequences. Read data acquired from a NGS sequencing technique was acquired as described herein (1310). In this example, sequencing data is obtained from bisulfite and enzymatically converted DNA fragments. Reads obtained from the sequencing data were then aligned to a reference sequence (1320). Reads that correspond to optical duplicates were then removed (1330). Following optical duplicate removal, PCR duplicates (also known as library duplicates) were then removed (1340). Finally, the deduplicated reads were quality filtered (1350), which resulted in the removal of additional reads.

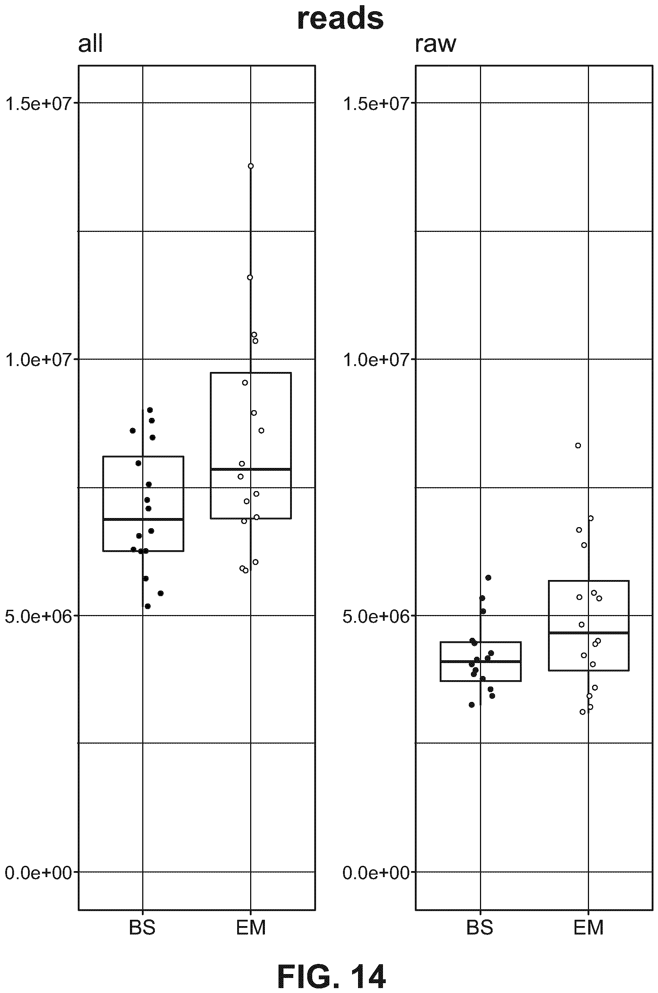

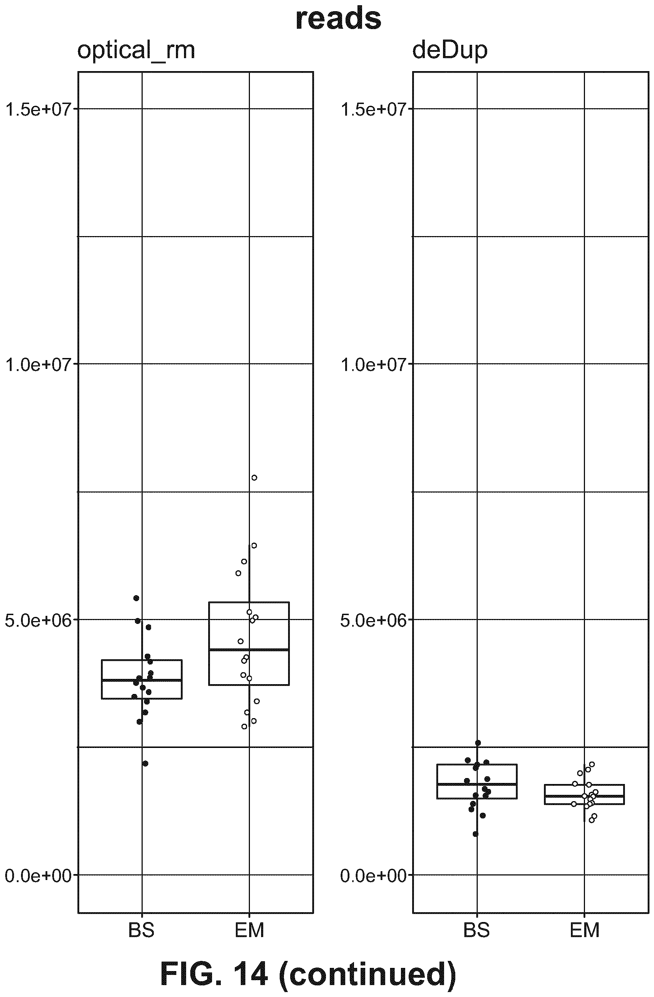

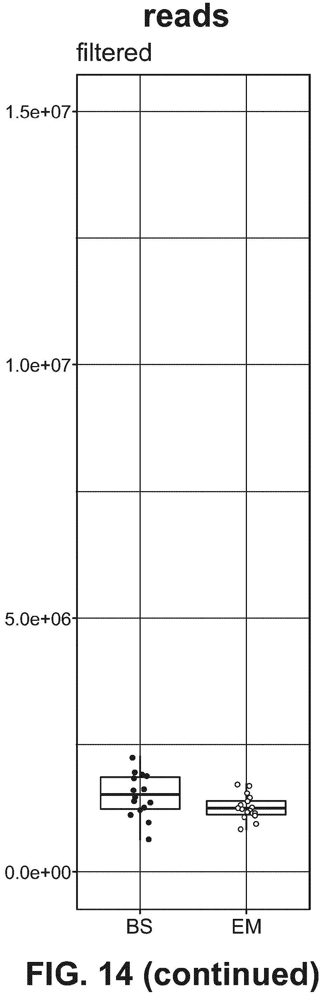

In this example, a comparative analysis was performed for 16 sample pairs prepared with bisulfite and enzymatic conversion in order to compare the performance of the two conversion methods. FIG. 14 shows series of panels comparing bisulfite (BS) and enzymatic (EM) conversion data quality after samples are subjected to different bioinformatics analyses. The y-axis indicates the number of reads present at various stages of the analysis. Samples treated with enzymatic conversion method initially presented with higher amount data (e.g., more reads) acquired as shown in the panel titled "all". After alignment, reads that do not align to target regions were removed from further analysis. The number of aligned reads are shown in the panel titled "raw". Fewer reads were present after alignment in both conditions, though there were still more reads (on average) present in the samples of the EM data set. Following read alignment, reads corresponding to optical duplicates were removed ("optical_rm"). The panel titled "optical_rm" shows the number of remaining reads after optical duplicates were removed. Following optical duplicate removal, PCR duplicates were removed ("deDup") from the samples. Finally, files were quality filtered ("filtered") to remove any remaining erroneous reads. As can be seen in FIG. 14, the number of reads remaining in both the bisulfite and enzymatic converted samples were similar.

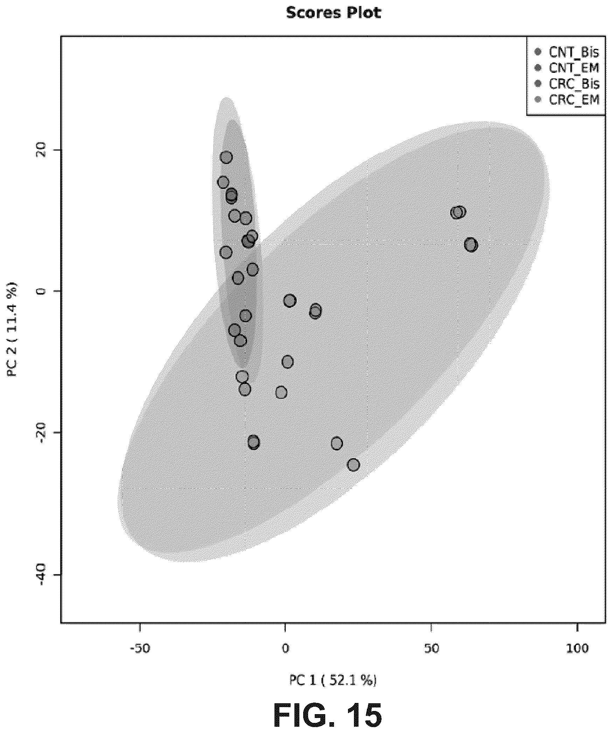

Then PCA (principle component analysis) was performed on the resulting data using a 203 CRC marker panel (FIG. 2). The different condition groups were defined as follows: CRC samples prepared with bisulfite conversion (CRC_Bis), CRC samples prepared with enzymatic conversion (CRC_EM), control samples prepared with bisulfite conversion (CNT_Bis) and control samples prepared with enzymatic conversion (CNT_EM). Note that each of the 16 samples were prepared using both bisulfite and enzymatic conversion. Ideally, results between the two groups should be the same, regardless of whether bisulfite or enzymatic conversion is used. As can be seen on the PCA plot in FIG. 15 the biggest separation can be seen between the two conditions (colorectal cancer and control samples) and not between the conversion methods. As can be seen in the PCA plot, the sample pairs appear plotted closely together.

Further analysis using prediction algorithm developed previously on the 68 samples resulted in sample pairs being classified similarly with similar prediction scores as shown in Table 18 below.

Table 18. Paired samples prepared with enzymatic bisulfite conversion.

| Sample | PredictionScore | Prediction | Reference | Match |
|---|---|---|---|---|
| UDX016525_Bis | 0.43 | CNT | CNT | TRUE |
| UDX016525_EM | 0.44 | CNT | CNT | TRUE |
| UDX016707_Bis | 0.68 | CRC | CRC | TRUE |
| UDX016707_EM | 0.69 | CRC | CRC | TRUE |
| UDX017209_Bis | 0.46 | CNT | CNT | TRUE |
| UDX017209_EM | 0.45 | CNT | CNT | TRUE |
| UDX017309_Bis | 0.45 | CNT | CNT | TRUE |
| UDX017309_EM | 0.47 | CNT | CNT | TRUE |

(continued)

| Sample | PredictionScore | Prediction | Reference | Match |
|---|---|---|---|---|
| UDX017397_Bis | 0.42 | CNT | CNT | TRUE |
| UDX017397_EM | 0.38 | CNT | CNT | TRUE |
| UDX017440_Bis | 0.60 | CRC | CRC | TRUE |
| UDX017440_EM | 0.57 | CRC | CRC | TRUE |
| UDX017732_Bis | 0.64 | CRC | CRC | TRUE |
| UDX017732_EM | 0.64 | CRC | CRC | TRUE |
| UDX018506_Bis | 0.91 | CRC | CRC | TRUE |
| UDX018506_EM | 0.91 | CRC | CRC | TRUE |
| UDX018688_Bis | 0.80 | CRC | CRC | TRUE |
| UDX018688_EM | 0.80 | CRC | CRC | TRUE |
| UDX018828_Bis | 0.44 | CNT | CNT | TRUE |
| UDX018828_EM | 0.46 | CNT | CNT | TRUE |
| UDX018948_Bis | 0.39 | CNT | CNT | TRUE |
| UDX018948_EM | 0.46 | CNT | CNT | TRUE |
| UDX019177_Bis | 0.35 | CNT | CNT | TRUE |
| UDX019177_EM | 0.34 | CNT | CNT | TRUE |
| UDX019715_Bis | 0.40 | CNT | CRC | FALSE |
| UDX019715_EM | 0.41 | CNT | CRC | FALSE |
| UDX019764_Bis | 0.38 | CNT | CNT | TRUE |
| UDX019764_EM | 0.44 | CNT | CNT | TRUE |

The sample name provide a unique sample ID, along with the method of preparation - enzymatic (_EM) conversion or bisulfite (_Bis) conversion. A prediction score for each sample was generated using a random forest (RF) prediction model. In this case, the RF prediction model is a colorectal cancer prediction model, which used the methylation status of the 203 markers of FIG. 2 to predict whether or not a particular subject was suffering from CRC. A prediction score above 0.5 was correlated with a subject having CRC, while a prediction score below 0.5 was correlated with a subject being in a control group. The group to which the sample is predicted to belong to is shown in the "prediction" column. The "reference" column is indicative of the group to which the subject actually belonged. As can be seen from the results, a bisulfite treated sample and the corresponding enzyme treated sample produced similar prediction scores and resulted in the same diagnosis. The single misclassified CRC sample pair (UDX019715_Bis and UDX019715_EM) is from a subject having stage II CRC.

Computer System and Network Environment

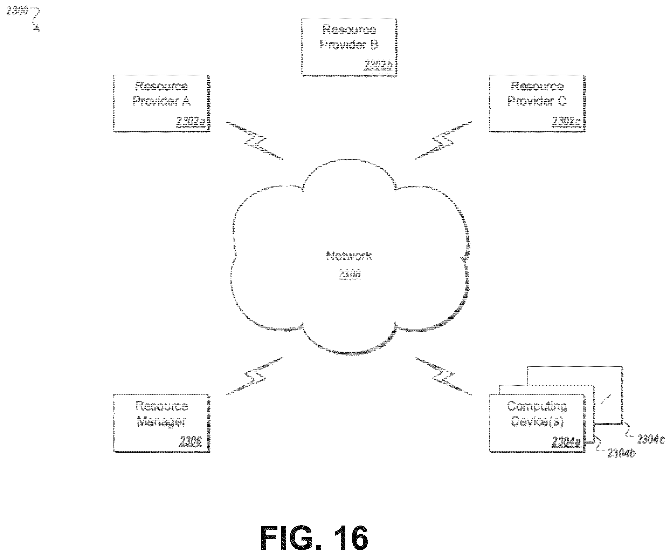

As shown in FIG. 16, an implementation of a network environment 2300 for use in providing systems, methods, and architectures for identifying biomarkers for detection of a disease or condition such as advanced adenoma, colorectal cancer, other cancers, or other diseases or conditions associated with an aberrant methylation status as described herein is shown and described. In brief overview, referring now to FIG. 16, a block diagram of an exemplary cloud computing environment 2300 is shown and described. The cloud computing environment 2300 may include one or more resource providers 2302a, 2302b, 2302c (collectively, 2302). Each resource provider 2302 may include computing resources. In some implementations, computing resources may include any hardware and/or software used to process data. For example, computing resources may include hardware and/or software capable of executing algorithms, computer programs, and/or computer applications. In some implementations, exemplary computing resources may include application servers and/or databases with storage and retrieval capabilities. Each resource provider 2302 may be connected to any other resource provider 2302 in the cloud computing environment 2300. In some implementations, the resource providers 2302 may be connected over a computer network 2308. Each resource provider 2302 may be connected to one or more computing device 2304a, 2304b, 2304c (collectively, 2304), over the computer network 2308.

The cloud computing environment 2300 may include a resource manager 2306. The resource manager 2306 may be connected to the resource providers 2302 and the computing devices 2304 over the computer network 2308. In some implementations, the resource manager 2306 may facilitate the provision of computing resources by one or more resource providers 2302 to one or more computing devices 2304. The resource manager 2306 may receive a request for a computing resource from a particular computing device 2304. The resource manager 2306 may identify one or more resource providers 2302 capable of providing the computing resource requested by the computing device 2304. The resource manager 2306 may select a resource provider 2302 to provide the computing resource. The resource manager 2306 may facilitate a connection between the resource provider 2302 and a particular computing device 2304. In some implementations, the resource manager 2306 may establish a connection between a particular resource provider 2302 and a particular computing device 2304. In some implementations, the resource manager 2306 may redirect a particular computing device 2304 to a particular resource provider 2302 with the requested computing resource.

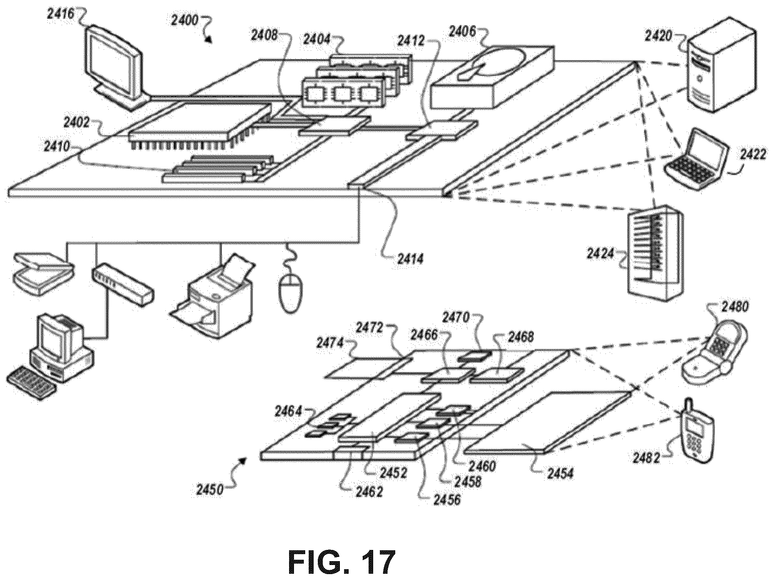

FIG. 17 shows an example of a computing device 2400 and a mobile computing device 2450 that can be used to implement the techniques described in this disclosure. The computing device 2400 is intended to represent various forms of digital computers, such as laptops, desktops, workstations, personal digital assistants, servers, blade servers, mainframes, and other appropriate computers. The mobile computing device 2450 is intended to represent various forms of mobile devices, such as personal digital assistants, cellular telephones, smart-phones, and other similar computing devices. The components shown here, their connections and relationships, and their functions, are meant to be examples only, and are not meant to be limiting.

The computing device 2400 includes a processor 2402, a memory 2404, a storage device 2406, a high-speed interface 2408 connecting to the memory 2404 and multiple high-speed expansion ports 2410, and a low-speed interface 2412 connecting to a low-speed expansion port 2414 and the storage device 2406. Each of the processor 2402, the memory 2404, the storage device 2406, the high-speed interface 2408, the high-speed expansion ports 2410, and the low-speed interface 2412, are interconnected using various busses, and may be mounted on a common motherboard or in other manners as appropriate. The processor 2402 can process instructions for execution within the computing device 2400, including instructions stored in the memory 2404 or on the storage device 2406 to display graphical information for a GUI on an external input/output device, such as a display 2416 coupled to the high-speed interface 2408. In other implementations, multiple processors and/or multiple buses may be used, as appropriate, along with multiple memories and types of memory. Also, multiple computing devices may be connected, with each device providing portions of the necessary operations (e.g., as a server bank, a group of blade servers, or a multiprocessor system). Thus, as the term is used herein, where a plurality of functions are described as being performed by "a processor", this encompasses embodiments wherein the plurality of functions are performed by any number of processors (one or more) of any number of computing devices (one or more). Furthermore, where a function is described as being performed by "a processor", this encompasses embodiments wherein the function is performed by any number of processors (one or more) of any number of computing devices (one or more) (e.g., in a distributed computing system). The memory 2404 stores information within the computing device 2400. In some implementations, the memory 2404 is a volatile memory unit or units. In some implementations, the memory 2404 is a non-volatile memory unit or units. The memory 2404 may also be another form of computer-readable medium, such as a magnetic or optical disk.

The storage device 2406 is capable of providing mass storage for the computing device 2400. In some implementations, the storage device 2406 may be or contain a computer-readable medium, such as a floppy disk device, a hard disk device, an optical disk device, or a tape device, a flash memory or other similar solid state memory device, or an array of devices, including devices in a storage area network or other configurations. Instructions can be stored in an information carrier. The instructions, when executed by one or more processing devices (for example, processor 2402), perform one or more methods, such as those described above. The instructions can also be stored by one or more storage devices such as computer- or machine-readable mediums (for example, the memory 2404, the storage device 2406, or memory on the processor 2402).

The high-speed interface 2408 manages bandwidth-intensive operations for the computing device 2400, while the low-speed interface 2412 manages lower bandwidth-intensive operations. Such allocation of functions is an example only. In some implementations, the high-speed interface 2408 is coupled to the memory 2404, the display 2416 (e.g., through a graphics processor or accelerator), and to the high-speed expansion ports 2410, which may accept various expansion cards (not shown). In the implementation, the low-speed interface 2412 is coupled to the storage device 2406 and the low-speed expansion port 2414. The low-speed expansion port 2414, which may include various communication ports (e.g., USB, Bluetooth®, Ethernet, wireless Ethernet) may be coupled to one or more input/output devices, such as a keyboard, a pointing device, a scanner, or a networking device such as a switch or router, e.g., through a network adapter.

The computing device 2400 may be implemented in a number of different forms, as shown in the figure. For example, it may be implemented as a standard server 2420, or multiple times in a group of such servers. In addition, it may be implemented in a personal computer such as a laptop computer 2422. It may also be implemented as part of a rack server system 2424. Alternatively, components from the computing device 2400 may be combined with other components in a mobile device (not shown), such as a mobile computing device 2450. Each of such devices may contain one or more of the computing device 2400 and the mobile computing device 2450, and an entire system may be made up of multiple computing devices communicating with each other.

The mobile computing device 2450 includes a processor 2452, a memory 2464, an input/output device such as a display 2454, a communication interface 2466, and a transceiver 2468, among other components. The mobile computing device 2450 may also be provided with a storage device, such as a micro-drive or other device, to provide additional storage. Each of the processor 2452, the memory 2464, the display 2454, the communication interface 2466, and the transceiver 2468, are interconnected using various buses, and several of the components may be mounted on a common motherboard or in other manners as appropriate.

The processor 2452 can execute instructions within the mobile computing device 2450, including instructions stored in the memory 2464. The processor 2452 may be implemented as a chipset of chips that include separate and multiple analog and digital processors. The processor 2452 may provide, for example, for coordination of the other components of the mobile computing device 2450, such as control of user interfaces, applications run by the mobile computing device 2450, and wireless communication by the mobile computing device 2450.

The processor 2452 may communicate with a user through a control interface 2458 and a display interface 2456 coupled to the display 2454. The display 2454 may be, for example, a TFT (Thin-Film-Transistor Liquid Crystal Display) display or an OLED (Organic Light Emitting Diode) display, or other appropriate display technology. The display interface 2456 may comprise appropriate circuitry for driving the display 2454 to present graphical and other information to a user. The control interface 2458 may receive commands from a user and convert them for submission to the processor 2452. In addition, an external interface 2462 may provide communication with the processor 2452, so as to enable near area communication of the mobile computing device 2450 with other devices. The external interface 2462 may provide, for example, for wired communication in some implementations, or for wireless communication in other implementations, and multiple interfaces may also be used.

The memory 2464 stores information within the mobile computing device 2450. The memory 2464 can be implemented as one or more of a computer-readable medium or media, a volatile memory unit or units, or a non-volatile memory unit or units. An expansion memory 2474 may also be provided and connected to the mobile computing device 2450 through an expansion interface 2472, which may include, for example, a SIMM (Single In Line Memory Module) card interface. The expansion memory 2474 may provide extra storage space for the mobile computing device 2450, or may also store applications or other information for the mobile computing device 2450. Specifically, the expansion memory 2474 may include instructions to carry out or supplement the processes described above, and may include secure information also. Thus, for example, the expansion memory 2474 may be provide as a security module for the mobile computing device 2450, and may be programmed with instructions that permit secure use of the mobile computing device 2450. In addition, secure applications may be provided via the SIMM cards, along with additional information, such as placing identifying information on the SIMM card in a non-hackable manner.

The memory may include, for example, flash memory and/or NVRAM memory (non-volatile random access memory), as discussed below. In some implementations, instructions are stored in an information carrier. that the instructions, when executed by one or more processing devices (for example, processor 2452), perform one or more methods, such as those described above. The instructions can also be stored by one or more storage devices, such as one or more computer- or machine-readable mediums (for example, the memory 2464, the expansion memory 2474, or memory on the processor 2452). In some implementations, the instructions can be received in a propagated signal, for example, over the transceiver 2468 or the external interface 2462. The mobile computing device 2450 may communicate wirelessly through the communication interface 2466, which may include digital signal processing circuitry where necessary. The communication interface 2466 may provide for communications under various modes or protocols, such as GSM voice calls (Global System for Mobile communications), SMS (Short Message Service), EMS (Enhanced Messaging Service), or MMS messaging (Multimedia Messaging Service), CDMA (code division multiple access), TDMA (time division multiple access), PDC (Personal Digital Cellular), WCDMA (Wideband Code Division Multiple Access), CDMA2000, or GPRS (General Packet Radio Service), among others. Such communication may occur, for example, through the transceiver 2468 using a radio-frequency. In addition, short-range communication may occur, such as using a Bluetooth®, Wi-Fi™, or other such transceiver (not shown). In addition, a GPS (Global Positioning System) receiver module 2470 may provide additional navigation- and location-related wireless data to the mobile computing device 2450, which may be used as appropriate by applications running on the mobile computing device 2450.

The mobile computing device 2450 may also communicate audibly using an audio codec 2460, which may receive spoken information from a user and convert it to usable digital information. The audio codec 2460 may likewise generate audible sound for a user, such as through a speaker, e.g., in a handset of the mobile computing device 2450. Such sound may include sound from voice telephone calls, may include recorded sound (e.g., voice messages, music files, etc.) and may also include sound generated by applications operating on the mobile computing device 2450.

The mobile computing device 2450 may be implemented in a number of different forms, as shown in the figure. For example, it may be implemented as a cellular telephone 2480. It may also be implemented as part of a smart-phone 2482, personal digital assistant, or other similar mobile device.

Various implementations of the systems and techniques described here can be realized in digital electronic circuitry, integrated circuitry, specially designed ASICs (application specific integrated circuits), computer hardware, firmware, software, and/or combinations thereof. These various implementations can include implementation in one or more computer programs that are executable and/or interpretable on a programmable system including at least one programmable processor, which may be special or general purpose, coupled to receive data and instructions from, and to transmit data and instructions to, a storage system, at least one input device, and at least one output device.

These computer programs (also known as programs, software, software applications or code) include machine instructions for a programmable processor, and can be implemented in a high-level procedural and/or object-oriented programming language, and/or in assembly/machine language. As used herein, the terms machine-readable medium and computer-readable medium refer to any computer program product, apparatus and/or device (e.g., magnetic discs, optical disks, memory, Programmable Logic Devices (PLDs)) used to provide machine instructions and/or data to a programmable processor, including a machine-readable medium that receives machine instructions as a machine-readable signal. The term machine-readable signal refers to any signal used to provide machine instructions and/or data to a programmable processor.

To provide for interaction with a user, the systems and techniques described here can be implemented on a computer having a display device (e.g., a CRT (cathode ray tube) or LCD (liquid crystal display) monitor) for displaying information to the user and a keyboard and a pointing device (e.g., a mouse or a trackball) by which the user can provide input to the computer. Other kinds of devices can be used to provide for interaction with a user as well; for example, feedback provided to the user can be any form of sensory feedback (e.g., visual feedback, auditory feedback, or tactile feedback); and input from the user can be received in any form, including acoustic, speech, or tactile input.

The systems and techniques described here can be implemented in a computing system that includes a back end component (e.g., as a data server), or that includes a middleware component (e.g., an application server), or that includes a front end component (e.g., a client computer having a graphical user interface or a Web browser through which a user can interact with an implementation of the systems and techniques described here), or any combination of such back end, middleware, or front end components. The components of the system can be interconnected by any form or medium of digital data communication (e.g., a communication network). Examples of communication networks include a local area network (LAN), a wide area network (WAN), and the Internet.

The computing system can include clients and servers. A client and server are generally remote from each other and typically interact through a communication network. The relationship of client and server arises by virtue of computer programs running on the respective computers and having a client-server relationship to each other.

Elements of different implementations described herein may be combined to form other implementations not specifically set forth above. Elements may be left out of the processes, computer programs, databases, etc. described herein without adversely affecting their operation. In addition, the logic flows depicted in the figures do not require the particular order shown, or sequential order, to achieve desirable results. Various separate elements may be combined into one or more individual elements to perform the functions described herein.

Throughout the description, where apparatus and systems are described as having, including, or comprising specific components, or where processes and methods are described as having, including, or comprising specific steps, it is contemplated that, additionally, there are apparatus, and systems of the present invention that consist essentially of, or consist of, the recited components, and that there are processes and methods according to the present invention that consist essentially of, or consist of, the recited processing steps.

It should be understood that the order of steps or order for performing certain action is immaterial so long as the invention remains operable. Moreover, two or more steps or actions may be conducted simultaneously.

**Claims**

1. A method of detecting colorectal cancer in a human subject, the method comprising:

   determining a methylation status of
   at least a portion of each of the following DMRs of Table 9:

| chr | start | end | SEQ ID NO. |
|---|---|---|---|
| 7 | 96997902 | 96999222 | SEQ ID NO.: 92 |
| 8 | 96145538 | 96145718 | SEQ ID NO.: 108 |
| 2 | 100322218 | 100322818 | SEQ ID NO.: 28 |

(continued)

| chr | start | end | SEQ ID NO. |
|---|---|---|---|
| 2 | 29115776 | 29116791 | SEQ ID NO.: 17 |

identified in DNA from a sample obtained from the subject, and
determining whether the subject has colorectal cancer based at least in part on the determined methylation status of at least a portion of each of the DMRs of Table 9.,

2. The method of claim 1, wherein the method comprises determining a methylation status of at least a portion of each of the following DMRs of Table 10:

| chr | start | end | SEQ ID NO. |
|---|---|---|---|
| 7 | 96997902 | 96999222 | SEQ ID NO.: 92 |
| 8 | 96145538 | 96145718 | SEQ ID NO.: 108 |
| 2 | 100322218 | 100322818 | SEQ ID NO.: 28 |
| 2 | 29115776 | 29116791 | SEQ ID NO.: 17 |
| 2 | 88765502 | 88766042 | SEQ ID NO.: 25 |
| 4 | 153249541 | 153249721 | SEQ ID NO.: 55 |
| 2 | 86790271 | 86790811 | SEQ ID NO.: 24 |
| 2 | 176094518 | 176094878 | SEQ ID NO.: 35 |
| 3 | 37453325 | 37453874 | SEQ ID NO.: 41 |

identified in DNA from a sample obtained from the subject, and
determining whether the subject has colorectal cancer based at least in part on the determined methylation status of at least a portion of each of the DMRs of Table 10.

3. The method of claims 1-2, wherein the method comprises determining a methylation status of at least a portion of each of the following DMRs of Table 11 :

| chr | start | end | SEQ ID NO. |
|---|---|---|---|
| 7 | 96997902 | 96999222 | SEQ ID NO.: 92 |
| 8 | 96145538 | 96145718 | SEQ ID NO.: 108 |
| 2 | 100322218 | 100322818 | SEQ ID NO.: 28 |
| 2 | 29115776 | 29116791 | SEQ ID NO.: 17 |
| 2 | 88765502 | 88766042 | SEQ ID NO.: 25 |
| 4 | 153249541 | 153249721 | SEQ ID NO.: 55 |
| 2 | 86790271 | 86790811 | SEQ ID NO.: 24 |
| 2 | 176094518 | 176094878 | SEQ ID NO.: 35 |
| 3 | 37453325 | 37453874 | SEQ ID NO.: 41 |
| 10 | 43105168 | 43105348 | SEQ ID NO.: 124 |
| 7 | 90596855 | 90597335 | SEQ ID NO.: 90 |
| 6 | 28988380 | 28988560 | SEQ ID NO.: 69 |
| 19 | 53254542 | 53254962 | SEQ ID NO.: 190 |
| 7 | 141072108 | 141073057 | SEQ ID NO.: 99 |
| 14 | 104117671 | 104117851 | SEQ ID NO.: 159 |

(continued)

| chr | start | end | SEQ ID NO. |
|---|---|---|---|
| 6 | 163413139 | 163413679 | SEQ ID NO.: 80 |
| 5 | 38555799 | 38556399 | SEQ ID NO.: 60 |
| 2 | 95025733 | 95026933 | SEQ ID NO.: 26 |
| 7 | 35257235 | 35257535 | SEQ ID NO.: 86 |
| 1 | 2132912 | 2133092 | SEQ ID NO.: 2 |
| 3 | 128491607 | 128492807 | SEQ ID NO.: 49 |
| 20 | 23049079 | 23049259 | SEQ ID NO.: 193 |
| 1 | 243917149 | 243917569 | SEQ ID NO.: 15 |
| 1 | 38045915 | 38046095 | SEQ ID NO.: 6 |

identified in DNA from a sample obtained from the subject, and
determining whether the subject has colorectal cancer based at least in part on the determined methylation status of at least a portion of each of the DMRs of Table 11.

4. The method of claim 1-3, wherein the method comprises determining a methylation status of at least a portion of each of the DMRs of Figure 2

identified in DNA from a sample obtained from the subject, and
determining whether the subject has colorectal cancer based at least in part on the determined methylation status of at least a portion of each of the DMRs of Figure 2.

5. The method of any one of claims 1-4, wherein the sample is a tissue sample, a blood sample, a stool sample, or a blood product sample.

6. The method of any one of claims 1-5, wherein the sample comprises DNA that is isolated from blood or plasma of the human subject.

7. The method of any one of claims 1-6, wherein the DNA is cell-free DNA (cfDNA) of the human subject.

8. The method of any one of claims 1-7, wherein the method comprises determining the methylation status of each of the one or more markers using next generation sequencing (NGS).

9. The method of any one of claims 1-8, wherein the method comprises using one or more capture baits that enrich for a target region to capture one or more corresponding methylation locus / loci.

10. The method of any one of claims 1-9, wherein each of the DMRs of Table 9, Table 10, Table 11 and/or Figure 2 is a methylation locus comprising at least a portion, each said portion comprising at least three (3) CpGs and each said methylation locus having a length equal to or less than 5000 bp.

11. The method according to claim 10, wherein each methylation locus is equal to or less than 3000bp in length.

**Patentansprüche**

1. Verfahren zum Nachweisen von kolorektalem Krebs in einem menschlichen Individuum, wobei das Verfahren umfasst:

Bestimmen eines Methylierungsstatus von mindestens einem Teil jeder der folgenden DMRs aus Tabelle 9:

| chr | Start | Ende | SEQ ID NO. |
|---|---|---|---|
| 7 | 96997902 | 96999222 | SEQ ID NO.: 92 |
| 8 | 96145538 | 96145718 | SEQ ID NO.: 108 |
| 2 | 100322218 | 100322818 | SEQ ID NO.: 28 |
| 2 | 29115776 | 29116791 | SEQ ID NO.: 17 |

die in der DNA einer von dem Individuum gewonnenen Probe identifiziert wurden, und
Bestimmen, ob das Individuum kolorektalen Krebs hat, zumindest teilweise basierend auf dem bestimmten Methylierungsstatus von zumindest einem Teil jeder der DMRs aus Tabelle 9.

2. Verfahren nach Anspruch 1, wobei das Verfahren das Bestimmen eines Methylierungsstatus von zumindest einem Teil jeder der folgenden DMRs aus Tabelle 10 umfasst:

| chr | Start | Ende | SEQ ID NO. |
|---|---|---|---|
| 7 | 96997902 | 96999222 | SEQ ID NO.: 92 |
| 8 | 96145538 | 96145718 | SEQ ID NO.: 108 |
| 2 | 100322218 | 100322818 | SEQ ID NO.: 28 |
| 2 | 29115776 | 29116791 | SEQ ID NO.: 17 |
| 2 | 88765502 | 88766042 | SEQ ID NO.: 25 |
| 4 | 153249541 | 153249721 | SEQ ID NO.: 55 |
| 2 | 86790271 | 86790811 | SEQ ID NO.: 24 |
| 2 | 176094518 | 176094878 | SEQ ID NO.: 35 |
| 3 | 37453325 | 37453874 | SEQ ID NO.: 41 |

die in der DNA einer von dem Individuum gewonnenen Probe identifiziert wurden, und
Bestimmen, ob das Individuum kolorektalen Krebs hat, zumindest teilweise basierend auf dem bestimmten Methylierungsstatus von zumindest einem Teil jeder der DMRs aus Tabelle 10.

3. Verfahren nach Anspruch 1-2, wobei das Verfahren das Bestimmen eines Methylierungsstatus von zumindest einem Teil jeder der folgenden DMRs aus Tabelle 11 umfasst:

| chr | Start | Ende | SEQ ID NO. |
|---|---|---|---|
| 7 | 96997902 | 96999222 | SEQ ID NO.:92 |
| 8 | 96145538 | 96145718 | SEQ ID NO.:108 |
| 2 | 100322218 | 100322818 | SEQ ID NO.:28 |
| 2 | 29115776 | 29116791 | SEQ ID NO.:17 |
| 2 | 88765502 | 88766042 | SEQ ID NO.:25 |
| 4 | 153249541 | 153249721 | SEQ ID NO.:55 |
| 2 | 86790271 | 86790811 | SEQ ID NO.:24 |
| 2 | 176094518 | 176094878 | SEQ ID NO.:35 |
| 3 | 37453325 | 37453874 | SEQ ID NO.:41 |
| 10 | 43105168 | 43105348 | SEQ ID NO.:124 |
| 7 | 90596855 | 90597335 | SEQ ID NO.:90 |
| 6 | 28988380 | 28988560 | SEQ ID NO.:69 |
| 19 | 53254542 | 53254962 | SEQ ID NO.:190 |

(continued)

| chr | Start | Ende | SEQ ID NO. |
|---|---|---|---|
| 7 | 141072108 | 141073057 | SEQ ID NO.:99 |
| 14 | 104117671 | 104117851 | SEQ ID NO.:159 |
| 6 | 163413139 | 163413679 | SEQ ID NO.:80 |
| 5 | 38555799 | 38556399 | SEQ ID NO.:60 |
| 2 | 95025733 | 95026933 | SEQ ID NO.:26 |
| 7 | 35257235 | 35257535 | SEQ ID NO.:86 |
| 1 | 2132912 | 2133092 | SEQ ID NO.:2 |
| 3 | 128491607 | 128492807 | SEQ ID NO.:49 |
| 20 | 23049079 | 23049259 | SEQ ID NO.:193 |
| 1 | 243917149 | 243917569 | SEQ ID NO.:15 |
| 1 | 38045915 | 38046095 | SEQ ID NO.:6 |

die in der DNA einer von dem Individuum gewonnenen Probe identifiziert wurden, und
Bestimmen, ob das Individuum kolorektalen Krebs hat, zumindest teilweise basierend auf dem bestimmten Methylierungsstatus von zumindest einem Teil jeder der DMRs aus Tabelle 11.

4. Verfahren nach Anspruch 1-3, wobei das Verfahren das Bestimmen eines Methylierungsstatus von zumindest einem Teil jeder der DMRs aus Figur 2 umfasst, die in DNA aus einer von dem Individuum gewonnenen Probe identifiziert wurden, und
Bestimmen, ob das Individuum kolorektalen Krebs hat, zumindest teilweise basierend auf dem bestimmten Methylierungsstatus von zumindest einem Teil jeder der DMRs aus Figur 2.

5. Verfahren nach einem der Ansprüche 1-4, wobei die Probe eine Gewebeprobe, eine Blutprobe, eine Stuhlprobe oder eine Blutproduktprobe ist.

6. Verfahren nach einem der Ansprüche 1-5, wobei die Probe DNA umfasst, die aus Blut oder Plasma des menschlichen Individuums isoliert wurde.

7. Verfahren nach einem der Ansprüche 1-6, wobei die DNA zellfreie DNA (cfDNA) des menschlichen Individuums ist.

8. Verfahren nach einem der Ansprüche 1-7, wobei das Verfahren das Bestimmen des Methylierungsstatus jedes des einen oder der mehreren Marker unter Verwendung von Sequenzierung der nächsten Generation (NGS) umfasst.

9. Verfahren nach einem der Ansprüche 1-8, wobei das Verfahren die Verwendung einer oder mehrerer Fängersonden umfasst, die zum Anreichern einer Zielregion dienen, um einen oder mehrere entsprechende/n Methylierungs-Locus/-Loci zu erfassen.

10. Verfahren nach einem der Ansprüche 1-9, wobei jede der DMRs aus Tabelle 9, Tabelle 10, Tabelle 11 und/oder Figur 2 ein Methylierungs-Locus ist, der zumindest einen Abschnitt umfasst, wobei jeder Abschnitt zumindest drei (3) CpGs umfasst und jeder Methylierungs-Locus eine Länge von 5000 bp oder weniger aufweist.

11. Verfahren nach Anspruch 10, wobei jeder Methylierungs-Locus eine Länge von 3000bp oder weniger aufweist.

**Revendications**

1. Procédé de détection de cancer colorectal chez un sujet humain, le procédé comprenant :

la détermination d'un état de méthylation d'au moins une partie de chacune des DMR suivantes du tableau 9 :

| chr | début | fin | SEQ ID N° |
|---|---|---|---|
| 7 | 96997902 | 96999222 | SEQ ID N° : 92 |
| 8 | 96145538 | 96145718 | SEQ ID N° : 108 |
| 2 | 100322218 | 100322818 | SEQ ID N° : 28 |
| 2 | 29115776 | 29116791 | SEQ ID N° : 17 |

identifiées dans de l'ADN provenant d'un échantillon obtenu à partir du sujet et

le fait de déterminer si le sujet a un cancer colorectal sur la base au moins en partie de l'état de méthylation déterminé d'au moins une partie de chacune des DMR du tableau 9.

2. Procédé selon la revendication 1, le procédé comprenant la détermination d'un état de méthylation d'au moins une partie de chacune des DMR suivantes du tableau 10 :

| chr | début | fin | SEQ ID N° |
|---|---|---|---|
| 7 | 96997902 | 96999222 | SEQ ID N° : 92 |
| 8 | 96145538 | 96145718 | SEQ ID N° : 108 |
| 2 | 100322218 | 100322818 | SEQ ID N° : 28 |
| 2 | 29115776 | 29116791 | SEQ ID N° : 17 |
| 2 | 88765502 | 88766042 | SEQ ID N° : 25 |
| 4 | 153249541 | 153249721 | SEQ ID N° : 55 |
| 2 | 86790271 | 86790811 | SEQ ID N° : 24 |
| 2 | 176094518 | 176094878 | SEQ ID N° : 35 |
| 3 | 37453325 | 37453874 | SEQ ID N° : 41 |

identifiées dans de l'ADN provenant d'un échantillon obtenu à partir du sujet et

le fait de déterminer si le sujet a un cancer colorectal sur la base au moins en partie de l'état de méthylation déterminé d'au moins une partie de chacune des DMR du tableau 10.

3. Procédé selon les revendications 1 à 2, le procédé comprenant la détermination d'un état de méthylation d'au moins une partie de chacune des DMR suivantes du tableau 11 :

| chr | début | fin | SEQ ID N° |
|---|---|---|---|
| 7 | 96997902 | 96999222 | SEQ ID N° :92 |
| 8 | 96145538 | 96145718 | SEQ ID N° :108 |
| 2 | 100322218 | 100322818 | SEQ ID N° :28 |
| 2 | 29115776 | 29116791 | SEQ ID N° :17 |
| 2 | 88765502 | 88766042 | SEQ ID N° :25 |
| 4 | 153249541 | 153249721 | SEQ ID N° :55 |
| 2 | 86790271 | 86790811 | SEQ ID N° :24 |
| 2 | 176094518 | 176094878 | SEQ ID N° :35 |
| 3 | 37453325 | 37453874 | SEQ ID N° :41 |
| 10 | 43105168 | 43105348 | SEQ ID N° :124 |
| 7 | 90596855 | 90597335 | SEQ ID N° :90 |
| 6 | 28988380 | 28988560 | SEQ ID N° :69 |
| 19 | 53254542 | 53254962 | SEQ ID N° :190 |

(continued)

| chr | début | fin | SEQ ID N° |
|---|---|---|---|
| 7 | 141072108 | 141073057 | SEQ ID N° :99 |
| 14 | 104117671 | 104117851 | SEQ ID N° :159 |
| 6 | 163413139 | 163413679 | SEQ ID N° :80 |
| 5 | 38555799 | 38556399 | SEQ ID N° :60 |
| 2 | 95025733 | 95026933 | SEQ ID N° :26 |
| 7 | 35257235 | 35257535 | SEQ ID N° :86 |
| 1 | 2132912 | 2133092 | SEQ ID N° :2 |
| 3 | 128491607 | 128492807 | SEQ ID N° :49 |
| 20 | 23049079 | 23049259 | SEQ ID N° :193 |
| 1 | 243917149 | 243917569 | SEQ ID N° :15 |
| 1 | 38045915 | 38046095 | SEQ ID N° :6 |

identifiées dans de l'ADN provenant d'un échantillon obtenu à partir du sujet et
le fait de déterminer si le sujet a un cancer colorectal sur la base au moins en partie de l'état de méthylation déterminé d'au moins une partie de chacune des DMR du tableau 11.

4.  Procédé selon la revendication 1 à 3, le procédé comprenant la détermination d'un état de méthylation d'au moins une partie de chacune des DMR de la figure 2 identifiées dans de l'ADN provenant d'un échantillon obtenu à partir du sujet et
le fait de déterminer si le sujet a un cancer colorectal sur la base au moins en partie de l'état de méthylation déterminé d'au moins une partie de chacune des DMR de la figure 2.

5.  Procédé selon l'une quelconque des revendications 1 à 4, dans lequel l'échantillon est un échantillon de tissu, un échantillon de sang, un échantillon de selles ou un échantillon de produit sanguin.

6.  Procédé selon l'une quelconque des revendications 1 à 5, dans lequel l'échantillon comprend de l'ADN qui est isolé à partir de sang ou de plasma du sujet humain.

7.  Procédé selon l'une quelconque des revendications 1 à 6, dans lequel l'ADN est de l'ADN acellulaire (cfDNA) du sujet humain.

8.  Procédé selon l'une quelconque des revendications 1 à 7, le procédé comprenant la détermination de l'état de méthylation de chacun des un ou plusieurs marqueurs à l'aide d'un séquençage de nouvelle génération (NGS).

9.  Procédé selon l'une quelconque des revendications 1 à 8, le procédé comprenant l'utilisation d'un ou plusieurs appâts de capture qui s'enrichissent en ce qui concerne une région cible afin de capturer un ou plusieurs loci de méthylation correspondants.

10. Procédé selon l'une quelconque des revendications 1 à 9, dans lequel chacune des DMR du tableau 9, du tableau 10, du tableau 11 et/ou de la figure 2 est un locus de méthylation comprenant au moins une partie, chaque dite partie comprenant au moins trois (3) CpG et chaque dit locus de méthylation ayant une longueur inférieure ou égale à 5 000 pb.

11. Procédé selon la revendication 10, dans lequel chaque locus de méthylation a une longueur inférieure ou égale à 3 000 pb.

**100**

110 — DNA extraction

120 — Conversion

130 — Library Construction

140 — Target Enrichment

150 — Sequencing

160 — Bioinformatics Analysis

## FIG. 1

| SEQ. ID.NO. | chr | start | end | width | Overlapping genes | enhancers | promoters | 1to5kb |
|---|---|---|---|---|---|---|---|---|
| SEQ. ID NO. 1 | 1 | 1062388 | 1062568 | 181 | No | | AL390719.1 | AL390719.1 |
| SEQ. ID NO. 2 | 1 | 2132912 | 2133092 | 181 | No | | PRKCZ | PRKCZ |
| SEQ. ID NO. 3 | 1 | 3073311 | 3073791 | 481 | No | | | PRDM16-DT |
| SEQ. ID NO. 4 | 1 | 32464123 | 32464603 | 481 | No | | AL033529.1,ZBTB8B | |
| SEQ. ID NO. 5 | 1 | 32753865 | 32754105 | 241 | No | | KIAA1522 | |
| SEQ. ID NO. 6 | 1 | 38045915 | 38046095 | 181 | No | | | MIR3659HG |
| SEQ. ID NO. 7 | 1 | 50417741 | 50418443 | 703 | No | | | |
| SEQ. ID NO. 8 | 1 | 98053745 | 98054105 | 361 | No | | | MIR137HG |
| SEQ. ID NO. 9 | 1 | 151721519 | 151721944 | 426 | No | | RIIAD1 | CELF3 |
| SEQ. ID NO. 10 | 1 | 158180920 | 158181460 | 541 | No | | | ELL2P1 |
| SEQ. ID NO. 11 | 1 | 159200739 | 159201039 | 301 | No | | | ACKR1 |
| SEQ. ID NO. 12 | 1 | 160083804 | 160084524 | 721 | No | | | |
| SEQ. ID NO. 13 | 1 | 160401102 | 160401282 | 181 | No | | | |
| SEQ. ID NO. 14 | 1 | 183185880 | 183186120 | 241 | No | | LAMC2 | |
| SEQ. ID NO. 15 | 1 | 243917149 | 243917569 | 421 | No | | LINC02774 | |
| SEQ. ID NO. 16 | 2 | 10080669 | 10080849 | 181 | No | | CYS1 | AC104794.2 |
| SEQ. ID NO. 17 | 2 | 29115776 | 29116791 | 1016 | No | 1 | | CLIP4 |
| SEQ. ID NO. 18 | 2 | 31136883 | 31138428 | 1546 | No | | GALNT14 | |
| SEQ. ID NO. 19 | 2 | 47369683 | 47369863 | 181 | No | | | EPCAM |
| SEQ. ID NO. 20 | 2 | 47569537 | 47570197 | 661 | No | 1 | | |
| SEQ. ID NO. 21 | 2 | 61145001 | 61145181 | 181 | No | | C2orf74,AC016747.1 | |
| SEQ. ID NO. 22 | 2 | 64204147 | 64204447 | 301 | No | | | AC012368.1 |
| SEQ. ID NO. 23 | 2 | 68319245 | 68319425 | 181 | No | | CNRIP1 | |
| SEQ. ID NO. 24 | 2 | 86790271 | 86790811 | 541 | No | | | |
| SEQ. ID NO. 25 | 2 | 88765502 | 88766042 | 541 | No | | ANKRD36BP2 | |
| SEQ. ID NO. 26 | 2 | 95025733 | 95026933 | 1201 | No | | MAL,AC103563.7 | |
| SEQ. ID NO. 27 | 2 | 95074648 | 95075068 | 421 | No | 1 | | |
| SEQ. ID NO. 28 | 2 | 100322218 | 100322818 | 601 | No | | LONRF2 | |
| SEQ. ID NO. 29 | 2 | 117835956 | 117836256 | 301 | No | | AC009404.1 | AC009404.1 |
| SEQ. ID NO. 30 | 2 | 130287611 | 130287911 | 301 | No | | | |
| SEQ. ID NO. 31 | 2 | 130372760 | 130372940 | 181 | No | | | |

**FIG. 2**

| 5UTRs | exons | introns | 3UTRs | cpg_islands | cpg_shores | cpg_shelves | cpg_inter |
|---|---|---|---|---|---|---|---|
| | AL645608.8 | AL390719.1 | | | 1 | | |
| | | PRKCZ | | | 1 | | |
| | | PRDM16 | | 1 | 1 | | |
| | | | | | 1 | | |
| KIAA1522 | KIAA1522 | KIAA1522 | | 1 | | | |
| | POU3F1 | | | 1 | | | |
| | DMRTA2 | | DMRTA2 | 1 | 1 | | |
| | AC104453.1 | AC104453.1 | | 1 | | | |
| | RIIAD1 | RIIAD1 | | 1 | 1 | | |
| CD1D | CD1D | CD1D | | 1 | | | |
| | CADM3,CADM3-AS1 | CADM3,CADM3-AS1 | CADM3 | | | | 1 |
| KCNJ9 | KCNJ9 | KCNJ9 | | 1 | 1 | | |
| | | VANGL2 | | | 1 | | |
| | | | | | | | 1 |
| | LINC02774 | LINC02774 | | 1 | 1 | | |
| | CYS1 | CYS1 | | 1 | | | |
| | | CLIP4 | | 1 | 1 | | |
| GALNT14 | GALNT14 | GALNT14 | | 1 | | | |
| | EPCAM | EPCAM | | 1 | | | |
| | KCNK12 | KCNK12,MSH2 | | 1 | 1 | | |
| C2orf74 | C2orf74 | AC016747.4 | | | | | 1 |
| | LINC00309 | AC012368.1,LINC00309 | | | | | 1 |
| CNRIP1 | CNRIP1 | CNRIP1 | | 1 | | | |
| | CD8A | CD8A | | 1 | | | |
| | ANKRD36BP2 | | | 1 | 1 | | |
| MAL | MAL,AC103563.7 | MAL,AC103563.7 | | 1 | 1 | | |
| | | | | | 1 | 1 | |
| LONRF2 | LONRF2 | | | 1 | 1 | | |
| | | AC009404.1 | | 1 | 1 | | |
| | | | | | 1 | | |
| | PTPN18 | PTPN18 | | 1 | 1 | | |

**FIG. 2 (continued)**

| SEQ. ID.NO. | chr | start | end | width | Overlapping genes | enhancers | promoters | 1to5kb |
|---|---|---|---|---|---|---|---|---|
| SEQ. ID NO. 32 | 2 | 144516784 | 144516964 | 181 | No | | ZEB2 | ZEB2-AS1,AC009951.3,AC009951.1,AC009951.2,ZEB2 |
| SEQ. ID NO. 33 | 2 | 159904622 | 159904992 | 371 | No | | LY75,LY75-CD302 | |
| SEQ. ID NO. 34 | 2 | 172736178 | 172736658 | 481 | No | | RAPGEF4-AS1 | RAPGEF4-AS1 |
| SEQ. ID NO. 35 | 2 | 176094518 | 176094878 | 361 | No | | | HOXD12 |
| SEQ. ID NO. 36 | 2 | 176638778 | 176638958 | 181 | No | | LINC01116 | LINC01116 |
| SEQ. ID NO. 37 | 2 | 216013535 | 216013835 | 301 | No | | MREG | |
| SEQ. ID NO. 38 | 2 | 222424114 | 222424294 | 181 | No | | SGPP2 | |
| SEQ. ID NO. 39 | 2 | 238848026 | 238848686 | 661 | No | | TWIST2 | |
| SEQ. ID NO. 40 | 3 | 14880783 | 14881083 | 301 | No | | | FGD5 |
| SEQ. ID NO. 41 | 3 | 37453325 | 37453874 | 550 | No | 1 | | |
| SEQ. ID NO. 42 | 3 | 38039106 | 38039524 | 419 | No | | DLEC1 | |
| SEQ. ID NO. 43 | 3 | 43894073 | 43894613 | 541 | No | 1 | | |
| SEQ. ID NO. 44 | 3 | 43998051 | 43998471 | 421 | No | 1 | | |
| SEQ. ID NO. 45 | 3 | 44022250 | 44022430 | 181 | No | | | |
| SEQ. ID NO. 46 | 3 | 54121546 | 54121726 | 181 | No | | CACNA2D3 | CACNA2D3 |
| SEQ. ID NO. 47 | 3 | 113441338 | 113441758 | 421 | No | | CFAP44 | |
| SEQ. ID NO. 48 | 3 | 119322448 | 119322772 | 325 | No | | ARHGAP31-AS1 | ARHGAP31 |
| SEQ. ID NO. 49 | 3 | 128491607 | 128492807 | 1201 | No | | GATA2 | GATA2 |
| SEQ. ID NO. 50 | 3 | 185363363 | 185363963 | 601 | No | | | |
| SEQ. ID NO. 51 | 4 | 41867059 | 41867539 | 481 | No | | | |
| SEQ. ID NO. 52 | 4 | 42151203 | 42151863 | 661 | No | | | |
| SEQ. ID NO. 53 | 4 | 143699914 | 143701174 | 1261 | No | | FREM3,AC107223.1 | |
| SEQ. ID NO. 54 | 4 | 145733372 | 145733552 | 181 | No | | | |
| SEQ. ID NO. 55 | 4 | 153249541 | 153249721 | 181 | No | 1 | | |
| SEQ. ID NO. 56 | 4 | 182448095 | 182448815 | 721 | No | | TENM3 | |
| SEQ. ID NO. 57 | 5 | 1875964 | 1876144 | 181 | No | | | |
| SEQ. ID NO. 58 | 5 | 31854956 | 31855556 | 601 | No | | | |
| SEQ. ID NO. 59 | 5 | 32713356 | 32713716 | 361 | No | | | |
| SEQ. ID NO. 60 | 5 | 38555799 | 38556399 | 601 | No | | LIFR-AS1 | LIFR-AS1 |

# FIG. 2 (continued)

EP 4 320 276 B1

| 5UTRs | exons | introns | 3UTRs | cpg_islands | cpg_sh ores | cpg_s helves | cpg_i nter |
|---|---|---|---|---|---|---|---|
| | | ZEB2 | | 1 | | | |
| LY75,LY75-CD302 | LY75,LY75-CD302 | | | 1 | 1 | | |
| | RAPGEF4-AS1 | RAPGEF4 | | 1 | 1 | | |
| | HOXD13 | | HOXD13 | | 1 | | |
| | | LINC01117 | | | 1 | | |
| MREG | MREG | MREG,PECR | | 1 | 1 | | |
| | | | | 1 | 1 | | |
| TWIST2 | TWIST2 | | | 1 | | | |
| | | FGD5 | | | | | 1 |
| | | ITGA9 | | | 1 | | |
| DLEC1 | DLEC1 | | | 1 | 1 | | |
| | | | | | | | 1 |
| | AC006058.3 | | | 1 | | | |
| | | | | 1 | 1 | | |
| | | | | 1 | | | |
| CFAP44 | CFAP44,AC112128.1 | CFAP44,AC112128.1 | AC112128.1 | 1 | 1 | | |
| | ARHGAP31-AS1 | ARHGAP31,ARHGAP 31-AS1 | | 1 | 1 | | |
| GATA2 | GATA2 | GATA2,GATA2-AS1 | | 1 | | | |
| MAP3K13 | MAP3K13 | MAP3K13 | | | | | 1 |
| | | | | 1 | 2 | | |
| | BEND4,AC111006.1 | BEND4,AC111006.1 | | 1 | | | |
| | FREM3,AC107223.1 | AC107223.1 | | 1 | 1 | | |
| | | C4orf51 | | 1 | 1 | | |
| | | TRIM2 | | | 1 | | |
| | | TENM3 | | 1 | 1 | | |
| | | | | 1 | | | |
| | PDZD2 | PDZD2 | | 1 | 1 | | |
| | | NPR3 | | 1 | | | |
| LIFR | LIFR | LIFR | | 1 | 1 | | |

## FIG. 2 (continued)

| SEQ. ID.NO. | chr | start | end | width | Overlapping genes | enhancers | promoters | 1to5kb |
|---|---|---|---|---|---|---|---|---|
| SEQ. ID NO. 61 | 5 | 88683649 | 88683949 | 301 | No | | | ZEB2- |
| SEQ. ID NO. 62 | 5 | 93621604 | 93621784 | 181 | No | | MIR2277 | |
| SEQ. ID NO. 63 | 5 | 173236238 | 173237198 | 961 | No | 1 | NKX2-5 | NKX2-5 |
| SEQ. ID NO. 64 | 5 | 175772822 | 175773002 | 181 | No | | | |
| SEQ. ID NO. 65 | 6 | 6004596 | 6005316 | 721 | No | | NRN1 | NRN1 |
| SEQ. ID NO. 66 | 6 | 10415249 | 10415489 | 241 | No | | TFAP2A | TFAP2A |
| SEQ. ID NO. 67 | 6 | 26550445 | 26550884 | 440 | No | 1 | | |
| SEQ. ID NO. 68 | 6 | 26754333 | 26755053 | 721 | No | | | |
| SEQ. ID NO. 69 | 6 | 28988380 | 28988560 | 181 | No | | | |
| SEQ. AL NO. 70 | 6 | 31308135 | 31308735 | 601 | No | 2 | AL671883.1 | |
| SEQ. ID NO. 71 | 6 | 37570393 | 37570813 | 421 | No | | | |
| SEQ. ID NO. 72 | 6 | 42726729 | 42727149 | 421 | No | 1 | | PRPH2 |
| SEQ. ID NO. 73 | 6 | 42771139 | 42771319 | 181 | No | 1 | | |
| SEQ. ID NO. 74 | 6 | 43174231 | 43174471 | 241 | No | | | |
| SEQ. ID NO. 75 | 6 | 44002777 | 44003257 | 481 | No | | | |
| SEQ. ID NO. 76 | 6 | 72622682 | 72623402 | 721 | No | | FO393414.3 | FO393414.3 |
| SEQ. KHNO. 77 | 6 | 73309532 | 73310552 | 1021 | No | | KHDC1 | |
| SEQ. ID NO. 78 | 6 | 79946660 | 79947835 | 1176 | No | | ELOVL4 | |
| SEQ. ID NO. 79 | 6 | 123803435 | 123804335 | 901 | No | | NKAIN2 | |
| SEQ. ID NO. 80 | 6 | 163413139 | 163413679 | 541 | No | 1 | QKI | QKI |
| SEQ. ID NO. 81 | 6 | 170736319 | 170736619 | 301 | No | | | |
| SEQ. ID NO. 82 | 7 | 24284003 | 24284543 | 541 | No | | NPY | NPY |
| SEQ. ID NO. 83 | 7 | 26377180 | 26377360 | 181 | No | | AC004540.2 | AC004540.2 |
| SEQ. ID NO. 84 | 7 | 27174216 | 27174696 | 481 | No | | HOXA10 | MIR196B,HOXA9,HOXA10 |
| SEQ. ID NO. 85 | 7 | 28957467 | 28957647 | 181 | No | | AC005013.1 | |
| SEQ. ID NO. 86 | 7 | 35257235 | 35257535 | 301 | Yes | | | TBX20 |
| SEQ. ID NO. 87 | 7 | 35259057 | 35259357 | 301 | Yes | | | TBX20 |
| SEQ. ID NO. 88 | 7 | 50303675 | 50303855 | 181 | No | | IKZF1 | IKZF1 |

## FIG. 2 (continued)

EP 4 320 276 B1

| 5UTRs | exons | introns | 3UTRs | cpg_islands | cpg_sh ores | cpg_s helves | cpg_i nter |
|---|---|---|---|---|---|---|---|
| | | LINC00461,MEF2C-AS2 | | | 1 | | |
| | | FAM172A | | 1 | 1 | | |
| | | | | 1 | 1 | | |
| | | | | | 1 | | |
| | | NRN1 | | 1 | 1 | | |
| TFAP2A | TFAP2A,TFAP2A-AS1 | TFAP2A,TFAP2A-AS1 | | | | 1 | |
| | | | | 1 | 1 | | |
| | | | | | | | 1 |
| | AL662791.1 | | | 1 | | | |
| | AL671883.1 | AL671883.1,HLA-B | | 1 | 1 | | |
| | AL353597.3 | | | | | | 1 |
| | | | | 1 | 1 | | |
| | | BICRAL | | | | | 1 |
| | | SRF | | 1 | 1 | | |
| | C6orf223 | AL109615.3 | C6orf223 | 1 | 1 | | |
| | | KCNQ5 | | 1 | 1 | | |
| KHDC1 | KHDC1 | KHDC1 | | 1 | 2 | | |
| ELOVL4 | ELOVL4 | ELOVL4 | | 1 | 1 | | |
| NKAIN2 | NKAIN2 | NKAIN2 | | 1 | 1 | | |
| | CAHM | | | 1 | 1 | | |
| | AL731684.1,AL672310.1 | AL731684.1,AL672310.1 | | | | | |
| NPY | NPY | NPY | | 1 | | | |
| | | | | 1 | 1 | | |
| | HOXA10 | HOXA10,HOXA9,AC004080.3 | | 1 | | | |
| | TRIL | | | 1 | | | |
| | | | | 1 | 1 | | |
| | | AC009531.1 | | 1 | 1 | | |
| | | | | 1 | | | |

# FIG. 2 (continued)

| SEQ. ID.NO. | chr | start | end | width | Overlapping genes | enhancers | promoters | 1to5kb |
|---|---|---|---|---|---|---|---|---|
| SEQ. ID NO. 89 | 7 | 64889447 | 64889627 | 181 | No | | | |
| SEQ. ID NO. 90 | 7 | 90596855 | 90597335 | 481 | No | | AC002456.1 | AC002456.1 |
| SEQ. ID NO. 91 | 7 | 93890873 | 93891053 | 181 | No | | TFPI2,AC002076.1 | |
| SEQ. ID NO. 92 | 7 | 96997902 | 96999222 | 1321 | No | | | |
| SEQ. ID NO. 93 | 7 | 97020672 | 97021092 | 421 | No | | | |
| SEQ. ID NO. 94 | 7 | 103988969 | 103989749 | 781 | No | | RELN | |
| SEQ. ID NO. 95 | 7 | 107660513 | 107660693 | 181 | No | | SLC26A4 | |
| SEQ. ID NO. 96 | 7 | 122300668 | 122301028 | 361 | No | | | FEZF1-AS1 |
| SEQ. ID NO. 97 | 7 | 134458200 | 134459460 | 1261 | No | 1 | AKR1B1 | |
| SEQ. ID NO. 98 | 7 | 139434580 | 139434880 | 301 | No | | | |
| SEQ. ID NO. 99 | 7 | 141072108 | 141073057 | 950 | No | | | TMEM178B |
| SEQ. ID NO. 100 | 7 | 143345452 | 143345795 | 344 | No | | | |
| SEQ. ID NO. 101 | 7 | 157336263 | 157336683 | 421 | No | | DNAJB6 | DNAJB6 |
| SEQ. ID NO. 102 | 8 | 23163898 | 23164198 | 301 | No | | TNFRSF10D | |
| SEQ. ID NO. 103 | 8 | 39107409 | 39107589 | 181 | No | | ADAM32 | |
| SEQ. ID NO. 104 | 8 | 41828830 | 41829010 | 181 | No | | | |
| SEQ. ID NO. 105 | 8 | 52564305 | 52566225 | 1921 | No | | ALKAL1 | |
| SEQ. ID NO. 106 | 8 | 69994687 | 69995047 | 361 | No | | | |
| SEQ. ID NO. 107 | 8 | 72004794 | 72005334 | 541 | No | 1 | | |
| SEQ. ID NO. 108 | 8 | 96145538 | 96145718 | 181 | No | | | AP003465.2 |
| SEQ. ID NO. 109 | 8 | 98951181 | 98951931 | 751 | No | | | |
| SEQ. ID NO. 110 | 8 | 103371382 | 103371833 | 452 | No | | CTHRC1 | CTHRC1 |
| SEQ. ID NO. 111 | 8 | 104367291 | 104367591 | 301 | No | | | |
| SEQ. ID NO. 112 | 8 | 108082141 | 108082671 | 531 | No | | RSPO2 | |
| SEQ. ID NO. 113 | 8 | 126556342 | 126556522 | 181 | No | | LRATD2,PCAT1 | PCAT1 |
| SEQ. ID NO. 114 | 9 | 23824147 | 23824447 | 301 | No | | | ELAVL2 |
| SEQ. ID NO. 115 | 9 | 109639950 | 109640490 | 541 | Yes | | PALM2AKAP2 | |
| SEQ. ID NO. 116 | 9 | 109640702 | 109641422 | 721 | Yes | | PALM2AKAP2 | |
| SEQ. ID NO. 117 | 9 | 114387376 | 114388005 | 630 | No | | AKNA | |
| SEQ. ID NO. 118 | 9 | 120869156 | 120869336 | 181 | No | | PHF19 | PHF19 |
| SEQ. ID NO. 119 | 9 | 124007671 | 124008151 | 481 | No | | | LHX2,AC006450.1 |
| SEQ. ID NO. 120 | 9 | 129258261 | 129258441 | 181 | No | | AL158151.1 | |

## FIG. 2 (continued)

EP 4 320 276 B1

| 5UTRs | exons | introns | 3UTRs | cpg_islands | cpg_shores | cpg_shelves | cpg_inter |
|---|---|---|---|---|---|---|---|
| | AC073349.1,ZNF273 | AC073349.1,ZNF273 | | 1 | | | |
| | AC002456.1 | CDK14,AC002456.1 | | 1 | 1 | | |
| TFPI2 | TFPI2,AC002076.1 | GNGT1 | | | 1 | | |
| | | DLX6-AS1 | | | | 1 | 1 |
| | DLX5 | DLX5 | DLX5 | 1 | 1 | | |
| RELN | RELN | RELN | | 1 | 1 | | |
| | | SLC26A4-AS1 | | | 1 | | |
| | | | | | 1 | | |
| AKR1B1 | AKR1B1 | AKR1B1 | | 1 | 2 | | |
| | | | | | | | 1 |
| | | | | 1 | 1 | | |
| | CLCN1 | CLCN1 | | 1 | 1 | | |
| DNAJB6 | DNAJB6 | DNAJB6 | | 1 | 1 | | |
| TNFRSF10D | TNFRSF10D | | | 1 | 1 | | |
| ADAM32 | ADAM32 | ADAM32 | | 1 | 1 | | |
| | AC027702.1 | ANK1 | | 1 | | | |
| ALKAL1 | ALKAL1 | ALKAL1 | | 1 | 2 | | |
| | | | | | | | 1 |
| | | MSC-AS1 | | 1 | 1 | | |
| | | GDF6 | | 1 | | | |
| | OSR2 | OSR2 | OSR2 | | 1 | 1 | |
| CTHRC1 | CTHRC1 | CTHRC1 | | 1 | | | |
| | | DPYS | | 1 | 1 | | |
| RSPO2 | RSPO2 | RSPO2 | | 1 | 1 | | |
| | LRATD2,PCAT1 | | LRATD2 | 1 | 1 | | |
| | | ELAVL2 | | | 1 | | |
| | | | | 1 | 1 | | |
| PALM2AKAP2 | PALM2AKAP2 | PALM2AKAP2 | | 1 | 1 | | |
| AKNA | AKNA | AKNA | | | | | 1 |
| | PHF19 | PHF19 | | 1 | | | |
| | AC006450.3 | AC006450.3,LHX2 | | | 1 | | |
| | AL158151.1 | AL158151.1 | | 1 | 1 | | |

## FIG. 2 (continued)

| SEQ. ID.NO. | chr | start | end | width | Overlapping genes | enhancers | promoters | 1to5kb |
|---|---|---|---|---|---|---|---|---|
| SEQ. ID NO. 121 | 9 | 133257345 | 133257645 | 301 | No | | | |
| SEQ. ID NO. 122 | 9 | 134407349 | 134407680 | 332 | No | | | |
| SEQ. ID NO. 123 | 9 | 135926952 | 135927312 | 361 | No | 1 | | |
| SEQ. ID NO. 124 | 10 | 43105168 | 43105348 | 181 | No | | | |
| SEQ. ID NO. 125 | 10 | 59817900 | 59818140 | 241 | No | | | |
| SEQ. ID NO. 126 | 10 | 75408705 | 75408885 | 181 | No | | AC010997.4 | |
| SEQ. ID NO. 127 | 10 | 96044290 | 96044470 | 181 | No | | | ENTPD1-AS1 |
| SEQ. ID NO. 128 | 10 | 98029739 | 98030219 | 481 | No | | | |
| SEQ. ID NO. 129 | 10 | 99329112 | 99330672 | 1561 | No | | | |
| SEQ. ID NO. 130 | 10 | 101134289 | 101134829 | 541 | No | | TLX1 | TLX1NB |
| SEQ. ID NO. 131 | 10 | 116272416 | 116272956 | 541 | No | | GFRA1 | AC012470.1 |
| SEQ. ID NO. 132 | 10 | 132786883 | 132787292 | 410 | No | 1 | NKX6-2 | NKX6-2 |
| SEQ. ID NO. 133 | 11 | 639416 | 639956 | 541 | No | | | |
| SEQ. ID NO. 134 | 11 | 8080670 | 8081150 | 481 | No | | TUB | AC116456.2 |
| SEQ. ID NO. 135 | 11 | 17351464 | 17351704 | 241 | No | | NCR3LG1,AC124798.2 | |
| SEQ. ID NO. 136 | 11 | 64060717 | 64061197 | 481 | No | | | |
| SEQ. ID NO. 137 | 11 | 67583928 | 67584405 | 478 | No | | GSTP1 | GSTP1 |
| SEQ. ID NO. 138 | 11 | 68855693 | 68855873 | 181 | No | 1 | | |
| SEQ. ID NO. 139 | 11 | 69637540 | 69637720 | 181 | No | | | CCND1 |
| SEQ. ID NO. 140 | 11 | 73309427 | 73309607 | 181 | No | | AP002761.3 | ARHGEF17 |
| SEQ. ID NO. 141 | 11 | 74311342 | 74311687 | 346 | No | | P4HA3,P4HA3-AS1 | |
| SEQ. ID NO. 142 | 11 | 74467133 | 74467313 | 181 | No | | | KCNE3 |
| SEQ. ID NO. 143 | 11 | 104163936 | 104164116 | 181 | No | | PDGFD | |
| SEQ. ID NO. 144 | 11 | 134332352 | 134332532 | 181 | No | | | |
| SEQ. ID NO. 145 | 12 | 3200277 | 3201237 | 961 | No | | TSPAN9 | |
| SEQ. ID NO. 146 | 12 | 3493022 | 3493802 | 781 | No | | | |
| SEQ. ID NO. 147 | 12 | 6540419 | 6540659 | 241 | No | | | AC006064.1,AC006064.4 |
| SEQ. ID NO. 148 | 12 | 40224354 | 40224954 | 601 | No | | LRRK2,LRRK2-DT | LRRK2-DT,LRRK2 |
| SEQ. ID NO. 149 | 12 | 63150967 | 63151627 | 661 | No | | AVPR1A,AC135584.1 | AVPR1A |
| SEQ. ID NO. 150 | 12 | 104456512 | 104457736 | 1225 | No | | CHST11 | |

# FIG. 2 (continued)

| 5UTRs | exons | introns | 3UTRs | cpg_islands | cpg_shores | cpg_shelves | cpg_inter |
|---|---|---|---|---|---|---|---|
| | ABO | ABO | | | 1 | | |
| | | RXRA | | 1 | 1 | | |
| | | | | | | | 1 |
| | RET | RET | | 1 | | | |
| | | CCDC6 | | | | | 1 |
| | ZNF503-AS2 | | | 1 | | | |
| CCNJ | CCNJ | CCNJ,ENTPD1-AS1 | | 1 | | | |
| | | CRTAC1 | | 1 | 1 | | |
| CNNM1 | CNNM1 | | | 1 | 1 | | |
| | TLX1 | TLX1 | | 1 | | | |
| GFRA1 | GFRA1 | GFRA1 | | 1 | | | |
| | | | | 1 | | | |
| | DRD4 | DRD4 | | 1 | | | |
| | | TUB | | 1 | 1 | | |
| | AC124798.2 | | | 1 | 1 | | |
| | | MACROD1,AP000721.2 | | | | | 1 |
| | GSTP1 | GSTP1 | | 1 | | | |
| | | | | 1 | 1 | | |
| | | | | 1 | | | |
| | ARHGEF17 | | | 1 | 1 | | |
| P4HA3 | P4HA3,P4HA3-AS1 | P4HA3,P4HA3-AS1 | | 1 | 2 | | |
| | | KCNE3,AP001372.1 | | 1 | | | |
| PDGFD | PDGFD | | | 1 | | | |
| | | GLB1L2 | | 1 | 1 | | |
| TSPAN9 | TSPAN9 | TSPAN9 | | 1 | 1 | | |
| | | PRMT8 | | 1 | | | |
| | IFFO1 | IFFO1 | IFFO1 | 1 | 1 | | |
| | LRRK2-DT | LRRK2 | | 1 | 1 | | |
| | AC135584.1 | AC135584.1 | | 1 | 1 | | |
| CHST11 | CHST11 | CHST11 | | 1 | | | |

## FIG. 2 (continued)

| SEQ. ID.NO. | chr | start | end | width | Overlapping genes | enhancers | promoters | 1to5kb |
|---|---|---|---|---|---|---|---|---|
| SEQ. ID NO. 151 | 12 | 132904844 | 132905024 | 181 | No | | CHFR | |
| SEQ. ID NO. 152 | 13 | 24271993 | 24272173 | 181 | No | 1 | SPATA13 | |
| SEQ. ID NO. 153 | 13 | 36297374 | 36297734 | 361 | No | | | |
| SEQ. ID NO. 154 | 13 | 102394503 | 102394683 | 181 | No | | FGF14-AS2,FGF14-IT1 | |
| SEQ. ID NO. 155 | 14 | 21068771 | 21069311 | 541 | No | | ARHGEF40 | ARHGEF40 |
| SEQ. ID NO. 156 | 14 | 53956625 | 53956805 | 181 | No | | BMP4 | BMP4 |
| SEQ. ID NO. 157 | 14 | 97412990 | 97413410 | 421 | No | | | |
| SEQ. ID NO. 158 | 14 | 101561321 | 101561561 | 241 | No | | MIR1247,DIO3,DIO3OS | MIR1247,DIO3OS |
| SEQ. ID NO. 159 | 14 | 104117671 | 104117851 | 181 | No | | MIR203B | |
| SEQ. ID NO. 160 | 15 | 52789319 | 52790159 | 841 | No | | ONECUT1 | |
| SEQ. ID NO. 161 | 15 | 79089695 | 79090115 | 421 | No | 1 | | |
| SEQ. ID NO. 162 | 15 | 84079746 | 84080646 | 901 | No | | EFL1P1,AC027807.1 | |
| SEQ. ID NO. 163 | 15 | 88803502 | 88803862 | 361 | No | | ACAN | AC103982.1 |
| SEQ. ID NO. 164 | 16 | 3151986 | 3152166 | 181 | No | | | |
| SEQ. ID NO. 165 | 16 | 23182247 | 23182634 | 388 | No | | SCNN1G | |
| SEQ. ID NO. 166 | 16 | 57091834 | 57092014 | 181 | No | 1 | CPNE2 | |
| SEQ. ID NO. 167 | 16 | 57536947 | 57537307 | 361 | No | | CCDC102A | |
| SEQ. ID NO. 168 | 16 | 68448640 | 68449000 | 361 | No | | SMPD3 | AC099521.2 |
| SEQ. ID NO. 169 | 16 | 68737031 | 68737271 | 241 | No | | CDH1 | |
| SEQ. ID NO. 170 | 16 | 72787676 | 72787856 | 181 | No | | | |
| SEQ. ID NO. 171 | 17 | 5500469 | 5500649 | 181 | No | | | |
| SEQ. ID NO. 172 | 17 | 15966446 | 15966686 | 241 | No | 1 | | |
| SEQ. ID NO. 173 | 17 | 17757263 | 17758043 | 781 | No | | | |
| SEQ. ID NO. 174 | 17 | 19744858 | 19745191 | 334 | No | | ALDH3A1 | ALDH3A1 |
| SEQ. ID NO. 175 | 17 | 19867961 | 19868141 | 181 | No | | ULK2,AC015726.2 | ULK2 |
| SEQ. ID NO. 176 | 17 | 29019967 | 29020267 | 301 | No | | | |
| SEQ. ID NO. 177 | 17 | 56834692 | 56835015 | 324 | No | | C17orf67,DGKE | C17orf67 |
| SEQ. ID NO. 178 | 18 | 46756320 | 46757040 | 721 | No | | ST8SIA5,AC090241.2 | AC090241.2 |
| SEQ. ID NO. 179 | 19 | 4328597 | 4329017 | 421 | No | | STAP2 | STAP2 |

<p align="center"><strong>FIG. 2 (continued)</strong></p>

| 5UTRs | exons | introns | 3UTRs | cpg_islands | cpg_sh ores | cpg_s helves | cpg_i nter |
|---|---|---|---|---|---|---|---|
| CHFR | CHFR | CHFR | | | | 1 | |
| | | SPATA13,AL359736. 1 | | | | | 1 |
| CCDC169,CCDC169- SOHLH2 | CCDC169,CCDC169- SOHLH2 | CCDC169,CCDC169- SOHLH2 | | 1 | 1 | | |
| | FGF14-AS2,FGF14-IT1 | FGF14 | | 1 | | | |
| | | NDRG2 | | 1 | 2 | | |
| BMP4 | BMP4 | BMP4 | | | 1 | | |
| | | | | | | | 1 |
| DIO3 | DIO3 | | | 1 | | | |
| | | | | 1 | | | |
| ONECUT1 | ONECUT1 | ONECUT1 | | 1 | | | |
| | | RASGRF1 | | 1 | | | |
| | EFL1P1,AC027807.1 | EFL1P1,AC027807.1 | | 1 | 2 | | |
| ACAN | ACAN | ACAN | | 1 | | | |
| | | | | | 1 | | |
| | | AC099482.2 | | 1 | 1 | | |
| | AC009090.2 | AC009090.2 | | | 1 | | |
| | | | | | 1 | | |
| SMPD3 | SMPD3 | | | 1 | 1 | | |
| CDH1 | CDH1 | | | 1 | 1 | | |
| | ZFHX3,AC004943.2 | | | 1 | 1 | | |
| | AC055839.2 | NLRP1 | | 1 | | | |
| | | ADORA2B | | | | | 1 |
| | | RAI1 | | | | | 1 |
| ALDH3A1 | ALDH3A1 | ALDH3A1 | | 1 | 1 | | |
| | | | | 1 | | | |
| | | PIPOX | | 1 | 1 | | |
| DGKE | DGKE | DGKE,C17orf67 | | 1 | | | |
| ST8SIA5 | ST8SIA5,AC090241.2 | ST8SIA5,AC090241.2 | | 1 | | | |
| | STAP2 | STAP2 | STAP2 | 1 | 1 | | |

# FIG. 2 (continued)

EP 4 320 276 B1

| SEQ. ID.NO. | chr | start | end | width | Overlapping genes | enhancers | promoters | 1to5kb |
|---|---|---|---|---|---|---|---|---|
| SEQ. ID NO. 180 | 19 | 12867684 | 12868104 | 421 | No | | MAST1 | |
| SEQ. ID NO. 181 | 19 | 15551391 | 15551571 | 181 | No | | | |
| SEQ. ID NO. 182 | 19 | 18438234 | 18438414 | 181 | No | | ISYNA1 | ISYNA1,AC010335.1 |
| SEQ. ID NO. 183 | 19 | 19618353 | 19618696 | 344 | No | | PBX4 | |
| SEQ. ID NO. 184 | 19 | 20238353 | 20238533 | 181 | No | | AC011447.3 | |
| SEQ. ID NO. 185 | 19 | 20424875 | 20425055 | 181 | No | | ZNF826P | |
| SEQ. ID NO. 186 | 19 | 47243022 | 47243202 | 181 | No | 1 | | |
| SEQ. ID NO. 187 | 19 | 47754579 | 47755119 | 541 | No | | SNORD23,NOP53 | SNORD23,NOP53 |
| SEQ. ID NO. 188 | 19 | 49142732 | 49143092 | 361 | No | | PPFIA3 | PPFIA3 |
| SEQ. ID NO. 189 | 19 | 50050361 | 50050716 | 356 | No | | AC010624.1 | |
| SEQ. ID NO. 190 | 19 | 53254542 | 53254962 | 421 | No | | ZNF677 | ZNF677,VN1R2 |
| SEQ. ID NO. 191 | 19 | 53866337 | 53866517 | 181 | No | | MYADM | MYADM |
| SEQ. ID NO. 192 | 19 | 53982150 | 53982330 | 181 | No | | MIR935 | |
| SEQ. ID NO. 193 | 20 | 23049079 | 23049259 | 181 | Yes | | | |
| SEQ. ID NO. 194 | 20 | 23049351 | 23051331 | 1981 | Yes | 1 | THBD | THBD |
| SEQ. ID NO. 195 | 20 | 43188826 | 43189486 | 661 | No | 1 | AL021395.1 | AL021395.1 |
| SEQ. ID NO. 196 | 20 | 48828207 | 48828567 | 361 | No | | PREX1 | |
| SEQ. ID NO. 197 | 20 | 63829922 | 63830102 | 181 | No | | | |
| SEQ. ID NO. 198 | 21 | 26843610 | 26845470 | 1861 | No | | ADAMTS1 | ADAMTS1 |
| SEQ. ID NO. 199 | 21 | 33071136 | 33071916 | 781 | No | | AP000282.1,OLIG1 | |
| SEQ. ID NO. 200 | 22 | 24654234 | 24654594 | 361 | No | | | AP000356.2 |
| SEQ. ID NO. 201 | 22 | 25282006 | 25282426 | 421 | No | | AL022332.1,AL022332.2,AL022324.4 | |
| SEQ. ID NO. 202 | X | 108732499 | 108733039 | 541 | No | | AL035425.2 | AL035425.1 |
| SEQ. ID NO. 203 | X | 114582244 | 114582664 | 421 | No | | | HTR2C |

# FIG. 2 (continued)

| 5UTRs | exons | introns | 3UTRs | cpg_islands | cpg_shores | cpg_shelves | cpg_inter |
|---|---|---|---|---|---|---|---|
| | MAST1 | MAST1,HOOK2 | | 1 | 1 | | |
| | CYP4F22 | | CYP4F22 | 1 | 1 | | |
| ISYNA1 | ISYNA1 | | | 1 | 1 | | |
| PBX4 | PBX4 | PBX4 | | 1 | | | |
| | AC011447.3 | AC011447.3 | | | | | 1 |
| | ZNF826P | | | | | | 1 |
| | | | | | 1 | | |
| | NOP53 | NOP53 | | | | | 1 |
| | PPFIA3 | PPFIA3 | PPFIA3 | 1 | 2 | | |
| | AC010624.1 | AC010624.3,ZNF473 | | | 1 | | |
| ZNF677 | ZNF677 | ZNF677 | | 1 | 1 | | |
| MYADM | MYADM | MYADM,MYADM-AS1 | | 1 | | | |
| | CACNG8,MIR935 | | | 1 | | | |
| | THBD | | | 1 | | | |
| THBD | THBD | | | 1 | | | |
| | | PTPRT | | 1 | 1 | | |
| | | AL133342.1 | | 1 | | | |
| | | ZBTB46 | | 1 | | | |
| ADAMTS1 | ADAMTS1 | ADAMTS1 | | 1 | 1 | | |
| | OLIG1 | OLIG1 | OLIG1 | 1 | | | |
| | | | | | | | 1 |
| | AL022332.1 | AL022332.1 | | | 1 | | |
| | IRS4,AL035425.2 | AL035425.2 | IRS4 | 1 | 2 | | |
| | | | | | 1 | | |

# FIG. 2 (continued)

| SEQ. ID NO. | reg_id | chr | start | end | width | Overlapping genes | enhancers |
|---|---|---|---|---|---|---|---|
| SEQ. ID NO. 204 | 19:56507424-56508024 | 19 | 56507424 | 56508024 | 601 | Yes | |
| SEQ. ID NO. 205 | 19:56508112-56508652 | 19 | 56508112 | 56508652 | 541 | Yes | |
| SEQ. ID NO. 206 | 19:36915828-36916968 | 19 | 36915828 | 36916968 | 1141 | No | |
| SEQ. ID NO. 207 | 3:151086591-151087491 | 3 | 151086591 | 151087491 | 901 | No | |
| SEQ. ID NO. 208 | 4:141132059-141132899 | 4 | 141132059 | 141132899 | 841 | No | |
| SEQ. ID NO. 209 | 7:35185861-35186281 | 7 | 35185861 | 35186281 | 421 | No | |
| SEQ. ID NO. 210 | 7:141072108-141073057 | 7 | 141072108 | 141073057 | 950 | No | |
| SEQ. ID NO. 211 | 9:21970722-21971382 | 9 | 21970722 | 21971382 | 661 | No | |
| SEQ. ID NO. 212 | 15:64823877-64824537 | 15 | 64823877 | 64824537 | 661 | No | |
| SEQ. ID NO. 213 | 15:79089695-79090115 | 15 | 79089695 | 79090115 | 421 | No | 1 |
| SEQ. ID NO. 214 | 16:70737472-70738072 | 16 | 70737472 | 70738072 | 601 | No | 1 |
| SEQ. ID NO. 215 | 21:31343623-31344643 | 21 | 31343623 | 31344643 | 1021 | No | |
| SEQ. ID NO. 216 | 10:75407325-75407565 | 10 | 75407325 | 75407565 | 241 | Yes | |
| SEQ. ID NO. 217 | 10:75407574-75408594 | 10 | 75407574 | 75408594 | 1021 | Yes | |
| SEQ. ID NO. 218 | 10:75408705-75408885 | 10 | 75408705 | 75408885 | 181 | Yes | |
| SEQ. ID NO. 219 | 1:159200739-159201039 | 1 | 159200739 | 159201039 | 301 | No | |
| SEQ. ID NO. 220 | 4:40198301-40198661 | 4 | 40198301 | 40198661 | 361 | No | |
| SEQ. ID NO. 221 | 7:100785927-100786167 | 7 | 100785927 | 100786167 | 241 | No | |
| SEQ. ID NO. 222 | 8:98948170-98948530 | 8 | 98948170 | 98948530 | 361 | No | |
| SEQ. ID NO. 223 | 15:96947637-96948417 | 15 | 96947637 | 96948417 | 781 | No | |
| SEQ. ID NO. 224 | 17:15966446-15966686 | 17 | 15966446 | 15966686 | 241 | No | 1 |
| SEQ. ID NO. 225 | 19:48568628-48568928 | 19 | 48568628 | 48568928 | 301 | No | |
| SEQ. ID NO. 226 | 3:134364186-134364534 | 3 | 134364186 | 134364534 | 349 | No | 1 |
| SEQ. ID NO. 227 | 7:27169523-27169934 | 7 | 27169523 | 27169934 | 412 | No | |
| SEQ. ID NO. 228 | 1:2132912-2133092 | 1 | 2132912 | 2133092 | 181 | Yes | |
| SEQ. ID NO. 229 | 1:2133236-2133416 | 1 | 2133236 | 2133416 | 181 | Yes | |

# FIG. 3

| promoters | 1to5kb | 5UTRs | exons |
|---|---|---|---|
| ZNF471 | ZNF471 | ZNF471 | ZNF471 |
|  | ZNF471 |  |  |
| ZNF568,ZNF829 |  | ZNF568,ZNF829 | ZNF568,ZNF829 |
| MED12L |  | MED12L | MED12L |
| RNF150 |  | RNF150 | RNF150 |
| DPY19L2P1 |  |  | DPY19L2P1 |
|  | TMEM178B |  |  |
| CDKN2A |  | CDKN2A | CDKN2A |
|  | PIF1 | PIF1 | PIF1 |
|  |  |  |  |
|  |  |  |  |
| TIAM1 |  | TIAM1 | TIAM1 |
|  | AC010997.4 |  | ZNF503-AS2 |
| AC010997.4 | AC010997.4 |  | ZNF503-AS2 |
| AC010997.4 |  |  | ZNF503-AS2 |
|  | ACKR1 |  | CADM3,CADM3-AS1 |
|  | RHOH |  |  |
|  |  |  | ZAN |
| OSR2 |  | OSR2 | OSR2 |
|  |  |  |  |
|  |  |  |  |
|  |  |  |  |
|  |  |  |  |
| MIR196B,HOXA9 | HOXA9 |  | MIR196B,HOXA9,HOXA10-AS |
| PRKCZ | PRKCZ |  |  |
| PRKCZ | PRKCZ |  |  |

## FIG. 3 (continued)

| introns | 3UTRs | cpg_islands | cpg_shores | cpg_shelves | cpg_inter |
|---|---|---|---|---|---|
| ZNF471 | | 1 | | | |
| ZNF471 | | 1 | 1 | | |
| ZNF568,ZNF829 | | 1 | 2 | | |
| MED12L | | 1 | 1 | | |
| RNF150 | | 1 | 1 | | |
| | | 1 | | | |
| | | 1 | 1 | | |
| CDKN2A,AL359922.1 | CDKN2A | 1 | 2 | | |
| PIF1 | | 1 | 1 | | |
| RASGRF1 | | 1 | | | |
| VAC14 | | | | | 1 |
| TIAM1 | | 1 | 2 | | |
| | | 1 | | | |
| | | 1 | | | |
| | | 1 | | | |
| CADM3,CADM3-AS1 | CADM3 | | | | 1 |
| RHOH | | | | | 1 |
| ZAN | | | | | 1 |
| OSR2 | | 1 | 1 | | |
| | | | | | 1 |
| ADORA2B | | | | | 1 |
| SULT2B1 | | | | | 1 |
| AMOTL2 | | 1 | 1 | | |
| HOXA9,AC004080.3,HOXA10-AS | | 1 | | | |
| PRKCZ | | | 1 | | |
| PRKCZ | | 1 | | | |

## FIG. 3 (continued)

| SEQ. ID NO. | reg_id | chr | start | end | width | Overlapping genes | enhancers |
|---|---|---|---|---|---|---|---|
| SEQ. ID NO. 230 | 3:185363027-185363207 | 3 | 185363027 | 185363207 | 181 | Yes | |
| SEQ. ID NO. 231 | 3:185363363-185363963 | 3 | 185363363 | 185363963 | 601 | Yes | |
| SEQ. ID NO. 232 | 7:96993058-96993418 | 7 | 96993058 | 96993418 | 361 | Yes | 1 |
| SEQ. ID NO. 233 | 7:96997902-96999222 | 7 | 96997902 | 96999222 | 1321 | Yes | |
| SEQ. ID NO. 234 | 13:24270525-24270945 | 13 | 24270525 | 24270945 | 421 | Yes | |
| SEQ. ID NO. 235 | 13:24271993-24272173 | 13 | 24271993 | 24272173 | 181 | Yes | 1 |
| SEQ. ID NO. 236 | 1:6447340-6447520 | 1 | 6447340 | 6447520 | 181 | No | |
| SEQ. ID NO. 237 | 1:19643753-19643933 | 1 | 19643753 | 19643933 | 181 | No | |
| SEQ. ID NO. 238 | 1:26360410-26360890 | 1 | 26360410 | 26360890 | 481 | No | |
| SEQ. ID NO. 239 | 1:31771901-31772621 | 1 | 31771901 | 31772621 | 721 | No | 1 |
| SEQ. ID NO. 240 | 1:32248455-32248635 | 1 | 32248455 | 32248635 | 181 | No | |
| SEQ. ID NO. 241 | 1:40317257-40317497 | 1 | 40317257 | 40317497 | 241 | No | |
| SEQ. ID NO. 242 | 1:92030047-92030410 | 1 | 92030047 | 92030410 | 364 | No | |
| SEQ. ID NO. 243 | 1:98045882-98046362 | 1 | 98045882 | 98046362 | 481 | No | |
| SEQ. ID NO. 244 | 1:121008795-121009695 | 1 | 121008795 | 121009695 | 901 | No | |
| SEQ. ID NO. 245 | 1:148302626-148302986 | 1 | 148302626 | 148302986 | 361 | No | |
| SEQ. ID NO. 246 | 1:154567391-154567691 | 1 | 154567391 | 154567691 | 301 | No | |
| SEQ. ID NO. 247 | 1:160083804-160084524 | 1 | 160083804 | 160084524 | 721 | No | |
| SEQ. ID NO. 248 | 1:160401102-160401282 | 1 | 160401102 | 160401282 | 181 | No | |
| SEQ. ID NO. 249 | 1:166920601-166921261 | 1 | 166920601 | 166921261 | 661 | No | |
| SEQ. ID NO. 250 | 1:180232819-180233599 | 1 | 180232819 | 180233599 | 781 | No | |
| SEQ. ID NO. 251 | 1:183185880-183186120 | 1 | 183185880 | 183186120 | 241 | No | |
| SEQ. ID NO. 252 | 1:197913075-197913255 | 1 | 197913075 | 197913255 | 181 | No | |
| SEQ. ID NO. 253 | 1:201539995-201540295 | 1 | 201539995 | 201540295 | 301 | No | 1 |

## FIG. 3 (continued)

| promoters | 1to5kb | 5UTRs | exons |
|---|---|---|---|
| MAP3K13 | | MAP3K13 | MAP3K13 |
| | | MAP3K13 | MAP3K13 |
| | | | |
| | | | |
| SPATA13 | SPATA13 | SPATA13 | SPATA13 |
| SPATA13 | | | |
| ESPN,AL031848.1 | ESPN | ESPN | ESPN |
| NBL1 | NBL1 | NBL1 | NBL1 |
| CRYBG2 | | | |
| | | | |
| | LCK | | FAM167B |
| COL9A2 | COL9A2 | COL9A2 | COL9A2 |
| | EPHX4 | EPHX4 | EPHX4 |
| MIR137,MIR137HG,MIR2682 | MIR2682,MIR137HG | | MIR137,MIR137HG |
| U1 | U1 | | LINC00623 |
| | | | |
| CHRNB2 | CHRNB2 | | |
| | | KCNJ9 | KCNJ9 |
| | | | |
| | ILDR2 | | ILDR2 |
| | | | |
| LAMC2 | | | |
| | LHX9 | LHX9 | LHX9 |
| | | | |

FIG. 3 (continued)

EP 4 320 276 B1

| introns | 3UTRs | cpg_islands | cpg_shores | cpg_shelves | cpg_inter |
|---|---|---|---|---|---|
| MAP3K13 | | | | | 1 |
| MAP3K13 | | | | | 1 |
| DLX6-AS1 | | | | | 1 |
| DLX6-AS1 | | | | 1 | 1 |
| SPATA13,AL359736.1 | | | | | 1 |
| SPATA13,AL359736.1 | | | | | 1 |
| ESPN | | 1 | | | |
| NBL1,MICOS10-NBL1 | | 1 | 1 | | |
| | | 1 | 1 | | |
| | | 1 | 1 | | |
| | FAM167B | | | | 1 |
| COL9A2 | | 1 | 1 | | |
| EPHX4 | | 1 | 1 | | |
| MIR137HG | | 1 | 1 | | |
| AC241377.2,LINC00623 | | 1 | | | |
| LINC01138,AC245100.1 | | | 1 | | |
| | | | 1 | | |
| KCNJ9 | | 1 | 1 | | |
| VANGL2 | | | 1 | | |
| ILDR2 | | 1 | | | |
| LHX4 | | 1 | | | |
| | | | | | 1 |
| LHX9 | | | 1 | | |
| RPS10P7 | | 1 | 1 | | |

## FIG. 3 (continued)

EP 4 320 276 B1

| SEQ. ID NO. | reg_id | chr | start | end | width | Overlapping genes | enhancers |
|---|---|---|---|---|---|---|---|
| SEQ. ID NO. 254 | 1:203629355-203629535 | 1 | 203629355 | 203629535 | 181 | No | |
| SEQ. ID NO. 255 | 1:228818226-228818526 | 1 | 228818226 | 228818526 | 301 | No | |
| SEQ. ID NO. 256 | 1:232805208-232805868 | 1 | 232805208 | 232805868 | 661 | No | |
| SEQ. ID NO. 257 | 2:3195914-3196214 | 2 | 3195914 | 3196214 | 301 | No | |
| SEQ. ID NO. 258 | 2:12719226-12719779 | 2 | 12719226 | 12719779 | 554 | No | |
| SEQ. ID NO. 259 | 2:19350886-19351666 | 2 | 19350886 | 19351666 | 781 | No | |
| SEQ. ID NO. 260 | 2:20667295-20667775 | 2 | 20667295 | 20667775 | 481 | No | |
| SEQ. ID NO. 261 | 2:45000833-45001613 | 2 | 45000833 | 45001613 | 781 | No | |
| SEQ. ID NO. 262 | 2:47569537-47570197 | 2 | 47569537 | 47570197 | 661 | No | 1 |
| SEQ. ID NO. 263 | 2:48530399-48530759 | 2 | 48530399 | 48530759 | 361 | No | |
| SEQ. ID NO. 264 | 2:88765502-88766042 | 2 | 88765502 | 88766042 | 541 | No | |
| SEQ. ID NO. 265 | 2:95024998-95025598 | 2 | 95024998 | 95025598 | 601 | No | |
| SEQ. ID NO. 266 | 2:95074648-95075068 | 2 | 95074648 | 95075068 | 421 | No | 1 |
| SEQ. ID NO. 267 | 2:130372760-130372940 | 2 | 130372760 | 130372940 | 181 | No | |
| SEQ. ID NO. 268 | 2:134719120-134719540 | 2 | 134719120 | 134719540 | 421 | No | |
| SEQ. ID NO. 269 | 2:176638778-176638958 | 2 | 176638778 | 176638958 | 181 | No | |
| SEQ. ID NO. 270 | 2:197786407-197786887 | 2 | 197786407 | 197786887 | 481 | No | |
| SEQ. ID NO. 271 | 2:236507319-236507799 | 2 | 236507319 | 236507799 | 481 | No | |
| SEQ. ID NO. 272 | 3:14880783-14881083 | 3 | 14880783 | 14881083 | 301 | No | |
| SEQ. ID NO. 273 | 3:32026520-32026820 | 3 | 32026520 | 32026820 | 301 | No | |
| SEQ. ID NO. 274 | 3:37453325-37453874 | 3 | 37453325 | 37453874 | 550 | No | 1 |
| SEQ. ID NO. 275 | 3:49277016-49277327 | 3 | 49277016 | 49277327 | 312 | No | |

## FIG. 3 (continued)

| promoters | 1to5kb | 5UTRs | exons |
|---|---|---|---|
|  |  |  |  |
|  |  |  |  |
|  |  |  | MAP10 |
|  | EIPR1 |  |  |
|  |  |  |  |
|  | LINC01376 |  | OSR1 |
|  |  |  | GDF7 |
|  |  |  |  |
|  |  |  | KCNK12 |
|  |  | STON1 | STON1 |
| ANKRD36BP2 |  |  | ANKRD36BP2 |
| MAL |  |  | AC103563.7 |
|  |  |  |  |
|  |  |  | PTPN18 |
| TMEM163 |  |  |  |
| LINC01116 | LINC01116 |  |  |
| BOLL | BOLL | BOLL | BOLL |
| IQCA1 | IQCA1 | IQCA1 | IQCA1 |
|  | FGD5 |  |  |
|  | RPL21P40 |  |  |
|  |  |  |  |
| C3orf62 | C3orf62 | C3orf62 | C3orf62 |

**FIG. 3 (continued)**

| introns | 3UTRs | cpg_islands | cpg_shores | cpg_shelves | cpg_inter |
|---|---|---|---|---|---|
| ATP2B4 | | 1 | | | |
| | | | | | 1 |
| | | 1 | 1 | | |
| EIPR1 | | | | | 1 |
| TRIB2 | | | 1 | | |
| | OSR1 | | 1 | | |
| GDF7 | | 1 | | | |
| | | 1 | | | |
| KCNK12,MSH2 | | 1 | 1 | | |
| STON1-GTF2A1L,STON1 | | 1 | 1 | | |
| | | 1 | 1 | | |
| AC103563.7 | | 1 | 1 | | |
| | | | 1 | 1 | |
| PTPN18 | | 1 | 1 | | |
| | | 1 | 1 | | |
| LINC01117 | | | 1 | | |
| BOLL | | 1 | 1 | | |
| | | 1 | 2 | | |
| FGD5 | | | | | 1 |
| OSBPL10 | | | | | 1 |
| ITGA9 | | | 1 | | |
| C3orf62 | | 1 | | | |

**FIG. 3 (continued)**

| SEQ. ID NO. | reg_id | chr | start | end | width | Overlapping genes | enhancers |
|---|---|---|---|---|---|---|---|
| SEQ. ID NO. 276 | 3:125357496-125357736 | 3 | 125357496 | 125357736 | 241 | No | |
| SEQ. ID NO. 277 | 3:126267526-126267946 | 3 | 126267526 | 126267946 | 421 | No | 1 |
| SEQ. ID NO. 278 | 3:128491607-128492807 | 3 | 128491607 | 128492807 | 1201 | No | |
| SEQ. ID NO. 279 | 3:134650612-134651272 | 3 | 134650612 | 134651272 | 661 | No | |
| SEQ. ID NO. 280 | 3:147407874-147408234 | 3 | 147407874 | 147408234 | 361 | No | |
| SEQ. ID NO. 281 | 3:160224533-160224833 | 3 | 160224533 | 160224833 | 301 | No | |
| SEQ. ID NO. 282 | 3:161105353-161105533 | 3 | 161105353 | 161105533 | 181 | No | |
| SEQ. ID NO. 283 | 3:173397661-173398081 | 3 | 173397661 | 173398081 | 421 | No | |
| SEQ. ID NO. 284 | 4:1404179-1404659 | 4 | 1404179 | 1404659 | 481 | No | |
| SEQ. ID NO. 285 | 4:1683738-1683918 | 4 | 1683738 | 1683918 | 181 | No | |
| SEQ. ID NO. 286 | 4:7938762-7938942 | 4 | 7938762 | 7938942 | 181 | No | |
| SEQ. ID NO. 287 | 4:7940020-7940200 | 4 | 7940020 | 7940200 | 181 | No | |
| SEQ. ID NO. 288 | 4:30720303-30721203 | 4 | 30720303 | 30721203 | 901 | No | |
| SEQ. ID NO. 289 | 4:41866325-41866745 | 4 | 41866325 | 41866745 | 421 | No | |
| SEQ. ID NO. 290 | 4:41878205-41878505 | 4 | 41878205 | 41878505 | 301 | No | |
| SEQ. ID NO. 291 | 4:54231766-54232486 | 4 | 54231766 | 54232486 | 721 | No | |
| SEQ. ID NO. 292 | 4:56530240-56530420 | 4 | 56530240 | 56530420 | 181 | No | |
| SEQ. ID NO. 293 | 4:165873652-165874192 | 4 | 165873652 | 165874192 | 541 | No | |
| SEQ. ID NO. 294 | 5:77210543-77210783 | 5 | 77210543 | 77210783 | 241 | No | |
| SEQ. ID NO. 295 | 5:93579016-93579376 | 5 | 93579016 | 93579376 | 361 | No | 1 |
| SEQ. ID NO. 296 | 5:93621604-93621784 | 5 | 93621604 | 93621784 | 181 | No | |
| SEQ. ID NO. 297 | 5:173236238-173237198 | 5 | 173236238 | 173237198 | 961 | No | 1 |

## FIG. 3 (continued)

| promoters | 1to5kb | 5UTRs | exons |
|---|---|---|---|
| ZNF148 | | ZNF148 | ZNF148 |
| | | | |
| GATA2 | GATA2 | GATA2 | GATA2 |
| KY | | KY | KY |
| | ZIC1,ZIC4 | | |
| C3orf80 | C3orf80 | | |
| B3GALNT1 | B3GALNT1 | B3GALNT1 | B3GALNT1 |
| NLGN1 | | NLGN1 | NLGN1 |
| | | | |
| AC016773.1 | | FAM53A | FAM53A |
| AC097381.1 | | | |
| AFAP1 | | | AC097381.1 |
| PCDH7 | PCDH7 | PCDH7 | PCDH7 |
| | | | |
| | | | |
| THEGL | | | |
| TLL1 | TLL1 | TLL1 | TLL1 |
| PDE8B | PDE8B | | |
| | NR2F1 | | |
| MIR2277 | | | |
| NKX2-5 | NKX2-5 | | |

# FIG. 3 (continued)

EP 4 320 276 B1

| introns | 3UTRs | cpg_islands | cpg_shores | cpg_shelves | cpg_inter |
|---|---|---|---|---|---|
| ZNF148 | | 1 | 1 | | |
| AC016924.1 | | 1 | 1 | | |
| GATA2,GATA2-AS1 | | 1 | | | |
| KY,EPHB1 | | 1 | 2 | | |
| ZIC1 | | | 1 | | |
| IL12A-AS1 | | | 1 | | |
| NMD3 | | 1 | 1 | | |
| NLGN1 | | 1 | 1 | | |
| NKX1-1 | | 1 | | | |
| FAM53A | | 1 | | | |
| AFAP1 | | 1 | 1 | | |
| | | 1 | 1 | | |
| | | 1 | | | |
| | | | 1 | | |
| LINC00682 | | 1 | 2 | | |
| PDGFRA,AC058822.1 | | 1 | | | |
| | | | 1 | | |
| TLL1 | | 1 | 1 | | |
| PDE8B,AC022414.1 | | 1 | | | |
| NR2F1-AS1 | | 1 | 1 | | |
| FAM172A | | 1 | 1 | | |
| | | 1 | 1 | | |

**FIG. 3 (continued)**

| SEQ. ID NO. | reg_id | chr | start | end | width | Overlapping genes | enhancers |
|---|---|---|---|---|---|---|---|
| SEQ. ID NO. 298 | 5:173283672-173284272 | 5 | 173283672 | 173284272 | 601 | No | 1 |
| SEQ. ID NO. 299 | 5:175772822-175773002 | 5 | 175772822 | 175773002 | 181 | No | |
| SEQ. ID NO. 300 | 6:17102332-17102512 | 6 | 17102332 | 17102512 | 181 | No | |
| SEQ. ID NO. 301 | 6:25041894-25042434 | 6 | 25041894 | 25042434 | 541 | No | |
| SEQ. ID NO. 302 | 6:28657067-28657555 | 6 | 28657067 | 28657555 | 489 | No | |
| SEQ. ID NO. 303 | 6:29553847-29554027 | 6 | 29553847 | 29554027 | 181 | No | |
| SEQ. ID NO. 304 | 6:31308135-31308735 | 6 | 31308135 | 31308735 | 601 | No | 2 |
| SEQ. ID NO. 305 | 6:42726729-42727149 | 6 | 42726729 | 42727149 | 421 | No | 1 |
| SEQ. ID NO. 306 | 6:43174231-43174471 | 6 | 43174231 | 43174471 | 241 | No | |
| SEQ. ID NO. 307 | 6:46735424-46735814 | 6 | 46735424 | 46735814 | 391 | No | |
| SEQ. ID NO. 308 | 6:56955981-56956161 | 6 | 56955981 | 56956161 | 181 | No | |
| SEQ. ID NO. 309 | 6:83708290-83708710 | 6 | 83708290 | 83708710 | 421 | No | |
| SEQ. ID NO. 310 | 6:98833379-98833919 | 6 | 98833379 | 98833919 | 541 | No | |
| SEQ. ID NO. 311 | 6:167351042-167351222 | 6 | 167351042 | 167351222 | 181 | No | |
| SEQ. ID NO. 312 | 6:170736319-170736619 | 6 | 170736319 | 170736619 | 301 | No | |
| SEQ. ID NO. 313 | 7:351113-351413 | 7 | 351113 | 351413 | 301 | No | 1 |
| SEQ. ID NO. 314 | 7:19118914-19119214 | 7 | 19118914 | 19119214 | 301 | No | |
| SEQ. ID NO. 315 | 7:28957779-28957959 | 7 | 28957779 | 28957959 | 181 | No | |
| SEQ. ID NO. 316 | 7:35254531-35255011 | 7 | 35254531 | 35255011 | 481 | No | |
| SEQ. ID NO. 317 | 7:54933072-54933372 | 7 | 54933072 | 54933372 | 301 | No | |
| SEQ. ID NO. 318 | 7:69599017-69599197 | 7 | 69599017 | 69599197 | 181 | No | |
| SEQ. ID NO. 319 | 7:76511041-76511341 | 7 | 76511041 | 76511341 | 301 | No | |
| SEQ. ID NO. 320 | 7:88219317-88219497 | 7 | 88219317 | 88219497 | 181 | No | |
| SEQ. ID NO. 321 | 7:91881001-91881181 | 7 | 91881001 | 91881181 | 181 | No | |

# FIG. 3 (continued)

| promoters | 1to5kb | 5UTRs | exons |
|---|---|---|---|
| | | | |
| | | | |
| | | | STMND1 |
| RIPOR2 | | RIPOR2 | AL133268.3,RIPOR2 |
| | | | |
| | | | OR2I1P |
| AL671883.1 | | | AL671883.1 |
| ATP6V0CP3 | PRPH2 | | |
| | | | |
| PLA2G7 | | PLA2G7 | PLA2G7 |
| | DST | | |
| SNAP91 | | SNAP91 | SNAP91 |
| POU3F2,AL589826.2 | POU3F2,AL589826.2 | | |
| | | | |
| | | | AL731684.1,AL672310.1 |
| | | | |
| AC003986.3 | TWIST1,AC003986.3 | | |
| | | | AC005013.1,TRIL |
| TBX20 | TBX20 | | |
| AC006971.1 | | | |
| AUTS2 | CT66 | AUTS2 | AUTS2 |
| UPK3B | | | UPK3B |
| AC003991.1 | | | AC003991.1 |
| MTERF1 | | | |

## FIG. 3 (continued)

| introns | 3UTRs | cpg_islands | cpg_shores | cpg_shelves | cpg_inter |
|---|---|---|---|---|---|
| | | 1 | 2 | | |
| | | | 1 | | |
| STMND1 | | | 1 | | |
| AL133268.3 | | | | | 1 |
| | | | | | 1 |
| | | 1 | | | |
| AL671883.1,HLA-B | | 1 | 1 | | |
| | | 1 | 1 | | |
| SRF | | 1 | 1 | | |
| PLA2G7 | | 1 | 1 | | |
| BEND6 | | | 1 | | |
| SNAP91 | | 1 | | | |
| | | 1 | | | |
| TTLL2 | | 1 | | | |
| AL731684.1,AL672310.1 | | | | | |
| | | | | 1 | |
| | | | 1 | | |
| AC005013.1 | | 1 | | | |
| | | 1 | | | |
| | | 1 | 1 | | |
| | | 1 | | | |
| UPK3B | | | | | 1 |
| SRI,AC003991.1 | | | 1 | | |
| | | 1 | 1 | | |

**FIG. 3 (continued)**

| SEQ. ID NO. | reg_id | chr | start | end | width | Overlapping genes | enhancers |
|---|---|---|---|---|---|---|---|
| SEQ. ID NO. 322 | 7:108456902-108457082 | 7 | 108456902 | 108457082 | 181 | No | |
| SEQ. ID NO. 323 | 7:122300668-122301028 | 7 | 122300668 | 122301028 | 361 | No | |
| SEQ. ID NO. 324 | 7:139483469-139483891 | 7 | 139483469 | 139483891 | 423 | No | |
| SEQ. ID NO. 325 | 7:143345452-143345795 | 7 | 143345452 | 143345795 | 344 | No | |
| SEQ. ID NO. 326 | 7:155803569-155804049 | 7 | 155803569 | 155804049 | 481 | No | |
| SEQ. ID NO. 327 | 7:157336263-157336683 | 7 | 157336263 | 157336683 | 421 | No | |
| SEQ. ID NO. 328 | 8:544064-544544 | 8 | 544064 | 544544 | 481 | No | |
| SEQ. ID NO. 329 | 8:37797956-37798676 | 8 | 37797956 | 37798676 | 721 | No | |
| SEQ. ID NO. 330 | 8:39107409-39107589 | 8 | 39107409 | 39107589 | 181 | No | |
| SEQ. ID NO. 331 | 8:71843830-71844010 | 8 | 71843830 | 71844010 | 181 | No | |
| SEQ. ID NO. 332 | 8:104367291-104367591 | 8 | 104367291 | 104367591 | 301 | No | |
| SEQ. ID NO. 333 | 8:123073280-123073580 | 8 | 123073280 | 123073580 | 301 | No | |
| SEQ. ID NO. 334 | 8:143355090-143355270 | 8 | 143355090 | 143355270 | 181 | No | |
| SEQ. ID NO. 335 | 8:143740045-143740225 | 8 | 143740045 | 143740225 | 181 | No | |
| SEQ. ID NO. 336 | 9:23824147-23824447 | 9 | 23824147 | 23824447 | 301 | No | |
| SEQ. ID NO. 337 | 9:27528212-27528524 | 9 | 27528212 | 27528524 | 313 | No | |
| SEQ. ID NO. 338 | 9:61862430-61863030 | 9 | 61862430 | 61863030 | 601 | No | |
| SEQ. ID NO. 339 | 9:77648213-77648393 | 9 | 77648213 | 77648393 | 181 | No | |
| SEQ. ID NO. 340 | 9:87148272-87148872 | 9 | 87148272 | 87148872 | 601 | No | |
| SEQ. ID NO. 341 | 9:93184774-93184954 | 9 | 93184774 | 93184954 | 181 | No | |
| SEQ. ID NO. 342 | 9:98709159-98709819 | 9 | 98709159 | 98709819 | 661 | No | |
| SEQ. ID NO. 343 | 9:109640702-109641422 | 9 | 109640702 | 109641422 | 721 | No | |
| SEQ. ID NO. 344 | 9:122218898-122219198 | 9 | 122218898 | 122219198 | 301 | No | |
| SEQ. ID NO. 345 | 10:7410763-7411078 | 10 | 7410763 | 7411078 | 316 | No | 1 |

## FIG. 3 (continued)

| promoters | 1to5kb | 5UTRs | exons |
|---|---|---|---|
| NRCAM | | | |
| | FEZF1-AS1 | | |
| KLRG2 | | KLRG2 | KLRG2 |
| | | | CLCN1 |
| | | | SHH |
| DNAJB6 | DNAJB6 | DNAJB6 | DNAJB6 |
| | | | |
| | | | |
| ADAM32 | | ADAM32 | ADAM32 |
| MSC-AS1,MSC | MSC | | MSC |
| | | | |
| | TBC1D31 | TBC1D31 | TBC1D31 |
| | | | |
| | | | |
| | ELAVL2 | | |
| AL451123.1 | AL451123.1 | | |
| AL391987.1 | | | AL391987.1 |
| GNA14 | | GNA14 | GNA14 |
| LINC02872 | | | LINC02872 |
| WNK2 | | WNK2 | WNK2 |
| GABBR2 | GABBR2 | GABBR2 | GABBR2 |
| PALM2AKAP2 | | PALM2AKAP2 | PALM2AKAP2 |
| | LHX6 | | |
| AL139125.1 | SFMBT2 | | |

**FIG. 3 (continued)**

| introns | 3UTRs | cpg_islands | cpg_shores | cpg_shelves | cpg_inter |
|---|---|---|---|---|---|
|  |  |  | 1 |  |  |
|  |  |  | 1 |  |  |
|  |  | 1 |  |  |  |
| CLCN1 |  | 1 | 1 |  |  |
| SHH |  | 1 |  |  |  |
| DNAJB6 |  | 1 | 1 |  |  |
| TDRP |  | 1 | 1 |  |  |
| ADGRA2 |  | 1 | 1 |  |  |
| ADAM32 |  | 1 | 1 |  |  |
| MSC-AS1 |  | 1 |  |  |  |
| DPYS |  | 1 | 1 |  |  |
| TBC1D31 |  |  | 1 |  |  |
| TOP1MT |  |  |  |  | 1 |
| IQANK1 |  | 1 |  |  |  |
| ELAVL2 |  |  | 1 |  |  |
| MOB3B |  | 1 | 1 |  |  |
| AL391987.1 |  |  | 1 |  |  |
|  |  | 1 |  |  |  |
|  |  | 1 | 2 |  |  |
|  |  | 1 |  |  |  |
|  |  | 1 |  |  |  |
| PALM2AKAP2 |  | 1 | 1 |  |  |
| LHX6 |  |  | 1 |  |  |
| SFMBT2 |  | 1 |  |  |  |

FIG. 3 (continued)

| SEQ. ID NO. | reg_id | chr | start | end | width | Overlapping genes | enhancers |
|---|---|---|---|---|---|---|---|
| SEQ. ID NO. 346 | 10:25174989-25175169 | 10 | 25174989 | 25175169 | 181 | No | |
| SEQ. ID NO. 347 | 10:43105168-43105348 | 10 | 43105168 | 43105348 | 181 | No | |
| SEQ. ID NO. 348 | 10:86959058-86959298 | 10 | 86959058 | 86959298 | 241 | No | |
| SEQ. ID NO. 349 | 10:99329112-99330672 | 10 | 99329112 | 99330672 | 1561 | No | |
| SEQ. ID NO. 350 | 10:103693303-103693603 | 10 | 103693303 | 103693603 | 301 | No | |
| SEQ. ID NO. 351 | 10:117534766-117535426 | 10 | 117534766 | 117535426 | 661 | No | 1 |
| SEQ. ID NO. 352 | 10:127196238-127196538 | 10 | 127196238 | 127196538 | 301 | No | |
| SEQ. ID NO. 353 | 10:132786224-132786524 | 10 | 132786224 | 132786524 | 301 | No | |
| SEQ. ID NO. 354 | 11:313954-314134 | 11 | 313954 | 314134 | 181 | No | |
| SEQ. ID NO. 355 | 11:639416-639956 | 11 | 639416 | 639956 | 541 | No | |
| SEQ. ID NO. 356 | 11:640311-640791 | 11 | 640311 | 640791 | 481 | No | |
| SEQ. ID NO. 357 | 11:9004093-9004273 | 11 | 9004093 | 9004273 | 181 | No | |
| SEQ. ID NO. 358 | 11:9091769-9091949 | 11 | 9091769 | 9091949 | 181 | No | |
| SEQ. ID NO. 359 | 11:12009476-12009656 | 11 | 12009476 | 12009656 | 181 | No | |
| SEQ. ID NO. 360 | 11:19713295-19713895 | 11 | 19713295 | 19713895 | 601 | No | |
| SEQ. ID NO. 361 | 11:35418738-35420778 | 11 | 35418738 | 35420778 | 2041 | No | |
| SEQ. ID NO. 362 | 11:62926056-62926596 | 11 | 62926056 | 62926596 | 541 | No | |
| SEQ. ID NO. 363 | 11:67583928-67584405 | 11 | 67583928 | 67584405 | 478 | No | |
| SEQ. ID NO. 364 | 11:74311342-74311687 | 11 | 74311342 | 74311687 | 346 | No | |
| SEQ. ID NO. 365 | 12:6548902-6549202 | 12 | 6548902 | 6549202 | 301 | No | |
| SEQ. ID NO. 366 | 12:48198272-48198932 | 12 | 48198272 | 48198932 | 661 | No | |
| SEQ. ID NO. 367 | 12:54053346-54053526 | 12 | 54053346 | 54053526 | 181 | No | |
| SEQ. ID NO. 368 | 12:104456512-104457736 | 12 | 104456512 | 104457736 | 1225 | No | |
| SEQ. ID NO. 369 | 12:114408286-114409006 | 12 | 114408286 | 114409006 | 721 | No | |

FIG. 3 (continued)

| promoters | 1to5kb | 5UTRs | exons |
|---|---|---|---|
| GPR158 | | GPR158 | GPR158 |
| | | | RET |
| SNCG | MMRN2 | | |
| | | CNNM1 | CNNM1 |
| | | | SH3PXD2A |
| | | | |
| INSYN2A | | | |
| NKX6-2 | NKX6-2 | | |
| IFITM1 | AC136475.1,AC136475.2 | IFITM1 | IFITM1 |
| | | | DRD4 |
| | | | DRD4 |
| NRIP3,AC079296.1 | | | AC079296.1 |
| SCUBE2 | KRT8P41,MIR5691 | | |
| DKK3 | DKK3 | DKK3 | DKK3 |
| NAV2,AC009549.1 | AC009549.1 | NAV2 | NAV2 |
| SLC1A2,AC090625.2 | SLC1A2 | SLC1A2 | SLC1A2,AC090625.2 |
| | CHRM1 | | |
| GSTP1 | GSTP1 | | GSTP1 |
| P4HA3,P4HA3-AS1 | | P4HA3 | P4HA3,P4HA3-AS1 |
| IFFO1 | | | IFFO1 |
| AC024257.3 | | | AC024257.3 |
| HOXC4 | FLJ12825 | | HOXC4 |
| CHST11 | | CHST11 | CHST11 |
| TBX5,TBX5-AS1 | TBX5 | TBX5 | TBX5,TBX5-AS1 |

## FIG. 3 (continued)

EP 4 320 276 B1

| introns | 3UTRs | cpg_islands | cpg_shores | cpg_shelves | cpg_inter |
|---|---|---|---|---|---|
| GPR158-AS1,GPR158 | | 1 | | | |
| RET | | 1 | | | |
| SNCG,MMRN2 | | | | | 1 |
| | | 1 | 1 | | |
| SH3PXD2A | | 1 | 1 | | |
| EMX2OS | | 1 | | | |
| DOCK1 | | 1 | | | |
| | | 1 | | | |
| IFITM1,IFITM2 | | | 1 | | |
| DRD4 | | 1 | | | |
| DRD4 | DRD4 | 1 | 1 | | |
| | | 1 | | | |
| SCUBE2 | | 1 | 1 | | |
| DKK3 | | | 1 | | |
| NAV2 | | 1 | 1 | | |
| SLC1A2,AC090625.2 | | 1 | 2 | | |
| | | 1 | | | |
| GSTP1 | | 1 | | | |
| P4HA3,P4HA3-AS1 | | 1 | 2 | | |
| IFFO1 | | | 1 | | |
| AC024257.3 | | 1 | | | |
| HOXC4 | | | 1 | | |
| CHST11 | | 1 | | | |
| TBX5-AS1 | | 1 | 1 | | |

**FIG. 3 (continued)**

| SEQ. ID NO. | reg_id | chr | start | end | width | Overlapping genes | enhancers |
|---|---|---|---|---|---|---|---|
| SEQ. ID NO. 370 | 14:36512000-36512700 | 14 | 36512000 | 36512700 | 701 | No | |
| SEQ. ID NO. 371 | 14:41606363-41607263 | 14 | 41606363 | 41607263 | 901 | No | 1 |
| SEQ. ID NO. 372 | 14:55129115-55129355 | 14 | 55129115 | 55129355 | 241 | No | |
| SEQ. ID NO. 373 | 14:64540512-64540932 | 14 | 64540512 | 64540932 | 421 | No | |
| SEQ. ID NO. 374 | 14:97412990-97413410 | 14 | 97412990 | 97413410 | 421 | No | |
| SEQ. ID NO. 375 | 14:104117671-104117851 | 14 | 104117671 | 104117851 | 181 | No | |
| SEQ. ID NO. 376 | 14:105364294-105364612 | 14 | 105364294 | 105364612 | 319 | No | |
| SEQ. ID NO. 377 | 15:52789319-52790159 | 15 | 52789319 | 52790159 | 841 | No | |
| SEQ. ID NO. 378 | 15:62165240-62165420 | 15 | 62165240 | 62165420 | 181 | No | |
| SEQ. ID NO. 379 | 15:67968089-67968329 | 15 | 67968089 | 67968329 | 241 | No | 1 |
| SEQ. ID NO. 380 | 15:69031565-69031745 | 15 | 69031565 | 69031745 | 181 | No | |
| SEQ. ID NO. 381 | 15:72319682-72319982 | 15 | 72319682 | 72319982 | 301 | No | |
| SEQ. ID NO. 382 | 15:78264397-78264697 | 15 | 78264397 | 78264697 | 301 | No | |
| SEQ. ID NO. 383 | 15:89486695-89486875 | 15 | 89486695 | 89486875 | 181 | No | |
| SEQ. ID NO. 384 | 15:98651103-98651343 | 15 | 98651103 | 98651343 | 241 | No | 1 |
| SEQ. ID NO. 385 | 16:1272246-1272546 | 16 | 1272246 | 1272546 | 301 | No | |
| SEQ. ID NO. 386 | 16:50841053-50841413 | 16 | 50841053 | 50841413 | 361 | No | |
| SEQ. ID NO. 387 | 16:57091834-57092014 | 16 | 57091834 | 57092014 | 181 | No | 1 |
| SEQ. ID NO. 388 | 16:57536947-57537307 | 16 | 57536947 | 57537307 | 361 | No | |
| SEQ. ID NO. 389 | 16:68737031-68737271 | 16 | 68737031 | 68737271 | 241 | No | |
| SEQ. ID NO. 390 | 16:87571366-87571666 | 16 | 87571366 | 87571666 | 301 | No | |
| SEQ. ID NO. 391 | 17:19744858-19745191 | 17 | 19744858 | 19745191 | 334 | No | |
| SEQ. ID NO. 392 | 17:20320942-20321122 | 17 | 20320942 | 20321122 | 181 | No | |
| SEQ. ID NO. 393 | 17:29761193-29761373 | 17 | 29761193 | 29761373 | 181 | No | |

## FIG. 3 (continued)

| promoters | 1to5kb | 5UTRs | exons |
|---|---|---|---|
| | | | |
| LRFN5 | LRFN5,AL121821.2 | | |
| LGALS3 | | LGALS3 | LGALS3 |
| HSPA2,AL049869.3 | AL049869.4,AL049869.3 | HSPA2 | HSPA2 |
| | | | |
| MIR203B | | | |
| | PACS2 | | |
| ONECUT1 | | ONECUT1 | ONECUT1 |
| C2CD4B | | C2CD4B | C2CD4B |
| | | | |
| NOX5 | | | NOX5 |
| CELF6,AC009690.3 | | CELF6,AC009690.3 | CELF6,AC009690.1,AC009690.3 |
| DNAJA4,AC090607.3 | DNAJA4 | DNAJA4 | DNAJA4 |
| | | | RHCG |
| | IRAIN | | |
| | | | |
| | | | |
| CPNE2 | | | AC009090.2 |
| CCDC102A | | | |
| CDH1 | | CDH1 | CDH1 |
| | | | |
| ALDH3A1 | ALDH3A1 | ALDH3A1 | ALDH3A1 |
| CCDC144CP | RNU6-467P,AC008088.1 | | |
| | | SSH2 | SSH2,AC023389.1 |

**FIG. 3 (continued)**

| introns | 3UTRs | cpg_islands | cpg_shores | cpg_shelves | cpg_inter |
|---|---|---|---|---|---|
| SFTA3,AL132857.1 | | | 1 | 1 | |
| | | 1 | 2 | | |
| LGALS3 | | 1 | | | |
| HSPA2 | | 1 | | | |
| | | | | | 1 |
| | | 1 | | | |
| PACS2 | | 1 | 1 | | |
| ONECUT1 | | 1 | | | |
| | | 1 | | | |
| | | | | | 1 |
| NOX5 | | 1 | 1 | | |
| AC009690.2 | AC009690.1 | 1 | | | |
| DNAJA4 | | 1 | | | |
| RHCG | | 1 | 1 | | |
| IGF1R | | 1 | 1 | | |
| | | | | | 1 |
| LINC02128 | | 1 | 2 | | |
| AC009090.2 | | | 1 | | |
| | | | 1 | | |
| | | 1 | 1 | | |
| | | | | | 1 |
| ALDH3A1 | | 1 | 1 | | |
| AC004702.1 | | 1 | | | |
| SSH2,AC023389.1 | | 1 | | | |

## FIG. 3 (continued)

| SEQ. ID NO. | reg_id | chr | start | end | width | Overlapping genes | enhancers |
|---|---|---|---|---|---|---|---|
| SEQ. ID NO. 394 | 17:40218777-40218957 | 17 | 40218777 | 40218957 | 181 | No | |
| SEQ. ID NO. 395 | 17:42421656-42421836 | 17 | 42421656 | 42421836 | 181 | No | 1 |
| SEQ. ID NO. 396 | 17:43211642-43211882 | 17 | 43211642 | 43211882 | 241 | No | |
| SEQ. ID NO. 397 | 18:9707922-9708102 | 18 | 9707922 | 9708102 | 181 | No | |
| SEQ. ID NO. 398 | 18:24139209-24139559 | 18 | 24139209 | 24139559 | 351 | No | |
| SEQ. ID NO. 399 | 19:2263624-2263924 | 19 | 2263624 | 2263924 | 301 | No | |
| SEQ. ID NO. 400 | 19:20167000-20167360 | 19 | 20167000 | 20167360 | 361 | No | |
| SEQ. ID NO. 401 | 19:20424875-20425055 | 19 | 20424875 | 20425055 | 181 | No | |
| SEQ. ID NO. 402 | 19:40613725-40614145 | 19 | 40613725 | 40614145 | 421 | No | |
| SEQ. ID NO. 403 | 19:40811045-40811585 | 19 | 40811045 | 40811585 | 541 | No | |
| SEQ. ID NO. 404 | 19:49142732-49143092 | 19 | 49142732 | 49143092 | 361 | No | |
| SEQ. ID NO. 405 | 19:50050361-50050716 | 19 | 50050361 | 50050716 | 356 | No | |
| SEQ. ID NO. 406 | 19:53159277-53159457 | 19 | 53159277 | 53159457 | 181 | No | |
| SEQ. ID NO. 407 | 19:56538908-56539088 | 19 | 56538908 | 56539088 | 181 | No | |
| SEQ. ID NO. 408 | 19:57888509-57888689 | 19 | 57888509 | 57888689 | 181 | No | |
| SEQ. ID NO. 409 | 19:58347014-58347734 | 19 | 58347014 | 58347734 | 721 | No | |
| SEQ. ID NO. 410 | 19:58562845-58563445 | 19 | 58562845 | 58563445 | 601 | No | |
| SEQ. ID NO. 411 | 20:3083167-3083587 | 20 | 3083167 | 3083587 | 421 | No | |
| SEQ. ID NO. 412 | 20:5504527-5504827 | 20 | 5504527 | 5504827 | 301 | No | |
| SEQ. ID NO. 413 | 20:45469465-45469765 | 20 | 45469465 | 45469765 | 301 | No | |
| SEQ. ID NO. 414 | 20:52103731-52104151 | 20 | 52103731 | 52104151 | 421 | No | |
| SEQ. ID NO. 415 | 20:60087400-60088000 | 20 | 60087400 | 60088000 | 601 | No | |
| SEQ. ID NO. 416 | 21:31558435-31558795 | 21 | 31558435 | 31558795 | 361 | No | |

## FIG. 3 (continued)

| promoters | 1to5kb | 5UTRs | exons |
|---|---|---|---|
| WIPF2 | WIPF2 | | |
| | | | |
| TMEM106A | TMEM106A | TMEM106A | NBR1,TMEM106A |
| RAB31 | | | |
| CABYR | AC090772.4 | CABYR | CABYR |
| | | | |
| ZNF486 | | ZNF486 | ZNF486 |
| ZNF826P | | | ZNF826P |
| LTBP4 | LTBP4 | | LTBP4 |
| CYP2T1P | | | CYP2T1P |
| PPFIA3 | PPFIA3 | | PPFIA3 |
| AC010624.1 | | | AC010624.1 |
| ZNF347 | AC092070.1 | | ZNF665 |
| ZFP28,AC005498.2 | | ZFP28 | ZFP28 |
| | | | |
| A1BG-AS1,A1BG | A1BG-AS1,AC012313.10 | | A1BG,AC012313.3 |
| | UBE2M | | MZF1 |
| | | | AVP |
| LINC00654 | | | LINC00654 |
| WFDC2 | WFDC2 | WFDC2 | WFDC2 |
| | | | |
| MIR646HG | MIR646HG,AL132822.1 | | MIR646HG |
| AP000251.1 | | | |

# FIG. 3 (continued)

| introns | 3UTRs | cpg_islands | cpg_shores | cpg_shelves | cpg_inter |
|---|---|---|---|---|---|
| | | | 1 | | |
| CAVIN1 | | 1 | 1 | | |
| | NBR1 | 1 | 1 | | |
| | | 1 | | | |
| CABYR | | 1 | | | |
| JSRP1 | | | | | 1 |
| AC011447.3 | | | | | 1 |
| | | | | | 1 |
| LTBP4 | | 1 | | | |
| CYP2T1P,AC008537.1 | | 1 | 1 | | |
| PPFIA3 | PPFIA3 | 1 | 2 | | |
| AC010624.3,ZNF473 | | | 1 | | |
| | | | 1 | | |
| | | 1 | | | |
| ZNF814,AC010326.2 | | 1 | 1 | | |
| A1BG,AC012313.3 | A1BG | 1 | 2 | | |
| MZF1-AS1,MZF1 | MZF1 | 1 | 1 | | |
| AVP | | 1 | 1 | | |
| | | | | | 1 |
| | | 1 | 1 | | |
| ZFP64 | | | 1 | | |
| | | 1 | 2 | | |
| TIAM1 | | 1 | | | |

**FIG. 3 (continued)**

| SEQ. ID NO. | reg_id | chr | start | end | width | Overlapping genes | enhancers |
|---|---|---|---|---|---|---|---|
| SEQ. ID NO. 418 | 22:37335098-37335278 | 22 | 37335098 | 37335278 | 181 | No | |
| SEQ. ID NO. 419 | 22:42432029-42432209 | 22 | 42432029 | 42432209 | 181 | No | |
| SEQ. ID NO. 420 | 22:44752220-44752460 | 22 | 44752220 | 44752460 | 241 | No | |
| SEQ. ID NO. 421 | 22:45867691-45867871 | 22 | 45867691 | 45867871 | 181 | No | |
| SEQ. ID NO. 422 | 22:50085187-50085994 | 22 | 50085187 | 50085994 | 808 | No | |
| SEQ. ID NO. 423 | X:781309-781729 | X | 781309 | 781729 | 421 | No | |

| promoters | 1to5kb | 5UTRs | exons |
|---|---|---|---|
| | | | |
| | | | |
| ARHGAP8 | | | |
| | | | |
| MLC1 | MOV10L1 | MLC1 | MLC1 |
| | | | |

| introns | 3UTRs | cpg_islands | cpg_shores | cpg_shelves | cpg_inter |
|---|---|---|---|---|---|
| | | 1 | | | |
| NFAM1 | | | | | 1 |
| PRR5-ARHGAP8 | | 1 | 1 | | |
| | | 1 | 1 | | |
| MLC1 | | | | 1 | 1 |
| | | | | | 1 |

# FIG. 3 (continued)

EP 4 320 276 B1

| SEQ. ID NO. | chr | start | end | genes | SEQ. ID NO. | seq_plus_30bp_padding,strand | strand |
|---|---|---|---|---|---|---|---|
| SEQ. ID NO.424 | chr1 | 114709462 | 114709702 | NRAS_p.A146 | SEQ. ID NO.451 | ctgaaagctgtaccatacctgtctggtcttGGCtgaggtttcaatgaatggaatcccgtaact | -1 |
| SEQ. ID NO.425 | chr1 | 114713788 | 114714028 | NRAS_p.Q61 | SEQ. ID NO.452 | gtattggtctctcatggcactgtactcttcTTGtccagctgtatccagtatgtccaacaaaca | -1 |
| SEQ. ID NO.426 | chr1 | 114716004 | 114716244 | NRAS_p.G13 | SEQ. ID NO.453 | tagctggattgtcagtgcgcttttcccaacACCacctgctccaaccaccaccagtttgtactc | -1 |
| SEQ. ID NO.427 | chr1 | 114716004 | 114716244 | NRAS_p.G12 | SEQ. ID NO.454 | ctggattgtcagtgcgcttttcccaacaccACCtgctccaaccaccaccagtttgtactcagt | -1 |
| SEQ. ID NO.428 | chr10 | 87933115 | 87933355 | PTEN_p.R159 | SEQ. ID NO.455 | caagaggccctagatttctatggggaagtaAGGaccagagacaaaaaggtaagttattttttg | 1 |
| SEQ. ID NO.429 | chr12 | 25225520 | 25225820 | KRAS_p.A146 | SEQ. ID NO.456 | tatttcagtgttacttacctgtcttgtcttTGCtgatgtttcaataaaaggaattccataact | -1 |
| SEQ. ID NO.430 | chr12 | 25225520 | 25225820 | KRAS_p.K117 | SEQ. ID NO.457 | tgtgtctactgttctagaaggcaaatcacaTTTatttcctactaggaccataggtacatcttc | -1 |
| SEQ. ID NO.431 | chr12 | 25227222 | 25227462 | KRAS_p.Q61 | SEQ. ID NO.458 | gtactggtccctcattgcactgtactcctcTTGacctgctgtgtcgagaatatccaagagaca | -1 |
| SEQ. ID NO.432 | chr12 | 25245228 | 25245468 | KRAS_p.G13 | SEQ. ID NO.459 | tagctgtatcgtcaaggcactcttgcctacGCCaccagctccaactaccacaagtttatattc | -1 |
| SEQ. ID NO.433 | chr12 | 25245228 | 25245468 | KRAS_p.G12 | SEQ. ID NO.460 | ctgtatcgtcaaggcactcttgcctacgccACCagctccaactaccacaagtttatattcagt | -1 |
| SEQ. ID NO.434 | chr3 | 179218185 | 179218425 | PIK3CA_p.E545 | SEQ. ID NO.461 | tctacacgagatcctctctctgaaatcactGAGcaggagaaagatttctatggagtcacagg | 1 |
| SEQ. ID NO.435 | chr3 | 179218185 | 179218425 | PIK3CA_p.Q546 | SEQ. ID NO.462 | acacgagatcctctctctgaaatcactgagCAGgagaaagatttctatggagtcacaggtaa | 1 |
| SEQ. ID NO.436 | chr3 | 179234177 | 179234417 | PIK3CA_p.H1047 | SEQ. ID NO.463 | gagtatttcatgaaacaaatgaatgatgcaCATcatggtggctggacaacaaaaatggattgg | 1 |
| SEQ. ID NO.437 | chr7 | 55160195 | 55160435 | EGFR_p.S492 | SEQ. ID NO.464 | gggacctccggtcagaaaaccaaaattataAGCaacagaggtgaaaacagctgcagtaagtca | 1 |
| SEQ. ID NO.438 | chr7 | 140753216 | 140753456 | BRAF_p.V600 | SEQ. ID NO.465 | aaactgatgggacccactccatcgagatttCACtgtagctagaccaaaatcacctattttac | -1 |
| SEQ. ID NO.439 | chr19 | 1223005 | 1223245 | STK11_p.F354 | SEQ. ID NO.466 | ctgcacggcgcggacgaggacgaggacctcTTCgacatcgaggatgacatcatctacactcag | 1 |
| SEQ. ID NO.440 | chr7 | 7673682 | 7673922 | TP53_p.R273 | SEQ. ID NO.467 | gcgccggtctctcccaggacaggcacaaacACGcacctcaaagctgttccgtcccagtagatt | -1 |
| SEQ. ID NO.441 | chr4 | 54727179 | 54727419 | KIT_p.M541 | SEQ. ID NO.468 | atcgtagctggcatgatgtgcattattgtgATGattctgacctacaaatatttacaggtaacc | 1 |
| SEQ. ID NO.442 | chr7 | 116700088 | 116700328 | MET_p.N375 | SEQ. ID NO.469 | ttccctatcaaatatgtcaacgacttcttcAACaagatcgtcaacaaaaacaatgtgagatgt | 1 |

| SEQ. ID NO. | mutAA | ids |
|---|---|---|
| SEQ. ID NO.443 | KPLRHH | NRAS_Q_-1_61_mid |
| SEQ. ID NO.444 | CRAV | NRAS_G_-1_13_mid |
| SEQ. ID NO.445 | CRAV | NRAS_G_-1_12_mid |
| SEQ. ID NO.446 | KPLRHH | KRAS_Q_-1_61_mid |
| SEQ. ID NO.447 | CRAV | KRAS_G_-1_13_mid |
| SEQ. ID NO.448 | CRAV | KRAS_G_-1_12_mid |
| SEQ. ID NO.449 | QAGV | PIK3CA_E_1_545_mid |
| SEQ. ID NO.450 | EKLPR | PIK3CA_Q_1_546_mid |

**FIG. 4A**

| SEQ.NO. | mutAA | ids |
|---|---|---|
| 00001 | P | NRAS_A_-1_146_left |
| 00002 | S | PTEN_R_1_159_mid |
| 00003 | P | KRAS_A_-1_146_left |
| 00004 | NN | KRAS_4_-1_177_mid |
| 00005 | LR | PIK3CA_H_1_1047_mid |
| 00006 | R | EGFR_S_1_492_mid |
| 00007 | EGA | BRAF_V_-1_600_mid |
| 00008 | L | STK11_F_1_354_mid |
| 00009 | GL | TP53_R_-1_273_mid |
| 00010 | L | KIT_M_1_541_mid |
| 00011 | S | MET_N_1_375_mid |

| SEQ.NO. | chr | i.start | i.end | SEQ.NO. | AA |
|---|---|---|---|---|---|
| 00012 | chr1 | 114709581 | 114709583 | 00031 | A |
| 00013 | chr1 | 114713907 | 114713909 | 00032 | Q |
| 00014 | chr1 | 114716122 | 114716124 | 00033 | G |
| 00015 | chr1 | 114716125 | 114716127 | 00034 | G |
| 00016 | chr10 | 87933234 | 87933236 | 00035 | R |
| 00017 | chr12 | 25225626 | 25225628 | 00036 | A |
| 00018 | chr12 | 25225713 | 25225715 | 00037 | K |
| 00019 | chr12 | 25227341 | 25227343 | 00038 | Q |
| 00020 | chr12 | 25245346 | 25245348 | 00039 | G |
| 00021 | chr12 | 25245349 | 25245351 | 00040 | G |
| 00022 | chr3 | 179218303 | 179218305 | 00041 | E |
| 00023 | chr3 | 179218306 | 179218308 | 00042 | Q |
| 00024 | chr3 | 179234296 | 179234298 | 00043 | H |
| 00025 | chr7 | 55160314 | 55160316 | 00044 | S |
| 00026 | chr7 | 140753335 | 140753337 | 00045 | V |
| 00027 | chr19 | 1223124 | 1223126 | 00046 | F |
| 00028 | chr7 | 7673801 | 7673803 | 00047 | R |
| 00029 | chr4 | 54727298 | 54727300 | 00048 | M |
| 00030 | chr7 | 116700207 | 116700209 | 00049 | N |

**FIG. 4B**

FIG. 5

**600**

**610**
Adaptase™
⊙ 17 minutes

**620**
Extension
⊙ 8 minutes

**630**
Ligation
⊙ 15 minutes

**640**
Indexing PCR
⊙ Time varies

Bisulfite Converted ssDNA Fragment

Tail
Truncated Adapter 1

Truncated Adapter 1 Primer

Truncated Adapter 2

Full-length Adapters

Indexed Library

This protocol sequentially attaches adapters to single-stranded DNA fragments.

The Adaptase step is a highly efficient, template-independent reaction that simultaneously performs tailing and ligation of truncated adapter 1 to 3' ends.

The Extension step is performed to generate a uracil-free library molecule.

The Ligation step is used to add truncated adapter 2 to the **bottom strand only.**

The Indexing PCR step increases yield and incorporates full length adapters for single or dual indexing.

Bead-based clean-ups are used to remove both oligonucleotides and small fragments, as well as to change enzymatic buffer composition.

**FIG. 6**

FIG. 7

**FIG. 8**

FIG. 9

**FIG. 10A**

FIG. 10B

FIG. 11

FIG. 12

**1300**

**FIG. 13**

reads

FIG. 14

# reads

FIG. 14 (continued)

FIG. 14 (continued)

Scores Plot

FIG. 15

**FIG. 16**

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10006925 B

- US 63011970 B

**Non-patent literature cited in the description**

- **HERMAN**. *Proc. Natl. Acad. Sci. USA*, 1992, vol. 93, 9821-9826
- **HUSSMANN**. *Methods Mol Biol.*, 2018, vol. 1708, 551-571
- **FACKLER**. *Methods Mol Biol.*, 2018, vol. 1708, 473-496
- **LIU**. *Nucleic Acids Res.*, 2017, vol. 45 (6), e39
- **GONZALGO**. *Nat Protoc.*, 2007, vol. 2 (8), 1931-6

- **FROMMER**. *Proc Natl Acad Sci U S A.*, 1992, vol. 89 (5), 1827-31
- **CAMPAN**. *Methods Mol Biol.*, 2018, vol. 1708, 497-513
- **SHAUKAT**. *N Engl J Med.*, 2013, vol. 369 (12), 1106-14
- AJCC Cancer Staging Manual